# EUROPÄISCHE PATENTSCHRIFT

(11) **EP 3 564 206 B1**
(45) Veröffentlichungstag und Bekanntmachung des Hinweises auf die Patenterteilung: **04.11.2020**
(21) Anmeldenummer: 19172865.8
(22) Anmeldetag: 06.05.2019
(51) Int. Cl.: C07C 69/86, C07C 69/92, C08F 283/00, C08F 290/14, A61C 7/00

(54) **NEUE POLYMERISIERBARE MONOMERE UND DEREN VERWENDUNG ALS REAKTIVVERDÜNNER IN HÄRTBAREN ZUSAMMENSETZUNGEN**
NOVEL POLYMERISABLE MONOMERS AND THEIR USE AS REACTIVE DILUENTS IN CURABLE COMPOSITIONS
NOUVEAUX MONOMÈRES POLYMÉRISABLES ET LEUR UTILISATION EN TANT QUE DILUANTS RÉACTIFS DANS DES COMPOSITIONS DURCISSABLES

(30) Priorität: 04.05.2018 US 201862667364 P; 05.12.2018 US 201862775762 P; 03.05.2019 WO PCT/US2019/030687
(43) Veröffentlichungstag der Anmeldung: 06.11.2019
(73) Patentinhaber: Align Technology, Inc., San Jose, CA 95134 (US)
(72) Erfinder: LISKA, Robert, 2123 Schleinbach (AT); GORSCHE, Christian, 1140 Wien (AT); KURY, Markus, 1060 Wien (AT); CHEN, Yan, San Jose, CA 95134 (US); LI, Chunhua, San Jose, CA 95134 (US); KAZA, Srinivas, San Jose, CA 95134 (US)
(74) Vertreter: Grünecker Patent- und Rechtsanwälte PartG mbB

(56) Entgegenhaltungen:
- WO-A1-2009/134488
- WO-A2-2010/064726
- JP-A- 2013 116 886
- C. S. JONE SELVAMALAR ET AL: "Homopolymer and copolymers of 4-benzyloxycarbonylphenyl acrylate with glycidyl methacrylate: Synthesis, characterization, reactivity ratios, and application as adhesive for leather", JOURNAL OF APPLIED POLYMER SCIENCE, Bd. 91, Nr. 6, 15. März 2004 (2004-03-15), Seiten 3604-3612, XP055621954, US ISSN: 0021-8995, DOI: 10.1002/app.13594
- SELVAMALAR C S J ET AL: "Copolymerization of 4-benzyloxycarbonylphenyl methacrylate with glycidyl methacrylate: synthesis, characterization, reactivity ratios and application as adhesives", REACTIVE AND FUNCTIONAL POLYMERS, ELSEVIER, AMSTERDAM, NL, Bd. 56, Nr. 2, 6. August 2003 (2003-08-06) , Seiten 89-101, XP004460494, ISSN: 1381-5148, DOI: 10.1016/S1381-5148(03)00028-2
- C. S. JONE SELVAMALAR ET AL: "Copolymers of 4-Benzyloxycarbonylphenyl Methacrylate with Methyl Methacrylate: Synthesis, Characterization, and Reactivity Ratios", JOURNAL OF MACROMOLECULAR SCIENCE , PART A - PURE AND APPLIED CHEMISTRY., Bd. 40, Nr. 10, 10. Januar 2003 (2003-01-10), Seiten 1019-1033, XP055621964, US ISSN: 1060-1325, DOI: 10.1081/MA-120024461
- P. S. VIJAYANAND ET AL: "Copolymerization of 4-Benzyloxycarbonylphenyl Acrylate with Methyl Methacrylate: Synthesis, Characterization, and Determination of Reactivity Ratios", JOURNAL OF MACROMOLECULAR SCIENCE , PART A - PURE AND APPLIED CHEMISTRY., Bd. 40, Nr. 2, 3. Januar 2003 (2003-01-03) , Seiten 125-140, XP055621966, US ISSN: 1060-1325, DOI: 10.1081/MA-120017256

## Beschreibung

### HINTERGRUND DER ERFINDUNG

**Zu** additiven Fertigungsverfahren (z.B. additive Fertigung auf Lithographiebasis (L-AM)) zählen verschiedene Verfahren zur Herstellung von Gegenständen, wie z. B. dreidimensionalen Gegenständen, aus photopolymerisierbaren Materialien. Aufgrund der Kosten, einfacher Handhabung und verschiedener anderer Faktoren wurden additive Fertigungsverfahren seit Langem eingesetzt, um Prototypen und funktionelle Gegenstände (z.B. durch "Rapid Prototyping") herzustellen und Gegenstände in großen Mengen zu produzieren. Viele additive Fertigungsverfahren umfassen das sukzessive Hinzufügen von Schichten aus polymerisierbarem Material und das Härten dieser Schichten durch gesteuerte Belichtung. Die photopolymerisierbaren Materialien umfassen oft reaktive Komponenten, die mit Licht gehärtet werden. Zu Beispielen für photopolymerisierbare Materialien, die mit additiver Fertigung kompatibel sind, zählen Acrylate, die z. B. mit radikalischer Polymerisation kompatibel sind, und Epoxide, die z. B. mit kationischer Polymerisation kompatibel sind. Beispielhafte Viskositäten von bestehenden Materialien, die für additive Fertigung verwendet werden, umfassen Viskositäten zwischen 20 und 40 Millipascalsekunden (mPa.s) (siehe I. Gibson, D.W. Rosen, B. Stucker et al., "Additive Manufacturing Technologies", Band 238, Springer Verlag (2010)).

Es hat sich bei zahlreichen medizinischen Vorrichtungen herkömmlicherweise als schwierig erwiesen, diese durch additive Fertigungsverfahren zu formen. Ein Problem besteht darin, dass bestehende Materialien, die für die additive Fertigung verwendet werden, nicht biokompatibel sind und noch weniger zur Verwendung im intraoralen Bereich oder einem anderen Teil des menschlichen Körpers geeignet sind. Ein weiteres Problem besteht darin, dass bestehende für additive Fertigung verwendete Materialien oft nicht ausreichend viskos sind, um die präzisen und/oder individualisierbaren Merkmale zu bilden, die für viele Vorrichtungen erforderlich sind. Außerdem weisen viele der aktuellen additive Fertigungsverfahren aus Sicherheits- und Kostenüberlegungen relative niedrige Härtungs- oder Reaktionstemperaturen auf, was bei medizinischen Vorrichtungen (einschließlich zahnärztlichen Vorrichtungen) verhindert, dass ein Produkt erzeugt werden kann, das bei der Temperatur des menschlichen Körpers und/oder darüber stabil ist.

Ein weiteres Problem besteht darin, dass bestehende Materialien, die für additive Fertigung verwendet werden, nicht die physikalischen, chemischen und/oder thermomechanischen Eigenschaften (Dehnung, Entspannung im Verlauf der Zeit, Modul, Beständigkeit, Zähigkeit etc.) aufweisen, die für Aligner, andere zahnärztlichen Vorrichtungen, Hörgeräte und/oder zahlreiche andere medizinische Vorrichtungen wünschenswert sind (siehe z.B. T. Swetly, J. Stampfl, G. Kempf und R.-M. Hucke, "Capabilities of Additive Manufacturing Technologies (AMT) in the validation of the automotive cockpit", RTejournal - Forum for Rapid Technology 2014 (1)). Bestehende Materialien, die für additive Fertigung verwendet werden, weisen zahlreiche Eigenschaften, die in medizinischen Vorrichtungen wünschenswert sind, nicht auf, wie z.B. Kräfte, Drehmomente, Momente und/oder andere Bewegungen auszuüben, die präzise sind und einem Behandlungsplan entsprechen.

Eine Erhöhung der Viskosität der Materialien kann bessere thermomechanische Eigenschaften für zahlreiche Anwendungen bereitstellen, indem die Vernetzung verringert wird, die physikalischen Wechselwirkungen zwischen Ketten erhöht wird, das Durchschnittsgewicht der Monomere erhöht wird usw. In der Folge kann es möglich sein, Materialien mit wünschenswerten thermomechanischen Eigenschaften und/ oder Viskositäten durch additive Fertigung zu zahnärztlichen und/oder medizinischen Vorrichtungen zu verarbeiten, indem die Verfahren durch Erhitzungsvorgänge erweitert werden. Beispielsweise offenbaren WO 2015/075094, WO 2016/078838 und WO 2018/032022 jeweils Stereolithographiesysteme, bei denen zu härtende Schichten aus photopolymerisierbarem Material erhitzt werden, um die Viskosität der Materialien zu verringern. Diese Verfahren können es ermöglichen, Materialien mit Harzen mit höheren Viskositäten zu verarbeiten, als es sonst möglich wäre. Viele dieser Verfahren können auch die Bandbreite von Monomeren und/oder Oligomeren, die für additive Fertigung verwendet werden, erweitern und die Verwendung einer größeren Vielfalt an Harzformulierungen ermöglichen. Diese Verfahren können auch die Bandbreite an Produkten erweitern, die durch Härtung der darin angeführten Formulierungen erhalten werden.

Um solche Eigenschaften zu erzielen, können oft hochmolekulare Präpolymere, wie z.B. Präpolymere mit einem Molekulargewicht von mehr als 5 kDa, in den oben angeführten Photopolymerisationsverfahren auf Basis von Hochtemperaturlithographie verarbeitet werden. Für eine reibungslose Verarbeitung solcher Harze, selbst bei Verarbeitungstemperaturen von beispielsweise 90 °C bis 120 °C oder von beispielsweise mehr als 120 °C, ist es oft wünschenswert, geeignete Reaktivverdünner zuzusetzen, z.B. härtbare Monomere, die, wenn sie mit den Harzen vermischt werden, die Viskosität der Formulierung senken und Teil der durch das Photopolymerisationsverfahren erhaltenen Polymere werden. Da jedoch die Polymere mitunter beträchtliche Mengen an Grundeinheiten des Reaktivverdünners enthalten, wäre es wünschenswert, dass die Grundeinheiten des Reaktivverdünners die gewünschten thermomechanischen Eigenschaften nicht beeinträchtigen, sondern diese eher unterstützen.

Aus dem Stand der Technik sind einige polymerisierbare Monomere auf Basis von 2- bzw. 4-(Meth)acryloyloxybenzoesäureestern sowie Derivaten und Homologen davon bekannt. So offenbart beispielsweise JP 2001/066777 A neben einer großen Anzahl anderer Monomere auch einige derartige Ester, darunter 2-(α-Chloracryloyloxy)benzoesäurecyclohexylester, zur Verwendung in Homopolymerisationsverfahren zur Herstellung von Homopolymeren, die in Kombination mit einem Photosäurebildner Photoresist-Zusammensetzungen ergeben sollen. US 2011/003909 A1 offenbart polyfunktionelle (Meth)acrylat-Monomere zur Verwendung in Nanoimprinting-Anwendungen zur Herstellung von Nanostrukturen, darunter neben einigen anderen auch 4-Acryloyloxybenzoesäure-2-cyclohexenylester. In JP 2013/100471 A wird eine Vielzahl polymerisierbarer Monomere zur Herstellung von Mikrostrukturen durch Resist Patterning beschrieben, darunter auch 4-(Meth)acryloyloxybenzoesäureester wie 4-Acryloyloxybenzoesäure-2,2-dimethyl-1,3-dioxolan-4-ylester. JP 2013/112631 A beschreibt zahlreiche Ausführungsformen polymerisierbarer Monomere auf Basis halogenierter Aromaten zur Herstellung von Flüssigkristallen mittels Homopolymerisation, darunter unter anderem die jeweiligen 4-Propylcyclohexylester von 3-Chlor-4-methacryloyloxybenzoesäure, 4-Acryloyloxy-3-chlorbenzoesäure und 4-Acryloyloxy-3,5-dichlorbenzoesäure. Ebenfalls zur Herstellung von Flüssigkristallen durch Homopolymerisation offenbaren US 5.362.315 A unter anderem 4-Methacryloyloxybenzoesäure-4-ethylphenylester und US 5.211.877 A und EP 358.208 A2 ebenfalls 4-substituierte 4-Methacryloyloxybenzoesäureester, darunter den 4-Methacryloyloxybenzoesäure-4-allyloxyphenylester und den 4-Methacryloyloxybenzoesäure-4-methacryloyloxyphenylester. Und schließlich werden von einer Arbeitsgruppe in mehreren Publikationen der 4-Acryloyloxy- und der 4-Methacryloyloxybenzoesäurebenzylester zur Herstellung von Homo- und Copolymeren mit Glycidyl- oder Methylmethacrylat zur Verwendung als Klebstoffe beschrieben; siehe C.S. Jone Selvamalar, T. Krithiga, A. Penlidis, S. Nanjundan, "Copolymerization of 4-benzyloxycarbonylphenyl methacrylate with glycidyl methacrylate: synthesis, characterization, reactivity ratios and application as adhesives", React. Funct. Polym. 56(2), 89-101 (2003); C.S. Jone Selvamalar, A. Penlidis, S. Nanjundan, "Copolymers of 4-Benzyloxycarbonylphenyl Methacrylate with Methyl Methacrylate: Synthesis, Characterization, and Reactivity Ratios", J. Macromol. Sci. Pure Appl. Chem. 40(10), 1019-1033 (2003); C.S. Jone Selvamalar, P.S. Vijayanand, A. Penlidis, S. Nanjundan, "Homopolymer and copolymers of 4-benzyloxycarbonylphenyl acrylate with glycidyl methacrylate: Synthesis, characterization, reactivity ratios, and application as adhesive for leather", J. Appl. Polym. Sci. 91, 3604-3612 (2004); und C.S. Jone Selvamalar, A. Penlidis, S. Nanjundan, "Copolymerization of 4-Benzyloxycarbonylphenyl Acrylate with Methyl Methacrylate: Synthesis, Characterization, and Determination of Reactivity Ratios", J. Macromol. Sci. Pure Appl. Chem. 40(2), 125-140 (2003). WO2010/064726A2 und WO2009/134488A1 offenbaren auch (Meth)acroyloxysubstituierte Benzoesäureester.

### ZUSAMMENFASSUNG DER ERFINDUNG

Die vorliegende Erfindung bezieht sich auf eine Verwendung nach Anspruch 1. Bevorzugte Ausführungsformen der Verwendung sind in den Unteransprüchen 2 bis 15 definiert. Weiter bezieht sich die vorliegende Erfindung auf eine kieferorthopädische Vorrichtung nach Anspruch 16. Eine bevorzugte Ausführungsform ist im Unteranspruch 17 definiert. Weiter bezieht sich die vorliegende Erfindung auf einen (Meth)acroyloxy-subsituierten Benzoesäureseter nach Anspruch 18.
In Bezug auf die hierin angeführten Probleme besteht das Ziel der vorliegenden Offenbarung in der Bereitstellung neuer polymerisierbarer Monomere. Diese polymerisierbaren Monomere können in verschiedenen Anwendungen eingesetzt werden, insbesondere jedoch als Reaktivverdünner in Photopolymerisationsverfahren, wie z.B. Photopolymerisationsverfahren auf Lithographiebasis (z.B. Hochtemperaturverfahren auf Lithographiebasis, die bei Temperaturen von mehr als 70 °C oder mehr als 90 °C durchgeführt werden). Die neuen polymerisierbaren Monomere sowie weitere hierin erläuterte Zusammensetzungen ermöglichen insbesondere bei Verwendung als Reaktivverdünner eine gute Verarbeitbarkeit in additiven Fertigungsverfahren und können gehärtete Produkte mit thermomechanischen Eigenschaften bereitstellen, die für verschiedene Anwendungen wünschenswert sind, einschließlich das Formen von medizinischen Vorrichtungen und/oder jenen Gegenständen, die in einem intraoralen Umfeld verwendet werden, wie z.B. Alignern, Expandern oder Spacern. Zusätzlich dazu besteht ein Ziel der vorliegenden Offenbarung in der Bereitstellung härtbarer Zusammensetzungen und von Vorläufern davon zur Verwendung in Photopolymerisationsverfahren auf Lithographiebasis zur Herstellung verschiedener Gegenstände, wie z.B. den als medizinische Vorrichtungen und/oder den in einem intraoralen Umfeld verwendeten Gegenständen, z.B. intraoralen Vorrichtungen wie Alignern, Expandern oder Spacern. Ein weiteres Ziel besteht in der Bereitstellung eines Verfahrens zum Polymerisieren einer härtbaren Zusammensetzung zum Erhalt von gegebenenfalls vernetzten Polymeren, wobei die polymerisierbaren Monomere als Reaktivverdünner verwendet werden, um vorteilhafte Eigenschaften in Bezug auf die Verarbeitbarkeit der Zusammensetzungen und die thermomechanischen Eigenschaften der dadurch erhaltenen Polymere zu erzielen.

In einem ersten Aspekt erreicht die vorliegende Offenbarung diese und weitere Ziele durch die Bereitstellung der Verwendung eines (Meth)acryloyloxy-substituierten Benzoesäureesters der allgemeinen Formel (III): 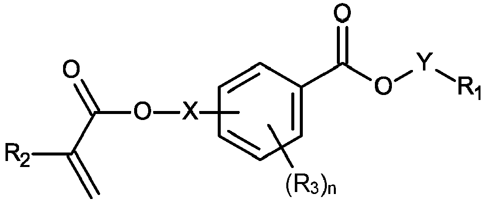 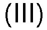 worin
R₁ für C₃-C₁₀-Cycloalkyl oder C₆-C₁₀-Aryl steht, wobei das C₃-C₁₀-Cycloalkyl und C₆-C₁₀-Aryl unsubstituiert oder mit einem oder mehreren Substituenten, ausgewählt aus C₁-C₆-Alkyl und C₁-C₆-Alkoxy substituiert sind;
R₂ für H oder CH₃ steht;
die R₃ jeweils unabhängig für C₁-C₆-Alkyl oder C₁-C₆-Alkoxy stehen;
n eine ganze Zahl von 0 bis 4 ist; und
X und Y jeweils unabhängig fehlen oder C₁-C₃-Alkylen sind, mit der Maßgabe, dass, wenn R₁ für C₆-C₁₀-Aryl steht, Y C₁-C₃-Alkylen ist;
als Reaktivverdünner in einem Verfahren zum Polymerisieren einer härtbaren Zusammensetzung, die zumindest eine Art von polymerisierbarer Spezies umfasst, wobei das Verfahren die Schritte des Bereitstellens der härtbaren Zusammensetzung, des Vermischens der härtbaren Zusammensetzung mit dem Reaktivverdünner und des Polymerisierens des Gemischs zu einem gegebenenfalls vernetzten Polymer umfasst

In bevorzugten Ausführungsformen steht in Formel (III) R₁ für gegebenenfalls substituiertes C₅-C₁₀-Cycloalkyl oder gegebenenfalls substituiertes Phenyl. Weitere bevorzugte Ausführungsformen sehen vor, dass in Formel (III) X und Y jeweils unabhängig entweder fehlen oder C₁-C₂-Alkylen sind, wobei besonders bevorzugt X fehlen kann.

Weitere bevorzugte Ausführungsformen sind durch eines oder mehrere der folgenden Merkmale gekennzeichnet:
das C₃-C₁₀-Cycloalkyl und C₆-C₁₀-Aryl von R₁ sind jeweils unsubstituiert oder mit einem oder mehreren Substituenten, ausgewählt aus C₁-C₃-Alkyl und C₁-C₃-Alkoxy substituiert; und/oder
die R₃ stehen jeweils unabhängig für C₁-C₃-Alkyl oder C₁-C₃-Alkoxy; und/oder
n = 0 oder 1.

In weiteren bevorzugten Ausführungsformen steht in Formel (III) R₁ entweder für unsubstituiertes Phenyl oder ist aus der aus 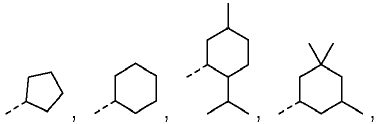 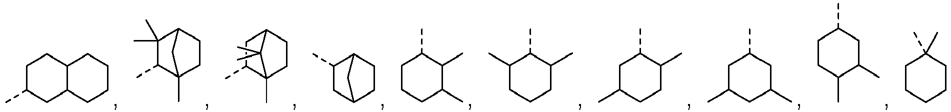 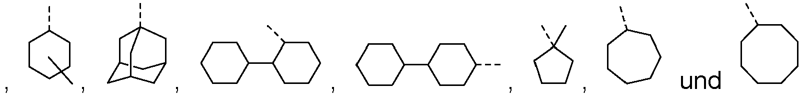 bestehenden Gruppe, noch bevorzugter aus der aus 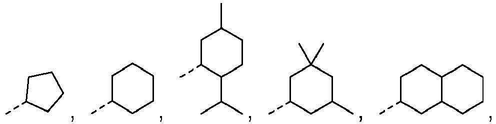 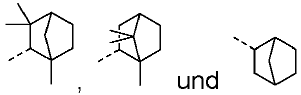 bestehenden Gruppe ausgewählt; wobei die gestrichelten Linien jeweils für die Stelle der Bindung an Y bzw. die Benzoat-Carboxylgruppe stehen.

In manchen Ausführungsformen ist in Formel (III) -Y-R₁ aus den folgenden Strukturen ausgewählt: 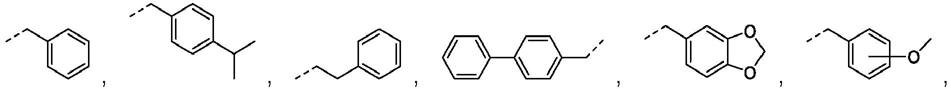 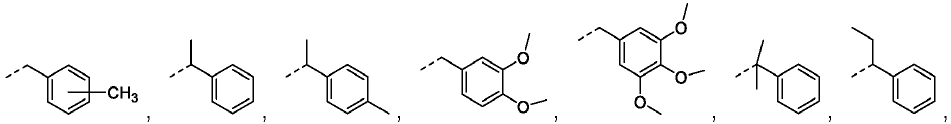 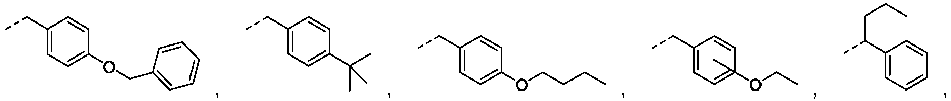 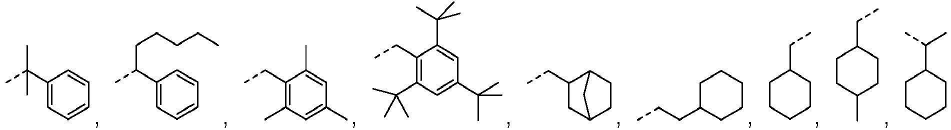 und 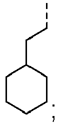 wobei die gestrichelten Linien jeweils für die Stelle der Bindung an die Benzoat-Carboxylgruppe stehen.

In besonders bevorzugten Ausführungsformen ist der (Meth)acryloyloxy-substituierte Benzoesäureester der allgemeinen Formel (III) aus den folgenden chemischen Verbindungen ausgewählt:
2-(Methacryloyloxy)benzoesäurecyclopentylester (1);
2-(Methacryloyloxy)benzoesäurecyclohexylester (2);
2-(Methacryloyloxy)benzoesäure-2-isopropyl-5-methylcyclohexylester (3);
3-(Methacryloyloxy)benzoesäure-2-isopropyl-5-methylcyclohexylester (4);
4-(Methacryloyloxy)benzoesäure-2-isopropyl-5-methylcyclohexylester (5);
2-(Methacryloyloxy)benzoesäure-3,3,5-trimethylcyclohexylester (6);
2-(Acryloyloxy)benzoesäure-3,3,5-trimethylcyclohexylester (7);
2-(Methacryloyloxy)benzoesäuredecahydronaphthalin-2-ylester (8);
2-(Methacryloyloxy)benzoesäure-1,3,3-trimethyl-2-bicyclo[2.2.1]heptanylester (9);
2-(Methacryloyloxy)benzoesäure-1,7,7-trimethyl-2-bicyclo[2.2.1]heptanylester (10);
2-(Methacryloyloxy)benzoesäurebicyclo[2.2.1]heptan-2-ylmethylester (11);
2-(Methacryloyloxy)benzoesäure-2-cyclohexylethylester (12);
2-(Methacryloyloxy)benzoesäurebenzylester (13);
4-(Methacryloyloxy)benzoesäurebenzester (14);
3-(Methacryloyloxy)benzoesäure-4-isopropylbenzylester (15);
2-(Acryloyloxy)benzoesäurebenzylester (16);
2-(Methacryloyloxy)benzoesäurephenethylester (17);
4-(Methacryloyloxy)-3-methoxybenzoesäure-3-methoxybenzylester (18);
2-(Methacryloyloxy)benzoesäure-1-phenylethylester (19);
4-((Methacryloyloxy)methyl)benzoesäurecycloheptylester (20);
2-(Methacryloyloxy)benzoesäurecyclohexylmethylester (21),
da die Erfinder bei Verwendung dieser Verbindungen als Reaktivverdünner durchwegs besonders gute Ergebnisse erzielt haben.

In weiteren bevorzugten Ausführungsformen wird der Schritt des Vermischens der härtbaren Zusammensetzung mit dem Reaktivverdünner nach Erhitzen der härtbaren Zusammensetzung und/oder des Reaktivverdünners durchgeführt. Außerdem kann die härtbare Zusammensetzung gemäß vorliegender Erfindung eine oder mehrere weitere Komponenten, ausgewählt aus der aus Polymerisationsinitiatoren, Polymerisationshemmern, Lösungsmitteln, Füllstoffen, Antioxidationsmitteln, Pigmenten, Farbstoffen, Oberflächenmodifikatoren und Gemischen davon bestehenden Gruppe umfassen. Die härtbare Zusammensetzung umfasst zumindest einen Photopolymerisationsinitiator und wird folglich bestrahlt, um im Polymerisationsschritt die Polymerisation zu initiieren, um die gegebenenfalls vernetzten Polymere herzustellen. Dabei wird die härtbare Zusammensetzung vorzugsweise zunächst auf eine vorab festgelegte erhöhte Verfahrenstemperatur im Bereich von 90 °C bis 120 °C erhitzt, bevor sie mit Licht bestrahlt wird, das eine geeignete Wellenlänge aufweist, um von dem Photoinitiator absorbiert zu werden, wodurch eine Spaltung des Photoinitiators bewirkt wird, um die Polymerisation der härtbaren Zusammensetzung zu induzieren.

Das Verfahren zum Polymerisieren der härtbaren Zusammensetzung ist gemäß vorliegender Erfindung vorzugsweise Teil eines additiven Fertigungsverfahrens oder eines 3D-Druckverfahrens.

Die härtbare Zusammensetzung umfasst dabei in bevorzugten Ausführungsformen zumindest ein mehrwertiges Monomer als die zumindest eine Art von polymerisierbarer Spezies und wird zu einem vernetzten Polymer polymerisiert, das besonders gute Eigenschaften, unter anderem Biokompatibilität, aufweisen kann.

In einem weiteren Aspekt stellt die vorliegende Erfindung ein durch die erfindungsgemäße Verwendung gemäß dem ersten Aspekt erhaltenes, vernetztes, Polymer bereit, das Gruppierungen der nachstehenden Formel (IV) als von dem (Meth)acryloyloxy-substituierten Benzoesäureester der Formel (III) abgeleitete Grundeinheiten umfasst: 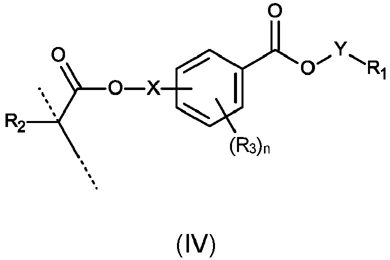 worin R₁, R₂, R₃, X, Y und n wie oben beschrieben definiert sind; und die gestrichelten Linien jeweils für eine Bindung an ein weiteres Kohlenstoffatom in der Polymerkette stehen.

Das so erhaltene Polymer ist ein vernetztes Polymer und dient zur Verwendung als kieferorthopädische Vorrichtung.

Folglich stellt die vorliegende Erfindung eine kieferorthopädische Vorrichtung bereit, die dadurch gekennzeichnet ist, dass sie ein solches vernetztes Polymer wie oben beschrieben umfasst, und bevorzugt ein Aligner, Expander oder Spacer ist.

Und schließlich stellt die Erfindung auch die von den Erfindern neu synthetisierten Substanzen bereit, die insbesondere, aber nicht ausschießlich, als Reaktivverdünner eingesetzt werden können. Genauer gesagt stellt sie also einen (Meth)acryloyloxysubstituierten Benzoesäureester der allgemeinen Formel (III) bereit: 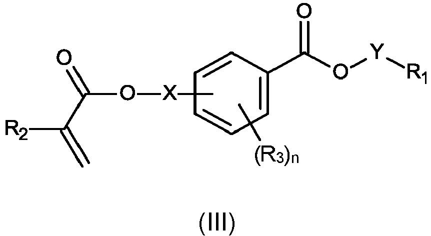 worin R₁, R₂, R₃, X, Y und n wie oben beschrieben definiert sind, der dadurch gekennzeichnet ist, dass R₁, R₂, R₃, X, Y und n jeweils so ausgewählt sind, dass sie eine der folgenden Verbindungen ergeben:
2-(Methacryloyloxy)benzoesäurecyclopentylester (1) 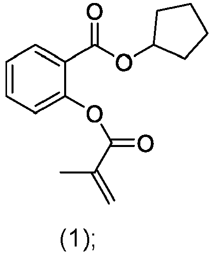 oder
2-(Methacryloyloxy)benzoesäurecyclohexylester (2) 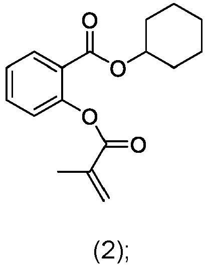 oder
2-(Methacryloyloxy)benzoesäure-2-isopropyl-5-methylcyclohexylester (3) 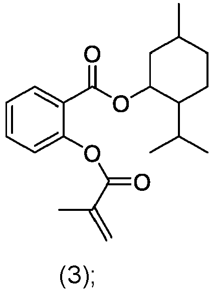 oder
3-(Methacryloyloxy)benzoesäure-2-isopropyl-5-methylcyclohexylester (4) 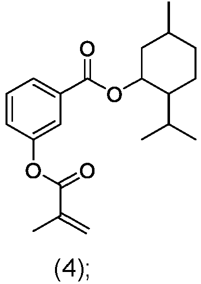 oder
4-(Methacryloyloxy)benzoesäure-2-isopropyl-5-methylcyclohexylester (5) 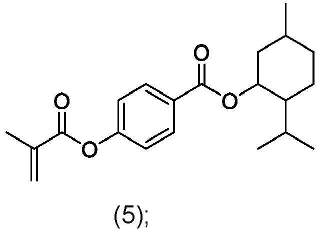 oder
2-(Methacryloyloxy)benzoesäure-3,3,5-trimethylcyclohexylester (6) 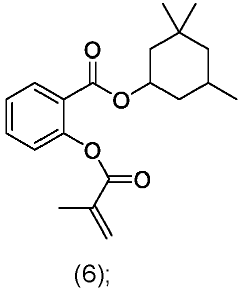 oder
2-(Acryloyloxy)benzoesäure-3,3,5-trimethylcyclohexylester (7) 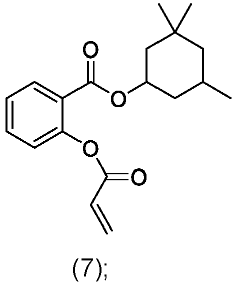 oder
2-(Methacryloyloxy)benzoesäuredecahydronaphthalin-2-ylester (8) 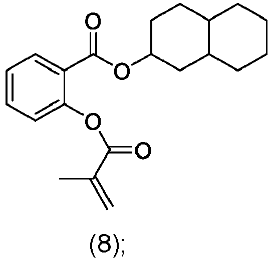 oder
2-(Methacryloyloxy)benzoesäure-1,3,3-trimethyl-2-bicyclo[2.2.1]heptanylester (9) 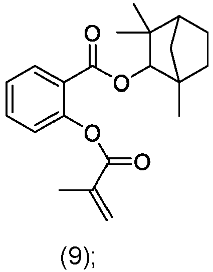 oder
2-(Methacryloyloxy)benzoesäure-1,7,7-trimethyl-2-bicyclo[2.2.1]heptanylester (10) 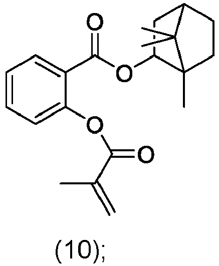 oder
2-(Methacryloyloxy)benzoesäurebicyclo[2.2.1]heptan-2-ylmethylester (11) 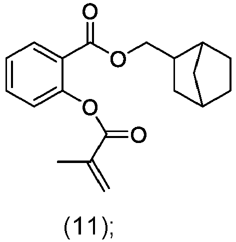 oder
2-(Methacryloyloxy)benzoesäure-2-cyclohexylethylester (12) 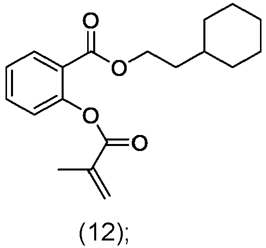 oder
2-(Methacryloyloxy)benzoesäurebenzylester (13) 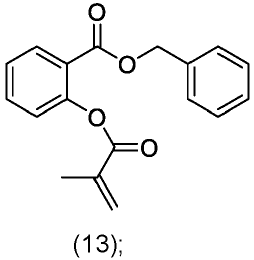 oder
3-(Methacryloyloxy)benzoesäure-4-isopropylbenzylester (15) 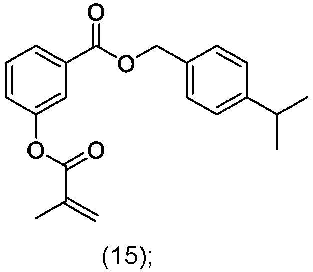 oder
2-(Acryloyloxy)benzoesäurebenzylester (16) 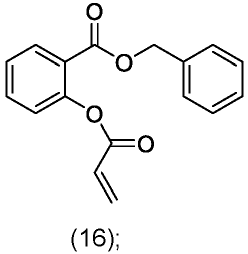 oder
2-(Methacryloyloxy)benzoesäurephenethylester (17) 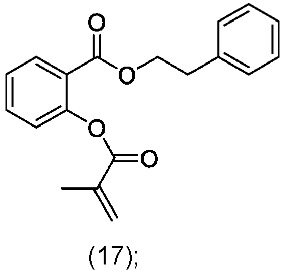 oder
4-(Methacryloyloxy)-3-methoxybenzoesäure-3-methoxybenzylester (18) 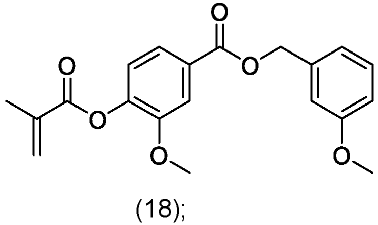 oder
2-(Methacryloyloxy)benzoesäure-1-phenylethylester (19) 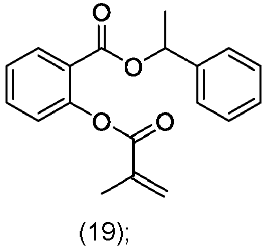 oder
4-((Methacryloyloxy)methyl)benzoesäurecycloheptylester (20) 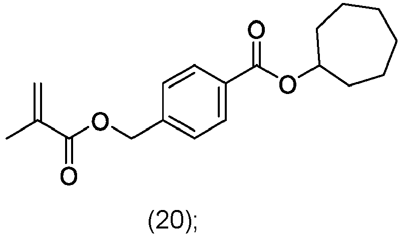 oder
2-(Methacryloyloxy)benzoesäurecyclohexylmethylester (21)

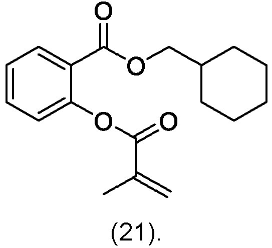

Die vorliegende Offenbarung umfasst jedoch neben der oben definierten Gruppe von (Meth)acryloyloxy-substituierten Benzoesäureestern der allgemeinen Formel (III) und deren Verwendung als Reaktivverdünnern auch noch weitere Verbindungen der Formel (III), in denen eine oder mehrere Gruppierungen, ausgewählt aus dem Benzolring der Benzoesäure, dem (Meth)acryloyl-Rest, den Alkylen-Linkern X und Y und dem Ester-Substituenten R₁ zusätzlich einen oder mehrere Substituenten aufweisen, solange dadurch die erfindungsgemäße Verwendung als Reaktivverdünner nicht beeinträchtigt wird, oder genauer gesagt, solange die Verträglichkeit des als Reaktivverdünner eingesetzten (Meth)acryloyloxy-substituierten Benzoesäureesters mit der zumindest einen Art von polymerisierbarer Spezies oder sonstigen Komponenten der härtbaren Zusammensetzung und der Ablauf der Polymerisationsreaktion nicht beeinträchtigt werden.

Diese optionalen weiteren Substituenten sind vorzugsweise aus der aus C₁-C₆-Alkyl, C₁-C₆-Alkoxy, C₃-C₇-Cycloalkyl, C₆-C₁₀-Aryl, C₁-C₆-Alkoxy-C₆-C₁₀-aryl, -O(CO)-(C₁-C₆)-Alkyl, -COO-(C₁-C₆)-Alkyl, =O, -F, -Cl und -Br bestehenden Gruppe ausgewählt, noch bevorzugter aus der aus C₁-C₆-Alkyl, C₁-C₆-Alkoxy, =O, -F, -Cl und -Br bestehenden Gruppe, noch bevorzugter aus der aus C₁-C₃-Alkyl, C₁-C₃-Alkoxy, -F, -Cl und -Br bestehenden Gruppe. Wie einschlägigen Fachleuten auf dem Gebiet der Erfindung klar sein wird, werden derartige, relativ kurzkettige oder einatomige Substituenten, insbesondere kurzkettige Substituenten mit nur 1 bis 6 oder 1 bis 3 Kohlenstoffatomen oder Halogenatome, mit hoher Wahrscheinlichkeit keine negativen Auswirkungen auf die erfindungsgemäße Verwendung solcher (Meth)acryloyloxy-substituierter Benzoesäureester als Reaktivverdünner bewirken.

Die vorliegende Offenbarung umfasst in einem Aspekt neue polymerisierbare Monomere, die jeweils ein 2-, 3- oder 4-(Meth)acryloyloxybenzoesäureester der Formel (I) sind: 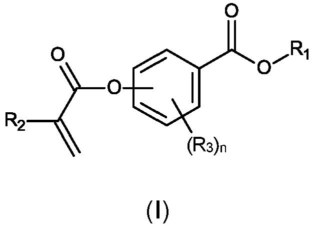
worin R₁ für einen cycloaliphatischen C₅-C₂₀-Rest steht, der aus der aus gegebenenfalls substituierten C₅-C₇-Cycloalkylresten bestehenden Gruppe ausgewählt ist;
R₂ für H oder CH₃ steht;
die R₃ jeweils unabhängig für C₁-C₃-Alkyl oder C₁-C₃-Alkoxy stehen; und
n eine ganze Zahl von 0 bis 4 ist.

Solche gegebenenfalls substituierte Cycloalkylester von 2-, 3- oder 4-(Meth)-acryloyloxybenzoesäure oder (Meth)acrylate von Cycloalkyl-2-, -3- oder -4-hydroxybenzoaten: (i) sind entweder bei Raumtemperatur flüssig oder weisen relative niedrige Schmelzpunkte auf, z.B. Schmelzpunkte, die unter den üblichen Verarbeitungstemperaturen von Hochtemperaturphotopolymerisationsverfahren auf Lithographiebasis, wie z.B. Hochtemperatur-3D-Druck, liegen, vorzugsweise Schmelzpunkte von < 90 °C, (ii) bewirken eine beträchtliche Reduktion der Viskosität einer Formulierung, zu der sie als Reaktivverdünner zugesetzt werden, (iii) sind selbst mit hochmolekularen Präpolymeren, die in solchen Formulierungen enthalten sind, verträglich und einfach copolymerisierbar, (iv) sind bei den üblichen Verarbeitungstemperaturen von Hochtemperaturphotopolymerisationsverfahren auf Lithographiebasis im Wesentlichen nicht flüchtig und (v) ergeben Polymere, die wünschenswert hohe Glasübergangstemperaturen und weitere wünschenswerte thermomechanische Eigenschaften aufweisen.

Insbesondere das gemeinsame Auftreten von niedrigen Schmelzpunkten und im Wesentlichen keiner Flüchtigkeit bei erhöhten Temperaturen war sehr überraschend, da z.B. Salicylate, d.h. 2-Hydroxybenzoesäureester, aufgrund ihres charakteristischen, an Kampfer erinnernden Geruchs schon lange als Geschmacksstoffe und Duftstoffe eingesetzt werden, was bedeutet, dass Salicylate bereits bei Raumtemperatur recht hohe Flüchtigkeit aufweisen. Es ist jedoch sehr überraschend, dass keines der (meth)acrylierten Salicylate gemäß der vorliegenden Offenbarung nennenswerte Flüchtigkeit aufwies, weder bei Raumtemperatur noch bei erhöhten Temperaturen von 90 °C, was sie für die Verwendung als Monomere in Hochtemperaturpolymerisationsverfahren besonders geeignet macht.

Dennoch sind optionale Substituenten von R₁ vorzugsweise kleine Substituenten, die das Polymerisationsverfahren nicht beeinflussen oder unerwünschte Nebenreaktionen mit etwaigen anderen Komponenten der härtbaren Harzformulierungen, die die polymerisierbaren Monomere gemäß der vorliegenden Offenbarung umfassen, auslösen, die die Schmelzpunkte nicht auf unerwünschte Weise erhöhen und die die viskositätssenkende Wirkung der neuen Monomere nicht beeinträchtigen. Aus diesem Grund sind in manchen Ausführungsformen die optionalen Substituenten der C₅-C₇-Cycloalkylreste von R₁ aus der aus C₁-C₆-Alkyl, C₁-C₆-Alkoxy, -O(CO)-(C₁-C₆)-Alkyl, -COO-(C₁-C₆)-Alkyl, =O, -F, -Cl, -Br und Gemischen davon bestehenden Gruppe ausgewählt.

Um das Molekulargewicht der polymerisierbaren Monomere gemäß Formel (I) relativ niedrig zu halten, ist R₁ in manchen Ausführungsformen der vorliegenden Offenbarung ein gegebenenfalls substituierter C₅-C₇-Cycloalkylrest mit insgesamt 5 bis 15, noch bevorzugter mit insgesamt 5 bis 12, insbesondere mit insgesamt 5 bis 10, Kohlenstoffatomen. In manchen Ausführungsformen ist R₁ ein C₅-C₇-Cycloalkylrest, der entweder unsubstituiert oder mit einer oder mehreren, unverzweigten oder verzweigten C₁-C₆-Alkylgruppen, noch bevorzugter mit einer oder mehreren C₁-C₃-Alkylgruppen, substituiert ist, wobei zwei der C₁-C₆-Alkylgruppen oder C₁-C₃-Alkylgruppen verbunden sein können, um gemeinsam mit den Kohlenstoffatomen, an die sie gebunden sind, und gegebenenfalls mit einem oder mehreren zusätzlichen, dazwischen liegenden Kohlenstoffatomen des Cyclohexylrings einen Ring zu bilden, da solche Monomere der Formel (I) bei Raumtemperatur entweder flüssig sind oder geeignet niedrige Schmelzpunkte aufweisen und eine ausgeprägte viskositätssenkende Wirkung aufweisen. Aus denselben Gründen ist R₁ in manchen Ausführungsformen ein substituierter Cyclohexylrest, der aus der aus folgenden Resten bestehenden Gruppe ausgewählt ist: 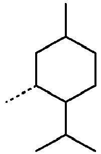
2-Isopropyl-5-methylcyclohexyl (Menthyl); 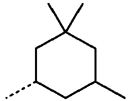
3,3,5-Trimethylcyclohexyl (Homomenthyl); 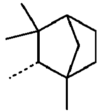
1,3,3-Trimethyl-2-norbornanyl, 1,3,3-Trimethyl-2-bicyclo[2.2.1]heptanyl (Fenchyl); und 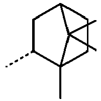
1,7,7-Trimethyl-2-bicyclo[2.2.1]heptanyl ((Iso)bornyl);
wobei die gestrichelten Linien in den oben angeführten Formeln jeweils die Bindung an das Estersauerstoffatom darstellen. Polymerisierbare Monomere gemäß der vorliegenden Offenbarung, die diese substituierten Cyclohexylreste als Estergruppierungen umfassen, haben bereits gute Ergebnisse geliefert, wie im experimentellen Teil gezeigt wird, und zusätzlich dazu sind die entsprechenden Alkohole, die zur Herstellung der jeweiligen (Meth)acryloyloxybenzoesäuresester eingesetzt werden, üblicherweise zu annehmbaren Kosten im Handel erhältlich.

In weiteren Ausführungsformen sind die C₁-C₃-Alkyl- oder C₁-C₃-Alkoxy-Substituenten an dem Benzolring in Formel (I), R₃, aus der aus Methyl, Methoxy, Ethyl und Ethoxy bestehenden Gruppe ausgewählt, und in weiteren Ausführungsformen ist n = 0 oder 1, was bedeutet, dass der Benzolring in Formel (I) unsubstituiert ist oder nur einen Substituenten R₃ an einer beliebigen substituierbaren Position aufweist. Besonders bevorzugt ist n jedoch 0.

In manchen Ausführungsformen der vorliegenden Offenbarung weisen die polymerisierbaren Monomere Schmelzpunkte von < 90 °C auf, damit sie bei den Temperaturen, die üblicherweise bei den derzeit verfügbaren additiven Fertigungsverfahren eingesetzt werden, flüssig und somit verarbeitbar sind. Besonders bevorzugt weisen sie Schmelzpunkte von < 50 °C oder < 30 °C auf, und besonders bevorzugt sind sie bei Raumtemperatur flüssig, was für eine niedrige Viskosität der Schmelzen und damit eine ausgeprägte viskositätssenkende Wirkung sorgt, wenn sie als Reaktivverdünner eingesetzt werden.

In manchen Ausführungsformen sind die polymerisierbaren Monomere der Formel (I) aus der aus folgenden Verbindungen bestehenden Gruppe ausgewählt:
2-(Methacryloyloxy)benzoesäurecyclopentylester (1);
2-(Methacryloyloxy)benzoesäurecyclohexylester (2);
2-(Methacryloyloxy)benzoesäure-2-isopropyl-5-methylcyclohexylester (3);
3-(Methacryloyloxy)benzoesäure-2-isopropyl-5-methylcyclohexylester (4);
4-(Methacryloyloxy)benzoesäure-2-isopropyl-5-methylcyclohexylester (5);
2-(Methacryloyloxy)benzoesäure-3,3,5-trimethylcyclohexylester (6);
2-(Acryloyloxy)benzoesäure-3,3,5-trimethylcyclohexylester (7);
2-(Methacryloyloxy)benzoesäuredecahydronaphthalin-2-ylester (8);
2-(Methacryloyloxy)benzoesäure-1,3,3-trimethyl-2-bicyclo[2.2.1]heptanylester (9);
2-(Methacryloyloxy)benzoesäure-1,7,7-trimethyl-2-bicyclo[2.2.1]heptanylester (10);
2-(Methacryloyloxy)benzoesäurebicyclo[2.2.1]heptan-2-ylmethylester (11);
2-(Methacryloyloxy)benzoesäure-2-cyclohexylethylester (12);
2-(Methacryloyloxy)benzoesäurebenzylester (13);
4-(Methacryloyloxy)benzoesäurebenzester (14);
3-(Methacryloyloxy)benzoesäure-4-isopropylbenzylester (15);
2-(Acryloyloxy)benzoesäurebenzylester (16);
2-(Methacryloyloxy)benzoesäurephenethylester (17);
4-(Methacryloyloxy)-3-methoxybenzoesäure-3-methoxybenzylester (18);
2-(Methacryloyloxy)benzoesäure-1-phenylethylester (19);
4-((Methacryloyloxy)methyl)benzoesäurecycloheptylester (20);
2-(Methacryloyloxy)benzoesäurecyclohexylmethylester (21);
die gute oder sogar hervorragende Ergebnisse geliefert haben, wie später noch ausführlicher im experimentellen Teil dargelegt ist.

In einem Aspekt stellt die vorliegende Offenbarung ein Verfahren zum Polymerisieren einer härtbaren Zusammensetzung bereit, die zumindest eine Art einer polymerisierbaren Spezies und gegebenenfalls eine oder mehrere weitere Komponenten umfasst, die aus der aus Polymerisationsinitiatoren, Polymerisationshemmern, Lösungsmitteln, Füllstoffen, Antioxidationsmitteln, Pigmenten, Farbstoffen, Oberflächenmodifikatoren und Gemischen davon bestehenden Gruppe ausgewählt sind, um ein gegebenenfalls vernetztes Polymer zu erhalten, wobei das Verfahren folgende Schritte umfasst: das Bereitstellen der härtbaren Zusammensetzung; das Vermischen der härtbaren Zusammensetzung mit einem Reaktivverdünner; und das Polymerisieren der Zusammensetzung, wobei das Reaktivverdünner aus den hierin beschriebenen polymerisierbaren Monomeren und beliebigen Gemischen davon ausgewählt ist. In einer Ausführungsform wird der Schritt des Vermischens nach Erhitzen der härtbaren Zusammensetzung durchgeführt. In einer Ausführungsform wird der Polymerisationsschritt durch Erhitzen und/oder Bestrahlen der Zusammensetzung durchgeführt.

In manchen Ausführungsformen des erfindungsgemäßen Verfahrens ist dieses Teil eines Hochtemperaturphotopolymerisationsverfahrens auf Lithographiebasis, wobei die härtbare Zusammensetzung zumindest einen Photopolymerisationsinitiator umfasst und erhitzt wird, bevor sie mit dem Reaktivverdünner vermischt wird, wobei es sich bei dem Hochtemperaturphotopolymerisationsverfahren auf Lithographiebasis noch bevorzugter um ein additives Fertigungsverfahren handelt und insbesondere um ein 3D-Druckverfahren.

Wie bereits erwähnt, sind die polymerisierbaren Monomere gemäß einem Aspekt der Offenbarung zur Verwendung als Reaktivverdünner geeignet, insbesondere in Hochtemperaturphotopolymerisationsverfahren auf Lithographiebasis, da sie durch (sehr) niedrige Schmelzpunkte und folglich niedrige Viskositäten ihrer Schmelzen und gleichzeitig durch im Wesentlichen keine Flüchtigkeit gekennzeichnet sind - selbst bei 90 °C. Hierin bedeutet "im Wesentlichen keine Flüchtigkeit" einen Massenverlust von < 1 Gew.-% bei der entsprechenden Temepratur, vorzugsweise bei 90 °C. Zusätzlich dazu bewirken die neuen polymerisierbaren Monomere der vorliegenden Offenbarung bei Verwendung als Reaktivverdünner keine Beeinträchtigung der thermomechanischen Eigenschaften der Polymere, deren Teil sie sind, sondern fördern diese vielmehr.

In weiteren Ausführungsformen des Verfahrens gemäß einem Aspekt der vorliegenden Offenbarung umfasst die härtbare Zusammensetzung zumindest einen Photopolymerisationsinitiator und wird auf eine vorab festgelegte erhöhte Verfahrenstemperatur im Bereich von 50 °C bis 120 °C erhitzt, wie z.B. auf 90 °C bis 120 °C, bevor sie mit Licht mit einer für die Absorption durch den Photoinitiator geeigneten Wellenlänge bestrahlt wird, wodurch eine Spaltung des Photoinitiators bewirkt wird, um die Polymerisation der härtbaren Zusammensetzung zu induzieren und das gegebenenfalls vernetzte Polymer zu erhalten. In weiteren Ausführungsformen umfasst die härtbare Zusammensetzung zumindest ein mehrwertiges Monomer und wird zu einem vernetzten Polymer polymerisiert.

Die auf diese Weise erhaltenen Polymere können beträchtliche Mengen an Grundeinheiten, beispielsweise Mengen von bis zu 40 Gew.-%, umfassen, die von den polymerisierbaren Monomeren der vorliegenden Offenbarung herrühren, und weisen hervorragende thermomechanische Eigenschaften auf, wie im experimentellen Teil gezeigt wird.

In einem Aspekt stellt die vorliegende Offenbarung solche gegebenenfalls vernetzte Polymere bereit, die durch das oben beschriebene Verfahren erhalten werden und Grundeinheiten umfassen, die von den polymerisierbaren Monomeren gemäß der Offenbarung herrühren.

Besonders bevorzugt sind diese vernetzten Polymere kieferorthopädische Vorrichtungen, z.B. kieferorthopädische Aligner-, Expander- oder Spacer-Vorrichtungen. In einer Ausführungsform ist das vernetzte Polymer biokompatibel und/oder bioinert. Das Verfahren gemäß einem Aspekt der vorliegenden Offenbarung bietet die Möglichkeit der raschen und einfachen Herstellung solcher kieferorthopädischen Vorrichtungen durch additive Fertigung, wie z.B. durch 3D-Druck, unter Verwendung von polymerisierbaren Monomeren als Reaktivverdünner.

In einem Aspekt stellt die vorliegende Offenbarung eine Gruppe neuer Verbindungen, d.h. spezifischer polymerisierbarer Monomere, bereit und zwar: 2-(Methacryloyloxy)benzoesäurecyclopentylester (1): 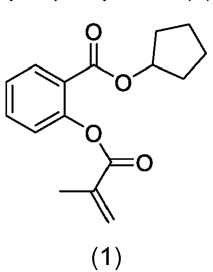
2-(Methacryloyloxy)benzoesäurecyclohexylester (2): 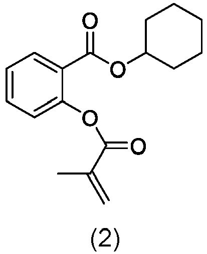
2-(Methacryloyloxy)benzoesäure-2-isopropyl-5-methylcyclohexylester (3): 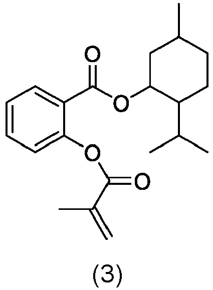
3-(Methacryloyloxy)benzoesäure-2-isopropyl-5-methylcyclohexylester (4): 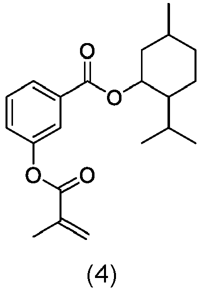
4-(Methacryloyloxy)benzoesäure-2-isopropyl-5-methylcyclohexylester (5): 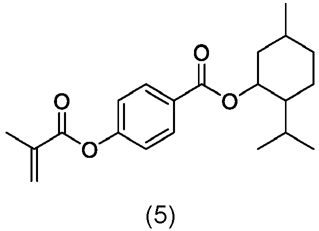
2-(Methacryloyloxy)benzoesäure-3,3,5-trimethylcyclohexylester (6): 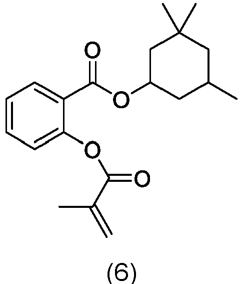
2-(Acryloyloxy)benzoesäure-3,3,5-trimethylcyclohexylester (7): 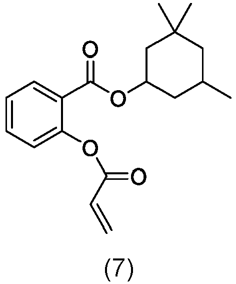
2-(Methacryloyloxy)benzoesäuredecahydronaphthalin-2-ylester (8): 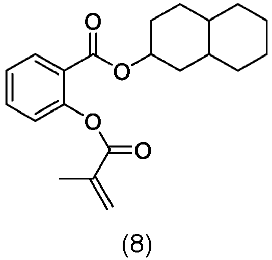
2-(Methacryloyloxy)benzoesäure-1,3,3-trimethyl-2-bicyclo[2.2.1]heptanylester (9): 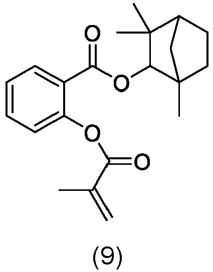
2-(Methacryloyloxy)benzoesäure-1,7,7-trimethyl-2-bicyclo[2.2.1]heptanylester (10): 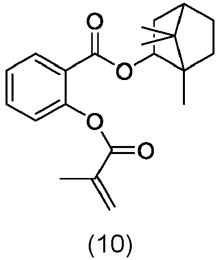
2-(Methacryloyloxy)benzoesäurebicyclo[2.2.1]heptan-2-ylmethylester (11): 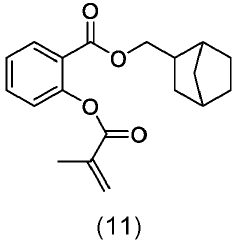
2-(Methacryloyloxy)benzoesäure-2-cyclohexylethylester (12): 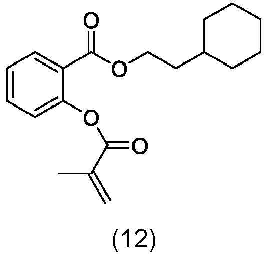
2-(Methacryloyloxy)benzoesäurebenzylester (13): 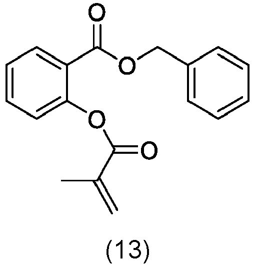
3-(Methacryloyloxy)benzoesäure-4-isopropylbenzylester (15): 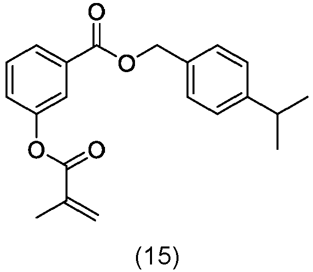
2-(Acryloyloxy)benzoesäurebenzylester (16): 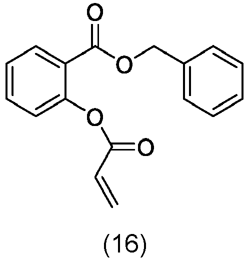
2-(Methacryloyloxy)benzoesäurephenethylester (17): 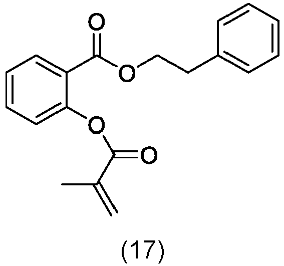
4-(Methacryloyloxy)-3-methoxybenzoesäure-3-methoxybenzylester (18): 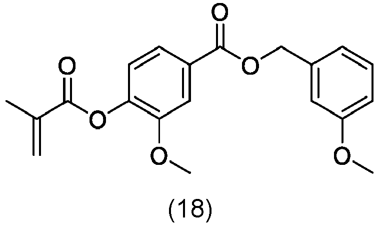
2-(Methacryloyloxy)benzoesäure-1-phenylethylester (19): 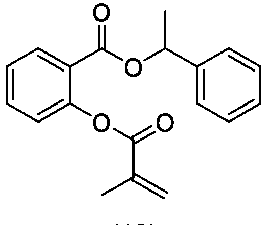 (19)
4-((Methacryloyloxy)methyl)benzoesäurecycloheptylester (20): 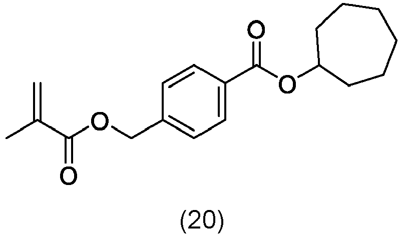
2-(Methacryloyloxy)benzoesäurecyclohexylmethylester (21):

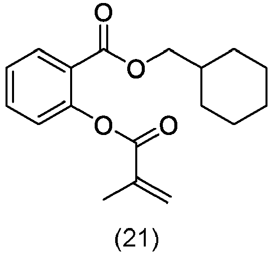

In manchen Aspekten stellt die vorliegende Offenbarung ein polymerisierbares Monomer bereit, das ein 2-, 3- oder 4-(Meth)acryloylbenzoesäureester der Formel (I) ist: 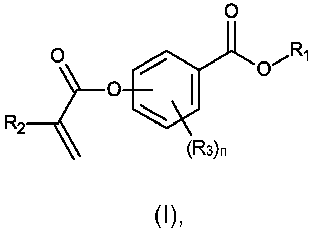 worin:
R₁ für C₃-C₁₀-Cycloalkyl steht, wobei das C₃-C₁₀-Cycloalkyl unsubstituiert ist; mit einem oder mehreren Substituenten, ausgewählt aus der aus -CH₃, C₁-C₆-Alkoxy, -O(CO)-(C₁-C₆)-Alkyl, -COO-(C₁-C₆)-Alkyl, =O, -F, -Cl und -Br bestehenden Gruppe substituiert ist; mit einem oder mehreren -CH₃ substituiert ist und gegebenenfalls außerdem mit einem oder mehreren Substituenten, ausgewählt aus der C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert ist; oder mit zwei oder mehr Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert ist;
R₂ für H oder C₁-C₆-Alkyl steht;
die R₃ jeweils unabhängig für Halogen, C₁-C₃-Alkyl, C₁-C₃-Alkoxy, Si(R₄)₃, P(O)(OR₅)₂ oder N(R₆)₂ stehen;
die R₄ jeweils unabhängig für C₁-C₆-Alkyl oder C₁-C₆-Alkoxy stehen;
die R₅ jeweils unabhängig für C₁-C₆-Alkyl oder C₆-C₁₀-Aryl stehen;
die R₆ jeweils unabhängig für H oder C₁-C₆-Alkyl stehen; und
n eine ganze Zahl von 0 bis 4 ist.

In manchen Aspekten stellt die vorliegende Offenbarung ein polymerisierbares Monomer bereit, das ein 2-, 3- oder 4-substituierter Benzoesäureester der Formel (II) ist: 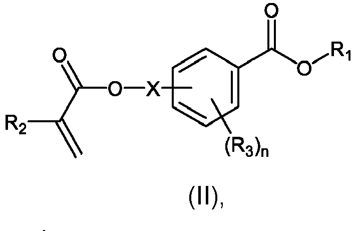 worin:
R₁ für C₃-C₇-Cycloalkyl oder 3- bis 7-gliedriges Heterocycloalkyl steht, wobei das C₃-C₇-Cycloalkyl und das 3- bis 7-gliedrige Heterocycloalkyl unsubstituiert sind; mit einem oder mehreren Substituenten, ausgewählt aus der aus -CH₃, C₁-C₆-Alkoxy, -O(CO)-(C₁-C₆)-Alkyl, -COO-(C₁-C₆)-Alkyl, =O, -F, -Cl und -Br bestehenden Gruppe substituiert ist; mit einem oder mehreren -CH₃ substituiert ist und gegebenenfalls außerdem mit einem oder mehreren Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert ist; oder mit zwei oder mehr Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert ist;
R₂ für H oder C₁-C₆-Alkyl steht;
die R₃ jeweils unabhängig für Halogen, C₁-C₃-Alkyl, C₁-C₃-Alkoxy, Si(R₄)₃, P(O)(OR₅)₂ oder N(R₆)₂ stehen;
die R₄ jeweils unabhängig für C₁-C₆-Alkyl oder C₁-C₆-Alkoxy stehen;
die R₅ jeweils unabhängig für C₁-C₆-Alkyl oder C₆-C₁₀-Aryl stehen;
die R₆ jeweils unabhängig für H oder C₁-C₆-Alkyl stehen;
X C₁-C₃-Alkylen, 1- bis 3-gliedriges Heteroalkylen oder (CH₂CH₂O)ₘ ist;
m eine ganze Zahl von 1 bis 4 ist; und
n eine ganze Zahl von 0 bis 4 ist.

In manchen Aspekten stellt die vorliegende Offenbarung ein polymerisierbares Monomer bereit, das ein 2-, 3- oder 4-substituierter Benzoesäureester der Formel (III) ist: 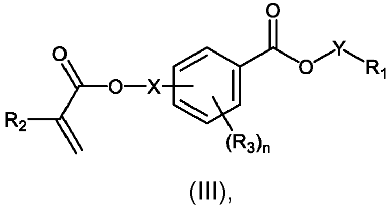 worin:
R₁ für C₃-C₇-Cycloalkyl oder C₆-C₁₀-Aryl steht, wobei das C₃-C₇-Cycloalkyl und C₆-C₁₀-Aryl unsubstituiert sind oder mit einem oder mehreren Substituenten, ausgewählt aus der aus C₁-C₆-Alkyl, C₁-C₆-Alkoxy, C₃-C₇-Cycloalkyl, C₆-C₁₀-Aryl, C₁-C₆-Alkoxy-C₆-C₁₀-aryl, -O(CO)-(C₁-C₆)-Alkyl, -COO-(C₁-C₆)-Alkyl, =O, -F, -Cl und -Br bestehenden Gruppe substituiert sind, mit der Maßgabe, dass der Benzoesäureester der Formel (III) 2- oder 3-substituiert ist, wenn R₁ unsubstituiert ist;
R₂ für H oder C₁-C₆-Alkyl steht;
die R₃ jeweils unabhängig für Halogen, C₁-C₃-Alkyl, C₁-C₃-Alkoxy, Si(R₄)₃, P(O)(OR₅)₂ oder N(R₆)₂ stehen;
die R₄ jeweils unabhängig für C₁-C₆-Alkyl oder C₁-C₆-Alkoxy stehen;
die R₅ jeweils unabhängig für C₁-C₆-Alkyl oder C₆-C₁₀-Aryl stehen;
die R₆ jeweils unabhängig für H oder C₁-C₆-Alkyl stehen;
X fehlt, C₁-C₃-Alkylen, 1- bis 3-gliedriges Heteroalkylen oder (CH₂CH₂O)ₘ ist;
Y C₁-C₆-Alkylen ist;
m eine ganze Zahl von 1 bis 4 ist; und
n eine ganze Zahl von 0 bis 4 ist.

In manchen Ausführungsformen ist Y für eine Verbindung der Formel (III) C₁-C₃-Alkylen. In manchen Ausführungsformen fehlt X. Für eine Verbindung der Formel (II) oder (III) kann X C₁-C₃-Alkylen sein. In manchen Ausführungsformen ist R₁ für eine Verbindung der Formel (I), (II) oder (III) gegebenenfalls substituiertes C₅-C₁₀-Cycloalkyl, wie z.B. gegebenenfalls substituiertes C₅-C₈-Cycloalkyl. R₁ kann mit einem oder mehreren, unverzweigten oder verzweigten C₁-C₆-Alkylgruppen substituiert sein. In manchen Ausführungsformen ist R₁ mit -CH₃ substituiert und gegebenenfalls außerdem mit einer oder mehreren, unverzweigten oder verzweigten C₁-C₆-Alkylgruppen substituiert. In manchen Ausführungsformen ist R₁ ein gegebenenfalls substituiertes C₅-C₇-Cycloalkyl mit insgesamt 5 bis 15 oder 5 bis 12 oder 5 bis 10 Kohlenstoffatomen. In manchen Ausführungsformen ist Cyclohexyl, das und gegebenenfalls außerdem mit einer oder mehreren, unverzweigten oder verzweigten C₁-C₆-Alkylgruppen substituiert ist, wobei zwei der C₁-C₆-Alkylgruppen verbunden sein können, um mit den Kohlenstoffatomen, an die sie gebunden sind, und gegebenenfalls einem oder mehreren zusätzlichen dazwischenliegenden Kohlenstoffatomen des Cyclohexylrings einen Ring zu bilden. In manchen Ausführungsformen ist R₁ Cyclohexyl, das mit -CH₃ und außerdem gegebenenfalls mit einer oder mehreren C₁-C₃ Alkylgruppen substituiert ist, wobei zwei der C₁-C₃ Alkylgruppen verbunden sein können, um mit dem Kohlenstoffatom, an das sie gebunden sind, und gegebenenfalls einem oder mehreren zusätzlichen dazwischen liegenden Kohlenstoffatomen des Cyclohexylrings einen Ring zu bilden. In manchen Ausführungsformen ist R₁ aus der aus folgenden bestehenden Gruppe ausgewählt: 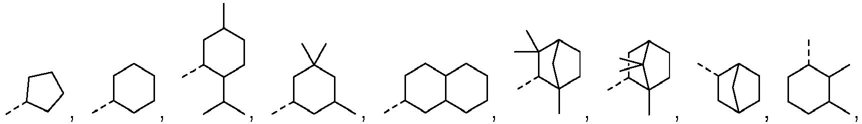 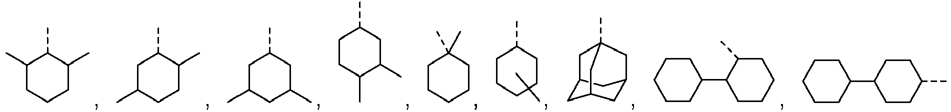 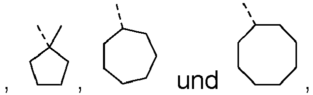 wie z.B. 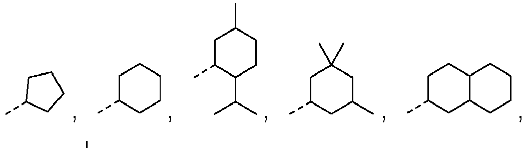 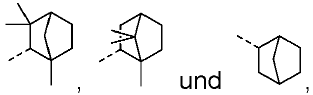 oder wie z.B.

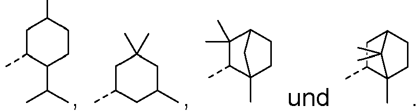

In manchen Ausführungsformen ist R₁ für eine Verbindung der Formel (III) gegebenenfalls substituiertes Phenyl. R₁ kann mit einem oder mehreren Substituenten, ausgewählt aus der aus C₁-C₄-Alkyl und C₁-C₄-Alkoxy bestehenden Gruppe substituiert sein. In manchen Ausführungsformen ist -Y-R₁ aus der aus 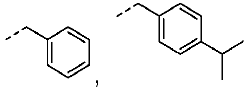 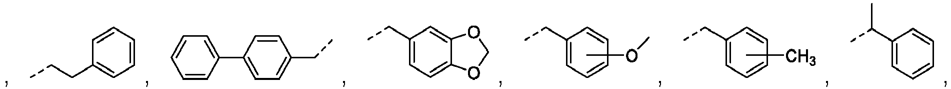 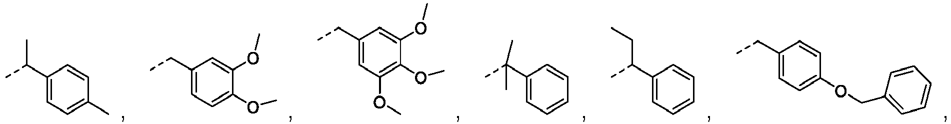 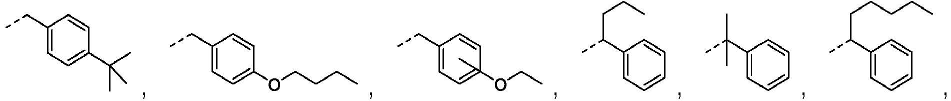 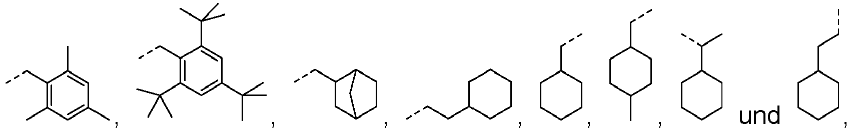 wie z.B. 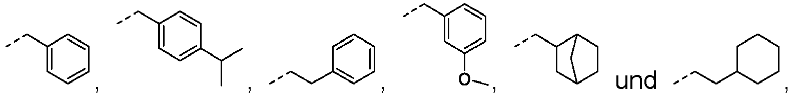 oder wie z.B. 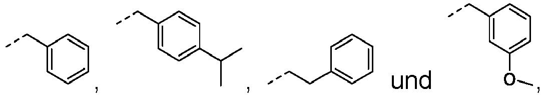 bestehenden Gruppe ausgewählt.

In manchen Ausführungsformen ist R₁ für eine Verbindung der (I), (II) oder (III), R₁ unsubstituiert. In manchen Ausführungsformen ist das Monomer ein 2- oder 3-substituierter Benzoesäureester. In manchen Ausführungsformen ist das Monomer ein 4-substituierter Benzoesäureester.

In manchen Ausführungsformen steht R₁ für eine Verbindung der Formel (I) für gegebenenfalls substituiertes C₅-C₇-Cycloalkyl; R₂ steht für H oder CH₃; die R₃ stehen jeweils unabhängig für C₁-C₃-Alkyl oder C₁-C₃-Alkoxy; und n ist eine ganze Zahl von 0 bis 4.

In manchen Ausführungsformen ist R₃ für eine Verbindung der Formel (I), (II) oder (III) aus der aus C₁-C₃-Alkyl und C₁-C₃-Alkoxy bestehenden Gruppe ausgewählt, wie z.B. Methyl, Methoxy, Ethyl und Ethoxy. In manchen Ausführungsformen ist n = 0 oder 1, wie z.B. n = 0. In manchen Ausführungsformen ist R₂ H oder CH₃. Vorzugsweise weist eine hierin beschriebene Verbindung einen Schmelzpunkt < 90 °C auf.

In manchen Aspekten stellt die vorliegende Offenbarung ein Verfahren zum Polymerisieren einer härtbaren Zusammensetzung bereit, die zumindest eine Art einer polymerisierbaren Spezies und gegebenenfalls eine oder mehrere weitere Komponenten umfasst, die aus der aus Polymerisationsinitiatoren, Polymerisationshemmern, Lösungsmitteln, Füllstoffen, Antioxidationsmitteln, Pigmenten, Farbstoffen, Oberflächenmodifikatoren und Gemischen davon bestehenden Gruppe ausgewählt sind, um ein gegebenenfalls vernetztes Polymer zu erhalten, wobei das Verfahren folgende Schritte umfasst: das Bereitstellen der härtbaren Zusammensetzung; das Vermischen der härtbaren Zusammensetzung mit einem Reaktivverdünner; und das Polymerisieren der Zusammensetzung, wobei das Reaktivverdünner aus einem hierin beschriebenen polymerisierbaren Monomer und beliebigen Gemischen davon ausgewählt ist. Das Vermischen kann nach dem Erhitzen der härtbaren Zusammensetzung durchgeführt werden. Das Polymerisieren kann durch Erhitzen und/oder Bestrahlen der Zusammensetzung durchgeführt werden. Ein hierin beschriebenes Verfahren kann Teil eines Hochtemperaturphotopolymerisationsverfahrens auf Lithographiebasis sein, wobei die härtbare Zusammensetzung zumindest einen Photopolymerisationsinitiator umfasst und erhitzt wird, bevor sie mit dem Reaktivverdünner vermischt wird. In manchen Ausführungsformen ist das Verfahren Teil eines additive Fertigungsverfahrens, wie z.B. eines 3D-Druckverfahrens. Die härtbare Zusammensetzung kann zumindest einen Photopolymerisationsinitiator umfassen und auf eine vorab festgelegte erhöhte Verfahrenstemperatur im Bereich von 50 °C bis 120 °C, wie z.B. von 90 °C bis 120 °C, erhitzt werden, bevor sie mit Licht bestrahlt wird, das eine geeignete Wellenlänge aufweist, um von dem Photoinitiator absorbiert zu werden. wodurch eine Spaltung des Photoinitiators hervorgerufen wird, um die Polymerisation der härtbaren Zusammensetzung zu induzieren, um das gegebenenfalls vernetzte Polymer zu erhalten. In manchen Ausführungsformen umfasst die härtbare Zusammensetzung zumindest ein mehrwertiges Monomer und wird zu einem vernetzten Polymer polymerisiert.

In manchen Aspekten stellt die vorliegende Erfindung ein gegebenenfalls vernetztes Polymer bereit, das durch ein hierin beschriebenes Verfahren erhalten wird und Gruppierungen eines hierin beschriebenen polymerisierbaren Monomers als Grundeinheiten umfasst. In manchen Aspekten stellt die vorliegende Offenbarung ein gegebenenfalls vernetztes Polymer bereit, das durch ein hierin beschriebenes Verfahren erhalten wird. In manchen Aspekten stellt die vorliegende Offenbarung ein gegebenenfalls vernetztes Polymer bereit, das ein hierin beschriebenes polymerisierbares Monomer umfasst. Das vernetzte Polymer ist vorzugsweise biokompatibel. In manchen Aspekten stellt die vorliegende Offenbarung eine kieferorthopädische Vorrichtung bereit, die ein hierin beschriebenes vernetztes Polymer umfasst, wie z.B. ein vernetztes Polymer, das ein polymerisierbares Monomer der vorliegenden Offenbarung umfasst. Die kieferorthopädische Vorrichtung kann ein Aligner, Expander oder Spacer sein.

In manchen Aspekten stellt die vorliegende Offenbarung eine kieferorthopädische Vorrichtung bereit, die ein Polymer umfasst, wobei das Polymer ein Monomer der Formel: 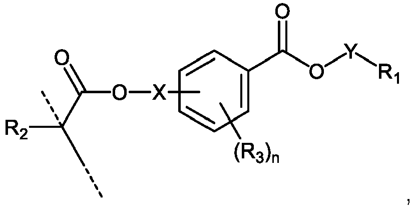 umfasst, worin:
R₁ für C₃-C₁₀-Cycloalkyl, 3- bis 10-gliedriges Heterocycloalkyl oder C₃-C₁₀-Aryl, steht, wobei das C₃-C₁₀-Cycloalkyl, das 3- bis 10-gliedrige Heterocycloalkyl und das C₃-C₁₀-Aryl unsubstituiert oder mit einem oder mehreren Substituenten, ausgewählt aus der aus C₁-C₆-Alkyl, C₁-C₆-Alkoxy, C₃-C₇-Cycloalkyl, C₆-C₁₀-Aryl, C₁-C₆-Alkoxy-C₆-C₁₀-aryl, -O(CO)-(C₁-C₆)-Alkyl, -COO-(C₁-C₆)-Alkyl, =O, -F, -Cl und -Br bestehenden Gruppe substituiert sind;
R₂ für H oder C₁-C₆-Alkyl steht;
die R₃ jeweils unabhängig für Halogen, C₁-C₃-Alkyl, C₁-C₃-Alkoxy, Si(R₄)₃, P(O)(OR₅)₂ oder N(R₆)₂ stehen;
die R₄ jeweils unabhängig für C₁-C₆-Alkyl oder C₁-C₆-Alkoxy stehen;
die R₅ jeweils unabhängig für C₁-C₆-Alkyl oder C₆-C₁₀-Aryl stehen;
die R₆ jeweils unabhängig für H oder C₁-C₆-Alkyl stehen;
X fehlt, C₁-C₃-Alkylen, 1- bis 3-gliedriges Heteroalkylen oder (CH₂CH₂O)ₘ ist;
Y fehlt, C₁-C₆-Alkylen, 1- bis 3-gliedriges Heteroalkylen oder (CH₂CH₂O)ₘ ist;
die m jeweils eine ganze Zahl von 1 bis 4 sind; und
n eine ganze Zahl von 0 bis 4 ist;
wobei jede gestrichelte Linie für eine Bindung an ein Kohlenstoffatom steht.

In manchen Ausführungsformen ist die kieferorthopädische Vorrichtung ein Aligner, ein Expander oder ein Spacer. Die kieferorthopädische Vorrichtung kann eine Vielzahl von zahnaufnehmenden Ausnehmungen umfassen, die ausgebildet sind, um Zähne aus einer ersten Anordnung in eine zweite Anordnung zu repositionieren. In manchen Ausführungsformen ist die kieferorthopädische Vorrichtung eine aus einer Vielzahl von kieferorthopädischen Vorrichtungen, die ausgebildet sind, um die Zähne aus einer Ausgangsanordnung in eine Zielanordnung, gegebenenfalls gemäß einem Behandlungsplan, zu repositionieren.

### KURZBESCHREIBUNG DER ZEICHNUNGEN

Die neuen Merkmale der Erfindung sind insbesondere in den nachstehenden Patentansprüchen dargelegt. Ein besseres Verständnis der Merkmale und Vorzüge der vorliegenden Offenbarung wird durch Bezugnahme auf die nachfolgende detaillierte Beschreibung, die veranschaulichende Ausführungsformen darlegt, in denen die Grundlagen der Erfindung in der Praxis eingesetzt werden, und die beiliegenden Zeichnungen erlangt, in denen:
Fig. 1A eine Zahnrepositionierungsvorrichtung in Übereinstimmung mit Ausführungsformen veranschaulicht.
Fig. 1B eine Zahnrepositionierungsvorrichtung in Übereinstimmung mit Ausführungsvorrichtungen veranschaulicht.
Fig. 1C ein Verfahren zur kieferorthopädischen Behandlung unter Verwendung verschiedener Vorrichtungen in Übereinstimmung mit Ausführungsformen veranschaulicht.
Fig. 2 ein Verfahren für die Konzeption einer kieferorthopädischen Vorrichtung in Übereinstimmung mit Ausführungsformen veranschaulicht.
Fig. 3 ein Verfahren für die digitale Planung einer kieferorthopädischen Behandlung in Übereinstimmung mit Ausführungsformen veranschaulicht.

### DETAILLIERTE BESCHREIBUNG DER ERFINDUNG

Alle Begriffe, chemischen Bezeichnungen, Ausdrücke und Benennungen behalten ihre übliche Bedeutung, die Fachleuten auf dem Gebiet der Erfindung wohlbekannt ist. Wie hierin verwendet, sind die Begriffe "umfassen" und "umfassend" als nicht einschränkend zu verstehen, d.h. andere Komponenten als die ausdrücklich angeführten können umfasst sein. Zahlenbereiche sind als inklusiv zu verstehen, d.h. die Ober- und Untergrenze einschließend.

Wie hierin verwendet, bezeichnet der Begriff "Polymer" ein Molekül, das aus sich wiederholenden und durch kovalente chemische Bindungen verbundenen Grundeinheiten besteht und das durch eine erhebliche Anzahl an Grundeinheiten (z.B. gleich oder größer als 10 Grundeinheiten und häufig gleich oder größer als 50 Grundeinheiten und häufig gleich oder größer als 100 Grundeinheiten) und ein hohes Molekulargewicht (z.B. größer als 5.000 Da, 10.000 Da oder 20.000 Da) gekennzeichnet ist. Polymere sind für gewöhnlich das Polymerisationsprodukt eines oder mehrerer Monomervorläufer. Der Begriff Polymer umfasst Homopolymere oder Polymere, die im Wesentlichen aus einer sich wiederholenden Monomeruntereinheit bestehen. Der Begriff Polymer umfasst auch Copolymere, die gebildet werden, wenn zwei oder mehr unterschiedliche Arten von Monomeren im selben Polymer verbunden sind. Copolymere können zwei oder mehr Monomeruntereinheiten umfassen und umfassen statistische Copolymere, Blockcopolymere, alternierende Copolymere, Segmentcopolymere, Pfropfcopolymere, Gradienten-Copolymere und andere Copolymere. "Vernetzte Polymere" bezeichnet Polymere mit einer oder mehreren Verbindungen zwischen zumindest zwei Polymerketten, die vorzugsweise dadurch entstehen, dass mehrwertige Monomere bei der Polymerisation Vernetzungsstellen bilden.

Hierin bezeichnet ein "Oligomer" ein Molekül, das aus sich wiederholenden und durch kovalente chemische Bindungen verbundenen Grundeinheiten besteht und durch eine geringere Anzahl von Grundeinheiten als die eines Polymers (z.B. gleich oder weniger als 10 Grundeinheiten) und einem geringerem Molekulargewicht als das eines Polymers (z.B. weniger als 5.000 Da oder 2.000 Da) gekennzeichnet ist. Oligomere können das Polymerisationsprodukt eines oder mehrerer Monomervorläufer sein. In einer Ausführungsform können ein Oligomer oder ein Monomer nicht als eigenständiges Polymer betrachtet werden.

Ein "Präpolymer" bezeichnet ein Polymer oder Oligomer, dessen Moleküle in der Lage sind durch reaktive Gruppen weitere Polymerisation zu erfahren.

Ein "Reaktivverdünner" bezeichnet, wie hierin verwendet, auf eine Substanz, die die Viskosität einer anderen Substanz, wie z.B. eines Monomers oder eines härtbaren Harzes, reduziert. Ein Reaktivverdünner kann Teil einer anderen Substanz werden, wie z.B. eines durch ein Polymerisationsverfahren erhaltenes Polymer. In manchen Beispielen ist ein Reaktivverdünner ein härtbares Monomer, das bei Vermischen mit einem härtbaren Harz die Viskosität der resultierenden Formulierung reduziert und in das Polymer, das das Ergebnis der Polymerisation der Formulierung ist, eingebaut wird.

Oligomer- und Polymergemische können durch Messungen des Molekulargewichts und der Molekulargewichtsverteilung charakterisiert und von anderen Oligomer- und Polymergemischen unterschieden werden.

Das mittlere Molekulargewicht (M) ist die mittlere Anzahl der Grundeinheiten n multipliziert mit dem Molekulargewicht oder der Molmasse (Mi) der Grundeinheit. Das zahlenmittlere Molekulargewicht (Mn) ist das arithmetische Mittel, das das Gesamtgewicht der vorliegenden Moleküle, dividiert durch die Gesamtanzahl an Molekülen darstellt.

Photoinitiatoren, die gemäß der Offenbarung zweckmäßig sind, umfassen jene, die mit Licht aktiviert werden können und die Polymerisation der polymerisierbaren Komponenten initiieren können. Wie hierin verwendet, kann sich die Bezeichnung "Photoinitiator" auf eine Verbindung beziehen, die Radikale erzeugen und/oder Radikalreaktionen bei Aussetzen gegenüber Strahlung (z.B. UV- oder sichtbarem Licht) unterstützen kann.

Photopolymerisation tritt auf, wenn geeignete Formulierungen Strahlung (z.B. UV- oder sichtbarem Licht) ausgesetzt werden, die eine ausreichende Energie und eine Wellenlänge aufweist, die zum Initiieren von Polymerisation in der Lage ist. Die Wellenlänge und die Energie von Strahlung, die zum Initiieren der Polymerisation geeignet ist, hängen von dem eingesetzten Photoinitiator ab. "Licht", wie hierin verwendet, umfasst eine beliebige Wellenlänge und Energie, die zum Initiieren von Polymerisation in der Lage ist. Manche Wellenlängen von Licht umfassen ultraviolettes (UV) oder sichtbares Licht. UV-Lichtquellen umfassen UVA (Wellenlänge von etwa 400 nm bis etwa 320 nm), UVB (etwa 320 nm bis etwa 290 nm) oder UVC (etwa 290 nm bis etwa 100 nm). Jede geeignete Quelle kann verwendet werden, einschließlich Laserquellen. Die Quelle kann breitbandig oder schmalbandig oder eine Kombination davon sein. Die Lichtquelle kann während des Verfahrens durchgehendes oder gepulstes Licht bereitstellen. Sowohl die Zeitdauer, die das System dem UV-Licht ausgesetzt ist, als auch die Intensität des UV-Lichts können variiert werden, um die idealen Reaktionsbedingungen zu bestimmen.

Additive Fertigung umfasst verschiedene Technologien, mit denen durch ein additives Verfahren dreidimensionale Gegenstände direkt von digitalen Modellen gefertigt werden. In manchen Aspekten werden sukzessive Materialschichten abgelagert und "örtlich ausgehärtet". Verschiedene Verfahren sind auf dem Gebiet der additiven Fertigung bekannt, einschließlich des selektiven Lasersinterns (SLS), Fused Deposition Modeling (FDM) sowie Aufspritzen oder Extrusion. In vielen Ausführungsformen umfasst das selektive Lasersintern die Verwendung eines Laserstrahls, um eine Schicht aus pulverförmigem Material in Übereinstimmung mit einer gewünschten Querschnittsform selektiv zu schmelzen und zu verschmelzen, um die Geometrie des Gegenstands aufzubauen. In zahlreichen Ausführungsformen umfasst Fused Deposition Modeling das Schmelzen und das schichtweise selektive Ablagern eines dünnen Filaments eines thermoplastischen Polymers, um einen Gegenstand zu formen. In einem weiteren Beispiel kann 3D-Druck verwendet werden, um die hierin beschriebenen Vorrichtungen zu erzeugen. In zahlreichen Ausführungsformen umfasst 3D-Druck das Aufspritzen oder Extrudieren von einem oder mehreren Materialien auf eine Aufbauplattform, um sukzessive Schichten der Geometrie des Gegenstands zu formen. In manchen Ausführungsformen können die hierin beschriebenen polymerisierbaren Monomere in Tintenstrahl- oder Beschichtungsanwendungen verwendet werden.

Photopolymere können durch "Wannen"-Verfahren erzeugt werden, bei denen Licht eingesetzt wird, um eine Wanne oder einen Behälter voller Photopolymer selektiv zu härten. Jede Schicht des Photopolymers kann selektiv belichtet werden - durch Einzelbelichtung oder indem ein Lichtstrahl über die Schicht geführt wird. Konkrete Verfahren umfassen Stereolithographie (SLA), Digital Light Processing (DLP) und durch zwei Photonen induzierte Photopolymerisation (TPIP).

Es wurde auch von kontinuierlichen Direktfertigungsverfahren für Photopolymere berichtet. Ein Direktfertigungsverfahren kann beispielsweise den kontinuierlichen Aufbau der Geometrie eines Gegenstands durch kontinuierliche Bewegung der Aufbauplattform (z.B. in vertikaler oder Z-Richtung) während der Bestrahlungsphase erreichen, so dass die Härtungstiefe des bestrahlten Photopolymers durch die Bewegungsgeschwindigkeit gesteuert wird. Dementsprechend kann eine kontinuierliche Polymerisation des Materials auf der Aufbauoberfläche erzielt werden. Solche Verfahren sind in US-Patent Nr. 7.892.474 beschrieben, dessen Offenbarung durch Verweis vollständig hierin aufgenommen ist. In einem weiteren Beispiel wird in einem kontinuierlichen direkten Fertigungsverfahren ein "Heliolithographie"-Ansatz angewandt, bei dem das flüssige Photopolymer mittels fokussierter Strahlung gehärtet wird, während die Aufbauplattform kontinuierlich rotiert und angehoben wird. Dementsprechend kann die Geometrie des Gegenstands entlang eines spiralförmigen Aufbauwegs kontinuierlich aufgebaut werden. Solche Verfahren sind in US-Patentveröffentlichung Nr. 2014/ 0265034 beschrieben, deren Offenbarung durch Verweis vollständig hierin aufgenommen ist. Es wurde auch von kontinuierlicher flüssiger Grenzflächenproduktion von 3D-Gegenständen berichtet (J. Tumbleston et al., Science, 347 (6228), S. 1349-1352 (2015)), das zur Beschreibung des Verfahrens vollständig durch Verweis hierin aufgenommen ist. Ein weiteres Beispiel für ein kontinuierliches Direktfertigungsverfahren kann das Extrudieren eines Verbundmaterials umfassen, das aus einem härtbaren flüssigen Material besteht, das einen festen Strang umgibt. Das Verbundmaterial kann entlang eines kontinuierlichen dreidimensionalen Pfads zur Ausbildung des Gegenstands extrudiert werden. Solche Verfahren sind in US-Patentveröffentlichung Nr. 2014/0061974 offenbart, deren Offenbarung durch Verweis hierin vollständig aufgenommen ist.

"Hochtemperaturlithographie" bezieht sich, wie hierin verwendet, auf ein beliebiges Photopolymerisationsverfahren auf Lithographiebasis, das das Erhitzen von photopolymerisierbarem Material/photopolymerisierbaren Materialien umfasst. Durch das Erhitzen kann die Viskosität des photopolymerisierbaren Materials/der photopolymerisierbaren Materialien vor und/oder während des Härtens gesenkt werden. Nicht einschränkende Beispiele für Hochtemperaturlithographieverfahren umfassen jene Verfahren, die in WO 2015/075094, WO 2016/078838 und WO 2018/032022 beschrieben sind. In manchen Ausführungen kann Hochtemperaturlithographie das Anwenden von Wärme auf ein Material zum Erreichen von Temperaturen zwischen 50 °C und 120 °C, wie z.B. 90 °C bis 120 °C, 100 °C bis 120 °C, 105 °C bis 115 °C, 108 °C bis 110 °C usw., umfassen. Das Material kann auf Temperaturen von mehr als 120 °C erhitzt werden. Es ist anzumerken, dass auch andere Temperaturbereiche angewandt werden können, ohne von Schutzumfang und Substanz des hierin beschriebenen erfinderischen Konzepts abzuweichen.

"Biokompatibel" bezieht sich auf ein Material, das keine immunologische Abstoßung oder schädliche Wirkung hervorruft, die hierin als ungünstige Immunantwort bezeichnet wird, wenn es in ein biologisches In-vivo-Umfeld gebracht wird. Beispielsweise verändert sich in Ausführungsformen ein biologischer Marker, der eine Immunantwort anzeigt, um weniger als 10 % oder weniger als 20 % oder weniger als 25 % oder weniger als 40 % oder weniger als 50 % in Bezug auf einen Grundlinienwert, wenn ein Mensch oder ein Tier dem biokompatiblen Material ausgesetzt wird oder mit diesem in Kontakt gebracht wird. Alternativ dazu kann die Immunantwort histologisch bestimmt werden, wobei eine lokale Immunantwort durch Sichtprüfung von Markern, einschließlich Immunzellen, oder Markern, die an dem Immunantwortsignalweg beteiligt sind, in dem und angrenzend an das Material bewertet wird. In einem Aspekt ruft ein biokompatibles Material oder eine biokompatible Vorrichtung keine erkennbare Immunantwort hervor, wie histologisch festgestellt wird. In manchen Ausführungsformen stellt die Offenbarung biokompatible Vorrichtungen bereit, die zur Langzeitverwendung, wie z.B. in der Größenordnung von Wochen bis Monaten, ausgebildet sind, ohne dass sie negative Immunantworten hervorrufen. Eine biologische Wirkung kann anfänglich durch Messung von Zytotoxizität, Sensibilisierung, Reizung und intrakutaner Reaktivität, akuter systemische Toxizität, Pyrogenität, subakuter/subchronischer Toxizität und/oder Implantation bewertet werden. Biologische Test für eine ergänzende Bewertung umfassen Tests in Bezug auf chronische Toxizität.

"Bioinert" bezieht sich auf ein Material, das keine Immunantwort bei Mensch oder Tier hervorruft, wenn es in einem biologischen In-vivo-Umfeld vorliegt. Beispielsweise bleibt ein biologischer Marker, der eine Immunantwort anzeigt, im Wesentlichen konstant (plus oder minus 5 % in Bezug auf den Grundlinienwert), wenn ein Mensch oder Tier dem bioinerten Material ausgesetzt oder mit diesem in Kontakt gebracht wird. In manchen Ausführungsformen stellt die Offenbarung bioinerte Vorrichtungen bereit.

Wie bereits erwähnt stellt die vorliegende Offenbarung in einem Aspekt eine Gruppe polymerisierbarer Monomere bereit, die jeweils unabhängig einen 2-, 3- oder 4-(Meth)acryloylbenzoesäureester der allgemeinen Formel (I) umfassen: 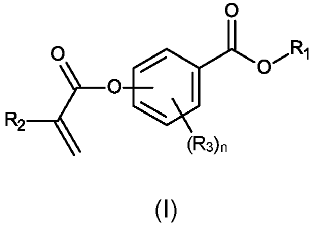
worin R₁ für einen cycloaliphatischen C₅-C₂₀-Hydrocarbylrest steht, der aus der aus gegebenenfalls substituierten C₅-C₇-Cycloalkylresten bestehenden Gruppe ausgewählt ist;
R₂ für H oder CH₃ steht;
die R₃ jeweils unabhängig für C₁-C₃-Alkyl oder C₁-C₃-Alkoxy stehen; und
n eine ganze Zahl von 0 bis 4 ist.

Etwaige optionale Substituenten an C₅-C₇-Cycloalkylresten von R₁ sollten vorzugsweise keine Funktionalitäten umfassen, die unerwünschte Nebenreaktionen mit anderen Komponenten der härtbaren Harzformulierungen, die die polymerisierbaren Monomere der vorliegenden Offenbarung umfassen, auslösen könnten. Außerdem sollten sie vorzugweise den Schmelzpunkt nicht übermäßig erhöhen oder die viskositätsreduzierende Wirkung der neuen Monomere beeinträchtigen. Aus diesem Grund sind die optionalen Substituenten der C₅-C₇-Cycloalkylreste von R₁ in manchen Ausführungsformen aus der aus C₁-C₆-Alkyl, C₁-C₆-Alkoxy, -O(CO)-(C₁-C₆)-Alkyl, -COO-(C₁-C₆)-Alkyl, =O, -F, -Cl, -Br und Gemischen davon bestehenden Gruppe ausgewählt, und noch bevorzugter handelt es sich um C₁-C₆ Alkylgruppen, insbesondere um C₁-C₃-Alkylgruppen, wobei zwei von diesen Gruppen verbunden sein können, um mit den Kohlenstoffatomen, an die sie gebunden sind, und gegebenenfalls mit einem oder mehreren zusätzlichen, dazwischenliegenden Kohlenstoffatomen des Cyclohexylrings einen Ring zu bilden. In weiteren Ausführungsformen ist R₁ ein gegebenenfalls substituierter C₅-C₇-Cycloalkylrest mit insgesamt 5 bis 15, noch bevorzugter mit insgesamt 5 bis 12 und insbesondere mit insgesamt 5 bis 10, Kohlenstoffatomen.

In besonders bevorzugten Ausführungsformen ist R₁ aus der aus folgenden Resten bestehenden Gruppe ausgewählt: 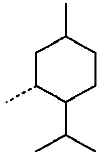
2-Isopropyl-5-methylcyclohexyl (Menthyl); 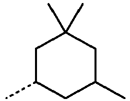
3,3,5-Trimethylcyclohexyl (Homomenthyl); 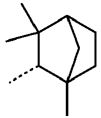
1,3,3-Trimethyl-2-norbornanyl, 1,3,3-Trimethyl-2-bicyclo[2.2.1]heptanyl (Fenchyl); und 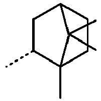
1,7,7-Trimethyl-2-bicyclo[2.2.1]heptanyl ((Iso)bornyl);
wobei die gestrichelten Linien in den oben angeführten Formeln jeweils die Bindung an das Estersauerstoffatom anzeigen.

Die entsprechenden Ester der Formel (I) lieferten bereits gute Ergebnisse, und die entsprechenden Alkohole, die zur Herstellung dieser Ester eingesetzt werden, sind im Handel erhältlich, wodurch potenziell komplexe und teure Herstellungsverfahren vermieden werden können. Bei diesen Alkoholen handelt es sich um folgende: Menthol 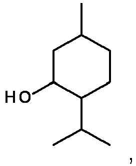 Homomenthol 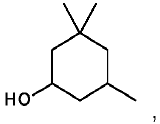 Fenchol 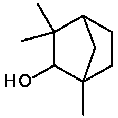 und (Iso)borneol 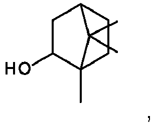 d.h. entweder (-)-Borneol 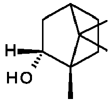 oder (+)-Isoborneol 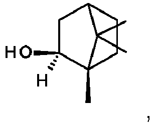 abhängig von der Molekülkonfiguration am asymmetrischen Kohlenstoffatom an Position 2 der Bicyclo[2.2.1]heptan-Grundstruktur.

In weiteren Ausführungsformen sind jedoch weitere Cycloalkylreste R₁, die durch die vorliegende Offenbarung in Betracht gezogen werden, beispielsweise jene, die von folgenden Alkoholen stammen:
unsubstituiertem Cyclopentanol 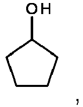 Cyclohexanol 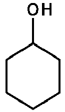 oder Cycloheptanol 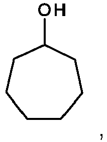 wobei diese Cycloalkanole mit einer oder mehreren C₁-C₆-Alkylgruppen, wie z.B. Methyl, Ethyl, Propyl, Isopropyl, n-Butyl, tert-Butyl, Amyl, Isoamyl, Hexyl, Isohexyl und beliebigen Kombinationen davon, an einem oder mehreren beliebigen Kohlenstoffatomen substituiert sind, einschließlich an Position C1, d.h. als Zwillingssubstituent für die funktionellen Alkoholgruppen, wie z.B. an einem beliebigen geeigneten Kohlenstoffatom mit einer oder mehreren weiteren Cycloalkylgruppen substituierte Cycloalkanole, wie z.B. Cyclopentylcyclohexanol oder Cyclohexylcyclohexanol, z.B. 4-Cyclopentylcyclohexanol 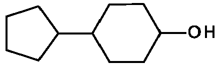 oder 4-Cyclohexylcyclohexanol 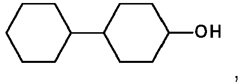 kondensierte Ringstrukturen, wie z.B. Decahydro-2-naphthol 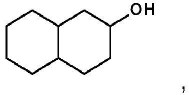 oder polyzyklische Gruppen wie 1- oder 2-Adamantanol 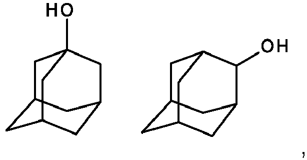 sowie bi- oder polyzyklische Strukturen, die an einem beliebigen substituierbaren Kohlenstoffatom mit einer oder mehreren C₁-C₆-Alkylgruppen substituiert sind, wie oben angeführt, usw.

Der Substituent des Benzolrings, R₃, ist, sofern vorhanden, vorzugsweise aus der aus Methyl, Methoxy, Ethyl und Ethoxy bestehenden Gruppe ausgewählt, und n ist vorzugsweise aus 0 oder 1 ausgewählt. Besonders bevorzugt ist n jedoch 0, d.h. der Benzolring ist unsubstituiert, wodurch wiederum vermieden wird, dass die entsprechenden Säuren als Ausgangsmaterialien für die Synthese der Ester der Formel (I) durch Um- oder Veresterung spezifisch synthetisiert werden müssen oder dass diese gekauft werden müssen, wenn sie überhaupt erhältlich sind, wobei sie typischerweise teurer sind als die unsubstituierten Säuren.

In manchen Aspekten stellt die vorliegende Offenbarung ein polymerisierbares Monomer bereit, das ein 2-, 3- oder 4-(Meth)acryloylbenzoesäureester der Formel (I) ist: 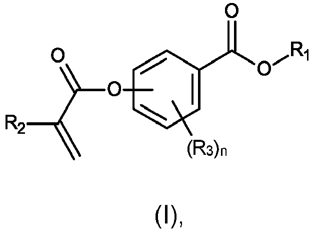 worin:
R₁ für C₃-C₁₀-Cycloalkyl steht, wobei das C₃-C₁₀-Cycloalkyl unsubstituiert ist; mit einem oder mehreren Substituenten, ausgewählt aus der aus -CH₃, C₁-C₆-Alkoxy, -O(CO)-(C₁-C₆)-Alkyl, -COO-(C₁-C₆)-Alkyl, =O, -F, -Cl und -Br bestehenden Gruppe substituiert ist; mit einem oder mehreren -CH₃ substituiert ist und gegebenenfalls außerdem mit einem oder mehreren Substituenten, ausgewählt aus der C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert ist; oder mit zwei oder mehr Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert ist;
R₂ für H oder C₁-C₆-Alkyl steht;
die R₃ jeweils unabhängig für Halogen, C₁-C₃-Alkyl, C₁-C₃-Alkoxy, Si(R₄)₃, P(O)(OR₅)₂ oder N(R₆)₂ stehen;
die R₄ jeweils unabhängig für C₁-C₆-Alkyl oder C₁-C₆-Alkoxy stehen;
die R₅ jeweils unabhängig für C₁-C₆-Alkyl oder C₆-C₁₀-Aryl stehen;
die R₆ jeweils unabhängig für H oder C₁-C₆-Alkyl stehen; und
n eine ganze Zahl von 0 bis 4 ist.

In manchen Aspekten stellt die vorliegende Offenbarung ein polymerisierbares Monomer bereit, das ein 2-, 3- oder 4-substituierter Benzoesäureester der Formel (II) ist: 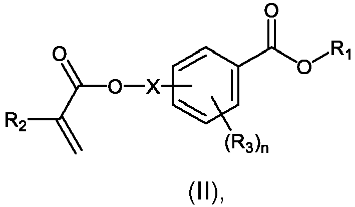 worin:
R₁ für C₃-C₇-Cycloalkyl oder 3- bis 7-gliedriges Heterocycloalkyl steht, wobei das C₃-C₇-Cycloalkyl und das 3- bis 7-gliedrige Heterocycloalkyl unsubstituiert sind; mit einem oder mehreren Substituenten, ausgewählt aus der aus -CH₃, C₁-C₆-Alkoxy, -O(CO)-(C₁-C₆)-Alkyl, -COO-(C₁-C₆)-Alkyl, =O, -F, -Cl und -Br bestehenden Gruppe substituiert ist; mit einem oder mehreren -CH₃ substituiert ist und gegebenenfalls außerdem mit einem oder mehreren Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert ist; oder mit zwei oder mehr Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert ist;
R₂ für H oder C₁-C₆-Alkyl steht;
die R₃ jeweils unabhängig für Halogen, C₁-C₃-Alkyl, C₁-C₃-Alkoxy, Si(R₄)₃, P(O)(OR₅)₂ oder N(R₆)₂ stehen;
die R₄ jeweils unabhängig für C₁-C₆-Alkyl oder C₁-C₆-Alkoxy stehen;
die R₅ jeweils unabhängig für C₁-C₆-Alkyl oder C₆-C₁₀-Aryl stehen;
die R₆ jeweils unabhängig für H oder C₁-C₆-Alkyl stehen;
X C₁-C₃-Alkylen, 1- bis 3-gliedriges Heteroalkylen oder (CH₂CH₂O)ₘ ist;
m eine ganze Zahl von 1 bis 4 ist; und
n eine ganze Zahl von 0 bis 4 ist.

In manchen Aspekten stellt die vorliegende Offenbarung ein polymerisierbares Monomer bereit, das ein 2-, 3- oder 4-substituierter Benzoesäureester der Formel (II) ist: 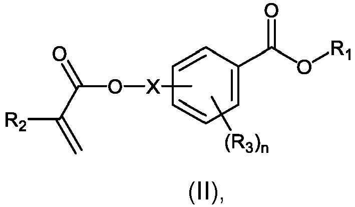 worin:
R₁ für C₃-C₈-Cycloalkyl oder 3- bis 7-gliedriges Heterocycloalkyl steht, wobei das C₃-C₈-Cycloalkyl und das 3- bis 7-gliedrige Heterocycloalkyl unsubstituiert sind; mit einem oder mehreren Substituenten, ausgewählt aus der aus -CH₃, C₁-C₆-Alkoxy, -O(CO)-(C₁-C₆)-Alkyl, -COO-(C₁-C₆)-Alkyl, =O, -F, -Cl und -Br bestehenden Gruppe substituiert ist; mit einem oder mehreren -CH₃ substituiert ist und gegebenenfalls außerdem mit einem oder mehreren Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert ist; oder mit zwei oder mehr Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert ist;
R₂ für H oder C₁-C₆-Alkyl steht;
die R₃ jeweils unabhängig für Halogen, C₁-C₃-Alkyl, C₁-C₃-Alkoxy, Si(R₄)₃, P(O)(OR₅)₂ oder N(R₆)₂ stehen;
die R₄ jeweils unabhängig für C₁-C₆-Alkyl oder C₁-C₆-Alkoxy stehen;
die R₅ jeweils unabhängig für C₁-C₆-Alkyl oder C₆-C₁₀-Aryl stehen;
die R₆ jeweils unabhängig für H oder C₁-C₆-Alkyl stehen;
X C₁-C₃-Alkylen, 1- bis 3-gliedriges Heteroalkylen oder (CH₂CH₂O)ₘ ist;
m eine ganze Zahl von 1 bis 4 ist; und
n eine ganze Zahl von 0 bis 4 ist.

In manchen Aspekten stellt die vorliegende Offenbarung ein polymerisierbares Monomer bereit, das ein 2-, 3- oder 4-substituierter Benzoesäureester der Formel (III) ist: 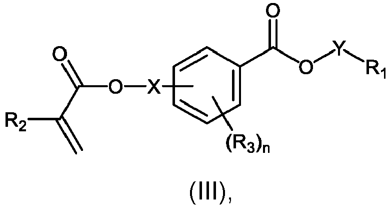 worin:
R₁ für C₃-C₇-Cycloalkyl oder C₆-C₁₀-Aryl steht, wobei das C₃-C₇-Cycloalkyl und C₆-C₁₀-Aryl unsubstituiert sind oder mit einem oder mehreren Substituenten, ausgewählt aus der aus C₁-C₆-Alkyl, C₁-C₆-Alkoxy, C₃-C₇-Cycloalkyl, C₆-C₁₀-Aryl, C₁-C₆-Alkoxy-C₆-C₁₀-aryl, -O(CO)-(C₁-C₆)-Alkyl, -COO-(C₁-C₆)-Alkyl, =O, -F, -Cl und -Br bestehenden Gruppe substituiert sind, mit der Maßgabe, dass der Benzoesäureester der Formel (III) 2- oder 3-substituiert ist, wenn R₁ unsubstituiert ist;
R₂ für H oder C₁-C₆-Alkyl steht;
die R₃ jeweils unabhängig für Halogen, C₁-C₃-Alkyl, C₁-C₃-Alkoxy, Si(R₄)₃, P(O)(OR₅)₂ oder N(R₆)₂ stehen;
die R₄ jeweils unabhängig für C₁-C₆-Alkyl oder C₁-C₆-Alkoxy stehen;
die R₅ jeweils unabhängig für C₁-C₆-Alkyl oder C₆-C₁₀-Aryl stehen;
die R₆ jeweils unabhängig für H oder C₁-C₆-Alkyl stehen;
X fehlt, C₁-C₃-Alkylen, 1- bis 3-gliedriges Heteroalkylen oder (CH₂CH₂O)ₘ ist;
Y C₁-C₆-Alkylen ist;
m eine ganze Zahl von 1 bis 4 ist; und
n eine ganze Zahl von 0 bis 4 ist.

In manchen Aspekten stellt die vorliegende Offenbarung ein polymerisierbares Monomer bereit, das ein 2-, 3- oder 4-substituierter Benzoesäureester der Formel (III) ist: 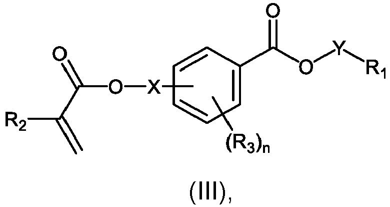 worin:
R₁ für C₃-C₈-Cycloalkyl oder C₆-C₁₀-Aryl steht, wobei das C₃-C₈-Cycloalkyl und C₆-C₁₀-Aryl unsubstituiert sind oder mit einem oder mehreren Substituenten, ausgewählt aus der aus C₁-C₆-Alkyl, C₁-C₆-Alkoxy, C₃-C₇-Cycloalkyl, C₆-C₁₀-Aryl, C₁-C₆-Alkoxy-C₆-C₁₀-aryl, -O(CO)-(C₁-C₆)-Alkyl, -COO-(C₁-C₆)-Alkyl, =O, -F, -Cl und -Br bestehenden Gruppe substituiert sind, mit der Maßgabe, dass der Benzoesäureester der Formel (III) 2- oder 3-substituiert ist, wenn R₁ unsubstituiert ist;
R₂ für H oder C₁-C₆-Alkyl steht;
die R₃ jeweils unabhängig für Halogen, C₁-C₃-Alkyl, C₁-C₃-Alkoxy, Si(R₄)₃, P(O)(OR₅)₂ oder N(R₆)₂ stehen;
die R₄ jeweils unabhängig für C₁-C₆-Alkyl oder C₁-C₆-Alkoxy stehen;
die R₅ jeweils unabhängig für C₁-C₆-Alkyl oder C₆-C₁₀-Aryl stehen;
die R₆ jeweils unabhängig für H oder C₁-C₆-Alkyl stehen;
X fehlt, C₁-C₃-Alkylen, 1- bis 3-gliedriges Heteroalkylen oder (CH₂CH₂O)ₘ ist;
Y C₁-C₆-Alkylen ist;
m eine ganze Zahl von 1 bis 4 ist; und
n eine ganze Zahl von 0 bis 4 ist.

In manchen Ausführungsformen ist R₁ für eine Verbindung der Formel (I), (II) oder (III), gegebenenfalls substituiertes C₅-C₇-Cycloalkyl. Das gegebenenfalls substituierte C₅-C₇-Cycloalkyl kann insgesamt 5 bis 15 Kohlenstoffatome aufweisen, wie z.B. 5 bis 12 oder 5 bis 10 Kohlenstoffatome. In manchen Ausführungsformen ist R₁ für eine Verbindung der Formel (I), (II) oder (III) gegebenenfalls substituiertes C₅-C₈-Cycloalkyl. Das gegebenenfalls substituierte C₅-C₈-Cycloalkyl kann insgesamt 5 bis 15 Kohlenstoffatomen aufweisen, wie z.B. 5 bis 12 oder 5 bis 10 Kohlenstoffatome. Manchen Ausführungsformen ist R₁ für eine Verbindung der Formel (II) aus gegebenenfalls substituiertem C₅-C₇-Cycloalkyl und gegebenenfalls substituiertem 5- bis 7-gliedrigem Heterocycloalkyl ausgewählt. In manchen Ausführungsformen ist R₁ für eine Verbindung der Formel (III) aus gegebenenfalls substituiertem C₅-C₇-Cycloalkyl und gegebenenfalls substituiertem C₆-C₁₀-Aryl ausgewählt, wie z.B. aus gegebenenfalls substituiertem Phenyl. Für eine Verbindung der Formel (I), (II) oder (III) kann R₁ ein monozyklisches Cycloalkyl sein, wie z.B. Cyclohexyl. In manchen Ausführungsformen ist R₁ ein bizyklisches Cycloalkyl, wie z.B. ein verbrücktes, kondensiertes oder spirozyklisches Cycloalkyl. Dieses umfasst beispielsweise Bicyclo[2.2.1]heptyl, Bicyclo[1.1.1]pentyl, Spiro[4.4]nonyl und Decahydronaphthyl, die jeweils gegebenenfalls substituiert sein können. In manchen Ausführungsformen ist R₁ unsubstituiert. Alternativ dazu ist R₁ mit zumindest einem Substituenten substituiert.

Zu Beispielen für optionale Substituenten von R₁ zählen C₁-C₆-Alkyl, C₁-C₆-Alkoxy, C₃-C₇-Cycloalkyl, C₆-C₁₀-Aryl, C₁-C₆-Alkoxy-C₆-C₁₀-aryl, -O(CO)-(C₁-C₆)-Alkyl, -COO-(C₁-C₆)-Alkyl, =O, -F, -Cl und -Br. In manchen Ausführungsformen ist R₁ mit einem oder mehreren Substituenten, ausgewählt aus der aus -CH₃, C₁-C₆-Alkoxy, -O(CO)-(C₁-C₆)-Alkyl, -COO-(C₁-C₆)-Alkyl, =O, -F, -Cl und -Br bestehenden Gruppe substituiert. R₁ kann mit zumindest einem -CH₃ substituiert sein. Beispielsweise ist R₁ in manchen Ausführungsformen mit einem oder mehreren -CH₃ und gegebenenfalls außerdem mit einem oder mehreren Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert. In manchen Ausführungsformen ist R₁ mit zwei oder mehr Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert. In manchen Ausführungsformen ist R₁ mit einer oder mehreren, unverzweigten oder verzweigten C₁-C₆-Alkylgruppen substituiert, wie z.B. Methyl, Ethyl, n-Propyl, Isopropyl, n-Butyl, sec-Butyl, Isobutyl oder tert-Butyl. R₁ kann mit -CH₃ und gegebenenfalls außerdem mit einer oder mehreren, unverzweigten oder verzweigten C₁-C₆-Alkylgruppen substituiert sein. Zwei Substituenten von R₁, wie z.B. zwei C₁-C₆-Alkylgruppen, können zu einem Ring verbunden sein. Beispielsweise können zwei Substituenten an einer Cyclohexylgruppe eine Brücke bilden, wie z.B. eine Methylenbrücke wie in Bicyclo[2.2.1]heptyl. In manchen Ausführungsformen ist R₁ mit einem oder mehreren Substituenten, ausgewählt aus der aus C₁-C₄-Alkyl und C₁-C₄-Alkoxy bestehenden Gruppe substituiert.

In manchen Ausführungsformen ist R₁ für eine Verbindung der Formel (I), (II) oder (III) Cyclohexyl, das mit -CH₃ und gegebenenfalls außerdem mit einer oder mehreren, unverzweigten oder verzweigten C₁-C₆-Alkylgruppen substituiert ist, wobei zwei der C₁-C₆-Alkylgruppen verbunden sein können, um zusammen mit den Kohlenstoffatomen, an die sie gebunden sind, und gegebenenfalls einem oder mehreren zusätzlichen, dazwischenliegenden Atomen des Cyclohexylrings einen Ring zu bilden.

Zu den Beispielen für R₁-Gruppen zählen folgende, ohne auf diese beschränkt zu sein: 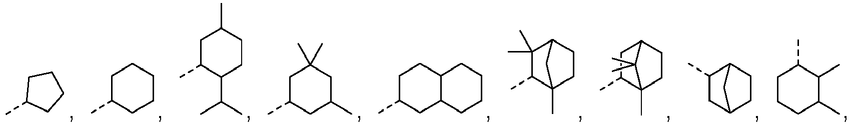 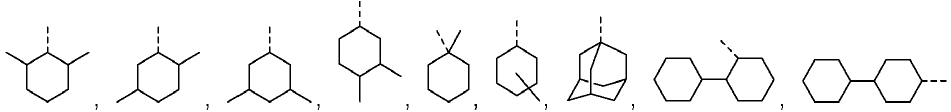 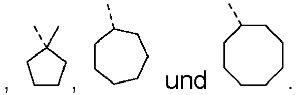 Die gestrichelte Linie wird hierin verwendet, um die Bindung an den Rest des Moleküls anzuzeigen (z.B. die Bindung an das Estersauerstoffatom aus den Formeln (I) und (II) oder die Bindung an den Linker Y aus der Formel (III)). Zu weiteren Beispielen für -Y-R₁-Gruppen einer Verbindung der Formel (III) zählen folgende, ohne auf diese eingeschränkt zu sein:

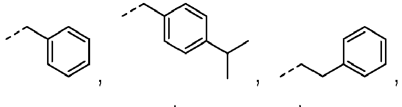

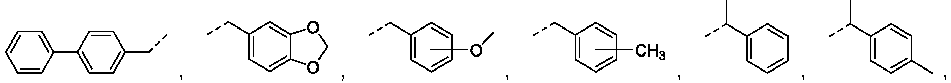

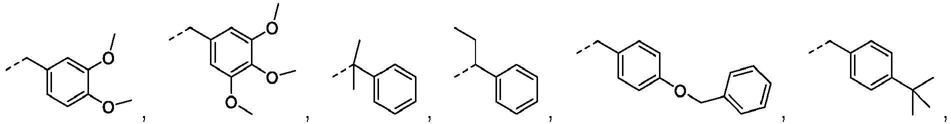

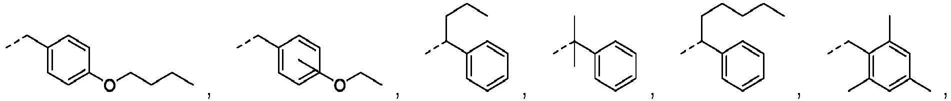

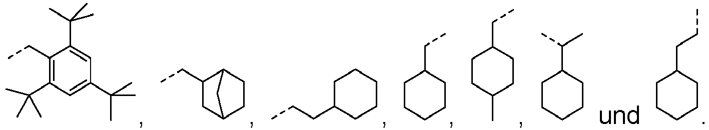

Ein polymerisierbares Monomer der Formel (I), (II) oder (III) kann 2-substituiert sein, wie in den Formeln (I-a), (II-a) und (III-a) dargestellt: 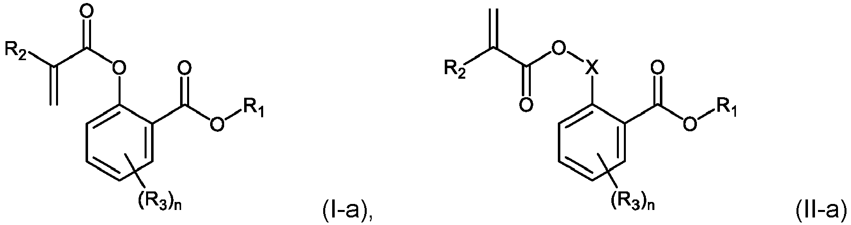 und 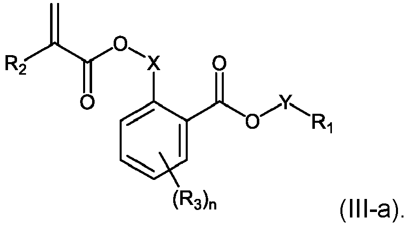 Ein polymerisierbares Monomer der Formel (I), (II) oder (III) kann 3-substituiert sein, wie in den Formeln (I-b), (II-b) und (III-b) dargestellt: 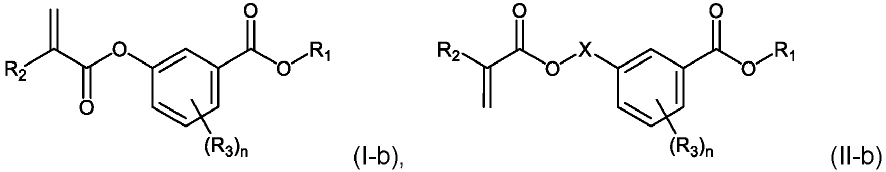 und 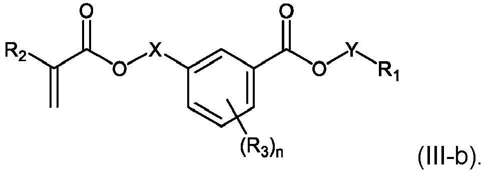 Ein polymerisierbares Monomer der Formel (I), (II) oder (III) kann 4-substituiert sein, wie in den Formeln (I-c), (II-c) und (III-c) dargestellt: 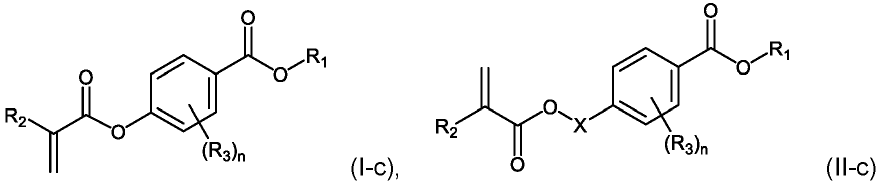 und

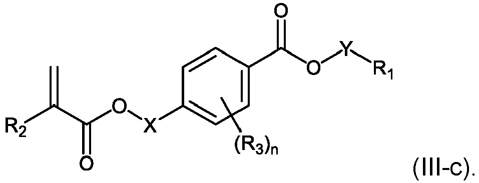

In manchen Ausführungsformen steht R₁ für eine Verbindung der Formel (I), (II) oder (III) für substituiertes C₅-C₇-Cycloalkyl; R₂ steht für H oder CH₃; die R₃ stehen jeweils unabhängig für C₁-C₃-Alkyl oder C₁-C₃-Alkoxy; und n ist eine ganze Zahl von 0 bis 4. In manchen Ausführungsformen ist R₃ aus der aus C₁-C₃-Alkyl und C₁-C₃-Alkoxy bestehenden Gruppe ausgewählt, wie z.B. aus -CH₃ oder -OCH₃. R₃ kann aus Methyl, Methoxy, Ethyl und Ethoxy ausgewählt sein.

In manchen Ausführungsformen ist n für eine Verbindung der Formel (I), (II) oder (III) 0 oder 1, wie z.B. n = 0. In manchen Ausführungsformen ist R₂ H oder CH₃. Für eine Verbindung der Formel (II) oder (III) kann X C₁-C₃-Alkylen sein, wie z.B. Methylen. In manchen Ausführungsformen fehlt X. In manchen Ausführungsformen für eine Verbindung der Formel (III) ist Y C₁-C₃-Alkylen.

Manche Eigenschaften der polymerisierbaren Monomere gemäß vorliegender Offenbarung in Zusammenhang mit ihrer möglichen Verwendung als Reaktivverdünner, umfassen das Aufweisen eines Schmelzpunkts, der niedriger ist als die in aktuellen Hochtemperaturphotopolymerisationsverfahren auf Lithographiebasis eingesetzten Verfahrenstemperaturen, die typischerweise im Bereich von 50 bis 120 °C, wie z.B. 90 bis 120 °C, liegen. Aus diesem Grund sollten polymerisierbare Monomere, die als Reaktivverdünner eingesetzt werden sollen, einen Schmelzpunkt von < 120 °C, vorzugsweise von < 90 °C, noch bevorzugter von < 70 °C und noch bevorzugter von < 50 °C oder < 30 °C, aufweisen, wodurch für niedrige Viskositäten der Schmelzen und in der Folge für eine deutlich ausgeprägtere viskositätsreduzierende Wirkung gesorgt wird, wenn sie als Reaktivverdünner für Harze eingesetzt werden, die durch Hochtemperaturpolymerisation auf Lithographiebasis gehärtet werden sollen. Besonders bevorzugt sind sie bei Raumtemperatur flüssig, was zusätzlich zu den oben angeführten Vorteilen ihre Handhabung erleichtert.

In insbesondere zu bevorzugenden Ausführungsformen der vorliegenden Offenbarung ist das polymerisierbare Monomer aus der aus folgenden Verbindungen bestehenden Gruppe ausgewählt: 2-Isopropyl-5-methylcyclohexyl-2-(methacryloyloxy)benzoat; 3,3,5-Trimethylcyclohexyl-2-(methacryloyloxy)benzoat; 1,3,3-Trimethyl-2-bicyclo[2.2.1]heptanyl-2-(methacryloyloxy)benzoat; 1,7,7-Trimethyl-2-bicyclo[2.2.1]-heptanyl-2-(methacryloyloxy)benzoat; 2-Isopropyl-5-methylcyclohexyl-3-(methacryloyloxy)benzoat; 2-Isopropyl-5-methylcyclohexyl-4-(methacryloyloxy)benzoat und 3,3,5-Trimethylcyclohexyl-2-(acryloyloxy)benzoat; wobei diese Verbindungen bereits gute oder sogar hervorragende Ergebnisse geliefert haben, weshalb diese sieben neuen Verbindungen Gegenstand eines Aspekts der Offenbarung sind. In manchen Ausführungsformen, ist das polymerisierbare Monomer aus den in Tabelle 1 angeführten Verbindungen 1 bis 21 ausgewählt.

Ein Aspekt der vorliegenden Offenbarung ist ein Verfahren zum Polymerisieren einer härtbaren Zusammensetzung, die zumindest eine Art einer polymerisierbaren Spezies und gegebenenfalls eine oder mehrere weitere Komponenten umfasst, die aus der aus Polymerisationsinitiatoren, Lösungsmitteln, Füllstoffen, Antioxidationsmitteln, Pigmenten, Farbstoffen, Oberflächenmodifikatoren und Gemischen davon bestehenden Gruppe ausgewählt sind, um ein gegebenenfalls vernetztes Polymer zu erhalten, wobei das Verfahren einen Schritt des Vermischens der härtbaren Zusammensetzung, gegebenenfalls nachdem sie erhitzt worden ist, mit einem Reaktivverdünner umfasst, bevor durch Erhitzen und/oder Bestrahlen der Zusammensetzung die Polymerisation initiiert wird; wobei der Reaktivverdünner aus den polymerisierbaren Monomeren gemäß einem Aspekt der Offenbarung und Gemischen davon ausgewählt ist.

Dieses Verfahren ist vorzugsweise Teil eines Hochtemperaturpolymerisationsverfahrens auf Lithographiebasis, wobei die härtbare Zusammensetzung zumindest einen Photopolymerisationinitator umfasst und erhitzt wird, bevor sie mit dem Reaktivverdünner vermischt wird, noch bevorzugter Teil eines additiven Fertigungsverfahrens, wie z.B. Teil eines 3D-Druckverfahrens. In manchen Ausführungsformen des Verfahrens umfasst die härtbare Zusammensetzung zumindest einen Photopolymerisationsinitiator und wird auf eine vorab festgeelgte erhöhte Verfahrenstemperatur von 50 °C bis 120 °C erhitzt, wie z. B. auf 90 °C bis 120 °C, bevor sie mit Licht mit einer geeigneten Wellenlänge belichtet wird, das von dem Photoinitator absorbiert wird und dadurch eine Spaltung des Photoinitators hervorruft, um die Polymerisation der härtbaren Zusammensetzung zu induzieren, um ein Polymer zu erhalten, bei dem es sich um ein vernetztes Polymer handeln kann, wenn die härtbare Zusammensetzung zumindest ein mehrwertiges Monomer umfasst, das im Verlauf der Polymerisation für ein gewisses Maß an Vernetzung sorgt.

Da die polymerisierbaren Monomere der vorliegenden Offenbarung im Polymerisationsverfahren des Verfahrens der vorliegenden Offenbarung copolymerisiert werden, ist das Ergebnis ein gegebenenfalls vernetztes Polymer, das Gruppierungen des polymerisierbaren Monomers als Grundeinheiten umfasst, was der Gegenstand eines Aspekts der vorliegenden Offenbarung ist. Vorzugsweise ist ein solches Polymer ein vernetztes Polymer, das typischerweise für die Verwendung als kieferorthopädische Vorrichtung bestens geeignet ist.

In manchen Aspekten stellt die vorliegende Offenbarung eine kieferorthopädische Vorrichtung bereit, die ein Polymer umfasst, wobei das Polymer ein Monomer der folgenden Formel umfasst: 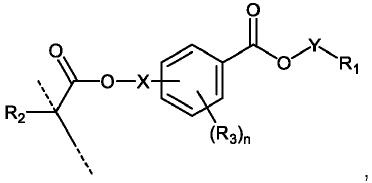 worin:
R₁ für C₃-C₁₀-Cycloalkyl, 3- bis 10-gliedriges Heterocycloalkyl oder C₃-C₁₀-Aryl steht, wobei das C₃-C₁₀-Cycloalkyl, das 3- bis 10-gliedrige Heterocycloalkyl und das C₃-C₁₀-Aryl unsubstituiert oder mit einem oder mehreren Substituenten, ausgewählt aus der aus C₁-C₆-Alkyl, C₁-C₆-Alkoxy, C₃-C₇-Cycloalkyl, C₆-C₁₀-Aryl, C₁-C₆-Alkoxy-C₆-C₁₀-aryl, -O(CO)-(C₁-C₆)-Alkyl, -COO-(C₁-C₆)-Alkyl, =O, -F, -Cl und -Br bestehenden Gruppe substituiert sind;
R₂ für H oder C₁-C₆-Alkyl steht;
die R₃ jeweils unabhängig für Halogen, C₁-C₃-Alkyl, C₁-C₃-Alkoxy, Si(R₄)₃, P(O)(OR₅)₂ oder N(R6)2 stehen;
die R₄ jeweils unabhängig für C₁-C₆-Alkyl oder C₁-C₆-Alkoxy stehen;
die R₅ jeweils unabhängig für C₁-C₆-Alkyl oder C₆-C₁₀-Aryl stehen;
die R₆ jeweils unabhängig für H oder C₁-C₆-Alkyl stehen;
X fehlt, C₁-C₃-Alkylen, 1- bis 3-gliedriges Heteroalkylen oder (CH₂CH₂O)ₘ ist;
Y fehlt, C₁-C₆-Alkylen, 1- bis 3-gliedriges Heteroalkylen oder (CH₂CH₂O)ₘ ist;
die m jeweils eine ganze Zahl von 1 bis 4 sind; und
n eine ganze Zahl von 0 bis 4 ist
wobei jede gestrichelte Linie für eine Bindung an ein Kohlenstoffatom steht.

In manchen Ausführungsformen
steht R₁ für C₃-C₁₀-Cycloalkyl, wobei das C₃-C₁₀-Cycloalkyl unsubstituiert ist; mit einem oder mehreren Substituenten, ausgewählt aus der aus -CH₃, C₁-C₆-Alkoxy, -O(CO)-(C₁-C₆)-Alkyl, -COO-(C₁-C₆)-Alkyl, =O, -F, -Cl und -Br bestehenden Gruppe substituiert ist; mit einem oder mehreren -CH₃ und gegebenenfalls außerdem mit einem oder mehreren Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert ist; oder mit zwei oder mehr Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert ist;
steht R₂ für H oder C₁-C₆-Alkyl;
stehen die R₃ jeweils unabhängig für Halogen, C₁-C₃-Alkyl, C₁-C₃-Alkoxy, Si(R4)3, P(O)(OR₅)₂ oder N(R6)2;
stehen die R₄ jeweils unabhängig für C₁-C₆-Alkyl oder C₁-C₆-Alkoxy;
stehen die R₅ jeweils unabhängig für C₁-C₆-Alkyl oder C₆-C₁₀-Aryl;
stehen die R₆ jeweils unabhängig für H oder C₁-C₆-Alkyl stehen;
fehlt X;
fehlt Y; und
ist n eine ganze Zahl von 0 bis 4.

In manchen Ausführungsformen
steht R₁ für C₃-C₇-Cycloalkyl oder 3- bis 7-gliedriges Heterocycloalkyl, wobei das C₃-C₇-Cycloalkyl und das 3- bis 7-gliedrige Heterocycloalkyl unsubstituiert sind; mit einem oder mehreren Substituenten, ausgewählt aus der aus -CH₃, C₁-C₆-Alkoxy, -O(CO)-(C₁-C₆)-Alkyl, -COO-(C₁-C₆)-Alkyl, =O, -F, -Cl und -Br bestehenden Gruppe substituiert sind; mit einem oder mehreren -CH₃ und gegebenenfalls außerdem mit einem oder mehreren Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert sind; oder mit zwei oder mehr Substituenten, ausgewählt aus der aus C₁-C₆-Alkyl, C₁-C₆-Alkoxy, -O(CO)-(C₁-C₆)-Alkyl, -COO-(C₁-C₆)-Alkyl, =O, -F, -Cl und -Br bestehenden Gruppe substituiert sind;
steht R₂ für H oder C₁-C₆-Alkyl;
stehen die R₃ jeweils unabhängig für Halogen, C₁-C₃-Alkyl, C₁-C₃-Alkoxy, Si(R4)3, P(O)(OR₅)₂ oder N(R6)2;
stehen die R₄ jeweils unabhängig für C₁-C₆-Alkyl oder C₁-C₆-Alkoxy;
stehen die R₅ jeweils unabhängig für C₁-C₆-Alkyl oder C₆-C₁₀-Aryl;
stehen die R₆ jeweils unabhängig für H oder C₁-C₆-Alkyl;
ist X C₁-C₃-Alkylen, 1- bis 3-gliedriges Heteroalkylen oder (CH₂CH₂O)ₘ-,
fehlt Y;
ist m eine ganze Zahl von 1 bis 4; und
ist n eine ganze Zahl von 0 bis 4.

In manchen Ausführungsformen
steht R₁ für C₃-C₇-Cycloalkyl oder C₆-C₁₀-Aryl, wobei das C₃-C₇-Cycloalkyl und das C₆-C₁₀-Aryl unsubstituiert sind oder mit einem oder mehreren Substituenten, ausgewählt aus der aus C₁-C₆-Alkyl, C₁-C₆-Alkoxy, C₃-C₇-Cycloalkyl, C₆-C₁₀-Aryl, C₁-C₆-Alkoxy-C₆-C₁₀-aryl, -O(CO)-(C₁-C₆)-Alkyl, -COO-(C₁-C₆)-Alkyl, =O, -F, -Cl und -Br bestehenden Gruppe substituiert sind, mit der Maßgabe, dass der Benzoesäureester der Formel (III) 2- oder 3-substituiert ist, wenn R₁ unsubstituiert ist;
steht R₂ für H oder C₁-C₆-Alkyl;
stehen die R₃ jeweils unabhängig für Halogen, C₁-C₃-Alkyl, C₁-C₃-Alkoxy, Si(R4)3, P(O)(OR₅)₂ oder N(R6)2;
stehen die R₄ jeweils unabhängig für C₁-C₆-Alkyl oder C₁-C₆-Alkoxy;
stehen die R₅ jeweils unabhängig für C₁-C₆-Alkyl oder C₆-C₁₀-Aryl;
stehen die R₆ jeweils unabhängig für H oder C₁-C₆-Alkyl;
fehlt X oder ist C₁-C₃-Alkylen, 1- bis 3-gliedriges Heteroalkylen oder (CH₂CH₂O)ₘ;
ist Y C₁-C₆-Alkylen;
ist m eine ganze Zahl von 1 bis 4; und
ist n eine ganze Zahl von 0 bis 4.

In manchen Ausführungsformen ist Y C₁-C₃-Alkylen. In manchen Ausführungsformen fehlt X. In manchen Ausführungsformen ist X C₁-C₃-Alkylen.

R₁ kann gegebenenfalls substitiuertes C₅-C₁₀-Cycloalkyl sein, wie z.B. gegebenenfalls substituiertes C₅-C₇-Cycloalkyl. In manchen Ausführungsformen ist R₁ mit einer oder mehreren, unverzweigten oder verzweigten C₁-C₆-Alkylgruppen substituiert. Beispielsweise kann R₁ mit -CH₃ und gegebenenfalls außerdem mit einer oder mehreren, unverzweigten oder verzweigten C₁-C₆-Alkylgruppen substituiert sein. In manchen Ausführungsformen ist R₁ gegebenenfalls substituiertes C₅-C₇-Cycloalkyl mit insgesamt 5 bis 15 oder 5 bis 12 oder 5 bis 10 Kohlenstoffatomen. In manchen Ausführungsformen ist R₁ Cyclohexyl, das mit -CH₃ und gegebenenfalls außerdem mit einer oder mehreren, unverzweigten oder verzweigten C₁-C₆-Alkylgruppen substituiert ist, wobei zwei der C₁-C₆-Alkylgruppen verbunden sein können, um zusammen mit den Kohlenstoffatomen, an die sie gebunden sind, und gegebenenfalls einem oder mehreren zusätzlichen, dazwischen liegenden Kohlenstoffatomen des Cyclohexylrings einen Ring zu bilden. In manchen Ausführungsformen ist R₁ Cyclohexyl, das mit -CH₃ und gegebenenfalls außerdem ist einer oder mehreren C₁-C₃ Alkylgruppen substituiert ist, wobei zwei der C₁-C₃ Alkylgruppen verbunden sein können, um zusammen mit den Kohlenstoffatomen, an die sie gebunden sind, und gegebenenfalls einem oder mehreren zusätzlichen, dazwischen liegenden Kohlenstoffatomen des Cyclohexylrings einen Ring zu bilden. In manchen Ausführungsformen ist R₁ aus der aus folgenden bestehenden Gruppe ausgewählt: 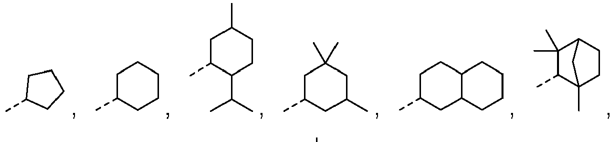 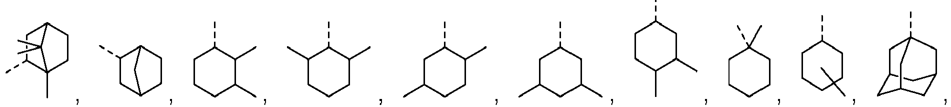 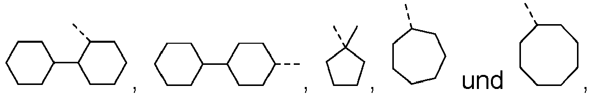 wie z.B. 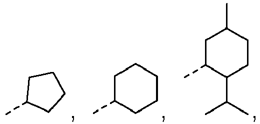 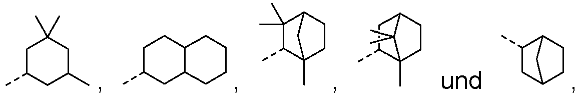 oder wie z.B. 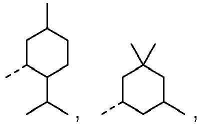 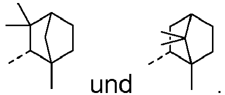 In manchen Ausführungsformen ist R₁ gegebenenfalls substituiertes Phenyl. In manchen Ausführungsformen ist R₁ mit einem oder mehreren Substituenten, ausgewählt aus der aus C₁-C₄-Alkyl und C₁-C₄-Alkoxy bestehenden Gruppe substituiert.

In manchen Ausführungsformen ist -Y-R₁ aus der aus folgenden bestehenden Gruppe ausgewählt: 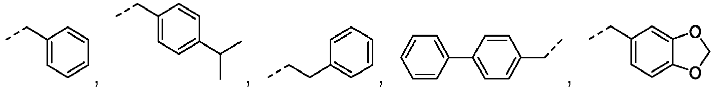 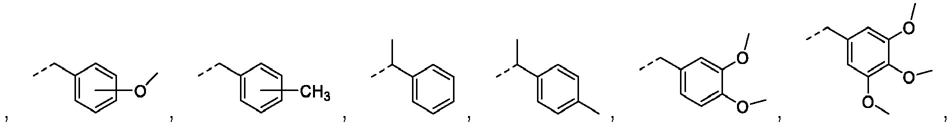 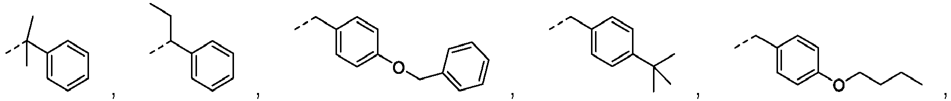 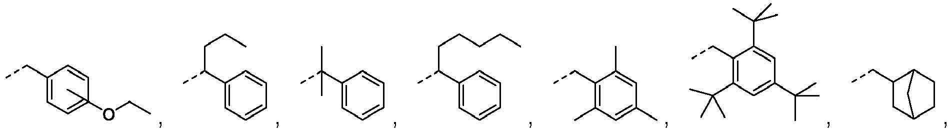 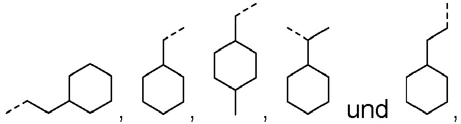 wie z.B. 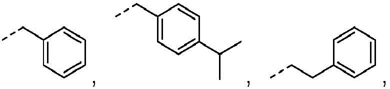 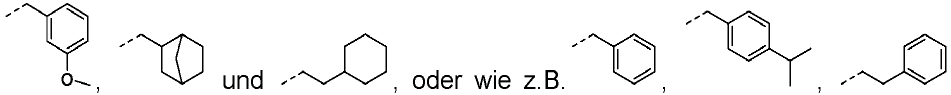 und 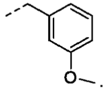 In manchen Ausführungsformen ist R₁ unsubstituiert.

In manchen Ausführungsformen ist das Monomer ein 2- oder 3-substituierter Benzoesäureester. In manchen Ausführungsformen ist das Monomer ein 4-substituierter Benzoesäureester.

In manchen Ausführungsformen steht R₁ für gegebenenfalls substituiertes C₅-C₇-Cycloalkyl; R₂ steht für H oder CH₃; die R₃ stehen jeweils unabhängig für C₁-C₃-Alkyl oder C₁-C₃-Alkoxy; und n ist eine ganze Zahl von 0 bis 4. In manchen Ausführungsformen ist R₃ aus der aus C₁-C₃-Alkyl und C₁-C₃-Alkoxy bestehenden Gruppe ausgewählt, wie z.B. Methyl, Methoxy, Ethyl und Ethoxy. In manchen Ausführungsformen ist n = 0 oder 1, beispielsweise ist n = 0. In manchen Ausführungsformen ist R₂ H oder CH₃.

Wie oben angeführt können die polymerisierbaren Monomere gemäß einem Aspekt der vorliegenden Offenbarung durch Veresterungs- oder Umesterungsreaktionen der entsprechenden im Handel erhältlichen 2-, 3-oder 4-Hydroxybenzoesäuren oder -ester und nachfolgende (Meth)acrylierung, vorzugsweise unter Verwendung von (Meth)acrylsäureanhydrid oder (Meth)acryloylchlorid, oder durch eine Veresterungs- oder Umesterungsreaktion der entsprechenden 2-, 3- oder 4-(Meth)acryloylbenzoesäuren oder -ester synthetisiert werden, die im Handel erhältlich sein oder in einem ersten Schritt durch eine Veresterungsreaktion der entsprechenden 2-, 3- oder 4-Hydroxybenzoesäuren oder -ester, wiederum vorzugsweise mit (Meth)acrylsäureanhydrid oder (Meth)acryloylchlorid, synthetisiert werden können.

Vorzugsweise werden diese Veresterungs- oder Umesterungsreaktionen in Gegenwart einer geeigneten Base, wie z.B. Triethylamin, Pyridin, Dimethylaminopyridin oder metallischem Natrium, und/oder einem geeigneten Lösungsmittel, wie z.B. CH₂Cl₂ oder einem beliebigen anderen Lösungsmittel für die Reaktanten, das vorzugsweise aprotisch ist und einen niedrigen Siedepunkt aufweist, durchgeführt. Es kann vorteilhaft sein, die Reaktanten gegebenenfalls zu erhitzen oder zu kühlen. Beispielsweise kann es für die Veresterung oder Umesterung der entsprechenden 2-, 3- oder 4-Hydroxybenzoesäuren (oder-säureester) bevorzugt sein, das Reaktionsgemisch auf erhöhte Temperaturen, wie z.B. 100 °C oder mehr, wie z.B. 120 °C oder 130 °C, zu erhitzen, und für die anschließenden Veresterungsreaktionen unter Verwendung von (Meth)acrylsäureanhydrid kann das Erhitzen ebenfalls vorteilhaft sein. Wird jedoch (Meth)acryloylchlorid zum Verestern der OH-Gruppe des Hydroxybenzoesäureesters verwendet, kann es empfehlenswert sein, die Reaktionsgemische, z.B. auf eine Temperatur von < 0 °C oder < -10 °C, abzukühlen, um eine mögliche Spaltung der entsprechenden Benzoesäureester zu vermeiden. Fachleuten auf dem Gebiet der organischen Synthese ist die beste Art der Durchführung solcher Veresterungs- oder Umesterungsreaktionen bekannt.

Wenn eine Umesterungsreaktion durchgeführt wird, um einen gewünschten 2-, 3- oder 4-(Meth)acryloylbenzoesäureester zu erhalten, wird vorzugsweise der entsprechende Methyl- oder Ethylester als Ausgangsmaterial eingesetzt, das als Nebenprodukt gebildetes Methanol oder Ethanol ziemlich einfach abdestilliert werden kann.

Beispiele für Verfahren zum Synthetisieren der polymerisierbaren Monomere der vorliegenden Offenbarung sind in den nachstehenden Reaktionsschemata angeführt. Auf einem ersten Syntheseweg wird entweder die entsprechende 2-, 3- oder 4-Hydroxybenzoesäure oder das entsprechende Methyl-2-, -3- oder -4-hydroxybenzoat als Ausgangsmaterial eingesetzt, das mit dem geeigneten Alkohol R₁-OH ver- oder umgeestert wird, gegebenenfalls während das als Nebenprodukt gebildete MeOH abdestilliert wird, um den gewünschten 2-, 3- oder 4-Hydroxybenzosäure-R₁-Ester zu erhalten, der schließlich unter Verwendung von (Meth)acrylsäureanhydrid oder (Meth)-acryloylchlorid, wie im nachstehenden Reaktionsschema angeführt, (meth)acryliert wird. 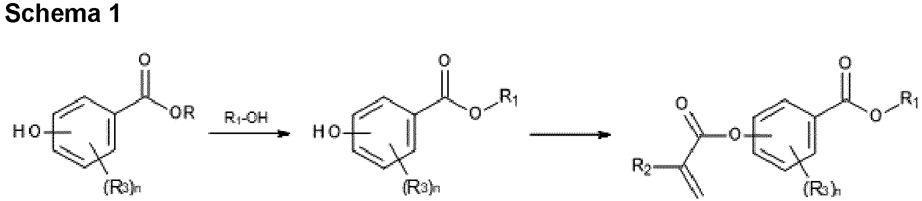 R: H oder -CH₃

Alternativ dazu kann die entsprechende 2-, 3- oder 4-Hydroxybenzoesäure oder das entsprechende Methyl-2-, -3- oder -4-hydroxybenzoat als Ausgangsmaterial eingesetzt werden, das zunächst (meth)acryliert wird, indem es mit (Meth)acrylsäureanhydrid oder (Meth)acryloylchlorid umgesetzt wird, um die entsprechende 2-, 3- oder 4-((Meth)acryloyloxy)benzoesäure oder deren Methylester zu ergeben, die/der ver- oder umgeestert wird, um gemäß dem nachstehenden Schema 2 das gewünschte Monomer der Formel (I) zu erhalten. 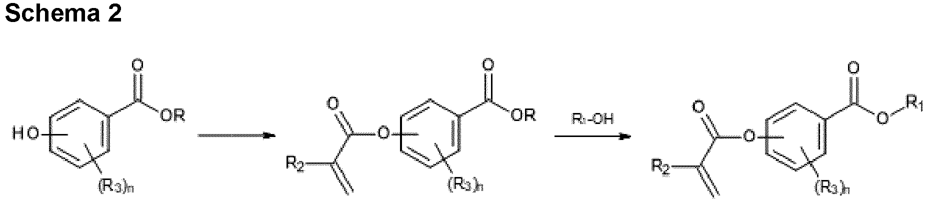 R: H oder -CH₃

Wenn die entsprechende 2-, 3- oder 4-Hydroxybenzoesäure als Ausgangsmaterial eingesetzt wird, kann die zweite Veresterungreaktion zunächst durch Umsetzen der als Zwischenprodukt erhaltenen (meth)acryloylierten Benzoesäure zum entsprechenden Benzoylchlorid unter Verwendung eines geeigneten Chlorierungsmittels, wie z.B. Thionylchlorid, und anschließendes Umsetzen des als Zwischenprodukt erhaltenen Benzoylchlorid mit dem entsprechenden Alkohol R₁-OH durchgeführt werden, um wie im nachstehenden Reaktionsschema dargestellt das Endprodukt zu ergeben.

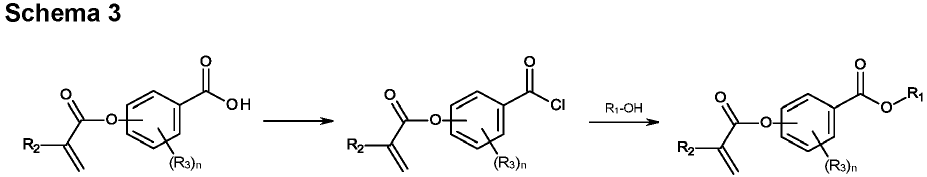

Solche Herstellungsverfahren zählen zur allgemeinen Praxis im Bereich der organischen Synthese, und Fachleute auf dem Gebiet der Erfindung sind in der Lage, das beste Verfahren zur Herstellung der jeweiligen polymerisierbaren Monomere gemäß der vorliegenden Offenbarung auszuwählen.

Ein polymerisierbares Monomer der vorliegenden Offenbarung, wie z.B. eine in **Tabelle 1** angeführte Verbindung, kann gemäß einem der in den **Schemata 1 bis 3** oder in den **Beispielen 1 bis 21** dargelegten Weg oder durch auf dem Gebiet der Erfindung allgemein bekannte Verfahren synthetisiert werden.

**Tabelle 1**

| **Nr.** | **Struktur** |
|---|---|
| **1** | 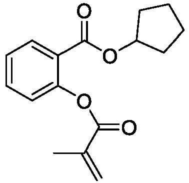 |
| **2** | 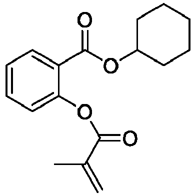 |
| **3** | 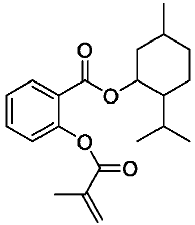 |
| **4** | 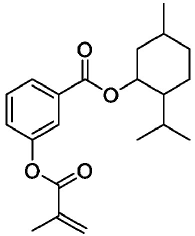 |
| **5** | 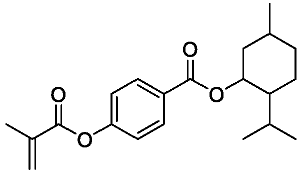 |
| **6** | 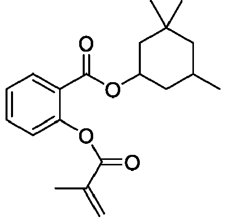 |
| **7** | 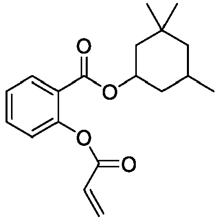 |
| **8** | 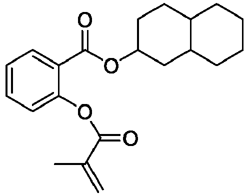 |
| **9** | 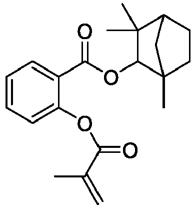 |
| **10** | 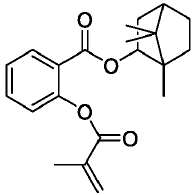 |
| **11** | 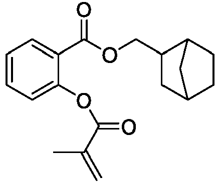 |
| **12** | 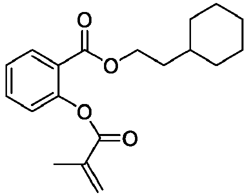 |
| **13** | 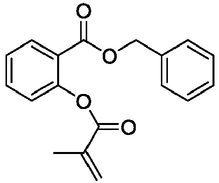 |
| **14** | 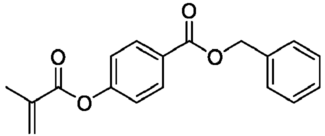 |
| **15** | 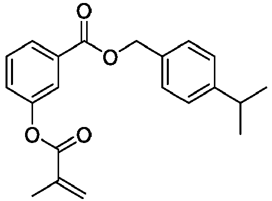 |
| **16** | 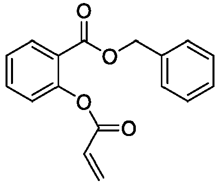 |
| **17** | 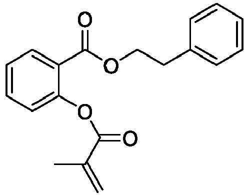 |
| **18** | 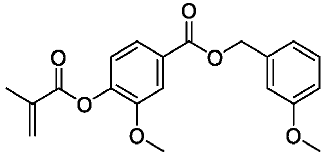 |
| **19** | 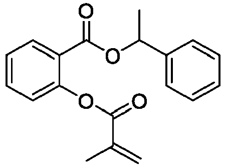 |
| **20** | 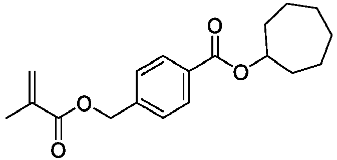 |
| **21** | 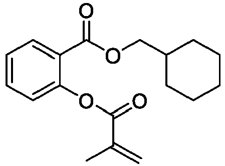 |
| **Ref1** | 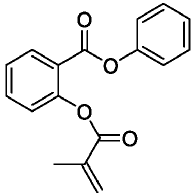 |
| **Ref2** | 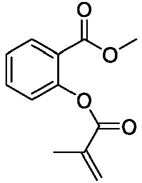 |
| **Ref3** | 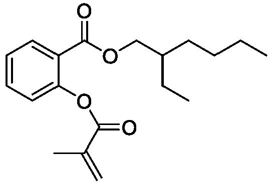 |
| **Ref4** | 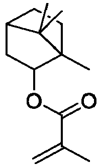 |
| **Ref5** | 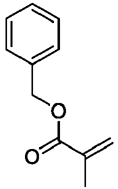 |

Das Verfahren gemäß einem Aspekt der vorliegenden Offenbarung, d.h. das Verfahren zum Polymerisieren einer härtbaren Zusammensetzung, die zumindest eine Art einer polymerisierbaren Spezies umfasst, um ein gegebenenfalls vernetztes Polymer zu erhalten, indem die härtbare Zusammensetzung, gegebenenfalls nachdem sie erhitzt wurde, mit einem Reaktivverdünner vermischt wird, wonach die Polymerisation induziert wird, indem die Zusammensetzung erhitzt und/oder bestrahlt wird, wobei der Reaktivverdünner zumindest ein polymerisierbares Monomer gemäß der vorliegenden Offenbarung ist, unterliegt keinen speziellen Einschränkungen. Das bedeutet, dass beliebige polymerisierbarer Monomere oder Präpolymere, die in der Lage sind, mit den polymerisierbaren Monomeren der Offenbarung zu copolymerisieren, als polymerisierbare Spezies eingesetzt werden können.

In manchen Ausführungsformen ist dieses Verfahren Teil eines Hochtemperaturphotolithographieverfahrens auf Lithographiebasis, wobei die härtbare Zusammensetzung zumindest einen Photopolymerisationsinitiator umfasst, der bei Besrahlung mit Licht mit einer geeigneten Wellenlänge, damit dieses absorbiert wird, gespalten wird, was Spaltprodukte ergibt, von denen zumindest eines in der Lage ist, die Polymerisation der härtbaren Zusammensetzung zu induzieren, wobei die Polymerisationsreaktion vorzugsweise Teil eines additiven Fertigungsverfahrens, noch bevorzugter eines 3D-Druckverfahrens, ist. Folglich sollte der Photoinitiator mit der zumindest einen polymerisierbaren Spezies und dem Reaktivverdünner, d.h. den polymerisierbaren Monomeren der Offenbarung, kompatibel sein. Als Teil eines Hochtemperaturphotopolymerisationsverfahrens umfassen manche Ausführungsformen des Verfahrens einen Schritt des Erhitzens der härtbaren Formulierung, die das polymerisierbare Monomer/die polymerisierbaren Monomere der Offenbarung als Reaktivverdünner enthält, auf eine vorab festgelegte erhöhte Verfahrenstemperatur im Bereich von 50 °C bis 120 °C, wie z.B. von 90 °C bis 120 °C, bevor es bestrahlt wird, um die Polymerisation zu induzieren, die gegebenenfalls vernetzte Polymere ergibt.

In weiteren Ausführungsformen umfasst das Verfahren das Polymerisieren einer härtbaren Zusammensetzung, die zumindest ein mehrwertiges Monomer umfasst und polymerisiert wird, um ein vernetztes Polymer zu liefern, das als Grundeinheiten Gruppierungen umfasst, die von dem oder den polymerisierbaren Monomeren der vorliegenden Offenbarung herrühren.

Solche gegebenenfalls vernetzte Polymere, die Grundeinheiten umfassen, die von dem oder den polymerisierbaren Monomeren der Offenbarung herrühren, sind Gegenstand eines Aspekts der vorliegenden Offenbarung, wobei die vernetzten Ausführungsformen dieser Polymere besonders geeignet für die Verwendung als kieferorthopädische Vorrichtungen, wie z.B. als Aligner, sind.

Zum Erhalt vernetzter Polymere, die besonders geeignet als kieferorthopädische Vorrichtungen sind, sollte die zumindest eine polymerisierbare Spezies, die in dem Verfahren gemäß der vorliegenden Offenbarung eingesetzt wird, im Hinblick auf mehrere thermomechanische Eigenschaften der resultierenden Polymere ausgewählt werden. Zunächst sollte zumindest eine, vorzugsweise jedoch mehr als eine, mehrwertige polymerisierbare Spezies inkludiert werden. Zweitens sollten die Mengen der polymerisierbaren Spezies und des Reaktivverdünners, d.h. des oder der polymerisierbaren Monomere der vorliegenden Offenbarung, gut abgestimmt sein. Drittens sollten das oder die polymerisierbaren Monomere der vorliegenden Offenbarung so ausgewählt werden, dass sie zu den thermomechanischen Eigenschaften der Polymere beitragen.

Wie in den nachstehenden Beispielen gezeigt sind die polymerisierbaren Monomere gemäß der vorliegenden Offenbarung als Reaktivverdünner für hochviskose härtbare Harze gut geeignet und ergeben gegebenenfalls vernetzte Polymere mit für die Verwendung als kieferorthopädische Vorrichtungen günstigen thermomechanischen Eigenschaften.

In manchen Ausführungsformen sind die vernetzten Polymere durch ein Zugspannungs-Dehnungsdiagramm gekennzeichnet, das eine Streckgrenze anzeigt, nach der die Länge des Prüflings weiterhin zunimmt, aber keine weitere Zunahme der Spannung vorliegt. Ein solches Streckgrenzenverhalten tritt typischerweise "nahe" der Glasübergangstemperatur auf, wo sich das Material zwischen einem glasartigen und gummiartigen Zustand befindet und als viskoelastisches Verhalten aufweisend charakterisiert werden kann. In manchen Ausführungsformen wird viskoelastisches Verhalten im Temperaturbereich von 20 °C bis 40 °C beobachtet. Die Streckspannung wird an der Streckgrenze bestimmt. In manchen Ausführungsformen wird der Modul aus der Anfangskurve des Spannungs-Dehnungs-Diagramms oder als Sekantenmodul bei 1 % Dehnung bestimmt (z.B. wenn es keinen linearen Abschnitt des Spannungs-Dehnungs-Diagramms gibt). Die Streckgrenzendehnung wird aus der Dehnung an der Streckgrenze bestimmt. Tritt die Streckgrenze bei einem Spannungshöchstmaß auf, ist die endgültige Zugfestigkeit geringer als die Streckspannung. Für einen Zugprüfling ist die Verformung durch In (l/l₀) definiert, was bei geringen Verformungen durch (l-l₀)/l₀ approximiert werden kann (z.B. weniger als etwa 10 %), und die Verlängerung ist l/l₀, wobei I die Messlänge nach dem Auftreten einer gewissen Verformung ist und l₀ die Anfangsmesslänge ist. Die Testtemperatur kann unterhalb der erwarteten Verwendungstemperatur für eine zahnärztliche Vorrichtung, wie etwa 35 °C bis 40 °C, liegen. In manchen Ausführungsformen beträgt die Testtemperatur 23 ± 2 °C.

In manchen Ausführungsformen kann die Spannungsrelaxion durch Überwachen der zeitabhängigen Spannung infolge einer stetigen Verformung/Belastung gemessen werden. Das Ausmaß der Spannungsrelaxion kann auch von der Temperatur, der relativen Feuchtigkeit und anderen herrschenden Bedingungen (z.B. Gegenwart von Wasser) abhängen. In manchen Ausführungsformen sind die Testbedingungen für die Spannungsrelaxion eine Temperatur von 37 ± 2 °C bei 100 % relativer Feuchtigkeit oder eine Temperatur von 37 ± 2 °C in Wasser.

Die dynamische Viskosität eines Fluids gibt dessen Widerstand gegenüber Scherflüssen an. Die SI-Einheit für die dynamische Viskosität ist das Poiseuille (Pa·s). Die dynamische Viskosität wird üblicherweise in Centipoise-Einheiten angegeben, wobei 1 Centipoise (cP) 1 mPa·s entspricht. Die kinematische Viskosität ist das Verhältnis der dynamischen Viskosität zur Dichte des Fluids; die SI-Einheit ist m²/s. Geräte zur Messung der Viskosität umfassen Viskosimeter und Rheometer.

Wie hierin verwendet sind die Begriffe "Steifigkeit" und "Starrheit" austauschbar, ebenso wie die entsprechenden Begriffe "steif" und "starr".

Wie hierin verwendet umfasst eine "Vielzahl von Zähnen" zwei oder mehr Zähne.

In zahlreichen Ausführungsformen umfassen ein oder mehrere posteriore Zähne einen oder mehrere aus einem Molar, einem Prämolar und einem Eckzahn, und ein oder mehrere anteriore Zähne umfassen einen oder mehr aus einem mittleren Schneidezahn, einem seitlichen Schneidezahn, einem Eckzahn, einem ersten Vormahlzahn und einem zweiten Vormahlzahn.

Die polymerisierbaren Monomere gemäß der vorliegenden Offenbarung sind als Reaktivverdünner für hochviskose härtbare Harze geeignet und ergeben gegebenenfalls vernetzte Polymere mit zur Verwendung als kieferorthopädische Vorrichtungen, beispielsweise zum Bewegen von einem oder mehreren Zähnen, günstigen thermomechanischen Eigenschaften.

Die hierin offenbarten Ausführungsformen können verwendet werden, um Gruppen aus einem oder mehreren Zähnen miteinander zu verbinden. Die Gruppen aus einem oder mehreren Zähnen können eine erste Gruppe aus einem oder mehreren anterioren Zähnen und eine zweite Gruppe aus einem oder mehreren posterioren Zähnen umfassen. Die erste Gruppe von Zähnen kann mit den hierin offenbarten Polymerschalenvorrichtungen mit der zweiten Gruppe von Zähnen verbunden werden.

Die hierin offenbarten Ausführungsformen sind gut geeignet, um einen oder mehrere Zähne aus der ersten Gruppe aus einem oder mehreren Zähnen zu bewegen oder um einen oder mehrere Zähne aus der zweiten Gruppe aus einem oder mehreren Zähnen zu bewegen, sowie Kombinationen davon.

Die hierin offenbarten Ausführungsformen sind für eine Kombination mit einem oder mehreren bekannten im Handel erhältlichen Zahnbewegungskomponenten, wie etwa Aufsätze oder Polymerschalenvorrichtungen, gut geeignet. In zahlreichen Ausführungsformen sind die Vorrichtung und ein oder mehrere Aufsätze ausgebildet, um einen oder mehrere Zähne entlang eines sechs Freiheitsgrade umfassenden Zahnbewegungsvektors zu bewegen, wobei drei Freiheitsgrade Rotationsfreiheitsgrade sind und drei Freiheitsgrade Translationsfreiheitsgrade sind.

Die vorliegende Offenbarung stellt kieferorthopädische Systeme und verwandte Verfahren für die Konzeption und Bereitstellung verbesserter oder wirksamerer Zahnbewegungssysteme bereit, um eine gewünschte Zahnbewegung und/oder Repositionierung von Zähnen in eine gewünschte Anordnung zu bewirken.

Obwohl auf eine Vorrichtung verwiesen wird, die eine Polymerschalenvorrichtung umfasst, sind die hierin offenbarten Ausführungsformen für die Verwendung mit zahlreichen zahnaufnehmenden Vorrichtungen geeignet, beispielsweise Vorrichtungen ohne ein/e oder mehrere Polymere oder Schalen. Die Vorrichtung kann aus einem oder mehreren von zahlreichen Materialien gefertigt sein, wie etwa z.B. Metall, Glas, Verstärkungsfasern, Kohlenstofffasern, Verbundstoffen, verstärkten Verbundstoffen, Aluminium, biologischen Materialien und Kombinationen davon. In manchen Fällen können die verstärkten Verbundstoffe beispielsweise eine mit Keramik oder Metallteilchen verstärkte Polymermatrix umfassen. Die Vorrichtung kann auf vielerlei Arten geformt werden, wie etwa z.B. durch Thermoformen oder direkte Fabrikation, wie hierin beschrieben. Alternativ oder in Kombination dazu kann die Vorrichtung durch maschinelle Bearbeitung erzeugt werden, wobei eine Vorrichtung mit rechnergestützter numerischer Steuerung aus einem Materialblock gefertigt wird. Vorzugsweise wird die Vorrichtung unter Verwendung eines polymerisierbaren Monomers gemäß der vorliegenden Offenbarung, wie z.B. unter Verwendung der Monomere als Reaktivverdünner für härtbare Harze, gefertigt.

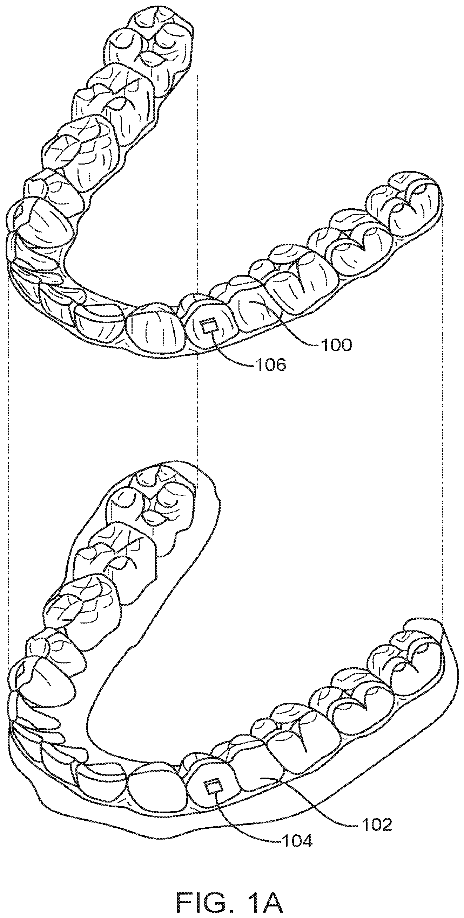

Nun auf die Zeichnungen Bezug nehmend, in denen die gleichen Zahlen in den verschiedenen Zeichnungen dieselben Elemente bezeichnen, veranschaulicht **Fig. 1A** eine(n) beispielhafte(n) Zahnrepositionierungsvorrichtung oder Aligner 100, die/der von einem Patienten getragen werden kann, um eine inkrementelle Repositionierung einzelner Zähne 102 im Kiefer zu erreichen. Die Vorrichtung kann eine Schale (z.B. eine durchgehende Polymerschale oder eine segmentierte Schale) umfassen, die zahnaufnehmende Ausnehmungen aufweist, die die Zähne aufnehmen und federnd repositionieren. Eine Vorrichtung oder (ein) Abschnitt(e) davon kann unter Verwendung eines physischen Zahnmodells indirekt hergestellt werden. Beispielsweise kann eine Vorrichtung (z.B. Polymervorrichtung) unter Verwendung eines physischen Zahnmodells und eines Bogens von geeigneten Schichten Polymermaterial gebildet werden. In manchen Ausführungsformen wird eine physische Vorrichtung direkt, z.B. unter Verwendung von Rapid-Prototyping-Fertigungsverfahren, aus einem digitalen Modell einer Vorrichtung hergestellt. Eine Vorrichtung kann über alle in einem Ober- oder Unterkiefer vorhandenen Zähne oder über weniger als alle Zähne passen. Die Vorrichtung kann so gestaltet sein, dass sie sich an die Zähne des Patienten anpasst (z.B. die Topographie der zahnaufnehmenden Ausnehmungen passt zu der Topographie der Zähne des Patienten), und kann basierend auf durch einen Abdruck, Scannen und dergleichen erzeugten, positiven oder negativen Modellen der Zähne des Patienten hergestellt werden. Alternativ dazu kann die Vorrichtung eine allgemeine Vorrichtung sein, die zum Aufnehmen der Zähne ausgebildet ist, aber nicht notwendigerweise geformt ist, um zur Topographie der Zähne des Patienten zu passen. In manchen Fällen werden nur gewisse von einer Vorrichtung aufgenommene Zähne von der Vorrichtung repositioniert, während andere Zähne eine Basis oder eine Ankerregion bereitstellen, um die Vorrichtung in Position zu halten, während sie gegen den Zahn oder die Zähne, auf den/die die Repositionierung abzielt, Kraft ausübt. In manchen Fällen werden manche, die meisten oder sogar alle Zähne zu einem gewissen Zeitpunkt während der Behandlung repositioniert. Zähne, die bewegt werden, können auch als Basis oder Anker dienen, um die Vorrichtung beim Tragen durch den Patienten festzuhalten. Typischerweise werden keine Drähte oder andere Mittel bereitgestellt, um eine Vorrichtung über den Zähnen in Position zu halten. In manchen Fällen kann es jedoch wünschenswert oder nötig sein, einzelne Aufsätze oder andere Ankerelemente 104 auf den Zähnen 102 mit entsprechenden Aufnehmern oder Öffnungen 106 in der Vorrichtung 100 bereitzustellen, so dass die Vorrichtung eine ausgewählte Kraft auf den Zahn ausüben kann. Beispielhafte Vorrichtungen, einschließlich der im Invisalign®-System verwendeten, sind in zahlreichen Patenten und Patentanmeldungen, die Align Technology, Inc. zugeordnet werden, beschrieben, z.B. in US-Patent Nr. 6.450.807 und 5.975.893, sowie auf der Firmen-Webseite, auf die über das World Wide Web zugegriffen werden kann (siehe z.B. "invisalign.com"). Beispiele für am Zahn angebrachte Aufsätze, die für die Verwendung mit kieferorthopädischen Vorrichtungen geeignet sind, sind ebenfalls in Patenten und Patenanmeldungen von Align Technology, Inc., beschrieben, einschließlich z.B. US-Patent Nr. 6.309.215 und 6.830.450.

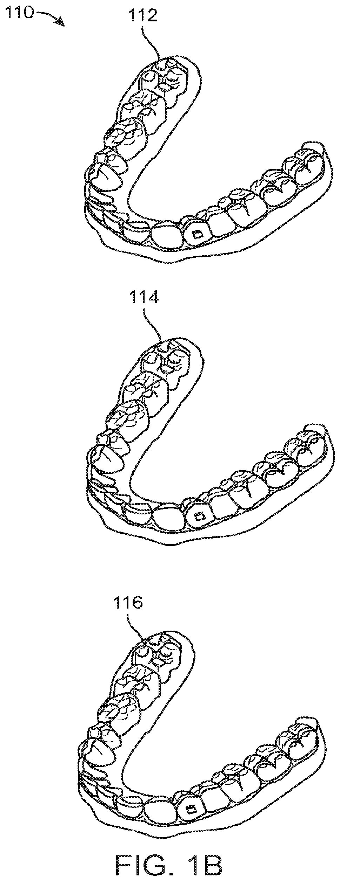

**Fig. 1B** veranschaulicht ein Zahnrepositionierungssystem 110, das eine Vielzahl von Vorrichtungen 112, 114, 116 umfasst. Jede der hierin beschriebenen Vorrichtungen kann als Teil eines Sets aus einer Vielzahl von Vorrichtungen, die in einem Zahnrepositionierungssystem verwendet werden, konzipiert und/oder bereitgestellt sein. Jede Vorrichtung kann so ausgebildet sein, das ihre zahnaufnehmende Ausnehmung eine Geometrie aufweist, die einer für die Vorrichtung vorgesehenen Zwischen- oder Endzahnanordnung entspricht. Die Zähne des Patienten können ausgehend von einer Anfangszahnanordnung sukzessive in eine Zielzahnanordnung repositioniert werden, indem eine Reihe inkrementeller Positionsanpassungsvorrichtungen über die Zähne des Patienten platziert werden. Beispielsweise kann das Zahnrepositionierungssystem 110 eine erste Vorrichtung 112, die einer Anfangszahnanordnung entspricht, eine oder mehrere Zwischenvorrichtungen, die einer oder mehreren Zwischenanordnungen entsprechen, und eine Endvorrichtung 116, die einer Zielanordnung entspricht, umfassen. Eine Zielzahnanordnung kann eine geplante Endzahnanordnung sein, die für die Zähne des Patienten am Ende der gesamten kieferorthopädischen Behandlung gewählt wurde. Alternativ dazu kann eine Zielanordnung eine von mehreren Zwischenanordnungen sein, die für die Zähne des Patienten während des Verlaufs einer kieferorthopädischen Behandlung ausgewählt wurde, die mehrere verschiedene Behandlungsszenarien umfassen kann, einschließlich, ohne darauf beschränkt zu sein, Fälle, in denen ein operativer Eingriff empfohlen wird, in denen eine interproximale Reduktion (IPR) angemessen ist, in denen eine Verlaufskontrolle geplant ist, wo die Anker platziert werden sollten, wo eine Gaumenexpansion wünschenswert ist, wo restaurative Zahnheilkunde beteiligt ist (z.B. Inlays, Onlays, Kronen, Brücken, Implantate, Veneers und dergleichen) etc. Somit versteht es sich, dass eine Zielzahnanordnung eine beliebige geplante Folgeanordnung für die Zähne eines Patienten sein, die auf eine oder mehrere inkrementelle Repositionierungsstufen folgt. Ebenso kann eine Anfangszahnanordnung eine beliebige Anfangsanordnung für die Zähne eines Patienten sein, auf die eine oder mehrere inkrementelle Repositionierungsstufen folgen.

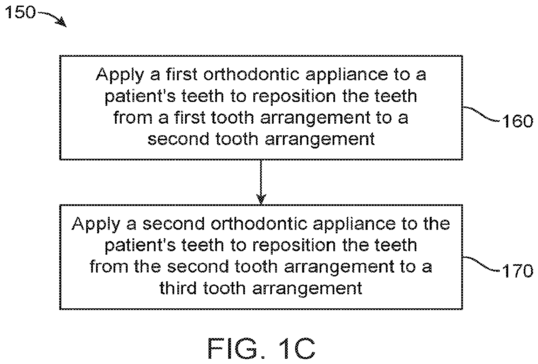

**Fig.** 1C veranschaulicht ein Verfahren 150 einer kieferorthopädischen Behandlung unter Verwendung einer Vielzahl von Vorrichtungen in Übereinstimmung mit Ausführungsformen. Das Verfahren 150 kann unter Verwendung jeder der hierin beschriebenen Vorrichtungen oder Vorrichtungssets durchgeführt werden. In Schritt 160 wird eine erste kieferorthopädische Vorrichtung an den Zähnen eines Patienten angebracht, um die Zähne ausgehend von einer ersten Zahnanordnung in eine zweite Zahnanordnung zu repositionieren. In Schritt 170 wird eine zweite kieferorthopädische Vorrichtung an den Zähnen eines Patienten angebracht, um die Zähne ausgehend von der zweiten Zahnanordnung in eine dritte Zahnanordnung zu repositionieren. Das Verfahren 150 kann gegebenenfalls unter Verwendung einer geeigneten Anzahl und Kombination sequentieller Vorrichtungen wiederholt werden, um die Zähne des Patienten ausgehend von einer Anfangsanordnung in eine Zielanordnung inkrementell zu repositionieren. Die Vorrichtungen können alle in derselben Stufe erzeugt werden oder in Sets oder Chargen (z.B. am Beginn einer Behandlungsstufe) oder die Vorrichtungen können einzeln gefertigt werden und der Patient kann jede Vorrichtung tragen, bis der Druck einer jeden Vorrichtung auf die Zähne nicht länger spürbar ist oder bis das Höchstmaß an Zahnbewegung für die jeweilige Stufe erreicht wurde. Eine Vielzahl verschiedener Vorrichtungen (z.B. ein Set) kann entworfen und sogar gefertigt werden, bevor der Patient eine Vorrichtung der Vielzahl trägt. Nachdem eine Vorrichtung für eine angemessene Zeitdauer getragen wurde, kann der Patient die aktuelle Vorrichtung durch die nächste Vorrichtung in der Serie ersetzen, bis keine weiteren Vorrichtungen verbleiben. Die Vorrichtungen sind im Allgemeinen nicht an den Zähnen befestigt und der Patient kann die Vorrichtungen zu jedem Zeitpunkt während des Verfahrens entfernen und wiedereinsetzen (z.B. vom Patienten entfernbare Vorrichtungen). Die Endvorrichtung oder mehrere Vorrichtungen in der Serie weisen eine Geometrie oder Geometrien auf, die zum Überkorrigieren der Zahnanordnung ausgewählt sind. Beispielsweise können eine oder mehrere Vorrichtungen eine Geometrie aufweisen, die (wenn sie vollständig erreicht wird) einzelne Zähne über die als "final" ausgewählte Zahnanordnung hinaus bewegen würde. Eine solche Überkorrektur kann erwünscht sein, um einem möglichen Rückfall nach Abschluss des Repositionierungsverfahrens vorzubeugen (z. B. Bewegung einzelner Zähne zurück in ihre Position vor der Korrektur erlauben). Eine Überkorrektur kann auch nützlich sein, um die Geschwindigkeit der Korrektur zu beschleunigen (z.B. eine Vorrichtung mit einer Geometrie, die über einer gewünschten Zwischen- oder Endposition hinaus positioniert ist, kann die einzelnen Zähne rascher hin Richtung der Position verschieben). In solchen Fällen kann die Verwendung einer Vorrichtung beendet werden, bevor die Zähne die durch die Vorrichtung definierten Positionen erreichen. Darüber hinaus kann eine Überkorrektur bewusst eingesetzt werden, um etwaige Ungenauigkeiten oder Einschränkungen der Vorrichtung zu kompensieren.

Die verschiedenen Ausführungsformen der hierin präsentierten kieferorthopädischen Vorrichtungen können auf zahlreiche verschiedene Arten hergestellt werden. In manchen Ausführungsformen können die kieferorthopädischen Vorrichtungen hierin (oder Teile davon) unter Verwendung von Direktfertigung, wie etwa additiven Fertigungsverfahren (hierin auch als "3D-Druck" bezeichnet" oder subtraktiven Fertigungsverfahren (z.B. Fräsen), hergestellt werden. In manchen Ausführungsformen umfasst Direktfertigung das Ausbilden eines Gegenstands (z.B. einer kieferorthopädischen Vorrichtung oder eines Teils davon), ohne eine physische Vorlage (z.B. Form, Maske etc.) zum Definieren der Geometrie des Gegenstands zu verwenden. Additive Fertigungsverfahren können wie folgt kategorisiert werden: (1) Wannenphotopolymerisation (z.B. Stereolithographie), bei der ein Gegenstand schichtweise aus einer Wanne mit flüssigem Pohotopolymerharz konstruiert wird; (2) "Material Jetting", ein Verfahren bei dem Material durch die Verwendung entweder eines kontinuierlichen oder Dropon-Demand- (DOD-) Ansatzes auf eine Bauplattform aufgespritzt wird; (3) "Binder Jetting", ein Verfahren bei dem alternierende Schichten eines Baumaterials (z.B. ein pulverbasiertes Material) und eines Bindematerials (z.B. eines flüssigen Bindemittels) durch einen Druckkopf abgelagert werden; (4) "Fused Deposition Modeling (FDM)", ein Verfahren bei dem Material durch eine Düse gezogen, erhitzt und schichtweise abgelagert wird; (5) Pulverbettfusion, einschließlich, ohne darauf beschränkt zu sein, des selektiven Heißsinterns (SHS), selektiven Laserschmelzens (SLM) und selektiven Lasersinterns (SLS); (6) Folienlaminierung, einschließlich, ohne darauf beschränkt zu sein, "Laminated Object Manufacturing (LOM)" und Ultraschall-Additiv-Fertigung (UAM); und (7) "Direct Energy Deposition (DED)", einschließlich, ohne darauf beschränkt zu sein, "Laser Engineering Net Shaping (LENS)", "Direct Metal Deposition" und 3D-Laser-Cladding. Beispielsweise kann Stereolithographie eingesetzt werden, um eine oder mehrere der Vorrichtungen hierin direkt herzustellen. In manchen Ausführungsformen umfasst Stereolithographie die selektive Polymerisation eines lichtempfindlichen Harzes (z.B. eines Photopolymers) mittels Licht (z.B. ultraviolettem Licht) in Übereinstimmung mit einer gewünschten Querschnittsform. Die Geometrie des Gegenstands kann durch sequentielles Polymerisieren einer Vielzahl von Querschnitten des Gegenstands schichtweise aufgebaut werden. Als weiteres Beispiel können die Vorrichtungen hierin mittels selektiven Lasersinterns direkt gefertigt werden. In manchen Ausführungsformen umfasst das selektive Lasersintern die Verwendung eines Laserstrahls, um eine Schicht aus pulverförmigem Material in Übereinstimmung mit einer gewünschten Querschnittsform selektiv zu schmelzen und zu verschmelzen, um die Geometrie des Gegenstands aufzubauen. Als noch ein weiteres Beispiel können die Vorrichtungen durch "Fused Deposition Modeling" direkt hergestellt werden. In manchen Ausführungsformen umfasst Fused Deposition Modeling das schichtweise Schmelzen und selektive Ablagern eines dünnen Filaments eines thermoplastischen Polymers, um einen Gegenstand zu bilden. In noch einem weiteren Beispiel kann "Material Jetting" eingesetzt werden, um die Vorrichtungen hierin direkt herzustellen. In manchen Ausführungsformen umfasst Material Jetting das Aufspritzen oder Extrudieren von einem oder mehreren Materialien auf eine Bauplattform, um sukzessive Schichten der Geometrie des Gegenstands zu bilden. In einem weiteren Beispiel können die Vorrichtungen hierin direkt durch Fused Deposition Modeling erzeugt werden. In machen Ausführungsformen umfasst Fused Deposition Modeling das Schmelzen und selektive schichtweise Abscheiden eines dünnen Filaments aus thermoplastischem Polymer zur Ausbildung eines Gegenstands. In einem weiteren Beispiel kann Material Jetting angewandt werden, um die Vorrichtungen hierin herzustellen. In manchen Ausführungsformen umfasst Material Jetting das Aufspritzen oder Extrudieren eines oder mehrerer Materialien zum Aufbau einer Oberfläche, um aufeinanderfolgende Schichten der Objektgeometrie zu bilden.

Alternativ dazu oder in Kombination damit können Ausführungsformen der Vorrichtungen hierin (oder Teile davon) unter Anwendung indirekter Fertigungsverfahren, wie z.B. Thermoformen mittels Positiv- oder Negativ-Form, hergestellt werden. Die indirekte Fertigung einer kieferorthopädische Vorrichtung kann das Herstellen einer Positiv- oder Negativ-Form der Zahnstruktur des Patienten in einer Zielanordnung (z.B. durch Rapid Prototying, Fräsen, etc.) und das Thermoformen einer oder mehrerer Materialbahnen über der Form zur Herstellung einer Vorrichtungshülle umfassen.

In manchen Ausführungsformen wird durch die hierin bereitgestellten direkten Fertigungsverfahren die Objektgeometrie schichtweise aufgebaut, wobei aufeinanderfolgende Schichten in eigenen Aufbauschritten ausgebildet werden. Alternativ dazu oder in Kombination damit können direkt Fertigungsverfahren angewandt werden, die den kontinuierlichen Aufbau einer Objektgeometrie ermöglichen und hierin als "kontinuierliche Direktfertigung" bezeichnet werden. Verschiedene Arten von kontinuierlichen Direktfertigungsverfahren können angewandt werden. Beispielsweise werden in manchen Ausführungsformen die Vorrichtungen hierin unter Anwendung von "kontinuierlichem Flüssiginterphasendruck" hergestellt, wobei ein Gegenstand aus einem Behälter mit photopolymerisierbarem Harz durch Ausbilden eines Gradienten von teilweise gehärtetem Harz zwischen der Aufbaufläche des Gegenstands und einer "toten Zone" mit gehemmter Polymerisation kontinuierlich aufgebaut wird. In manchen Ausführungsformen wird eine semipermeable Membran eingesetzt, um den Transport eines Photopolymerisationshemmers (z.B. Sauerstoff) in die tote Zone zu steuern, um den Polymerisationsgradienten auszubilden. Kontinuierlicher Flüssiginterphasendruck kann Fertigungsgeschwindigkeiten erzielen, die 25- bis 1000-mal schneller sind als andere direkte Fertigungsverfahren, und um das bis zu etwa 1000-Fache gesteigerte Geschwindigkeiten können durch den Einbau von Kühlsystemen erzielt werden. Kontinuierlicher Flüssiginterphasendruck ist in US-Patentveröffentlichung Nr. 2015/ 0097315, 2015/0097316 und 2015/0102532 beschrieben.

Als weiteres Beispiel kann ein kontinuierliches Direktfertigungsverfahren den kontinuierlichen Aufbau einer Objektgeometrie durch kontinuierliche Bewegung der Aufbauplattform (z.B. in vertikaler oder Z-Richtung) während der Bestrahlungsphase erzielen, so dass die Härtungstiefe des bestrahlten Photopolymers durch die Bewegungsgeschwindigkeit gesteuert wird. Dementsprechend kann eine kontinuierliche Polymerisation des Materials auf der Aufbauoberfläche erzielt werden. Solche Verfahren sind in US-Patent Nr. 7,892.474 beschrieben.

In einem weiteren Beispiel kann ein kontinuierliches Direktfertigungsverfahren das Extrudieren eines Verbundmaterials umfassen, das aus einem härtbaren flüssigen Material besteht, das einen festen Strang umgibt. Das Verbundmaterial kann entlang eines durchgehenden dreidimensionalen Pfads extrudiert werden, um den Gegenstand zu formen. Solche Verfahren sind in US-Patentveröffentlichung Nr. 2014/ 0061974 beschrieben.

In einem weiteren Beispiel wird in einem kontinuierlichen Direktfertigungsverfahren ein "Heliolithographie"-Ansatz genutzt, bei dem das flüssige Photopolymer mit fokussierter Strahlung gehärtet wird, während die Aufbauplattform kontinuierlich gedreht und angehoben wird. Dementsprechend kann die Objektgeometrie entlang eines spiralförmigen Aufbaupfads kontiniuerlich aufgebaut werden. Solche Verfahren sind in US-Patentveröffentlichung Nr. 2014/0265034 beschrieben.

Die hierin bereitgestellten Direktfertigungsansätze sind mit einer breiten Palette von Materialien kompatibel, einschließlich, aber nicht ausschließlich ein oder mehrere der folgenden Materialien: einem Polyester, einem Copolyester, einem Polycarbonat, einem thermoplastischen Polyurethan, einem Polypropylen, einem Polyethylen, einem Polypropylen und Polyethylencopolymer, einem Acryl, einem zyklischen Blockcopolymer, einem Polyetherketon, einem Polyamid, einem Polyethylenterephthalat, einem Polybutylenterephthalat, einem Polyetherimid, einem Polyethersulfon, einem Polytrimethylenterephthalat, einem Styrol-Blockcopolymer (SBC), einem Silikonkautschuk, einer Elastomerlegierung, einem thermoplastischen Elastomer (TPE), einem thermoplastischen Vulkanisat- (TPV-) Elastomer, einem Polyurethanelastomer, einem Blockcopolymer-Elastomer, einem Polyolefingemisch-Elastomer, einem thermoplastischen Copolyester-Elastomer, einem thermoplastischen Polyamid-Elastomer, einem duroplastischen Material oder Kombinationen davon. Die für Direktfertigung eingesetzten Materialien können in einer ungehärteten Form (z.B. als Flüssigkeit, Harz, Pulver usw.) bereitgestellt und gehärtet werden (z.B. durch Photopolymerisation, Lichthärten, Gashärten, Laserhärten, Vernetzen usw.), um eine kieferorthopädische Vorrichtung oder einen Teil davon zu bilden. Die Eigenschaften des Materials vor dem Härten können sich von den Eigenschaften des Materials nach dem Härten unterscheiden. Nach dem Härten können die Materialien hierin ausreichende Festigkeit, Steifigkeit, Haltbarkeit, Biokompatibilität usw. für eine Verwendung in kieferorthopädischen Vorrichtungen aufweisen. Die Eigenschaften der eingesetzten Materialien nach dem Härten können abhängig von den gewünschten Eigenschaften für die jeweiligen Teile der Vorrichtung ausgewählt werden.

In manchen Ausführungsformen können relative starre Abschnitte der kieferorthopädischen Vorrichtung durch Direktfertigung unter Verwendung eines oder mehrerer der folgenden Materialien ausgebildet werden: eines Polyesters, eines Copolyesters, eines Polycarbonats, eines thermoplastischen Polyurethans, eines Polypropylens, eines Polyethylens, eines Polypropylen-Polyethylen-Copolymers, eines Acryls, eines zyklischen Blockcopolymers, eines Polyetherketons, eines Polyamids, eines Polyethylenterephthalats, eines Polybutylenterephthalats, eines Polyetherimids, eines Polyethersulfons und/oder eines Polytrimethylenterephthalats.

In manchen Ausführungsformen können relative elastische Abschnitte der kieferorthopädischen Vorrichtung durch Direktfertigung unter Verwendung eines oder mehrerer der folgenden Materialien ausgebildet werden: eines Styrol-Blockcopolymers (SBC), eines Silikonkautschuks, einer Elastomerlegierung, eines thermoplastischen Elastomers (TPE), eines thermoplastischen Vulkanisat- (TPV-) Elastomers, eines Polyurethan-Elastomers, eines Blockcopolymer-Elastomers, eines Polyolefingemisch-Elastomers, eines thermoplastischen Copolyester-Elastomers und/oder eines thermoplastischen Polyamid-Elastomers.

Die Maschinenparameter können Härtungsparameter umfassen. Für Härtungssysteme, die auf Digital Light Processing (DLP) basieren, können die Härtungsparameter, Leistung, Härtungsdauer und/oder Graustufen des Vollbildes umfassen. Für laserbasierte Härtungssysteme können die Härtungsparameter Leistung, Geschwindigkeit, Strahlgröße, Strahlform und/oder Leistungsverteilung des Strahls umfassen. Für Drucksysteme können die Härtungsparameter Materialtropfengröße, Viskosität und/oder Härtungsleistung umfassen. Diese Maschinenparameter können als Teil der Verfahrenssteuerung der Fertigungsmaschine überwacht und regelmäßig angepasst werden (z.B. manche Parameter alle 1-x Schichten und manche Parameter nach jedem Aufbau). Verfahrenssteuerung kann durch das Einbauen eines Sensors in die Maschine erzielt werden, der Leistung und andere Strahleigenschaften für jede Schicht oder alle paar Sekunden misst und diese durch eine Feedback-Schleife automatisch anpasst. Für DLP-Maschinen können Graustufen vor, während und/oder am Ende jedes Aufbaus und/oder in vorbestimmten zeitlichen Abständen (z.B. bei jedem n-ten Aufbau, einmal pro Stunde, einmal pro Tag, einmal pro Woche usw.) abhängig von der Stabilität des Systems gemessen und kalibriert werden. Zusätzlich dazu können Materialeigenschaften und/oder Photo-Eigenschaften in Bezug auf die Fertigungsmaschine bereitgestellt werden, und ein Maschinenverfahrensteuermodul kann diese Parameter nutzen, um die Maschinenparameter (z.B. Leistung, Graustufe usw.) anzupassen, um die Variabilität von Materialeigenschaften auszugleichen. Durch das Umsetzen von Verfahrenssteuerung für die Fertigungsmaschine kann eine geringere Variabilität in Bezug auf Auftragspräzision und Eigenspannung erzielt werden.

Gegebenenfalls ermöglichen die hierin beschriebenen Direktfertigungsverfahren die Herstellung einer Vorrichtung, die mehrere Materialien umfasst, was hierin als "Multimaterial-Direktfertigung" bezeichnet wird. In manchen Ausführungsformen umfasst ein Multimaterial-Direktfertigungsverfahren das gleichzeitige Formen eines Gegenstands aus mehreren Materialien in einem einzigen Fertigungsschritt. Beispielsweise kann eine Extrusionsvorrichtung mit mehreren Düsen verwendet werden, um selektiv mehrere Materialtypen aus separaten Materialversorgungsquellen abzugeben, um ein Gegenstand aus einer Vielzahl verschiedener Materialien zu fertigen. Solche Verfahren werden in US-Patent Nr. 6.749.414 beschrieben. Alternativ dazu oder in Kombination damit kann ein Multimaterialdirektfertigungsverfahren das Ausbilden eines Gegenstands aus mehreren Materialien in einer Vielzahl aufeinanderfolgender Fertigungsschritte umfassen. Beispielsweise kann ein erster Abschnitt des Gegenstands aus einem ersten Material gemäß einem der Direktfertigungsverfahren hierin ausgebildet werden, wonach ein zweiter Abschnitt des Gegenstands durch aus einem zweiten Material gemäß den hierin beschriebenen Verfahren ausgebildet werden kann usw., bis der vollständige Gegenstand ausgebildet wurde.

Direktfertigung kann verschiedene Vorteile im Vergleich zu anderen Fertigungsverfahren bieten. Beispielsweise ermöglicht Direktfertigung im Gegensatz zu indirekter Fertigung die Produktion einer kieferorthopädischen Vorrichtung ohne die Verwendung von Formen oder Schablonen für die Formgebung der Vorrichtung, wodurch die Anzahl der Fertigungsschritte reduziert und die Auflösung und Präzision der letztendlichen Geometrie der Vorrichtung verbessert wird. Zusätzlich dazu ermöglicht Direktfertigung eine präzise Steuerung der dreidimensionalen Geometrie der Vorrichtung, wie z.B. der Dicke der Vorrichtung. Komplexe Strukturen und/oder Hilfskomponenten können einstückig mit der Vorrichtungshülle in einem einzigen Fertigungsschritt ausgebildet werden, anstatt in einem separaten Fertigungsschritt zu der Hülle hinzugefügt zu werden. In manchen Ausführungsformen wird Direktfertigung angewandt, um Vorrichtungsgeometrien zu erzeugen, die unter Anwendung alternativer Fertigungsverfahren schwierig herzustellen wären, wie z.B. Vorrichtungen mit sehr kleinen oder feinen Merkmalen, komplexe geometrische Formen, Hinterschnitte, interproximale Strukturen, Hüllen mit variabler Dicke und/oder internen Strukturen (z.B. zur Verbesserung der Festigkeit bei reduziertem Gewicht und Materialverbrauch). In manchen Ausführungsformen ermöglichen die Direktfertigungsansätze hierin beispielsweise die Fertigung einer kieferorthopädischen Vorrichtung mit Merkmalen in der Größenordnung von etwa 5 µm oder weniger oder in einem Bereich von etwa 5 µm bis etwa 50 µm oder in einem Bereich von etwa 20 µm bis etwa 50 µm.

Die Direktfertigungstechniken, die hierin beschrieben sind, können angewandt werden, um Vorrichtungen mit im Wesentlichen isotropen Materialeigenschaften herzustellen, wie z.B. mit Festigkeiten, die im Wesentlichen in allen Richtungen gleich oder ähnlich sind. In manchen Ausführungsformen ermöglichen die Direktfertigungsansätze hierin die Herstellung einer kieferorthopädischen Vorrichtung mit einer Festigkeit, die in allen Richtungen um nicht mehr als etwa 25 %, etwa 20 %, etwa 15 %, etwa 10 %, etwa 5 %, etwa 1 % oder etwa 0,5 % variiert. Zusätzlich dazu können die Direktfertigungsansätze hierin angewandt werden, um kieferorthopädische Vorrichtungen im Vergleich zu anderen Fertigungstechniken rascher herzustellen. In manchen Ausführungsformen ermöglichen die Direktfertigungsansätze hierin die Produktion einer kieferorthopädischen Vorrichtung in einem Zeitintervall von etwa 1 h, etwa 30 min, etwa 25 min, etwa 20 min, etwa 15 min, etwa 10 min, etwa 5 min, etwa 4 min, etwa 3 min, etwa 2 min, etwa 1 min oder etwa 30 s oder weniger. Solche Fertigungsgeschwindigkeiten ermöglichen eine rasche Herstellung individuell angepasster Vorrichtungen "am Behandlungsstuhl", z.B. während eines Routinetermins oder einer Kontrolle.

In manchen Ausführungsformen werden in den hierin beschriebenen Direktfertigungsverfahren Verfahrenskontrollen für verschiedene Maschinenparameter eines Direktfertigungssystems oder einer Direktfertigungsvorrichtung umgesetzt, um sicherzustellen, dass die resultierenden Vorrichtungen mit höchster Präzision gefertigt werden. Eine solche Präzision kann vorteilhaft sein, um sicherzustellen, dass das gewünschte Kraftsystem präzise auf die Zähne ausgeübt wird, um auf wirksame Weise Zahnbewegungen zu bewirken. Verfahrenskontrolle kann umgesetzt werden, um Verfahrensvariabilität aufgrund mehrerer Faktoren, wie z.B. Materialeigenschaften, Maschinenparametern, Umweltvariablen und/oder Nachbearbeitungsparametern, gerecht zu werden.

Materialeigenschaften können in Abhängigkeit von den Eigenschaften der Rohmaterialien und/oder Verfahrensvariablen während des Vermischens der Rohmaterialien variieren. In zahlreichen Ausführungsformen sollten Harze oder andere Materialien für die Direktfertigung unter strenger Verfahrenskontrolle hergestellt werden, um geringe Variabilität in Bezug auf Photoeigenschaften, Materialeigenschaften (z.B. Viskosität, Oberflächenspannung), physikalische Eigenschaften (z.B. Modul, Festigkeit, Dehnung) und/oder thermische Eigenschaften (z.B. Glasübergangstemperatur, Formbeständigkeitstemperatur) sicherzustellen. Verfahrenskontrolle für Materialfertigungsverfahren kann durch Screening der Rohmaterialien in Bezug auf ihre physikalischen Eigenschaften und/oder Steuerung von Temperatur, Feuchtigkeit und/oder anderen Verfahrensparamatern während des Mischverfahrens erzielt werden. Durch Umsetzung der Prozesskontrolle für das Materialfertigungsverfahren können eine geringere Variabilität der Verfahrensparameter und einheitliche Materialeigenschaften für jede Materialcharge erzielt werden. Eine Restvariabilität der Materialeigenschaften kann durch Verfahrenskontrolle an der Maschine erzielt werden, wie hierin ausführlicher erläutert wird.

Maschinenparameter können Härtungsparameter umfassen. Für Härtungssysteme auf der Basis von Digital Light Processing (DLP) können die Härtungsparameter die Leistung, Härtungsdauer und/oder Graustufen des Vollbilds umfassen. Für laserbasierte Härtungssysteme können die Härtungsparameter Leistung, Geschwindigkeit, Strahlgröße, Strahlform und/oder Leistungsverteilung des Strahls umfassen. Für Drucksysteme können die Härtungsparameter Materialtropfengröße, Viskosität und/ oder Härtungsleistung umfassen. Diese Maschinenparameter können als Teil der Verfahrenssteuerung der Fertigungsmaschine überwacht und regelmäßig angepasst werden (z.B. manche Parameter alle 1-x Schichten und manche Parameter nach jedem Aufbau). Verfahrenssteuerung kann durch Einbau eines Sensors in die Maschine erzielt werden, der die Leistung und andere Strahleigenschaften für jede Schicht oder alle paar Sekunden misst und diese durch eine Feedback-Schleife automatisch anpasst. Für DLP-Maschinen können Graustufen am Ende jedes Aufbaus gemessen und kalibriert werden. Zusätzlich dazu können Materialeigenschaften und/oder Photo-Eigenschaften in Bezug auf die Fertigungsmaschine bereitgestellt werden, und ein Maschinenverfahrensteuermodul kann diese Parameter nutzen, um die Maschinenparameter (z.B. Leistung, Graustufe usw.) anzupassen, um die Variabilität von Materialeigenschaften auszugleichen. Durch das Umsetzen von Verfahrenssteuerung für die Fertigungsmaschine kann eine geringere Variabilität in Bezug auf Auftragspräzision und Eigenspannung erzielt werden.

In zahlreichen Ausführungsformen werden Umweltvariablen (z.B. Temperatur, Feuchtigkeit, Sonnenlicht oder Aussetzen gegenüber anderen Energie-/Härtungsquellen) in einem engen Bereich gehalten, um die Variabilität in Bezug auf die Dicke der Vorrichtung und/oder andere Eigenschaften zu reduzieren. Gegebenenfalls können Maschinenparameter angepasst werden, um Umweltvariablen auszugleichen.

In zahlreichen Ausführungsformen umfasst die Nachbearbeitung von Vorrichtungen Reinigungs-, Nachhärtungs- und/oder Trägerentfernungsverfahren. Relevante Nachbearbeitungsparameter können Reinheit des Reinigungsmittels, Reinigungsdruck und/oder -temperatur, Reinigungsdauer, Nachhärtungsenergie und/oder -dauer und/oder Art des Trägerentfernungsverfahrens umfassen. Diese Parameter können als Teil eines Verfahrenskontrollschemas gemessen und angepasst werden. Zusätzlich dazu können die physikalischen Eigenschaften der Vorrichtung durch Modifizieren der Nachbearbeitungsparameter variiert werden. Das Anpassen von Nachbearbeitungsmaschinenparametern kann eine weitere Möglichkeit zum Ausgleich der Variabilität in Bezug auf Materialeigenschaften und/oder Maschineneigenschaften bereitstellen.

Die Konfiguration der kieferorthopädische Vorrichtungen hierin kann gemäß einem Behandlungsplan für einen Patienten bestimmt werden, z.B. gemäß einem Behandlungsplan, der die aufeinanderfolgende Behandlung mit einer Vielzahl von Vorrichtungen umfasst, die die Zähne schrittweise neu positionieren. Computer-basierte Behandlungsplanung und/oder Vorrichtungsfertigungsverfahren können angewandt werden, um die Gestaltung und Fertigung der Vorrichtungen zu erleichtern. Beispielsweise kann eine oder können mehrere der Vorrichtungskomponenten, die hierin beschrieben werden, digital gestaltet und unter Einsatz computergesteuerter Fertigungsvorrichtungen (z.B. Computer-Numerical-Control- (CNC-) Fräsen, computergesteuertes Rapid Prototyping, wie z.B. 3D-Druck, usw.) gefertigt werden. Die computerbasierten Verfahren, die hierin vorgestellt werden, können die Präzision, Flexibilität und Annehmlichkeit der Fertigung der Vorrichtungen verbessern.

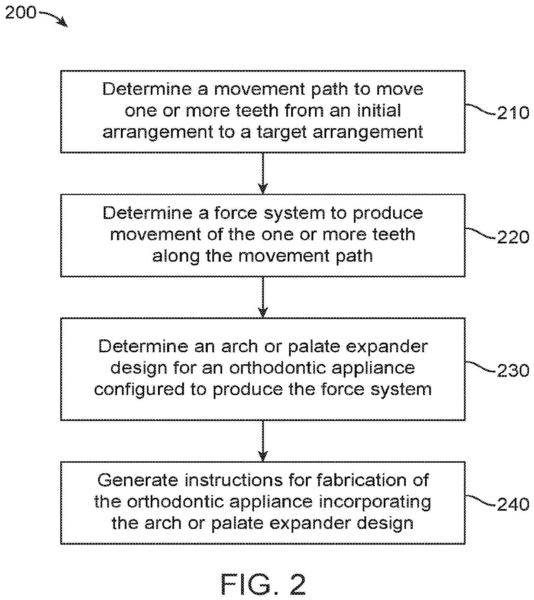

**FIG. 2** zeigt ein Verfahren 200 zur Gestaltung einer durch Direktfertigung herzustellenden kieferorthopädischen Vorrichtung gemäß den Ausführungsformen. Das Verfahren 200 kann auf jede beliebige Ausführungsform der hierin beschriebenen kieferorthopädischen Vorrichtungen angewandt werden. Manche oder alle der Schritte von Verfahren 200 können durch ein beliebiges geeignetes Datenverarbeitungssystem oder eine beliebige geeignete Datenverarbeitungsvorrichtung, z.B. einen oder mehrere Prozessoren, die durch geeignete Befehle konfiguriert sind, durchgeführt werden.

In Schritt 210 wird ein Bewegungspfad für die Bewegung eines oder mehrerer Zähne aus einer Ausgangsanordnung in eine Zielanordnung bestimmt. Die Ausgangsanordnung kann anhand eines Abdrucks oder eines Scans der Zähne oder des Mundgewebes des Patienten bestimmt werden, z.B. unter Verwendung von Wachsbissen, Direktkontaktscannen, Röntgenbildgebung, tomographischer Bildgebung, sonographischer Bildgebung und weiterer Verfahren zum Erhalt von Informationen in Bezug auf die Position und Struktur von Zähnen, Kiefer, Zahnfleisch und weiterem kieferorthopädisch relevantem Gewebe. Anhand der erfassten Daten kann ein digitaler Datensatz abgeleitet werden, die die Ausgangsanordnung der Zähne und anderen Gewebe des Patienten (z.B. vor der Bahandlung) repräsentiert. Gegebenenfalls wird der Ausgangsdatensatz verarbeitet, um die Gewebekomponenten von einander zu trennen. Beispielsweise können Datenstrukturen erzeugt werden, die einzelne Zahnkronen digital abbilden. Vorteilhafterweise können digitale Modelle ganzer Zähne, einschließlich gemessener oder extrapolierter versteckter Flächen und Wurzelstrukturen, sowie der umgebenden Knochen- und Weichgewebestrukturen erstellt werden.

Die Zielanordnung der Zähne (z.B. ein gewünschtes und beabsichtigtes Endergebnis der kieferorthopädischen Behandlung) kann vom behandelnden Arzt in Form einer Verschreibung bezogen werden, anhand grundlegender Prinzipien der Kieferorthopädie berechnet und/oder mittels Computer anhand einer klinischen Verschreibung extrapoliert werden. Mit einer Beschreibung der gewünschten Endpositionen der Zähne und einer digitalin Darstellung der Zähne selbst können die Endposition und die Oberflächengeometrie jedes Zahns angegeben werden, um ein vollständiges Modell der Zahnanordnung zum Zeitpunkt des gewünschten Endes der Behandlung zu erstellen.

Gibt es für jeden Zahn eine Ausgangs- und eine Zielposition, kann ein Bewegungspfad für die Bewegung jedes Zahnes definiert werden. In manchen Ausführungsformen sind die Bewegungspfade konfiguriert, um die Zähne möglichst schnell mit möglichst wenig Umwegen zu bewegen, um sie von ihren Ausgangspositionen in die gewünschten Endpositionen zu bringen. Die Zahnpfade können gegebenenfalls unterteilt werden, und die einzelnen Segmente können so berechnet werden, dass die Bewegung jedes Zahns innerhalb eines Segments innerhalb der Grenzwerte der linearen und Drehverschiebung bleibt. Auf diese Weise können die Endpunkte jedes Pfadsegments eine klinisch realisierbare Neupositionierung darstellen, und die Summe der Segmentendpunkte kann eine klinisch realisierbare Abfolge von Zahnpositionen darstellen, so dass die Bewegung von einem Punkt zum nächsten in der Abfolge keine Kollision von Zähnen bewirkt.

In Schritt 220 wird ein Kraftsystem bestimmt, um die Bewegung des einen Zahns oder der mehreren Zähne entlang des Bewegungspfads hervorzurufen. Ein Kraftsystem kann eine oder mehrere Kräfte und/oder ein oder mehrere Drehmomente umfassen. Verschiedene Kraftsysteme können verschiedene Arten von Zahnbewegungen hervorrufen, wie z.B. Kippen, Verschieben, Drehen, Verschieben nach außen, Verschieben nach innen, Wurzelbewegung usw. Biomechanische Grundsätze, Modellerstellungsverfahren, Kraftberechungs-/-messverfahren und dergleichen, einschließlich des Wissens und der Ansätze, die auf dem Gebiet der Kieferorthopädie üblicherweise eingesetzt werden, können angewandt werden, um das geeignete Kraftsystem zu bestimmen, das auf die Zähne anzuwenden ist, um die Zahnbewegung zu erzielen. Bei der Bestimmung des anzuwendenden Kraftsystems können Quellen einschließlich Literatur, durch Experimente oder virtuelle Modellerstellung bestimmte Kraftsysteme, computerbasierte Modellerstellung, klinische Erfahrung, die Minimierung unerwünschter Kräfte usw. in Betracht gezogen werden.

Die Bestimmung des Kraftsystems kann Einschränkungen in Bezug auf die zulässigen Kräfte umfassen, wie z.B. zulässige Richtungen und Größenordnungen, sowie erwünschte Bewegungen, die durch die ausgeübten Kräfte hervorgerufen werden sollen. Beispielsweise können in der Fertigung von Gaumenexpandern verschiedene Bewegungsstrategien für verschiedene Patienten wünschenswert sein. Das Ausmaß an Kraft, das erforderlich ist, um den Gaumen zu erweitern, kann beispielsweise vom Alter des Patienten abhängig sein, da sehr junge Patienten möglicherweise noch keine vollständig ausgebildete Gaumennaht haben. Demnach kann bei jugendlichen Patienten und solchen mit nicht vollständig geschlossener Gaumennaht die Erweiterung des Gaumens mit geringeren Kraftgrößenordnungen erzielt werden. Eine langsamere Bewegung des Gaumens kann dabei helfen, dass Knochen wächst, der die sich erweiternde Naht füllt. Bei anderen Patienten kann eine raschere Erweiterung wünschenswert sein, was durch die Anwendung größerer Kräfte erzielt werden kann. Diese Anforderungen können nach Bedarf berücksichtigt werden, um die Struktur und Materialien für Vorrichtungen auszuwählen; beispielsweise durch die Auswahl von Gaumenexpandern, die in der Lage sind, starke Kräfte auszuüben, um die Gaumennaht aufzubrechen und/oder eine rasche Erweiterung des Gaumens hervorzurufen. Spätere Vorrichtungsstufen können so gestaltet sein, dass sie verschiedene Kraftausmaße ausüben, wobei z.B. zunächst eine große Kraft angewandt wird, um die Naht aufzubrechen, und dann kleinere Kräfte angewandt werden, um die Naht offen zu halten oder den Gaumen und/oder den Gaumenbogen allmählich zu erweitern.

Das Bestimmen des Kraftsystems kann eine Modellerstellung der Gesichtsstrukturen des Patienten umfassen, wie z.B. der Skelettstruktur von Kiefer und Gaumen. Scandaten des Gaumens und des Bogens, wie z.B. Röntgendaten oder Daten von optischem 3D-Scannen, können verwendet werden, um Parameter des Skelett- und Muskelsystems des Munds des Patienten zu ermitteln, um Kräfte zu bestimmen, die ausreichen, um für eine gewünschte Expansion des Gaumens und/oder Bogens zu sorgen. In manchen Ausführungsformen kann die Dicke und/oder Dichte der Naht in der Mitte des Gaumens gemessen oder durch einen behandelnden Arzt eingegeben werden. In anderen Ausführungsformen kann der behandelnde Arzt eine geeignete Behandlung auf Grundlage von physiologischen Eigenschaften des Patienten festlegen. Beispielsweise können die Eigenschaften des Gaumens auch aufgrund von Faktoren wie dem Alter des Patienten eingeschätzt werden; beispielsweise brauchen junge jugendliche Patienten typischerweise geringere Kräfte, um die Naht zu erweitern, als ältere Patienten, da die Naht bei den jüngeren Patienten noch nicht vollständig ausgebildet ist.

In Schritt 230 wird die Gestaltung eines Bogen- oder Gaumenexpanders für eine kieferorthopädische Vorrichtung ermittelt, die konfiguriert ist, um das Kraftsystem zu erzeugen. Die Bestimmung der Gestaltung des Bogen- oder Gaumenexpanders, die Geometrie der Vorrichtung, die Materialzusammenseztung und/oder -eigenschaften kann unter Anwendung einer Behandlungs- oder Kraftanwendungssimulationsumgebung durchgeführt werden. Eine Simulationsumgebung kann z.B. Computermodellerstellungssysteme, biomechanische Systeme oder Vorrichtungen und dergleichen umfassen. Gegebenenfalls können digitale Modelle der Vorrichtung und/oder der Zähne erzeugt werden, wie z.B. Finite-Elemente-Modelle. Die Finite-Elemente-Modelle können unter Verwendung einer Computerprogrammanwendungssoftware erstellt werden, die von verschiedenen Anbietern bezogen werden können. Zur Herstellung von soliden Geometriemodellen können computergestützte Engineering- (CAE-) oder computergestützte Design- (CAD-) Programme verwendet werden, wie z.B. die Auto-CAD®-Softwareprodukt von Autodesk, Inc., aus San Rafael, CA. Zum Erstellen von Finite-Elemente-Modellen und deren Analyse können Programmprodukte von verschiedenen Anbietern verwendet werden, einschließlich die Finite-Elemente-Analysepakete von ANSYS, Inc., aus Canonsburg, PA, und SIMULIA- (Abaqus-) Softwareprodukte von Dassault Systemes aus Waltham, MA.

Gegebenenfalls können ein oder mehrere Bogen- oder Gaumenexpanderdesigns für Tests oder Kraftmodellerstellung ausgewählt werden. Wie oben angemerkt können eine gewünschte Zahnbewegung sowie ein für das Hervorrufen der gewünschte Zahnbewegung erforderliches oder gewünschtes Kraftsystem identifiziert werden. Unter Nutzung der Simulationsumgebung kann ein Kandidaten-Bogen- oder Gaumenexpanderdesign analysiert werden, oder ein entsprechendes Modell kann erstellt werden, um ein tatsächliches Kraftsystem zu bestimmen, das aus der Verwendung der Kandidatenvorrichtung resultiert. Eine oder mehrere Modifikationen können gegebenenfalls an einer Kandidatenvorrichtung vorgenommen werden, und eine Kraftmodellerstellung kann weiter wie beschrieben analysiert werden, z.B. um die Gestaltung der Vorrichtung, die das gewünschte Kraftsystem erzeugt, schrittweise festzulegen.

In Schritt 240 werden Befehle für die Fertigung der kieferorthopädischen Vorrichtung, die das Bogen- oder Gaumenexpanderdesign umfasst, erstellt. Die Befehle können so konfiguriert sein, dass ein Fertigungssystem oder eine Fertigungsvorrichtung so gesteuert wird, dass die kieferorthopädische Vorrichtung mit dem festgelegten Bogen- oder Gaumenexpanderdesign erzeugt wird. In manchen Ausführungsformen sind die Befehle so konfiguriert, dass die kieferorthopädische Vorrichtung durch Direktfertigung (z.B. Stereolithographie, selektives Laser-Sintern, Fused Deposition Modeling, 3D-Druck, kontinuierliche Direktfertigung, Direktfertigung aus mehreren Materialien usw.) gemäß den verschiedenen hierin vorgestellten Verfahren erzeugt wird. In alternativen Ausführungsformen können die Befehle für die indirekte Fertigung der Vorrichtung, z.B. durch Thermoformen, konfiguriert sein.

Verfahren 200 kann zusätzliche Schritte umfassen: 1) Der obere Bogen und Gaumen des Patienten wird intraoral gescannt, um dreidimensionale Daten des Gaumens und des oberen Bogens zu erfassen. 2) Das dreidimensionale Formprofil der Vorrichtung wird ermittelt, um Abstands- und Zahneingriffsstrukturen wie hierin beschrieben bereitzustellen.

Wenngleich die oben beschriebenen Schritte ein Verfahren 200 zur Gestaltung einer kieferorthopädischen Vorrichtung gemäß manchen Ausführungsformen veranschaulichen, sind für Fachleute auf dem Gebiet der Erfindung auf Grundlage der hierin beschriebenen Lehre einige Variationen ersichtlich. Manche der Schritte können untergeordnete Schritte umfassen. Manche der Schritte können beliebig oft wiederholt werden. Einer oder mehrere Schritte des Verfahrens 200 können mit einem beliebigen Fertigungssystem oder einer beliebigen Fertigungsvorrichtung durchgeführt werden, wie z.B. mit den hierin beschriebenen Ausführungsformen. Manche der Schritte können optional sein, und die Reihenfolge der Schritte kann nach Wunsch variiert werden.

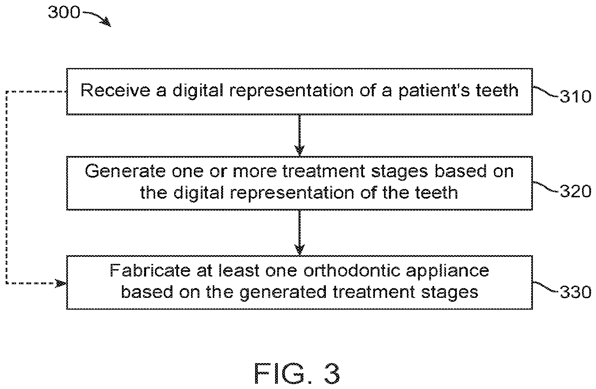

**FIG. 3** zeigt ein Verfahren 300 zur digitalen Planung einer kieferorthopädischen Behandlung und/oder zur Gestaltung oder Fertigung einer Vorrichtung gemäß den Ausführungsformen. Das Verfahren 300 kann auf ein beliebiges der hierin beschriebenen Behandlungsverfahren angewandt werden und durch ein beliebiges geeignetes Datenverarbeitungssystem durchgeführt werden.

In Schritt 310 wird eine digitale Darstellung der Zähne eines Patienten erhalten. Die digitale Darstellung kann Oberflächentopographiedaten für den Mundraum des Patienten umfassen (einschließlich Zähne, Zahnfleisch usw.). Die Oberflächentopographiedaten können durch direktes Scannen der Mundhöhle, anhand eines physischen Modells (positive oder negative) der Mundhöhle oder durch einen Abdruck der Mundhöhle unter Verwendung einer geeigneten Scanvorrichtung (z.B. eines handgeführten Scanners, eines Desktop-Scanners usw.) erfasst werden.

In Schritt 320 werden eine oder mehrere Behandlungsstufen auf Grundlage der digitalen Darstellung der Zähne erstellt. Die Behandlungsstufen können schrittweise Repositionierungsstufen eines kieferorthopädischen Behandlungsverfahrens sein, das so gestaltet ist, dass ein oder mehrere der Zähne des Patienten aus einer Ausgangsanordnung in eine Zielanordnung bewegt werden. Beispielsweise können die Behandlungsstufen durch Bestimmen der Ausgangszahnanordnung, die durch die digitale Darstellung angegeben ist, die Bestimmung einer Zielzahnanordnung und das Bestimmen von Bewegungspfaden eines oder mehrerer Zähne in der Ausgangsanordnung, die zum Erreichen der Zielanordnung der Zähne notwendig ist, festgelegt werden. Der Bewegungspfad kann auf Grundlage des Minimierens der Gesamtdistanz der Bewegung optimiert werden, wobei Kollisionen zwischen Zähnen vermieden werden, Zahnbewegungen vermieden werden, die zu schwierig zu bewerkstelligen sind, oder andere geeignete Kriterien angewandt werden.

In Schritt 330 wird zumindest eine kieferorthopädische Vorrichtung auf Grundlage der ermittelten Behandlungsstufen gefertigt. Beispielsweise kann ein Satz von Vorrichtungen gefertigt werden, die jeweils gemäß einer durch die Behandlungsstufen festgelegten Zahnanordnung entsprechen, so dass die Vorrichtungen nacheinander vom Patienten getragen werden können, um schrittweise die Zähne aus der Ausgangsanordnung in die Zielanordnung neu zu positionieren. Der Vorrichtungssatz kann eine oder mehrere der hierin beschriebenen kieferorthopädischen Vorrichtungen umfassen. Die Fertigung der Vorrichtung kann das Gestalten eines digitalen Modells der Vorrichtung umfassen, das als Eingabe für ein computergesteuertes Fertigungssystem verwendet wird. Die Vorrichtung kann unter Anwendung von Direktfertigungsverfahren, indirekten Fertigungsverfahren oder Kombinationen davon nach Wunsch gefertigt werden.

In manchen Fällen kann es sein, dass es nicht erforderlich ist, mehrere Anordnungen oder Behandlungsstufen durchzuführen, um eine Vorrichtung zu gestalten und/oder zu fertigen. Wie durch die gestrichelte Linie in **FIG. 3** dargestellt, können die Gestaltung und/oder Fertigung einer kieferorthopädischen Vorrichtung und möglicherweise eine bestimmte kieferorthopädische Behandlung die Verwendung einer Darstellung der Zähne des Patienten umfassen (z.B. das Empfangen einer digitalen Darstellung der Zähne des Patienten 310), gefolgt von der Gestaltung und/oder Fertigung einer kieferorthopädischen Vorrichtung auf Grundlage einer Darstellung der Zähne des Patienten in der durch die empfangene Darstellung dargestellten Anordnung.

### BEISPIELE

Die folgenden Beispiele werden zum Zweck der Veranschaulichung verschiedener Ausführungsformen der Erfindung angegeben und sollen die vorliegende Offenbarung in keiner Weise einschränken. Die vorliegenden Beispiele sind zusammen mit den hierin beschriebenen Verfahren in der gegebenen Form repräsentativ für manche Ausführungsformen, sind beispielhaft und sind nicht als Einschränkungen des Schutzumfangs der Erfindung zu verstehen. Änderungen daran und andere Verwendungen, die im Geiste der Erfindung, wie durch den Schutzumfang der Ansprüche definiert, umfasst sind, erschließen sich Fachleuten auf dem Gebiet der Erfindung.

Alle Chemikalien wurden aus kommerziellen Quellen erstanden und ohne Reinigung eingesetzt, wenn nicht anders angegeben.

¹H-NMR- und ¹³C-NMR-Spektren wurden auf einem BRUKER-AC-E-200-FT-NMR-Spektrometer oder einem BRUKER-Avance-DRX-400-FT-NMR-Spektrometer aufgenommen. Die chemischen Verschiebungen sind in ppm (s: Singulett, d: Dublett, t: Triplett, q: Quartett, m: Multiplett) angegeben. Die verwendeten Lösungsmittel waren deuteriertes Chloroform (CDCl₃, 99,5 % Deuterierung) und deuteriertes DMSO (d₆-DMSO, 99,8 % Deuterierung).

### Referenzbeispiel 1: Synthese von Phenyl-2-(methacryloyloxy)benzoat (Ref1).

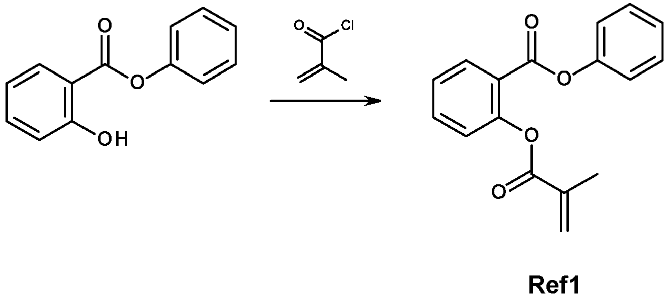

4,28 g (20 mmol) Phenylsalicylat und 3,34 g (33 mmol) Triethylamin wurden in 50 ml trockenem CH₂Cl₂ in einem 100-ml-Dreihalsrundkolben gelöst, gründlich mit Ar gespült und auf -15 °C abgekühlt. Währenddessen wurden 2,30 g (22 mmol) frisch destilliertes Methacryloylchlorid in 20 ml trockenem CH₂Cl₂ gelöst, auch mit Ar gespült und dann innerhalb 1 h zu dem Reaktionsgemisch zugetropft, ohne eine Temperatur von 0 °C zu überschreiten. Als die Zugabe abgeschlossen war, wurde das Reaktionsgemisch 1 h lang bei einer Temperatur unter 0 °C gerührt und dann langsam auf Raumtemperatur erwärmt und 18 h lang gerührt, wonach sich ein Niederschlag gebildet hatte, der abfiltriert wurde. Das Filtrat wurde zweimal mit Wasser, dreimal mit wässriger 10%iger HCl und zweimal mit Kochsalzlösung gewaschen. Dann wurde die organische Phase über Na₂SO₄ getrocknet, durch ein Silicabett filtriert und mit 250 ppm BHT stabilisiert, und dann wurde das Lösungsmittel abgedampft. Die resultierenden weißen Kristalle wurden aus Diethylether umkristallisiert, um 5,10 g (90 %) **Ref1** zu erhalten. Fp.: 99-100 °C; ¹H-NMR (200 MHz, CDCl₃; δ, ppm): 8,22 (d, 1H; Ar-H), 7,63 (t, 1H; Ar-H), 7,37 (m, 3H; Ar-H), 7,27-7,11 (m, 4H; Ar-H), 6,36 (s, 1H; =CH₂), 5,70 (m, 1H; =CH₂), 2,03 (s, 3H; CH₂=CH₂-CH₃); APT-¹³C-NMR (50,3 MHz, CDCl₃; δ, ppm): 166,0 (C4), 163,2 (C4), 151,3 (C4), 150,7 (C4), 135,6 (C4), 134,5 (C3), 132,4 (C3), 129,6 (C3), 127,9 (C2), 126,2 (C3), 126,1 (C1), 124,2 (C3), 123,1 (C4), 121,8 (C3), 18,5 (C1).

### Referenzbeispiel 2: Synthese von Methyl-2-(methacryloyloxy)benzoat (Ref2).

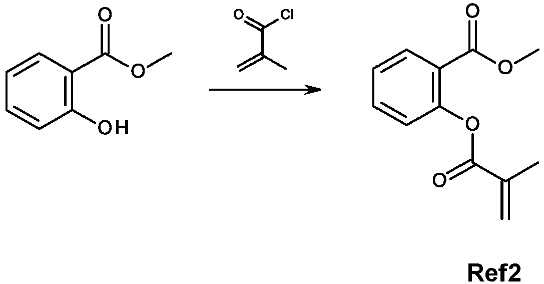

Die Herstellung war ähnlich zu Referenzbeispiel 1, wobei 3,04 g (20 mmol) Methylsalicylat eingesetzt wurden, mit Ausnahme der Reinigung des Rohprodukts nach Abdampfen des Lösungsmittels, die mittels Silica-Chromatographie (Elutionsmittel PE:EE = 6:1) durchgeführt wurde, um 3,72 g (84 %) **Ref2** als transparente Flüssigkeit zu erhalten. ¹H-NMR (200 MHz, CDCl₃; δ, ppm): 8,03 (d, 1H; Ar-H), 7,55 (t, 1H; Ar-H), 7,30 (t, 1H; Ar-H), 7,15 (d, 1H; Ar-H), 6,37 (s, 1H; =CH₂), 5,77 (m, 1H; =CH₂), 3,82 (s, 3H; COO-CH₃), 2,09 (s, 3H; CH₂=CH₂-CH₃); APT-¹³C-NMR (50,3 MHz, CDCl₃, δ, ppm): 166,0 (C4), 165,0 (C4), 150,9 (C4), 135,8 (C4), 133,8 (C3), 131,8 (C3), 127,4 (C2), 126,0 (C3), 123,9 (C3), 123,4 (C4), 52,2 (C3), 18,4 (C1).

### Referenzbeispiel 3: Synthese von 2-Ethylhexyl-2-(methacryloyloxy)benzoat (Ref3).

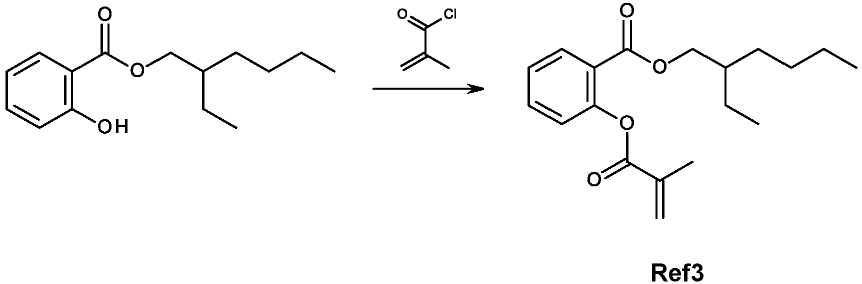

Die Herstellung war ähnlich zu Referenzbeispiel 1, wobei 5,02 g (20 mmol) 2-Ethylhexylsalicylat eingesetzt wurden, einschließlich der Reinigung des Rohprodukts nach Abdampfen des Lösungsmittels mittels Silica-Chromatographie (Elutionsmittel PE:EE = 6:1), um 5,29 g (83 %) **Ref3** als transparente Flüssigkeit zu erhalten. ¹H-NMR (200 MHz, CDCl₃, δ, ppm): 8,01 (d, 1H; Ar-H), 7,50 (t, 1H; Ar-H), 7,28 (t, 1H; Ar-H), 7,11 (d, 1H; Ar-H), 6,37 (d, 1H; =CH₂), 5,75 (d, 1H; =CH₂), 4,16 (d, 2H; COO-CH₂-), 2,07 (s, 3H; CH₂=CH₂-CH₃), 1,64 (m, 1H, COO-CH₂-CH-(CH₂)₂), 1,42-1,25 (m, 8H), 0,88 (t, 6H; -CH₂-CH₃); APT-¹³C-NMR (50,3 MHz, CDCl₃, δ, ppm): 165,9 (C4), 164,7 (C4, CO), 150,9 (C4, CO), 135,8 (C4), 133,6 (C1), 131,7 (C1), 129. (C4), 127,5 (C2), 125,9 (C1), 123,9 (C1), 67,6 (C2), 38,8 (C3), 30,4 (C2), 29,0 (C2), 23,8 (C2), 23,0 (C3), 14,1 (C1), 11,0 (C1).

### Referenzbeispiel 4: Isobornylmethacrylat (Ref4).

Als Referenzbeispiel 4 wurde im Handel erhältliches Isobornylmethacrylat (**Ref4**) von Sigma Aldrich erstanden.

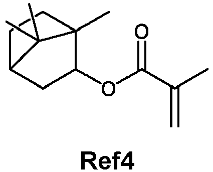

### Referenzbeispiel 5: Benzylmethacrylat (Ref5).

**Als** Referenzbeispiel 5 wurde Benzylmethacrylat **(Ref5)** von TCI erstanden.

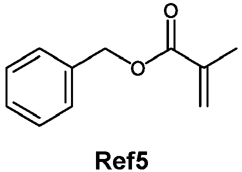

### Beispiel 1: Synthese von Cyclopentyl-2-(methacryloyloxy)benzoat (Cyclopentylsalicylatmethacrylat) (1).

### Stufe 1: Herstellung von Cyclopentylsalicylat.

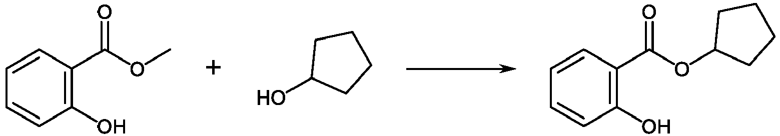

3,45 g (40 mmol) Pentanol und 1,15 g (50 mmol) metallisches Natrium wurden unter Rühren auf 130 °C erhitzt, bis eine Homogenität der Schmelze erreicht war. Dann wurden 4,57 g (30 mmol) Methylsalicylat zugesetzt. Methanol, das sich als Nebenprodukt gebildet hatte, wurde abdestilliert. Nach abgeschlossener Destillation wurde die Reaktion auf Raumtemperatur abkühlen gelassen. Der resultierende amorphe, rotbräunliche Feststoff wurde in CH₂Cl₂ gelöst und unlösliche Komponenten nach 4 h abfiltriert. Das Lösungsmittel wurde abgedampft, um 6,24 g eines rotbräunlichen Rohprodukts zu erhalten, das mittels Silica-Chromatographie (Elutionsmittel PE:EE = 6:1) gereinigt wurde, was 1,92 g (30 %) Cyclopentylsalicylat als transparente Flüssigkeit ergab. ¹H-NMR (400 MHz, CDCl₃, δ, ppm): 10,92 (s, 1H; Ar-OH) 7,81 (d, 1H; Ar-H), 7,43 (t, 1H; Ar-H), 6,97 (d, 1H; Ar-H), 6,86 (t, 1H; Ar-H), 5,43 (m, 1H; COO-CH-), 2,01-1,57 (m, 8H); APT-¹³C-NMR (100,6 MHz, CDCl₃, δ, ppm): 170,1 (C4, CO), 161,8 (C4, CO), 135,5 (C3), 130,0 (C3), 119,1 (C3), 117,6 (C3), 113,1 (C4), 78,5 (C3), 48,7 (C4), 32,8 (C2), 23,9 (C2).

### Stufe 2: Herstellung von Cyclopentylsalicylatmethacrylat (1).

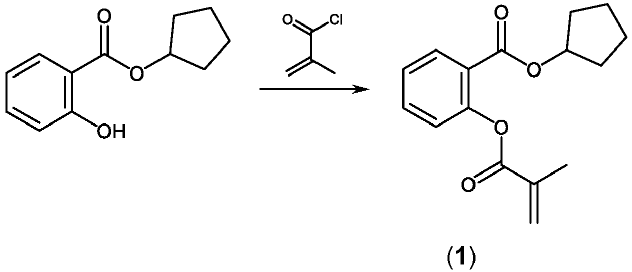

Die Herstellung war ähnlich zu Referenzbeispiel 1, wobei 1,84 g (9 mmol) Cyclopentylsalicylat eingesetzt wurden und das Rohprodukt mittels Silica-Chromatographie (Elutionsmittel PE:EE = 6:1) gereinigt wurde, was 2,15 g (87 %) der Titelverbindung **(1)** als transparente Flüssigkeit ergab, die vor dem Abdampfen der Lösungsmittel durch Zusetzen von 250 ppm BHT stabilisiert worden war. ¹H-NMR (200 MHz, CDCl₃, δ, ppm): 8,00 (d, 1H; Ar-H), 7,53 (t, 1H; Ar-H), 7,29 (t, 1H; Ar-H), 7,12 (d, 1H; Ar-H), 6,39 (s, 1H; =CH₂), 5,78 (s, 1H; =CH₂), 5,33 (s, 1H; COO-CH-), 2,08 (s, 3H; CH₂=CH₂-CH₃), 1,94-1,50 (m, 8H).

### Beispiel 2: Synthese von Cyclohexyl-2-(methacryloyloxy)benzoat (Cyclohexylsalicylatmethacrylat) (2).

### Stufe 1: Herstellung von Cyclohexylsalicylat.

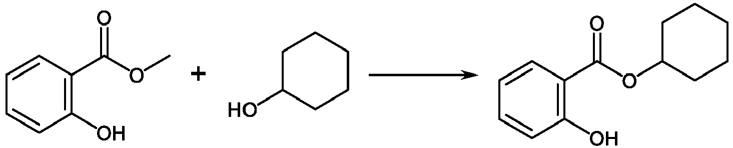

Cyclohexylsalicylat wurde gemäß der Synthesevorschrift von Beispiel **(1)** (Stufe 1) durch Umsetzen von 4,00 g (40 mmol) Cyclohexanol, 1,15 g (50 mmol) metallischem Natrium und 6,85 g (45 mmol) Methylsalicylat hergestellt. Das bräunliche Rohprodukt wurde mittels Silica-Chromatographie (Elutionsmittel PE:EE = 6:1) gereinigt, was 3,80 g (33 %) Cyclohexylsalicylat als transparente Flüssigkeit ergab. ¹H-NMR (400 MHz, CDCl₃, δ, ppm): 10,94 (s, 1H; Ar-OH) 7,87 (d, 1H; Ar-H), 7,44 (t, 1H; Ar-H), 6,98 (d, 1H; Ar-H), 6,87 (t, 1H; Ar-H), 5,07 (sept, 1H; COO-CH-), 1,98-1,93 (m, 2H), 1,84-1,78 (m, 2H), 1,66-1,33 (m, 6H); APT-¹³C-NMR (100,6 MHz, CDCl₃, δ, ppm): 169,8 (C4, CO), 161,8 (C4, CO), 135,6 (C3), 130,0 (C3), 119,1 (C3), 117,6 (C3), 113,2 (C4), 73,9 (C3), 31,6 (C2), 25,5 (C2), 23,7 (C2).

### Stufe 2: Herstellung von Cyclohexylsalicylatmethacrylat (2).

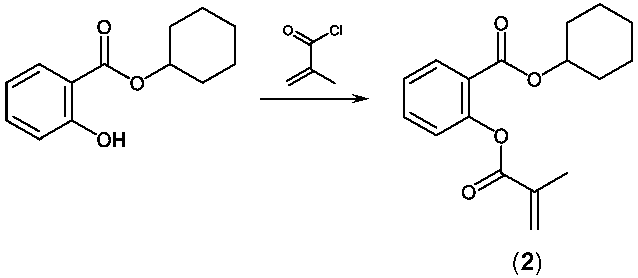

Die Herstellung war ähnlich zu Referenzbeispiel 1, wobei 3,30 g (15 mmol) Cyclohexylsalicylat, 16 mmol Methacryloylchlorid und 22 mmol Triethylamin eingesetzt wurden. Reinigung des Rohprodukts wurde mittels Silica-Chromatographie (Elutionsmittel PE:EE = 6:1) durchgeführt, was 3,45 g (80 %) der Titelverbindung **(2)** als transparente Flüssigkeit ergab, die vor dem Abdampfen der Lösungsmittel durch Zusetzen von 250 ppm BHT stabilisiert worden war. ¹H-NMR (400 MHz, CDCl₃, δ, ppm): 8,03 (d, 1H; Ar-H), 7,55 (t, 1H; Ar-H), 7,32 (t, 1H; Ar-H), 7,13 (d, 1H; Ar-H), 6,40 (s, 1H; =CH₂), 5,80 (s, 1H; =CH₂), 4,95 (sept, 1H; COO-CH-), 2,10 (s, 3H; CH₂=CH₂-CH₃), 1,95-1,91 (m, 2H), 1,78-1,75 (m, 2H), 1,62-1,24 (m, 6H); APT-¹³C-NMR (100,6 MHz, CDCl₃, δ, ppm): 165,9 (C4, CO), 164,2 (C4, CO), 150,7 (C4), 135,9 (C4), 133,5 (C3), 131,9 (C3), 127,7 (C2), 126,0 (C3), 124,5 (C4), 123,8 (C3), 73,7 (C3), 31,7 (C2), 25,5 (C2), 24,0 (C2), 18,5 (C1).

### Beispiel 3: Synthese von 2-Isopropyl-5-methylcyclohexyl-2-(methacryloyloxy)-benzoat (Menthylsalicylatmethacrylat) (3).

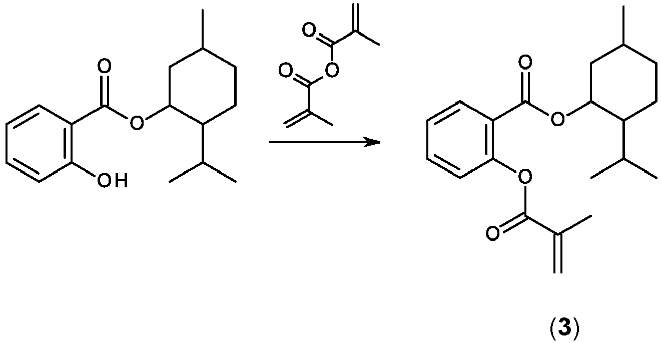

5,55 g Menthylsalicylat (20 mmol), 5,27 g Methacrylsäureanhydrid (34 mmol) und 0,12 g Dimethylaminopyridin (1 mmol, DMAP) wurden in einen 50-ml-Rundkolben gefüllt. Der Kolben wurde mit Ar gespült, auf 120 °C (Ölbadtemperatur) erhitzt und 24 h lang gerührt. Die Reaktion wurde mittels DC überwacht. Bei vollständigem Umsatz wurden das überschüssige Methacrylsäureanhydrid und Nebenprodukte im Vakuum abdestilliert. Das Rohprodukt wurde mittels Silica-Chromatographie (Elutionsmittel PE:EE = 6:1) gereinigt, um 5,67 g (83 %) der Titelverbindung **(3)** als transparente Flüssigkeit zu erhalten, die unter Verwendung von 250 ppm BHT stabilisiert wurde. ¹H-NMR (200 MHz, CDCl₃, δ, ppm): 8,01 (d, 1H; Ar-H), 7,52 (t, 1H; Ar-H), 7,29 (t, 1H; Ar-H), 7,29 (d, 1H; Ar-H), 6,39 (d, 1H; =CH₂), 5,78 (d, 1H; =CH₂), 4,87 (q, 1H; COO-CH-(CH₂)₂), 2,08 (s, 3H; CH₂=CH₂-CH₃), 1,93-1,00 (m, 9H) 0,84 (d, 6H; (CH₂)₂-CH-(CH₃)₂), 0,75 (d, 3H; (CH₂)₂-CH-CH₃); APT-¹³C-NMR (50,3 MHz, CDCl₃, δ, ppm): 165,7 (C4, CO), 164,2 (C4, CO), 150,6 (C4), 135,7 (C4), 133,4 (C3), 131,7 (C3), 128,9 (C4), 127,4 (C2), 125,9 (C3), 123,8 (C3), 74,9 (C3), 47,1 (C3), 40,8 (C2), 34,3 (C2), 31,4 (C3), 26,1 (C3), 23,3 (C2), 22,0 (C1), 20,8 (C1), 18,4 (C1), 16,1 (C1).

### Beispiel 4: Synthese von 2-Isopropyl-5-methylcyclohexyl-3-(methacryloyloxy)-benzoat (Menthyl-3-(methacryloyloxy)benzoat) (4).

### Stufe 1: Herstellung von 3-(Methacryloyloxy)benzoesäure.

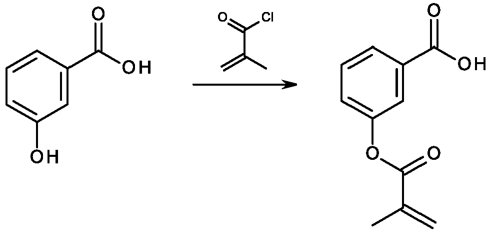

Zunächst wurde eine basische Lösung von 2,40 g (60 mmol) Natriumhydroxid in 50 ml entionisiertem Wasser hergestellt, worin 4,15 g (30 mmol) Benzoesäure gelöst wurden. Zu dieser Lösung wurde eine Lösung von 3,46 g (33 mmol) frisch destilliertem Methacryloylchlorid in 20 ml trockenem Dioxan unter 30-minütigem heftigem Rühren zugetropft. Als die Zugabe abgeschlossen war, wurde die Reaktion bei Raumtemperatur 5 h lang gerührt und mit Salzsäure (10 %) angesäuert, wobei ein weißer Feststoff ausfiel, der abfiltriert, mit Salzsäure (10 %, 1 x 50 ml) und warmem, entionisiertem Wasser (3 x 50 ml) gewaschen und schließlich aus Ethanol umkristallisiert wurde, um 4,21 g (68 %) der Titelverbindung als weißen Feststoff zu erhalten. ¹H-NMR (400 MHz, DMSO-d₆, δ, ppm): 13,00 (s, 1H; -COOH), 7,98 (d, 1H; Ar-H), 7,77 (t, 1H; Ar-H), 7,46 (t, 1H; Ar-H), 7,28 (m, 1H; Ar-H), 6,38 (s, 1H; =CH₂), 5,77 (t, 1H; =CH₂), 2,07 (s, 3H; CH₂=CH₂-CH₃); APT-¹³C-NMR (100,6 MHz, CDCl₃, δ, ppm): 166,5 (C4, CO), 165,2 (C4, CO), 150,6 (C4), 135,1 (C4), 132,4 (C3), 129,9 (C3), 128,0 (C2), 126,7 (C3), 126,4 (C4), 122,6 (C3), 18,0 (C1).

### Stufe 2: Herstellung von Menthyl-3-(methacryloyloxy)benzoat (4).

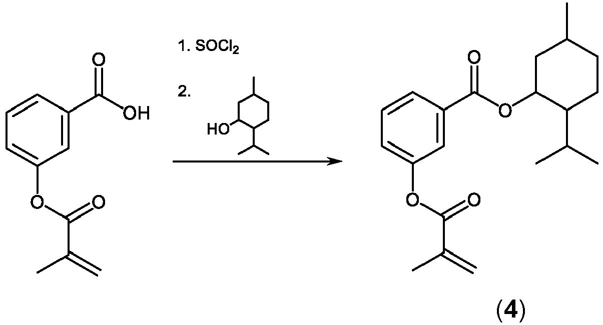

3-(Methacryloyloxy)benzoesäure (2,49 g, 12 mmol) wurde in 20 ml Thionylchlorid gelöst, und ein Tropfen DMF wurde zugesetzt. Diese Lösung wurde 3 h lang rückflusserhitzt, woraufhin das überschüssige Thionylchlorid abdestilliert wurde. Das so erhaltene 3-(Methacryloyloxy)benzoylchlorid wurde in trockenem Dichlormethan (20 ml) gelöst und zu einer Lösung von 3,3,5-Trimethylcyclohexanol (3,13 g, 20 mmol) und Pyridin (3,2 g, 40 mmol) in 50 ml trockenem Dichlormethan bei 0 °C zugetropft. Nach dem Zusetzen wurde die Reaktion auf Raumtemperatur erwärmt und über Nacht gerührt. Ein weißer Niederschlag, der sich gebildet hatte, wurde abfiltriert, und das Filtrat wurde mit Salzsäure (10 %, 3 x 30 ml), entionisiertem Wasser (1 x 30 ml) und Kochsalzlösung (2 x 30 ml) gewaschen. Die organische Phase wurde über Na₂SO₄ getrocknet, filtriert und bis zur Trockene eingeengt. Das Rohprodukt wurde mittels Säulenchromatographie gereinigt (SiO₂, PE:EE = 6:1), um 2,02 g (49 %) (**4**) als transparente Flüssigkeit zu erhalten. ¹H-NMR (400 MHz, CDCl₃, δ, ppm): 7,95 (d, 1H; Ar-H), 7,77 (t, 1H; Ar-H), 7,46 (t, 1H; Ar-H), 7,30 (m, 1H; Ar-H), 6,37 (s, 1H; =CH₂), 6,58 (t, 1H; =CH₂), 4,96 (m, 1H; COO-CH-), 2,06 (s, 3H; CH₂=CH₂-CH₃), 2,00-0,77 (m, 18H); APT-¹³C-NMR (100,6 MHz, CDCl₃, δ, ppm): 165,7 (C4, CO), 165,2 (C4, CO), 151,0 (C4), 135,7 (C4), 132,5 (C4), 129,4 (C3), 127,7 (C2), 127,0 (C3), 126,3 (C3), 122,9 (C3), 75,2 (C3), 47,3 (C3), 41,1 (C2), 34,4 (C2), 31,5 (C3), 26,5 (C3), 23,6 (C2), 22,1 (C1), 20,9 (C1), 18,4 (C1), 16,6 (C1).

### Beispiel 5: Synthese von 2-Isopropyl-5-methylcyclohexyl-4-(methacryloyloxy)-benzoat (Menthyl-4-(methacryloyloxy)benzoat) (5).

### Stufe 1: Herstellung von 4-(Methacryloyloxy)benzoesäure.

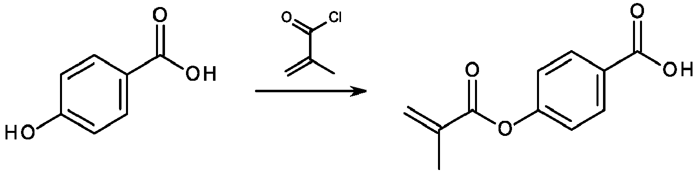

Die Herstellung war ähnlich zu Beispiel 4 (Stufe 1), wobei 4,14 g (30 mmol) 4-Hydroxybenzoesäure eingesetzt wurden und das Rohprodukt durch Umkristallisation aus Ethanol gereinigt wurde, was 4,01 g (65 %) der Titelverbindung als weißen Feststoff ergab. ¹H-NMR (400 MHz, DMSO-d₆, δ, ppm): 13,04 (s, 1H; -COOH), 8,03 (d, 2H; Ar-H), 7,33 (d, 2H; Ar-H), 6,30 (s, 1H; =CH₂), 5,93 (t, 1H; =CH₂), 2,10 (s, 3H; CH₂=CH₂-CH₃); APT-¹³C-NMR (100,6 MHz, DMSO-d₆, δ, ppm): 166,6 (C4, CO), 164,9 (C4, CO), 154,1 (C4), 135,7 (C4), 130,9 (C3), 128,4 (C2), 128,2 (C4), 122,0 (C3), 18,0 (C1).

### Stufe 2: Herstellung von Homomenthyl-4-(methacryloyloxy)benzoat (5).

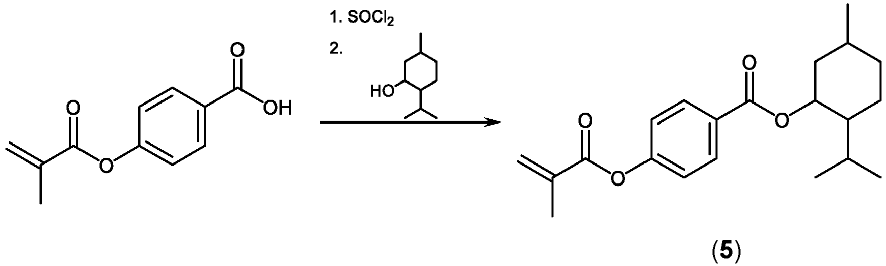

Die Herstellung war ähnlich zu Beispiel 4 (Stufe 2), wobei 2,51 g (12 mmol) 4-(Methacryloyloxy)benzoesäure eingesetzt wurden und das Rohprodukt durch Säulenchromatographie (SiO₂, PE:EE = 6:1) gereinigt wurde, was 2,71 g (66 %) **(5)** als farblose transparente Flüssigkeit ergab. ¹H-NMR (400 MHz, CDCl₃, δ, ppm): 8,12 (d, 2H; Ar-H), 7,22 (d, 2H; Ar-H), 6,37 (s, 1H; =CH₂), 5,79 (t, 1H; =CH₂), 4,95 (m, 1H; COO-CH-), 2,07 (s, 3H; CH₂=CH₂-CH₃), 2,00-0,78 (m, 18H); APT-¹³C-NMR (100,6 MHz, CDCl₃, δ, ppm): 165,4 (C4, 2xCO*), 154,5 (C4), 135,7 (C4), 131,2 (C3), 128,4 (C2), 127,8 (C4), 121,7 (C3), 75,1 (C3), 47,4 (C3), 41,1 (C2), 34,4 (C2), 31,5 (C3), 26,6 (C3), 23,7 (C2), 22,1 (C1), 20,9 (C1), 18,4 (C1), 16,6 (C1). (*Überlappung der 2 CO-Peaks wurde mittels HMQC-NMR bestätigt.).

### Beispiel 6: Synthese von 3,3,5-Trimethylcyclohexyl-2-(methacryloyloxy)-benzoat (Homomenthylsalicylatmethacrylat) (6).

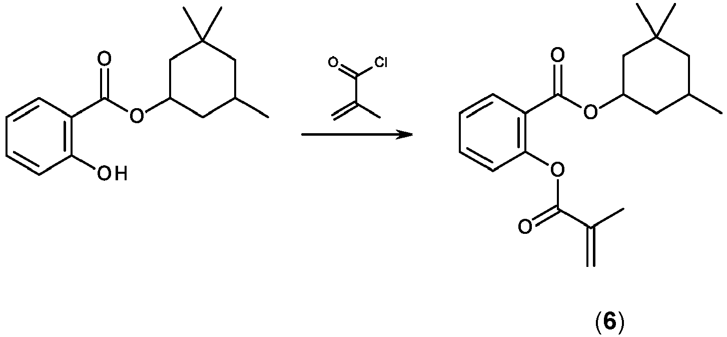

Die Herstellung war ähnlich zu Referenzbeispiel 1, wobei 7,89 g (30 mmol) Homomenthylsalicylat (3,3,5-Trimethylcyclohexylsalicylat; Gemisch aus cis- und trans-Isomeren) eingesetzt wurden und das Rohprodukt mittels Silica-Chromatographie (Elutionsmittel PE:EE = 6:1) gereinigt wurde, um 8,00 g (81 %) der Titelverbindung **(6)** als transparente Flüssigkeit zu erhalten, die unter Verwendung von 250 ppm BHT stabilisiert wurde. ¹H-NMR (400 MHz, CDCl₃, δ, ppm): 8,00 (d, 1H; Ar-H), 7,54 (t, 1H; Ar-H), 7,30 (t, 1H; Ar-H), 7,10 (d, 1H; Ar-H), 6,40 (d, 1H; =CH₂), 5,80 (d, 1H; =CH₂), 5,30-5,05 (m, 1H; COO-CH-(CH₂)₂), 2,10 (s, 3H; CH₂=CH₂-CH₃), 1,76-1,00 (m, 7H) 0,96 (d, 6H; (CH₂)₂-CH-(CH₃)₂), 0,90 (d, 3H; (CH₂)₂-CH-CH₃); APT-¹³C-NMR (100,6 MHz, CDCl₃, δ, ppm): 165,7 (C4, CO), 164,3 (C4, CO), 150,6 (C4), 135,8 (C4), 133,4 (C3), 131,9 (C3), 127,6 (C2), 125,9 (C3), 124,5 (C4), 123,8 (C3), 72,0 (C3), 47,6 (C2), 43,9 (C2), 40,4 (C2), 33,1 (C3), 32,3 (C4), 27,1 (C3), 22,3 (C1), 18,5 (C1).

### Beispiel 7: Synthese von 3,3,5-Trimethylcyclohexyl-2-(acryloyloxy)benzoat (Homomenthylsalicylatacrylat) (7).

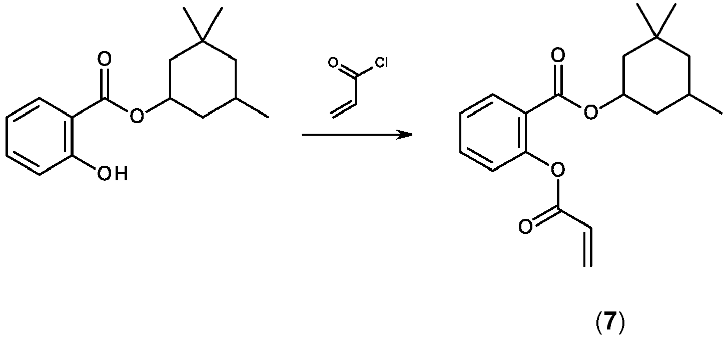

Die Herstellung war ähnlich zu Referenzbeispiel 1, wobei 5,25 g (30 mmol) Homomenthylsalicylat (3,3,5-Trimethylcyclohexylsalicylat; Gemisch aus cis- und trans-Isomeren) und 3,09 g (34 mmol) frisch destilliertes Acryloylchlorid eingesetzt wurden und das Rohprodukt mittels Silica-Chromatographie (Elutionsmittel PE:EE = 6:1) gereinigt wurde, um 3,90 g (62 %) der Titelverbindung **(7)** als transparente Flüssigkeit zu erhalten, die unter Verwendung von 250 ppm BHT stabilisiert wurde. ¹H-NMR (200 MHz, CDCl₃, δ, ppm): 8,02 (d, 1H; Ar-H), 7,55 (t, 1H; Ar-H), 7,31 (t, 1H; Ar-H), 7,13 (d, 1H; Ar-H), 6,69 (dd, 1H; =CH₂), 6,40 (q, 1H; -CH=CH₂), 6,07 (dd, 1H; =CH₂), 5,29-5,01 (m, 1H; COO-CH-(CH₂)₂), 1,76-1,00 (m, 7H) 0,96 (d, 6H; (CH₂)₂-CH-(CH₃)₂), 0,90 (d, 3H; (CH₂)₂-CH-CH₃); APT-¹³C-NMR (50,3 MHz, CDCl₃, δ, ppm): 164,6 (C4, CO), 164,3 (C4, CO), 150,2 (C4), 133,6 (C3), 132,8 (C2), 132,0 (C3), 128,1 (C3), 126,2 (C3), 124,5 (C2), 123,8 (C3), 72,2 (C3), 47,7 (C2), 43,1 (C2), 40,5 (C2), 33,2 (C3), 32,4 (C4), 27,2 (C3), 25,7 (C1), 22,4 (C1).

### Beispiel 8: Synthese von Decahydronaphthalin-2-yl-2-(methacryloyloxy)-benzoat (8).

### Stufe 1: Herstellung von Decahydronaphthalin-2-yl-2-hydroxybenzoat.

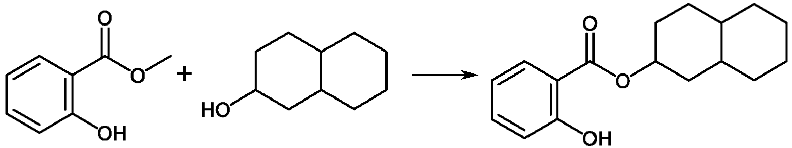

Die Synthese wurde gemäß Beispiel (**1**), Stufe 1 unter Verwendung von 7,40 g (48 mmol) Decahydronaphthalin-2-ol, 1,38 g (60 mmol) metallischem Natrium und 7,76 g (51 mmol) Methylsalicylat durchgeführt. Das Rohprodukt wurde mittels Silica-Chromatographie (Elutionsmittel PE:EE = 6:1) gereinigt, was 5,12 g (39 %) **(8)** als transparente Flüssigkeit ergab. ¹H-NMR (400 MHz, CDCl₃, δ, ppm): 10,95 (s, 1H; Ar-OH) 7,89 (d, 1H; Ar-H), 7,43 (t, 1H; Ar-H), 6,98 (d, 1H; Ar-H), 6,86 (t, 1H; Ar-H), 5,27-4,96 (m, 1H; COO-CH-), 2,14-0,87 (m, 16H).

### Stufe 2: Herstellung von Decahydronaphthalin-2-yl-2-(methacryloyloxy)benzoat (8).

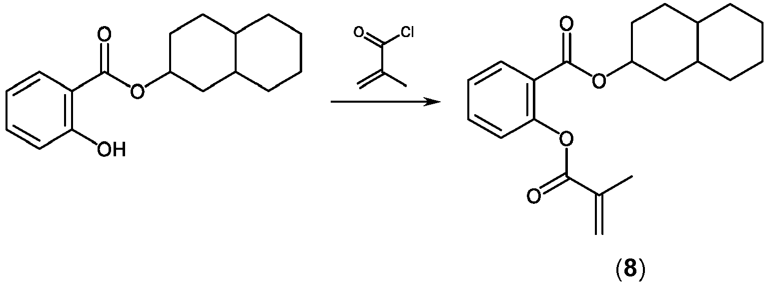

Die Herstellung war ähnlich zu Referenzbeispiel 1, wobei 4,66 g (17 mmol) Decahydronaphthalin-2-yl-2-hydroxybenzoat eingesetzt wurden und das Rohprodukt mittels Silica-Chromatographie (Elutionsmittel PE:EE = 6:1) gereinigt wurde, was 4,18 g (72 %) der Titelverbindung **(8)** als transparente viskose Flüssigkeit ergab, die vor dem Abdampfen der Lösungsmittel durch Zusetzen von 250 ppm BHT stabilisiert worden war. ¹H-NMR (400 MHz, CDCl₃, δ, ppm): 8,02 (d, 1H; Ar-H), 7,53 (t, 1H; Ar-H), 7,30 (t, 1H; Ar-H), 7,11 (d, 1H; Ar-H), 6,39 (s, 1H; =CH₂), 5,78 (s, 1H; =CH₂), 5,15-4,90 (m, 1H; COO-CH-), 2,09 (s, 3H; CH₂=CH₂-CH₃), 1,97-0,84 (m, 16H).

### Beispiel 9: Synthese von 1,3,3-Trimethyl-2-bicyclo[2,2,1]heptanyl-2-(methacryloyloxy)benzoat (Fenchylsalicylatmethacrylat) (9).

### Stufe 1: Herstellung von Fenchylsalicylat.

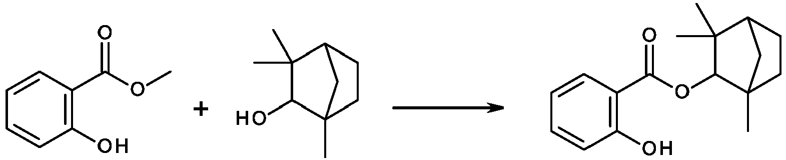

Fenchylsalicylat wurde gemäß Beispiel (**1**), Stufe 1 durch Umsetzen von 7,05 g (46 mmol) Fenchol, 1,38 g (60 mmol) metallischem Natrium und 7,41 g (49 mmol) Methylsalicylat hergestellt. Das rotbräunliche Rohprodukt wurde mittels Silica-Chromatographie (Elutionsmittel PE:EE = 6:1) gereinigt, was 3,42 g (27 %) Fenchylsalicylat als schwach bräunliche Flüssigkeit ergab. ¹H-NMR (400 MHz, CDCl₃, δ, ppm): 10,95 (s, 1H; Ar-OH) 7,90 (d, 1H; Ar-H), 7,47 (t, 1H; Ar-H), 7,00 (d, 1H; Ar-H), 6,91 (t, 1H; Ar-H), 4,65 (s, 1H; COO-CH-), 1,94-1,24 (m, 8H) 1,21 (s, 3H; (CH₂)₂-CH-(CH₃)₂), 1,14 (s, 3H; (CH₂)₂-CH-(CH₃)₂), 0,87 (s, 3H; (CH₂)₂-CH-CH₃); APT-¹³C-NMR (100,6 MHz, CDCl₃, δ, ppm): 170,6 (C4, CO), 161,9 (C4, CO), 135,6 (C3), 129,8 (C3), 119,2 (C3), 117,7 (C3), 112,9 (C4), 87,4 (C3), 48,7 (C4), 48,5 (C3), 41,5 (C2), 40,0 (C4), 29,7 (C1), 27,0 (C2), 26,0 (C2), 20,4 (C1), 19,6 (C1).

### Stufe 2: Herstellung von Fenchylsalicylatmethacrylat (9).

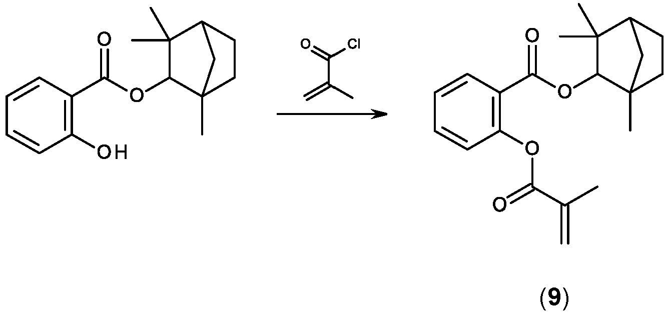

Die Herstellung war ähnlich zu Referenzbeispiel 1, wobei 2,50 g (9 mmol) Fenchylsalicylat eingesetzt wurden und das Rohprodukt mittels Silica-Chromatographie (Elutionsmittel PE:EE = 6:1) gereinigt wurde, was 2,56 g (83 %) der Titelverbindung **(9)** als weißen Feststoff ergab, der vor dem Abdampfen der Lösungsmittel durch Zusetzen von 250 ppm BHT stabilisiert worden war. Fp.: 43-45 °C; ¹H-NMR (400 MHz, CDCl₃, δ, ppm): 8,03 (d, 1H; Ar-H), 7,56 (t, 1H; Ar-H), 7,32 (t, 1H; Ar-H), 6,39 (s, 1H; =CH₂), 5,77 (s, 1H; =CH₂), 4,60 (s, 1H; COO-CH-), 2,10 (s, 3H; CH₂=CH₂-CH₃), 2,01-1,22 (m, 8H) 1,16 (s, 3H; (CH₂)₂-CH-(CH₃)₂), 1,10 (s, 3H; (CH₂)₂-CH-(CH₃)₂), 0,85 (s, 3H; (CH₂)₂-CH-CH₃); APT-¹³C-NMR (100,6 MHz, CDCl₃, δ, ppm): 166,0 (C4, CO), 164,7 (C4, CO), 151,0 (C4), 135,9 (C4), 133,4 (C3), 131,3 (C3), 127,5 (C2), 125,9 (C3), 124,3 (C4), 123,9 (C3), 86,8 (C3), 48,6 (C4), 48,5 (C3), 41,6 (C2), 39,8 (C4), 29,8 (C1), 26,9 (C2), 26,0 (C2), 20,4 (C1), 19,5 (C1), 18,5 (C1).

### Beispiel 10: Synthese von 1,7,7-Trimethyl-2-bicyclo[2,2,1]heptanyl-2-(methacryloyloxy)benzoat ((Iso)bornylsalicylatmethacrylat) (10).

### Stufe 1: Herstellung von (Iso)bornylsalicylat.

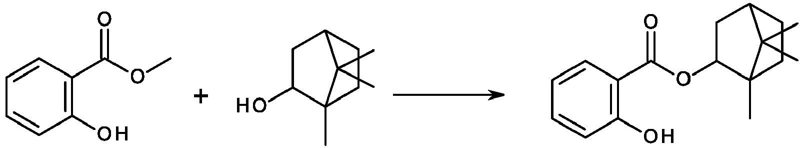

(Iso)bornylsalicylat wurde gemäß der Synthesevorschrift von Beispiel 1 (Stufe 1) umgesetzt, wobei 6,64 g (44 mmol) Methylsalicylat, 5,93 g (39 mmol) (Iso)borneol und 1,05 g (46 mmol) metallischem Natrium umgesetzt wurden. Das Rohprodukt wurde mittels Silica-Chromatographie (Elutionsmittel PE:EE = 6:1) gereinigt, was 3,18 g (30 %) (Iso)bornylsalicylat ergab. ¹H-NMR (400 MHz, CDCl₃, δ, ppm): 10,96 (s, 1H; Ar-OH) 7,78 (d, 1H; Ar-H), 7,45 (t, 1H; Ar-H), 6,98 (d, 1H; Ar-H), 6,88 (t, 1H; Ar-H), 4,95 (s, 1H; COO-CH-), 2,10 (s, 3H; CH₂=CH₂-CH₃), 1,76-1,00 (m, 7H), 1,13 (s, 3H; C-CH₃), 0,96 (s, 3H; (CH₂)₂-CH-(CH₃)₂), 0,91 (s, 3H; C-CH₃); APT-¹³C-NMR (50,3 MHz, CDCl₃, δ, ppm): 169,8 (C4, CO), 161,9 (C4, CO), 135,5 (C3), 129,7 (C3), 119,2 (C3), 117,7 (C3), 113,1 (C4), 82,3 (C3), 49,2 (C4), 47,1 (C4), 45,2 (C3), 38,9 (C2), 33,8 (C2), 27,1 (C2), 20,2 (C1), 20,1 (C1), 11,7 (C1).

### Stufe 2: Herstellung von (iso)Bornylsalicylatmethacrylat (10).

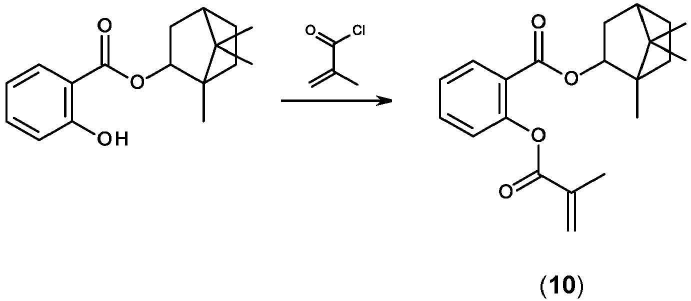

Die Herstellung war ähnlich zu Referenzbeispiel 1, wobei 3,29 g (12 mmol) (Iso)bornylsalicylat eingesetzt wurden und das Rohprodukt mittels Silica-Chromatographie (Elutionsmittel PE:EE = 6:1) gereinigt wurde, wodurch 3,22 g (78 %) der Titelverbindung **(10)** als weißer Feststoff erhalten wurde, der vor dem Abdampfen der Lösungsmittel durch Zusetzen 250 ppm BHT stabilisiert worden war. Fp.: 78-80 °C; ¹H-NMR (400 MHz, CDCl₃, δ, ppm): 7,95 (d, 1H; Ar-H), 7,55 (t, 1H; Ar-H), 7,30 (t, 1H; Ar-H), 7,14 (d, 1H; Ar-H), 6,39 (s, 1H; =CH₂), 6,78 (t, 1H; =CH₂), 4,87 (s, 1H; COO-CH-), 2,10 (s, 3H; CH₂=CH₂-CH₃), 1,88-1,09 (m, 8H) 1,07 (s, 3H; C-CH₃), 0,91 (s, 3H; (CH₂)₂-CH-(CH₃)₂), 0,87 (s, 3H; C-CH₃); APT-¹³C-NMR (100,6 MHz, CDCl₃, δ, ppm): 166,0 (C4, CO), 163,7 (C4, CO), 151,2 (C4), 135,9 (C4), 133,5 (C3), 131,3 (C3), 127,6 (C2), 126,0 (C3), 124,2 (C4), 124,0 (C3), 81,6 (C3), 49,2 (C4), 47,2 (C4), 45,2 (C3), 38,9 (C2), 33,9 (C2), 27,2 (C2), 20,2 (C1), 18,6 (C1), 11,7 (C1).

### Beispiel 11: Synthese von Bicyclo[2,2,1]heptan-2-ylmethyl-2-(methacryloyloxy)benzoat (11).

### Stufe 1: Herstellung von Bicyclo[2,2,1]heptan-2-ylmethyl-2-hydroxybenzoat.

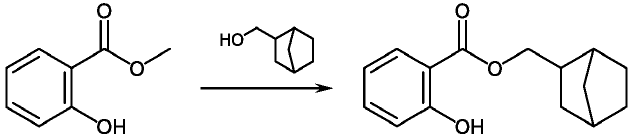

5,68 g (45 mmol) Norbornan-2-methanol und 1,38 g (60 mmol) Natrium wurden in Substanz unter Rühren auf 130 °C erhitzt, bis alles geschmolzen war. Dann wurden 7,60 g (50 mmol) Methylsalicylat zu der Reaktion zugesetzt. Nach einiger Zeit hatte sich Methanol als Nebenprodukt gebildet, das abdestilliert wurde. Als die Destillation abgeschlossen war, wurde die Reaktion auf Raumtemperatur abgekühlt. Der amorphe rotbräunliche Feststoff wurde dann 2 h lang in CH₂Cl₂ gelöst und ungelöste Rückstände wurden abfiltriert. Das Lösungsmittel wurde abgedampft, um 7,21 g eines rotbräunlichen Rohprodukts zu erhalten. Schließlich wurde das Produkt durch Säulenchromatographie (PE:EE = 6:1) gereinigt, wodurch 3,01 g (27 %) des farblosen Produkts erhalten wurden. ¹H-NMR (400 MHz, CDCl₃, δ, ppm): 10,84 (s, 1H; Ar-OH), 7,85 (t, 1H; Ar-H), 7,44 (t, 1H; Ar-H), 6,99 (d, 1H; Ar-H), 6,87 (t, 1H; Ar-H), 4,39 (q, 1H; -COO-CH₂-), 4,22 (t, 1H; -COO-CH₂-), 2,33-1,11 (m, 11H; Cycloaliphat). APT-¹³C-NMR (100,6 MHz, CDCl₃, δ, ppm): 170,3 (C4, CO), 161,8 (C4, CO), 135,7 (C3), 130,0 (C3), 119,2 (C3), 117,7 (C3), 112,8 (C4), 122,0 (C3), 67,3 (C2), 39,8 (C2), 38,8 (C3), 38,4 (C3), 36,8 (C3), 33,6 (C2), 30,0 (C2), 22,8 (C2).

### Stufe 2: Herstellung von Bicyclo[2,2,1]heptan-2-ylmethyl-2-(methacryloyloxy)-benzoat (11).

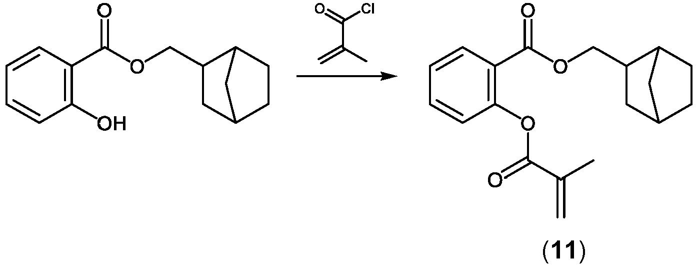

Die Herstellung war ähnlich zu Referenzbeispiel 1, wobei 2,51 g (10 mmol) Bicyclo[2,2,1]heptan-2-ylmethyl-2-hydroxybenzoat und 1,14 g (11 mmol) Methacryloylchlorid in 30 ml trockenem Dichlormethan sowie 1,52 g (15 mmol) Trimethylamin eingesetzt wurden. Die Reinigung des Rohprodukts wurde durch Säulenchromatographie (SiO₂, PE:EE = 6:1) durchgeführt, wodurch 2,71 g (86 %) **(11)** als farblose transparente Flüssigkeit erhalten und mit 250 ppm MEHQ stabilisiert wurden. ¹H-NMR (400 MHz, CDCl₃, δ, ppm): 8,01 (t, 1H; Ar-H) 7,54 (t, 1H; Ar-H), 7,30 (t, 1H; Ar-H), 7,12 (d, 1H; Ar-H), 6,38 (s, 1H; =CH₂), 5,77 (t, 1H; =CH₂), 4,28 (q, 1H; -COO-CH₂-), 4,12 (t, 1H; -COO-CH₂-), 2,08 (s, 3H; COO-CH₂-Ar), 2,08 (s, 3H; CH₂=CH₂-CH₃), 2,26-1,09 (m, 11H; Cycloaliphat); APT-¹³C-NMR (100,6 MHz, CDCl₃, δ, ppm): 165,9 (C4, CO), 164,7 (C4, CO), 150,9 (C4), 135,7 (C4), 133,7 (C3), 131,9 (C3), 129,1 (C4), 127,6 (C4), 126,0 (C3), 123,9 (C3), 67,2 (C2), 39,8 (C2), 38,8 (C3), 38,4 (C3), 36,8 (C3), 33,7 (C2), 29,9 (C2), 22,7 (C2), 18,5 (C1).

### Beispiel 12: Synthese von 2-Cyclohexylethyl-2-(methacryloyloxy)benzoat (12).

### Stufe 1: Herstellung von 2-Cyclohexylethyl-2-hydroxybenzoat.

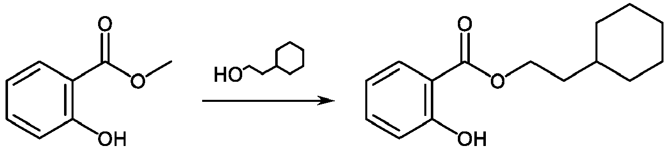

Die Titelverbindung **(12)** wurde gemäß Beispiel 1 (Stufe 1) durch Umsetzen von 5,77 g (45 mmol) Cyclohexyl-2-ethanol, 1,38 g (60 mmol) Natrium und 7,59 g (50 mmol) Methylsalicylat synthetisiert. Das Rohprodukt wurde durch Säulenchromatographie (PE:EE = 6:1) gereinigt, wodurch 3,01 g (27 %) des farblosen flüssigen Produkts erhalten wurden. ¹H-NMR (400 MHz, CDCl₃, δ, ppm): 10,86 (s, 1H; Ar-OH), 7,85 (t, 1H; Ar-H), 7,44 (t, 1H; Ar-H), 6,99 (d, 1H; Ar-H), 6,88 (t, 1H; Ar-H), 4,38 (t, 2H; -COO-CH₂-), 1,78-1,67 (m, 7H), 1,48-0,95 (m, 6H); APT-¹³C-NMR (100,6 MHz, CDCl₃, δ, ppm): 170,3 (C4, CO), 161,8 (C4, CO), 135,6 (C3), 130,0 (C3), 119,2 (C3), 117,7 (C3), 112,8 (C4), 63,8 (C2), 36,0 (C2), 34,8 (C1), 33,3 (C2), 26,6 (C2), 26,3 (C2).

### Stufe 2: Herstellung von 2-Cyclohexylethyl 2-(methacryloyloxy)benzoat (12).

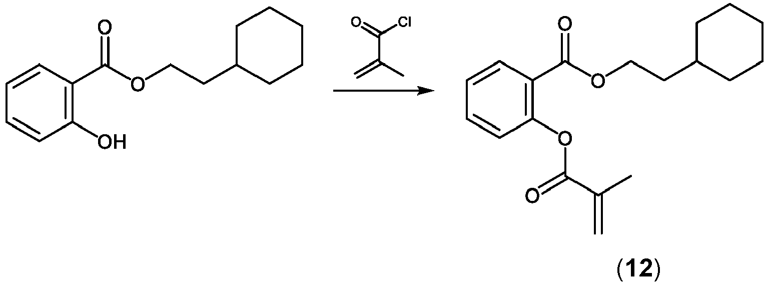

Die Herstellung war ähnlich zu Referenzbeispiel 1, wobei 2,51 g (10 mmol) Cyclohexylethanolsalicylat und 1,14 g (11 mmol) Methacryloylchlorid in 30 ml trockenem Dichlormethan sowie 1,52 g (15 mmol) Trimethylamin eingesetzt wurden. Die Reinigung des Rohprodukts wurde mittels Säulenchromatographie (SiO₂, PE:EE = 6:1) durchgeführt, wodurch 2,71 g (86 %) **(12)** als farblose transparente Flüssigkeit erhalten wurden, die mit 250 ppm MEHQ stabilisiert wurde. ¹H-NMR (400 MHz, CDCl₃, δ, ppm): 8,03 (t, 1H; Ar-H) 7,54 (t, 1H; Ar-H), 7,31 (t, 1H; Ar-H), 7,13 (d, 1H; Ar-H), 6,38 (s, 1H; =CH₂), 5,77 (t, 1H; =CH₂), 4,27 (t, 1H; -COO-CH₂-), 2,08 (s, 3H; CH₂=CH₂-CH₃), 1,76-1,55 (m, 7H), 1,41-0,88 (m, 6H); APT-¹³C-NMR (100,6 MHz, CDCl₃, δ, ppm): 165,9 (C4, CO), 164,8 (C4, CO), 150,8 (C4), 135,8 (C4), 133,6 (C3), 131,9 (C3), 129,0 (C4), 127,5 (C4), 126,0 (C3), 123,9 (C3), 63,5 (C2), 36,0 (C2), 34,5 (C3), 33,2 (C2), 26,5 (C2), 26,2 (C2), 18,5 (C1).

### Beispiel 13: Synthese von Benzyl-2-(methacryloyloxy)benzoat (Benzylsalicylatmethacrylat) (13).

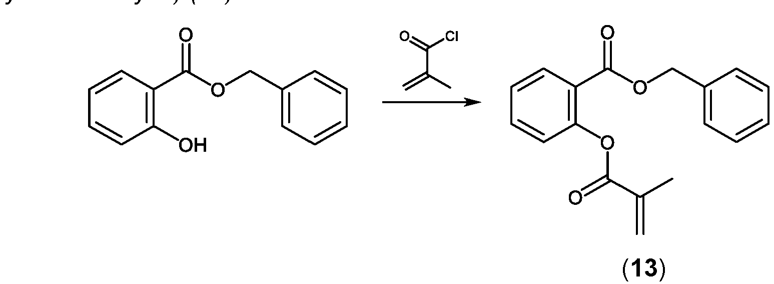

Die Herstellung war ähnlich zu Referenzbeispiel 1, wobei 5,55 g Benzylsalicylat (20 mmol), 5,27 g Methacryloylchlorid (34 mmol) und 0,12 g Trimethylamin (1 mmol) in trockenem Dichlormethan unter Argonschutz eingesetzt wurden. Das Rohprodukt wurde mittels Silica-Chromatographie (Elutionsmittel PE:EE = 6:1) gereinigt, um 5,67 g (83 %) der Titelverbindung **(13)** als transparente Flüssigkeit zu erhalten, die mit 250 ppm BHT stabilisiert wurde. ¹H-NMR (200 MHz, CDCl₃, δ, ppm): 8,12 (dd, 2H; Ar-H), 7,58 (td, 2H; Ar-H), 7,42-7,29 (m, 6H; Ar-H), 7,17 (dd, 1H; Ar-H), 6,31 (s, 1H; =CH₂), 5,68 (t, 1H; =CH₂), 5,30 (s, 2H; COO-CH₂-Ar), 2,00 (s, 3H; CH₂=CH₂-CH₃). APT-¹³C-NMR (50,3 MHz, CDCl₃, δ, ppm): 165,9 (C4, CO), 164,5 (C4, CO), 150,9 (C4), 135,7 (C4), 135,6 (C4), 133,9 (C3), 132,0 (C3), 128,6 (C3), 128,5 (C3), 128,4 (C3), 127,6 (C2), 126,0 (C3), 124,0 (C3), 123,6 (C4), 67,1 (C2), 18,3 (C1).

### Beispiel 14: Synthese von Benzyl-4-(methacryloyloxy)benzoat (14).

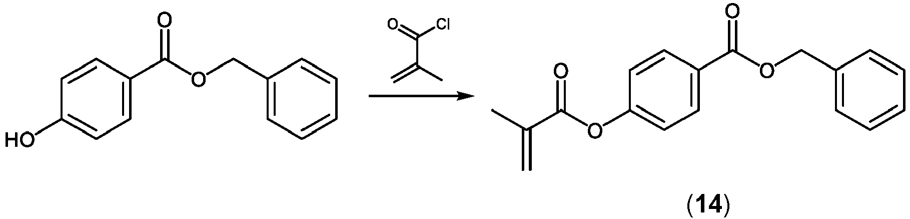

Verbindung **(14)** wurde durch Umsetzen von 4,57 g (20 mmol) Benzyl-4-hydroxybenzoat und 2,30 g (22 mmol) Methacryloylchlorid in trockenem Dichlormethan unter Verwendung eines leichten Überschusses an Trimethylamin als Säurefänger synthetisiert. Das Rohprodukt wurde mittels Silica-Chromatographie (Elutionsmittel PE:EE = 6:1) gereinigt, wodurch 5,15 g (87 %) Benzyl-4-(methacryloyloxy)benzoat (**14**) als transparente Flüssigkeit erhalten wurden, die mit 250 ppm MEHQ stabilisiert wurde. ¹H-NMR (400 MHz, CDCl₃, δ, ppm): 8,18 (d, 2H; Ar-H) 7,50-7,38 (m, 5H; Ar-H), 7,25 (d, 2H; Ar-H), 6,40 (s, 1H; =CH₂), 5,80 (t, 1H; =CH₂), 5,41 (s, 2H; COO-CH₂-Ar), 2,10 (s, 3H; CH₂=CH₂-CH₃). APT-¹³C-NMR (100,6 MHz, CDCl₃, δ, ppm): 165,6 (C4, CO), 165,2 (C4, CO), 154,7 (C4), 136,0 (C4), 135,6 (C4), 131,3 (C3), 128,6 (C3), 128,3 (C3), 128,2 (C3), 127,9 (C2), 127,6 (C4), 121,7 (C3), 66,8 (C2), 18,3 (C1).

### Beispiel 15: Synthese von Isopropylbenzyl-3-(methacryloyloxy)benzoat (15).

### Stufe 1: Herstellung von 3-(Methacryloyloxy)benzoesäure.

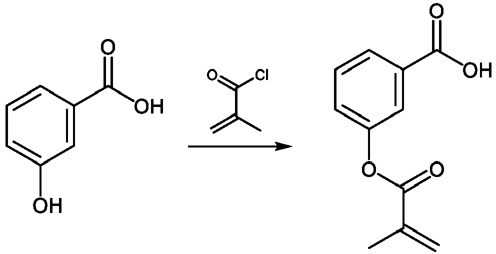

Die Synthese wurde gemäß Beispiel 4, Stufe 1 durchgeführt.

### Stufe 2: Herstellung von Isopropylbenzyl-3-(methacryloyloxy)benzoat (15).

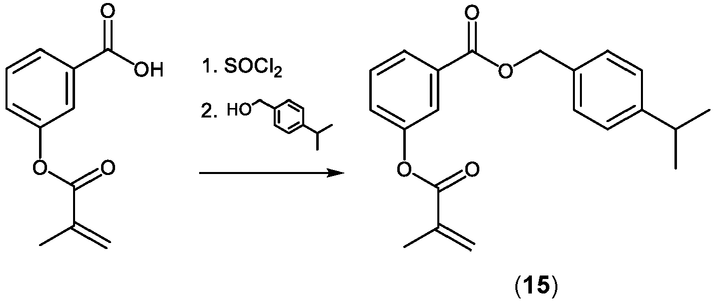

Die Titelverbindung (**15**) wurde gemäß Beispiel 4, Stufe 2 synthetisiert. 3-(Methacryloyloxy)benzoesäure (3,09 g, 15 mmol), 4-Isopropylbenzylalkohol (3,00 g, 20 mmol) und Pyridin (3,2 g, 40 mmol) wurden eingesetzt. Das Rohprodukt wurde mittels Säulenchromatographie gereinigt (SiO₂, PE:EE = 6:1), um 3,71 g (73 %) (**15**) als transparente Flüssigkeit zu erhalten, die mit 250 ppm BHT stabilisiert wurde. ¹H-NMR (400 MHz, CDCl₃, δ, ppm): 8,00 (d, 1H; Ar-H), 7,82 (t, 1H; Ar-H), 7,51-7,25 (m, 6H; Ar-H), 6,37 (s, 1H; =CH₂), 5,79 (t, 1H; =CH₂), 5,35 (s, 1H; COO-CH₂-), 2,94 (q, 1H; Ar-CH-(CH₃)₂), 2,08 (s, 3H; CH₂=CH₂-CH₃), 1,29 (s, 3H; CH-(CH₃)₂), 1,26 (s, 3H; CH-(CH₃)₂). APT-¹³C-NMR (100,6 MHz, CDCl₃, δ, ppm): 165,7 (C4, CO), 165,6 (C4, CO), 151,0 (C4), 149,3 (C4), 135,7 (C4), 133,3 (C4), 131,9 (C4), 129,5 (C3), 128,6 (C3), 127,7 (C2), 127,2 (C3), 126,8 (C3), 126,5 (C3), 123,0 (C3), 67,0 (C2), 34,0 (C1), 24,0 (C1), 18,4 (C1).

### Beispiel 16: Synthese von Benzyl-2-(acryloyloxy)benzoat (Benzylsalicylatacrylat) (16).

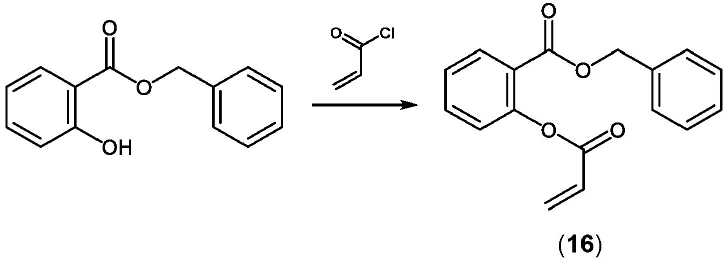

Die Herstellung war ähnlich zu Referenzbeispiel 1, wobei 4,57 g (20 mmol) Benzylsalicylat, 2,00 g (22 mmol) Acryloylchlorid sowie 2,53 g (25 mmol) Trimethylamin eingesetzt wurden und das Rohprodukt mittels Silica-Chromatographie (Elutionsmittel PE:EE = 6:1) gereinigt wurde, wodurch 3,67 g (65 %) der Titelverbindung (**16**) als transparente, farblose Flüssigkeit erhalten wurden, die vor dem Abdampfen der Lösungsmittel durch Zusetzen von 250 ppm BHT stabilisiert worden war. ¹H-NMR (400 MHz, CDCl₃, δ, ppm): 8,13 (dd, 1H; Ar-H) 7,59 (td, 1H; Ar-H), 7,43-7,33 (m, 6H; Ar-H), 7,19 (dd, 1H; Ar-H), 6,57 (dd, 1H; =CH₂), 6,28 (q, 1H; -CH=CH₂), 5,94 (dd, 1H; =CH₂), 5,32 (s, 2H; COO-CH₂-Ar). APT-¹³C-NMR (100,6 MHz, CDCl₃, δ, ppm): 164,6 (C4, CO), 164,4 (C4, CO), 150,4 (C4), 135,6 (C4), 133,9 (C3), 132,6 (C2), 132,0 (C3), 128,5 (C3, d), 128,3 (C3), 127,4 (C3), 126,1 (C3), 123,9 (C3), 123,4 (C4), 67,0 (C2).

### Beispiel 17: Synthese von Phenethyl-2-(methacryloyloxy)benzoat (Phenethylsalicylatmethacrylat) (17).

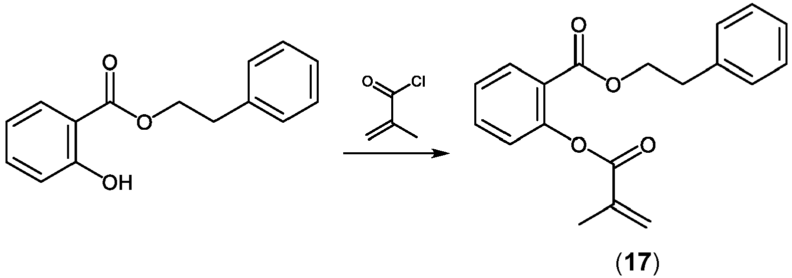

Die Herstellung war ähnlich zu Referenzbeispiel 1, wobei 7,89 g (30 mmol) Phenethylsalicylat eingesetzt wurden und das Rohprodukt durch Umkristallisation aus Et₂O gereinigt wurde, um 8,00 g (81 %) der Titelverbindung (**17**) als weiße Kristalle zu erhalten, die unter Verwendung von 250 ppm BHT stabilisiert wurden. ¹H-NMR (200 MHz, CDCl₃, δ, ppm): 8,02 (dd, 2H; Ar-H), 7,59 (td, 2H; Ar-H), 7,36-7,24 (m, 6H; Ar-H), 7,18 (dd, 1H; Ar-H), 6,40 (s, 1H; =CH₂), 5,80 (t, 1H; =CH₂), 4,48 (t, 2H; COO-CH₂--CH₂-Ar), 3,03 (t, 2H; COO-CH₂-CH₂Ar), 2,10 (s, 3H; CH₂=CH₂-CH₃). APT-¹³C-NMR (50,3 MHz, CDCl₃, δ, ppm): 166,0 (C4, CO), 164,5 (C4, CO), 150,9 (C4), 137,7 (C4), 135,8 (C4), 133,8 (C3), 131,8 (C3), 129,0 (C3), 128,6 (C3), 127,6 (C2), 126,7 (C3), 126,0 (C3), 123,9 (C3), 123,7 (C4), 65,7 (C2), 35,1 (C2), 18,5 (C1).

### Beispiel 18: Synthese von 4-(Methacryloyloxy)-3-methoxybenzoesäure (Methoxybenzylvanillatmethacrylat) (18).

### Stufe 1: Herstellung von (Methacryloyloxy)vanillinsäure.

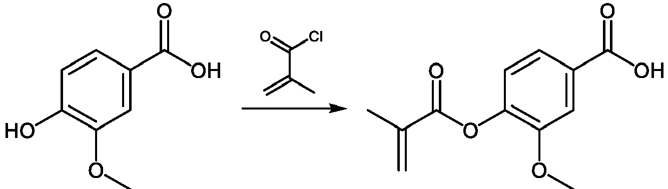

Die Synthese wurde gemäß Beispiel 4, Stufe 1 durchgeführt, wobei 20 mmol Vanillinsäure und 2,30 g (22 mmol) frisch destilliertes Methacryloylchlorid eingesetzt wurden, wodurch 3,12 g (66 %) des Produkts erhalten wurden. ¹H-NMR (200 MHz, DMSO-d₆, δ, ppm): 13,10 (s, 1H; -COOH), 7,61-7,56 (m, 2H; Ar-H), 6,28 (s, 1H; =CH₂), 5,92 (s, 1H; =CH₂), 3,82 (s, 1H; Ar-O-CH₃), 1,99 (s, 3H; CH₂=CH₂-CH₃).

### Stufe 2: Herstellung von 4-(Methacryloyloxy)-3-methoxybenzoesäure (18).

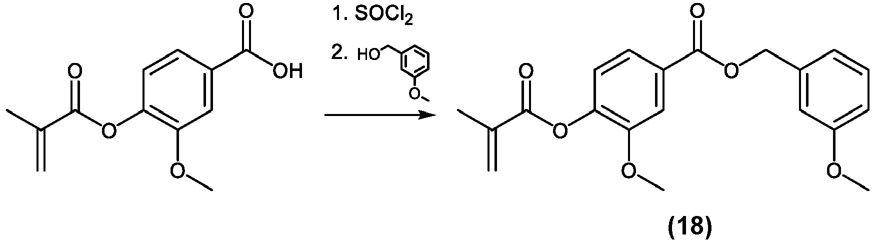

Die Titelverbindung (**18**) wurde gemäß Beispiel 4, Stufe 2 synthetisiert. (Methacryloyloxy)vanillinsäure (3,09 g, 15 mmol), 4-Methoxybenzylalkohol (3,00 g, 20 mmol) und Pyridin (3,2 g, 40 mmol) wurden eingesetzt. Das Rohprodukt wurde mittels Säulenchromatographie gereinigt (SiO₂, PE:EE = 3:1), um 3,71 g (73 %) (**18**) als transparente Flüssigkeit zu erhalten, die mit 250 ppm BHT und 100 ppm Phenothiazin stabilisiert wurde. ¹H-NMR (400 MHz, CDCl₃, δ, ppm): 7,72-7,68 (m, 2H; Ar-H), 7,41 (s, 1H; Ar-H), 7,38 (s, 1H; Ar-H), 7,13 (d, 1H; Ar-H), 6,94 (s, 1H; Ar-H), 6,92 (s, 1H; Ar-H), 6,38 (s, 1H; =CH₂), 5,78 (t, 1H; =CH₂), 5,31 (s, 2H; COO-CH₂-), 3,87 (s, 3H; Ar-O-CH₃), 3,82 (s, 3H; Ar-O-CH₃), 2,08 (s, 3H; CH₂=CH₂-CH₃).

### Beispiel 19: Synthese von 1-Phenylethyl-2-(methacryloyloxy)benzoat (19).

### Stufe 1: Herstellung von 1-Phenylethyl-2-hydroxybenzoat.

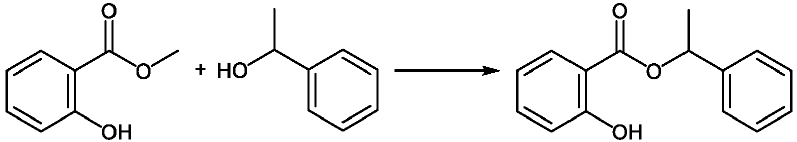

Die Reaktion wurde gemäß Beispiel 1, Stufe 1 durchgeführt. Dazu wurden 4,55 g (30 mmol) Methylsalicylat, 3,66 g (30 mmol) 1-Phenylethylalkohol und 1,15 g (50 mmol) metallisches Natrium umgesetzt, um nach Reinigung mittels Säulenchromatographie (PE:EE=6:1) 1,30 g (18 %) des Salicylatprodukts zu erhalten. ¹H-NMR (400 MHz, CDCl₃, δ, ppm): 10,81 (s, 1H; Ar-OH), 7,97 (d, 1H; Ar-H), 7,48-7,32 (m, 6H; Ar-H), 7,00 (d, 1H; Ar-H), 6,93 (t, 1H; Ar-H), 6,17 (q, 1H; -COO-CH(CH₃)-), 1,72 (d, 3H; -COO-CH(CH₃)-).

### Stufe 2: Herstellung von 1-Phenylethyl-2-(methacryloyloxy)benzoat (19).

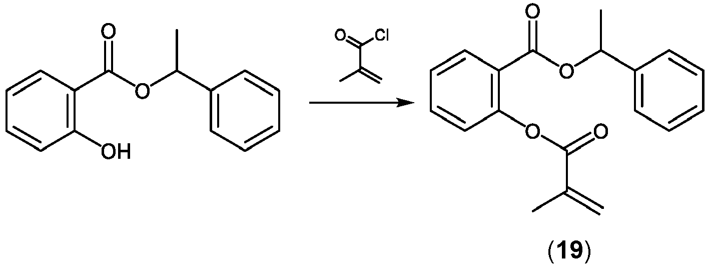

Die Reaktion wurde gemäß Referenzbeispiel 1 durchgeführt, wobei 1,20 g (5 mmol) 1-Phenylethyl-2-hydroxybenzoat, 0,57 g (5,5 mmol) Methacryloylchlorid und 0,81 g (8 mmol) Triethylamin eingesetzt wurden. Nach der Säulenchromatographie (PE:EE=6:1) wurden 1,10 g (71 %) der Titelverbindung (**19**) isoliert. ¹H-NMR (400 MHz, CDCl₃, δ, ppm): 8,07 (d, 1H; Ar-H), 7,58 (t, 1H; Ar-H), 7,40-7,27 (m, 6H; Ar-H), 7,14 (d, 1H; Ar-H), 6,36 (s, 1H; =CH₂), 6,10 (q, 1H; -COO-CH(CH₃)-), 5,74 (t, 1H; =CH₂), 2,03 (s, 3H; CH₂=CH₂-CH₃), 1,62 (d, 3H; -COO-CH(CH₃)-).

### Beispiel 20: Synthese von Cycloheptyl-4-((methacryloyloxy)methyl)benzoat (20).

### Stufe 1: Herstellung von 4-((Methacryloyloxy)methyl)benzoesäure.

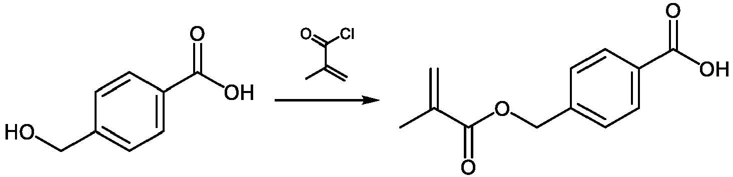

3,05 g (20 mmol) 4-Hydroxymethylbenzoesäure und 3,04 (66 mmol) Triethylamin wurden in 50 ml trockenem Acetonitril gelöst. Die Lösung wurde mit Ar entgast und auf unter 0 °C abgekühlt. Daraufhin wurden 2,30 g (22 mmol) frisch destilliertes Methacryloylchlorid in 20 ml trockenem Acetonitril gelöst, entgast und unter Argonatmosphäre zu der Reaktionslösung zugetropft. Nach dem Zusetzen wurde die Reaktion eine weitere Stunde lang bei einer Temperatur unter 0 °C gerüht, dann auf Raumtemperatur erwärmt und über Nacht gerührt. Am nächsten Tag wurde die Reaktionslösung mit 250 ppm BHT stabilisiert und das Lösungsmittel wurde abgedampft. Dann wurde die Aufschlämmung erneut in CH₂Cl₂ gelöst, und die organische Phase wurde mit 10 Vol.-% HCl (3x25 ml) und Kochsalzlösung (3x25 ml) gewaschen. Die organische Phase wurde über Na₂SO₄ getrocknet, filtriert und mit 50 ppm Phenothiazin stabilisiert, bevor das Lösungsmittel erneut entfernt wurde. Der erhaltene rohe weiße Feststoff wurde mittels Säulenchromatographie (SiO₂, PE:EE = 1:1) gereinigt, um 1,91 g (43 %) weiße Kristalle zu erhalten. ¹H-NMR (400 MHz, DMSO-d₆, δ, ppm): 12,97 (s, 1H; -COOH), 7,96 (d, 2H; Ar-H), 7,51 (d, 2H; Ar-H), 6,11 (s, 1H; =CH₂), 5,74 (t, 1H; =CH₂), 5,25 (s, 2H; Ar-CH₂-OOC-), 1,92 (s, 3H; CH₂=CH₂-CH₃).

### Stufe 2: Herstellung von Cycloheptyl-4-((methacryloyloxy)methyl)benzoat (20).

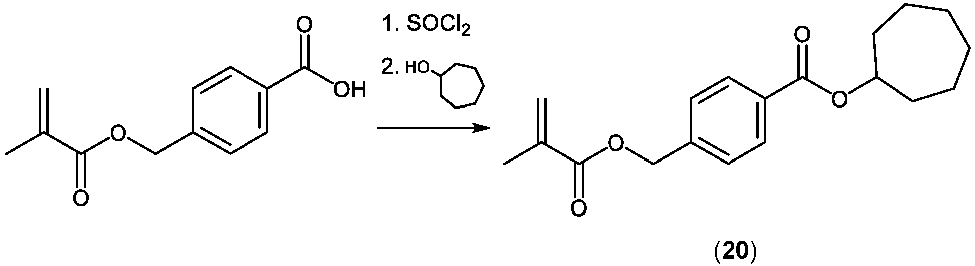

Die Titelverbindung **20** wurde gemäß der Synthesevorschrift von Beispiel 4, Stufe 2 synthetisiert, wobei 1,55 g (7 mmol) 4-((Methacryloyloxy)methyl)benzoesäure und 5 ml Thionylchlorid in 10 ml Toluol für die erste Stufe sowie 1,13 g (10 mmol) Cycloheptanol und 1,60 g (20 mmol) Pyridin in Dichlormethan für die zweite Stufe eingesetzt wurden. Das Rohprodukt wurde mittels Säulenchromatographie gereinigt (PE:EE=6:1), um 0,51 g (23 %) der Titelverbindung **20** zu erhalten. ¹H-NMR (200 MHz, CDCl₃, δ, ppm): 8,05 (d, 2H; Ar-H), 7,44 (d, 2H; Ar-H), 6,17 (s, 1H; =CH₂), 5,60 (t, 1H; =CH₂), 5,23 (s, 2H; Ar-CH₂-OOC-), 5,23-5,13 (m, 1H; -COO-CH(CH₂)₂-), 1,97 (s, 3H; CH₂=CH₂-CH₃), 2,07-1,03 (m, 14H; Cycloaliphat).

### Beispiel 21: Synthese von Cyclohexylmethyl-2-(methacryloyloxy)benzoat (21).

### Stufe 1: Herstellung von Cyclohexylmethyl-2-hydroxybenzoat.

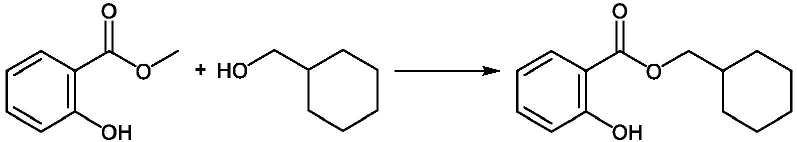

Die Reaktion wurde gemäß Beispiel 1, Stufe 1 durchgeführt. Daher wurden 5,33 g (35 mmol) Methylsalicylat, 4,00 g (35 mmol) Cyclohexylmethanol und 1,39 g (60 mmol) metallisches Natrium umgesetzt, um nach Reinigung mittels Säulenchromatographie (PE:EE=6:1) 1,71 g (21 %) des Salicylatprodukts zu erhalten. ¹H-NMR (400 MHz, CDCl₃, δ, ppm): 10,86 (s, 1H; Ar-OH), 7,89 (dd, 1H; Ar-H), 7,46 (td, 1H; Ar-H), 7,01 (dd, 1H; Ar-H), 6,89 (td, 1H; Ar-H), 4,19 (d, 1H; -COO-CH₂-), 1,86-1,74 (m, 6H; Cycloaliphat), 1,42-0,97 (m, 5H; Cycloaliphat).

### Stufe 2: Herstellung von Cyclohexylmethyl-2-(methacryloyloxy)benzoat (21).

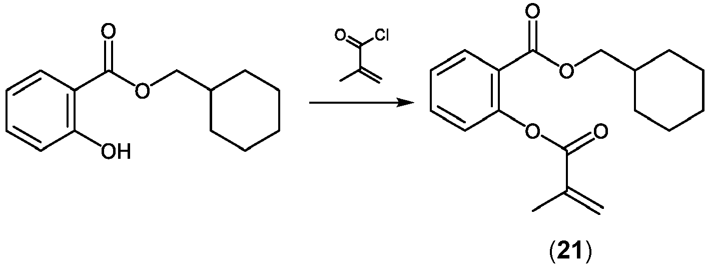

Die Reaktion wurde gemäß Referenzbeispiel 1 durchgeführt, wobei 1,05 g (4,5 mmol) Cyclohexylmethyl-2-hydroxybenzoat, 0,57 g (5,5 mmol) Methacryloylchlorid und 0,91 g (9 mmol) Triethylamin eingesetzt wurden. Nach der Säulenchromatographie (PE:EE=6:1) wurden 0,98 g (72 %) der Titelverbindung **21** isoliert. ¹H-NMR (200 MHz, CDCl₃, δ, ppm): 8,06 (d, 1H; Ar-H), 7,57 (t, 1H; Ar-H), 7,33 (t, 1H; Ar-H), 7,16 (d, 1H; Ar-H), 6,40 (s, 1H; =CH₂), 5,79 (t, 1H; =CH₂), 4,08 (d, 2H; -COO-CH₂-), 2,10 (s, 3H; CH₂=CH₂-CH₃), 1,80-1,60 (m, 6H; Cycloaliphat), 1,35-0,97 (m, 5H; Cycloaliphat).

### Beispiel 22: Schmelzpunktmessungen.

Bei Verwendung als Reaktivverdünner in härtbaren Formulierungen ist es wünschenswert, dass die hierin offenbarten polymerisierbaren Monomere im flüssigen Zustand bei Verarbeitungstemperatur vorliegen. Dies impliziert, dass die Untergrenze des Verarbeitungsfensters von Reaktivverdünnern entweder durch die Viskosität (wenn noch flüssig) oder durch den Schmelzpunkt der Substanz definiert ist. Für Hochtemperaturanwendungen wird die Obergrenze für Schmelzpunkte empirisch mit 90 °C definiert.

Daher wurden Monomere, die bei Raumtemperatur fest sind, unter Verwendung einer OptiMelt-Schmelzpunktvorrichtung von Stanford Research Systems analysiert, wobei eine Heizrate von 1 K/min eingesetzt wurde. Das Schmelzpunktintervall wurde mittels eines optischen Sensors bestimmt. Zusätzlich dazu wurden STA- (simultaner thermischer Analysator) Messungen auf einer DSC-Vorrichtung STA 449F1 Jupiter von Netzsch, die einen Temperaturbereich von -50 °C bis zu 400 °C abdeckt, unter Verwendung einer Heizrate von 10 K/min durchgeführt. Für die hierin umfassten Zwecke wurden die DSC-Daten verwendet, um die Schmelzpunkte der synthetisierten Verbindungen zu bestimmen. Aufgrund der relativ hohen Heizrate wurde der Schmelzbeginn als Bezugspunkt verwendet.

In nachstehender Tabelle 2 sind die Ergebnisse beider Schmelzpunktbestimmungen für die Beispiele der Offenbarung wie oben synthetisiert und fünf Referenzbeispiele angeführt,

**Tabelle 2: Schmelzpunkte ("-" flüssig bei Raumtemperatur)**

| **Beispiel** | **Fp (Optimelt) [°C]** | **Fp (Beginn, STA-Plot) [°C]** |
|---|---|---|
| **1** | - | - |
| **2** | - | - |
| **3** | - | - |
| **4** | - | - |
| **5** | - | - |
| **6** | - | - |
| **7** | - | - |
| **8** | - | - |
| **9** | 43-45 | 45 |
| **10** | 79-81 | 81 |
| **11** | - | - |
| **12** | - | - |
| **13** | - | - |
| **14** | 23-25 | 25 |
| **15** | - | - |
| **16** | - | - |
| **17** | 65-66 | 64 |
| **18** | - | - |
| **19** | - | - |
| **20** | - | - |
| **Ref1** | 99-100 | 99 |
| **Ref2** | - | - |
| **Ref3** | - | - |
| **Ref4** | - | - |
| **Ref5** | - | - |

Die Ergebnisse in Tabelle 2 zeigen, dass ein Referenzbeispiel und vier Beispiele der vorliegenden Offenbarung bei Raumtemperatur fest sind. Die anderen getesteten Substanzen sind Flüssigkeiten.

**Ref1** weist einen Schmelzpunkt von 99-100 °C auf, der etwa 10 °C über der definierten Obergrenze liegt. Ein solcher hoher Schmelzpunkt schließt wahrscheinlich aus, dass **Ref1** und möglicherweise andere Arylsalicylat(meth)acrylate als reaktive(s) Monomer(e) in Lithographie-basierten Photopolymerisationsprozessen eingesetzt werden.

Die Beispiele **9** und **10,** welche die verbrückten bizyklischen Ester, d.h. Fenchyl- und (Iso)bornylsalicylatmethacrylat, darstellen, sind bei Raumtemperatur fest. Allerdings besitzt (**9**) einen relativ niedrigen Schmelzpunkt (45 °C), während (**10**) einen signifikant höheren Schmelzpunkt von ∼80 °C aufweist, der immer noch deutlich unter der Obergrenze liegt. Der Umstand, dass alle anderen cycloaliphatischen Beispiele der vorliegenden Offenbarung, einschließlich der meta- und para-Methacryloyloxy-Derivate, bei Raumtemperatur Flüssigkeiten sind, zeigt die Wirkung der an den Ester gebundenen cycloaliphatischen Gruppe auf den Aggregationszustand der jeweiligen Verbindungen.

Was die Benzyl- und die Phenethylsalicylatmethacrylate betrifft, so sind die Beispiele (**14**) und (**17**) bei Raumtemperatur fest. Allerdings weist (**14**) einen Schmelzpunkt bei etwa Raumtemperatur auf. Beobachtungsgemäß erfolgt eine Kristallisation von Verbindung (**14**) nur im Kühlschrank, während sie bei 20 °C ihren flüssigen Aggregatzustand tagelang beibehält. Daher kann (**14**) vorbehaltlos als Monomer eingesetzt werden. Das Phenethylsalicylatmethacrylat (**17**) mit seinem Schmelzpunkt von 65 °C erfüllt auch die gewünschten Eigenschaften zur Verwendung als Reaktivverdünner. Alle anderen Benzylbeispiele, die verschiedene Substitutionsmuster umfassen, ergeben bei Raumtemperatur flüssige Substanzen und kommen daher als Reaktivverdünner-Monomere in der Photopolymerisation in Frage.

Aus diesen Ergebnissen kann der Fachmann schließen, dass die überwiegende Mehrheit anderer polymerisierbarer Monomere gemäß der vorliegenden Offenbarung, einschließlich gegebenenfalls substituierter Cycloalkylreste, Cycloalkylreste mit aliphatischem Linker und Benzyl- oder Phenethylresten als Estergruppierungen R₁, auch flüssige Verbindungen oder solche mit vergleichsweise niedrigem Schmelzpunkt sein werden.

### Beispiel 23: Gegenwart oder Abwesenheit von Geruch.

Wie oben bereits erwähnt, weisen Salicylate für gewöhnlich einen charakteristischen campherartigen Geruch auf, der von bereits bei Raumtemperatur relativ hoher Flüchtigkeit herrührt, weshalb sie lange Zeit als Aroma- und Duftstoffe eingesetzt wurden. Folglich kann eine einfache Detektion der Gegenwart oder Abwesenheit von Geruch als erste Untersuchung der Flüchtigkeit der Beispiele der vorliegenden Offenbarung und der Referenzbeispiele dienen.

Zu diesem Zweck wurden je 100±5 mg der polymerisierbaren Monomere (**1**) bis (**20**) der Offenbarung und von **Ref1-5** in eine 10-ml-Phiole eingewogen. Die Phiole wurde mit einem Deckel verschlossen und 30 min lang bei Raumtemperatur stehen gelassen. Daraufhin wurde die Kappe von der Phiole entfernt, und eine mögliche Geruchsentwicklung in der Probe wurde durch Riechen an der Probe detektiert. Die in Tabelle 3 angeführten Ergebnisse wurden wie folgt bewertet:
A: kein Geruch detektiert
B: schwacher bis mittlerer Geruch detektiert
C: starker Geruch detektiert

**Tabelle 3: Geruchsdetektion**

| **Beispiel** | **Bewertung** |
|---|---|
| **1** | A |
| **2** | A |
| **3** | A |
| **4** | A |
| **5** | A |
| **6** | A |
| **7** | A |
| **8** | A |
| **9** | A |
| **10** | A |
| **11** | A |
| **12** | A |
| **13** | A |
| **14** | A |
| **15** | A |
| **16** | A |
| **17** | A |
| **18** | A |
| **19** | A |
| **20** | A |
| **Ref1** | A |
| **Ref2** | B |
| **Ref3** | A |
| **Ref4** | C |
| **Ref5** | C |

Wie aus den Ergebnissen in Tabelle 3 ersichtlich ist, wiesen alle Beispiele der vorliegenden Offenbarung (**1**) bis (**20**), **Ref1** und **Ref3** eine Abwesenheit von Geruch auf, so dass von einer sehr niedrigen Flüchtigkeit ausgegangen werden kann. Für **Ref2** wurde ein leicht süßlicher Geruch, der mit "B" bewertet wurde, festgestellt, und für die niedermolekularen Monomere **Ref4 und Ref5** wurde ein starker Geruch, der mit "C" bewertet wurde, festgestellt, was typisch für sehr flüchtige Verbindungen ist. Somit sind **Ref2, Ref4** und **Ref5** für Anwendungen bei erhöhten Temperaturen weniger geeignet.

### Beispiel 24: Flüchtigkeit und thermische Stabilität.

Die Flüchtigkeit und thermische Stabilität wurden mittels simultaner thermischer Analyse (STA) getestet, die eine Kombination aus Thermogravimetrie (TG) und Differential-Scanning-Kalorimetrie (DSC) darstellt. Um die Flüchtigkeit und thermische Stabilität der Beispiele zu bestimmen, wurden STA-Messungen auf einem STA 449F1 Jupiter von Netzsch, das einen Temperaturbereich von -50 °C bis 400 °C abdeckt, unter Verwendung einer Heizrate von 10 K/min durchgeführt. Die Temperaturen, bei denen einen ein 5%iger Masseverlust bzw. ein 10%iger Masseverlust für die Monomere detektiert wurde, wurden als Maß für die Flüchtigkeit herangezogen. Die thermische Stabilität wurde von den aufgezeichneten DSC-Daten abgeleitet, d. h. jener Temperatur, bei der eine durch thermische Polymerisation der Monomere ausgelöste Energiefreisetzung detektiert wird. Hierin wird die Temperatur zu Beginn der exothermen thermischen Polymerisation als Maß für die thermische Stabilität herangezogen. Zusätzlich dazu wurde der Masseverlust der Reaktivverdünner bei einer konstanten Temperatur von 90 °C über eine Zeitspanne von 2 h beobachtet. In diesem Fall wurde ein Masseverlust unter 1 % festgelegt, um Monomere zu identifizieren, die als Reaktivverdünner für Hochtemperaturanwendungen am besten geeignet sind. Die Ergebnisse sind in der nachstehenden Tabelle 4 angeführt.

**Tabelle 4: Flüchtigkeit und thermische Stabilität ("-" nicht beobachtet, "n.d." nicht bestimmt)**

| **Beispiel** | **5 % Masseverlust [°C]** | **10 % Masseverlust [°C]** | **Beginn der thermischen Polymerisation [°C]** | **Masseverlust bei 90 °C nach 2 h [%]** |
|---|---|---|---|---|
| **1** | n.d. | n.d. | n.d. | 0,60 |
| **2** | 164 | 179 | - | 0,90 |
| **3** | 179 | 194 | - | 0,42 |
| **4** | n.d. | n.d. | n.d. | 0,13 |
| **5** | 190 | 209 | - | 0,40 |
| **6** | 171 | 187 | - | 0,82 |
| **7** | 172 | 186 | 225 | 0,43 |
| **8** | n.d. | n.d. | n.d. | 0,08 |
| **9** | 177 | 191 | - | 0,68 |
| **10** | 176 | 192 | - | 0,86 |
| **11** | n.d. | n.d. | n.d. | 0,43 |
| **12** | n.d. | n.d. | n.d. | n.d. |
| **13** | 185 | 200 | - | 0,05 |
| **14** | 195 | 210 | - | 0,18 |
| **15** | 214 | 230 | - | 0,56 |
| **16** | 184 | 198 | - | 0,23 |
| **17** | 196 | 210 | - | 0,21 |
| **18** | n.d. | n.d. | n.d. | 0,22 |
| **19** | n.d. | n.d. | n.d. | 0,42 |
| **20** | n.d. | n.d. | n.d. | 0,80 |
| **Ref1** | 178 | 191 | - | 0,96 |
| **Ref2** | 119 | 132 | - | 47,99 |
| **Ref3** | n.d. | n.d. | n.d. | 0,89 |
| **Ref4** | 91 | 104 | - | 80,30 |
| **Ref5** | n.d. | n.d. | n.d. | >95 (nach 85 min) |

Tabelle 4 zeigt, dass im Handel erhältliches **Ref4** nach 2 h bei 90 °C einen Masseverlust von mehr als 80 % aufwies, was diese Verbindung für Hochtemperaturanwendungen ungeeignet macht. Dasselbe gilt für **Ref5,** das einen Masseverlust von mehr als 95 % aufwies. Methylsalicylatmethacrylat **(Ref2)** ist aufgrund seiner kurzen aliphatischen Seitenkette auch relativ flüchtig. **Ref3** zeigte bei 90 °C einen Masseverlust von nur 0,89 %, was an seiner relativ langen aliphatischen Kette liegt, die seine Flüchtigkeit verringert. Allerdings führen solche azyklischen aliphatischen Ketten üblicherweise zu (Photo-)Polymeren mit eher schlechten thermomechanischen Eigenschaften. Vorteilhafterweise zeigten die polymerisierbaren Monomere (**1**) bis (**20**) gemäß der Offenbarung sowie **Ref1** nach 2 h bei 90 °C auch Masseverluste von unter 1 %. Während sich **Ref1** jedoch immer noch im festen Zustand befand, waren alle Monomere der vorliegenden Offenbarung bei dieser Temperatur bereits flüssig. Das einzige Monomer, für das ein thermischer Polymerisationsbeginn detektiert wurde, war Beispiel (7). Der Beginn der thermischen Polymerisation wurde bei einer Temperatur von 225 °C detektiert, was weit über dem Bereich von 90-120 °C liegt, der üblicherweise als Verarbeitungstemperatur für Lithographie-basierte Hochtemperatur-Photopolymerisationsprozesse festgelegt wird. Alle anderen Beispiele waren thermisch stabil und verdampften, bevor thermische Polymerisation erfolgte. Folglich sind alle polymerisierbaren Monomere (**1**) bis (**20**) der vorliegenden Offenbarung als reaktive, wenig flüchtige Monomere in solchen Prozessen gut geeignet.

### Beispiel 25: Viskosität.

Der Hauptgrund für die Zugabe eines Reaktivverdünners zu einer Harzformulierung besteht darin, ihre Viskosität zu verringern und so die Verarbeitbarkeit des Harzes zu verbessern. Daher wurden Rheologiemessungen der reinen Monomere durchgeführt, wobei ein Temperaturbereich von 25 °C bis 100 °C abgedeckt wurde. Die Messungen wurden auf einer Anton-Paar-MCR-301-Vorrichtung durchgeführt, die mit einem CTD-450-Ofen und einem CP-25-1-Messsystem ausgestattet war, wobei ein Spaltabstand zwischen Stempel (Kegel) und Bodenplatte von 48 µm und eine konstante Scherrate von 50 s⁻¹ eingesetzt wurden.

**Tabelle 5: Viskositäten bei variierenden Temperaturen ("-" bei der angegebenen Temperatur noch in festem Zustand)**

| **Beispiel** | **η bei 25 °C [mPa.s]** | **η bei 40 °C [mPa.s]** | **η bei 50 °C [mPa.s]** | **η bei 70 °C [mPa.s]** | **η bei 90 °C [mPa.s]** | **η bei 100 °C [mPa.s]** |
|---|---|---|---|---|---|---|
| **2** | 217 | 57 | 29 | 11 | 6 | 5 |
| **3** | 1350 | 195 | 72 | 20 | 9 | 6 |
| **4** | 645 | 135 | 62 | 20 | 13 | 10 |
| **5** | 1160 | 191 | 79 | 23 | 10 | 7 |
| **6** | 1330 | 225 | 95 | 28 | 13 | 9 |
| **7** | 610 | 112 | 49 | 15 | 7 | 5 |
| **8** | 1850 | 259 | 104 | 28 | 11 | 8 |
| **9** | - | - | 85 | 25 | 11 | 8 |
| **10** | - | - | - | - | 9 | 7 |
| **11** | 58 | 23 | 14 | 7 | 4 | 3 |
| **12** | 92 | 36 | 22 | 10 | 6 | 5 |
| **13** | 70 | 24 | 14 | 7 | 5 | 4 |
| **14** | 90 | 31 | 19 | 9 | 5 | 4 |
| **15** | 123 | 43 | 25 | 11 | 6 | 5 |
| **16** | 90 | 31 | 18 | 9 | 5 | 4 |
| **17** | - | - | - | 17 | 11 | 9 |
| **18** | 5450 | 360 | 226 | 52 | 20 | |
| **19** | 325 | 82 | 40 | 15 | 7 | 6 |
| **Ref1** | - | - | - | - | - | 6 |
| **Ref2** | 24 | 11 | 8 | 4 | 2 | 2 |
| **Ref3** | 37 | 16 | 10 | 5 | 3 | 2 |
| **Ref4** | 7 | 5 | 4 | 3 | 2 | 2 |
| **Ref5** | 2 | 2 | 2 | 1 | 1 | 1 |

Während die Referenzbeispiele **(Ref2), (Ref4)** und **(Ref5)** bei 25 °C niedrige Viskositäten zeigen, die mit steigenden Temperaturen abnehmen, zeigen diese Verbindungen signifikante Masseverluste bei erhöhten Temperaturen, was ihre Verwendung für Hochtemperaturanwendungen ausschließt. **(Ref1),** das einen Schmelzpunkt von 99-100 °C aufweist, ist bei Temperaturen unter 100 °C fest. Bei 100 °C zeigt es allerdings eine Viskosität der Schmelze von 6 mPa·s. **(Ref3)** hingegen kann bereits bei Raumtemperatur als Reaktivverdünner fungieren. Dennoch wird davon ausgegangen, dass die lange verzweigte aliphatische Seitenkette voraussichtlich Photopolymere mit schlechten thermomechanischen Eigenschaften ergibt.

Von den Beispielen der vorliegenden Offenbarung sind die Beispiele (**2**) bis (**8**), bei denen ein cycloaliphatischer Ring direkt mit der entsprechenden (Meth)acryloylbenzoesäure verestert ist, bei Raumtemperatur flüssig und weisen bei 25 °C Viskositäten zwischen 0,2 und 1,8 Pa·s auf, die der Definition eines Reaktivverdünners (d. h. < 40 mPa·s) nicht entsprechen. Besonders hervorzuheben ist, dass (**2**) mit dem unsubstituierten Cyclohexylring sowie das Acrylat-Derivat (**7**) und das *meta*-Derivat (**4**) niedrigere Viskositätswerte zeigen. Eine Erhöhung der Temperatur führt zu einem exponentiellen Abfall der Viskosität und damit zu Viskositäten unter 40 mPa·s bei 70 °C und ∼10 mPa·s bei 90 °C, was für die Verwendung als Reaktivverdünner bestens geeignet ist. Letzteres gilt auch für alle anderen getesteten Beispiele, d. h. die verbrückten cycloaliphatischen Salicylatmethacrylate (**9**) und (**10**), die bei Raumtemperatur fest sind, aber zweifellos das Potential aufweisen, im geschmolzenen Zustand als Reaktivverdünner zu fungieren. Interessanterweise zeigen die cycloaliphatischen Ester der Beispiele (**11**) und (**12**) bei 25 °C Viskositäten unter 100 mPa·s und nähern sich bereits durch leichtes Erwärmen Viskositäten unter 40 mPa·s an. Dies ist anscheinend auf den kurzen C1- bzw. C2-Alkyl-Linker zurückzuführen, der den cycloaliphatischen Ring mit dem Benzoat verbindet.

Bei den Benzyl-Derivaten (**13**) bis (**16**) wurden ähnliche Ergebnisse beobachtet. Während das Phenylsalicylatmethacrylat **(Ref1)** einen hohen Schmelzpunkt aufweist, sind die Benzylbeispiele bei 25 °C Flüssigkeiten mit Viskositäten knapp über (Beispiel (**15**)), aber größtenteils niedriger als 100 mPa·s (Beispiele (**13**), (**14**), (**16**)). Bereits bei 40 °C erfüllen sie die Anforderungen hinsichtlich der Viskosität für Anwendungen als Reaktivverdünner und stellen ein großes Verarbeitungsfenster z. B. für Stereolithographie-, Tintenstrahl- oder Beschichtungsanwendungen bereit. Und schließlich weist das Phenethylbeispiel (**17**) schon unmittelbar nach dem Schmelzen bei 65 °C eine niedrige Viskosität auf.

Zusammenfassend kann gesagt werden, dass alle Beispiele der vorliegenden Offenbarung die Anforderungen zur Verwendung als Reaktivverdünner leicht erfüllen.

### Beispiel 26: Homopolymerisation und Photoreaktivität.

Um ausreichende Photoreaktivitäten der polymerisierbaren Monomere zu gewährleisten, wurden Photo-Differentialscanningkalorimetrie- (Photo-DSC-) Messungen für eine Auswahl der synthetisierten Substanzen durchgeführt. Zu diesem Zweck wurden die Monomere 15 min lang mit 1 Gew.-% eines im Handel erhältlichen Photoinitiators (TPO-L, Ethyl-(2,4,6-trimethylbenzoyl)phenylphosphinat) in einem Ultraschallbad bei 50 °C (oder oberhalb der der Schmelztemperatur) vermischt. Dann wurden 10±1 mg des entsprechenden Monomers in einen Aluminium-DSC-Tiegel genau eingewogen und unter Verwendung eines Autosamplers in eine DSC-204-F1-Vorrichtung von Netzsch ugeführt, die mit einer Breitband-UV-Lichtquelle (320-500 nm) aus einem Exfo OmniCure™ Series 2000 verbunden war. Die Probe wurde unter N₂-Atmosphäre (N₂-Strom: 20 ml/min) unter Verwendung einer Intensität von 1 W/cm² am Ausgang der Lichtleitfaser, die etwa 20 mW/cm² auf der Probenoberfläche entspricht, 5 min lang bestrahlt. Nach der Messung wurden manche der gehärteten Proben in CDCl₃ gelöst, und ¹H-NMR-Spektren wurden mittels eines Avance-DRX-400-FT-NMR-Spektrometers von BRUKER aufgenommen. Die Doppelbindungsumsätze (DBC; in %) der entsprechenden Monomere wurden auf Basis der entsprechenden integrierten Doppelbindungs-Peakflächen berechnet und sind in den folgenden Tabellen 6 bis 8 zusammen mit den entsprechenden Werten für tₘₐₓ und t₉₅, den Zeiten (in s), bis die maximale Polymerisationstemperatur bzw. 95 % des Umsatzes erreicht wurden, angeführt.

**Tabelle 6: Photo-DSC bei 90 °C**

| **Beispiel** | **tₘₐₓ [s]** | **t₉₅ [s]** | **DBC [%]** |
|---|---|---|---|
| **3** | 8,3 | 35,4 | 87 |
| **6** | 9,3 | 47,5 | 94 |
| **7** | 3,1 | 23,7 | >99 |
| **9** | 7,1 | 38,2 | 81 |
| **10** | 8,0 | 40,2 | 85 |

Wie aus Tabelle 6 ersichtlich ist, zeigten die neuen Methacrylate **(3), (6), (9)** und **(10)** relativ ähnliche Ergebnisse, wobei die Werte für tₘₐₓ im Bereich von etwa 7 bis 9 s, für t₉₅ im Bereich von 35 bis 48 s und für den DBC über 80 % lagen, wobei der höchste Wert für den DBC, 94 %, von **(6)** erreicht wird. Allgemein bestätigen diese Ergebnisse gute Photoreaktivitäten der neuen Methacrylate. Die höchste Photoreaktivität wurde allerdings von **(7)** erreicht, das eine tₘₐₓ von 3,1 s, eine t₉₅ von 23,7 s und einen DBC > 99 % aufwies, was insofern nicht überraschend war, als Acrylate typischerweise höhere Photoreaktivitäten als Methacrylate aufweisen.

Um die Photoreaktivität der höher schmelzenden Verbindung **Ref1,** Phenylsalicylatmethacrylat, messen zu können, wurden weitere Messungen bei 100 °C durchgeführt, wobei die Photoreaktivitäten von **Ref1** mit jenen von **(3)** als repräsentatives cycloaliphatisches Salicylatmethacrylat verglichen wurden.

**Tabelle 7: Photo-DSC bei 100 °C**

| **Beispiel** | **tₘₐₓ [s]** | **t₉₅ [s]** | **DBC [%]** |
|---|---|---|---|
| **3** | 6,5 | 25,9 | 92 |
| **Ref1** | 16,3 | 67,0 | 92 |

Wie in Tabelle 7 gezeigt, zeigt **(3)** eine weit höhere Photoreaktivität als **Ref1.** Die DBCs beider Substanzen waren jedoch identisch (92 %). Diese Ergebnisse zeigen, dass **(3),** als Vertreter der neuen cycloaliphatischen Salicylatmethacrylate, eine deutlich höhere Photoreaktivität als **Ref1** besitzt.

Der hohe Masseverlust der Referenzbeispiele **Ref2, Ref4** und **Ref5** bei 90 °C erforderte eine niedrigere Härtungstemperatur bei den Photo-DSC-Messungen. Die gewählte Temperatur waren 70 °C, da davon ausgegangen wurde, dass ein Verdampfen der Referenzbeispiele während der kurzen Messdauer akzeptabel war. Zum Vergleich wurden **(3)** und **(11)** als Vertreter der cycloaliphatischen Salicylatmethacrylate eingesetzt, wobei **(11)** einen C1-Alkyl-Linker zwischen dem Ester und dem cycloaliphatischen Ring besitzt. Darüber hinaus wurden mehrere Benzyl- und Phenethyl-Derivate **(13)** bis **(17)** unter Verwendung einer Omnicure-LX400-LED-Lichtquelle mit einer Wellenlänge von 365 nm bestrahlt, die auf eine Intensität von 100 % eingestellt war.

**Tabelle 8: Photo-DSC bei 70 °C**

| **Beispiel** | **tₘₐₓ [s]** | **t₉₅ [s]** |
|---|---|---|
| **3** | 8,6 | 36,1 |
| **11** | 13,0 | 74,3 |
| **13** | 15,4 | 41,3 |
| **14** | 9,0 | 77,6 |
| **15** | 12,3 | 41,4 |
| **16** | 7,4 | 39,0 |
| **17** | 15,0 | 45,2 |
| **Ref2** | 53,0 | 70,0 |
| **Ref4** | 21,0 | 38,4 |
| **Ref5** | 13,0 | 74,3 |

Tabelle 8 zeigt, dass **(3),** das eine tmax von 8,6 s und eine t₉₅ von 36,1 s aufweist, was viel schneller war als **Ref2** (53 s / 70 s), **Ref4** (21 s / 38 s) und **Ref5** (13 s / 74 s) polymerisierte. Allerdings weist der zweite cycloaliphatische Vetreter **(11)** eine Reaktivität im Bereich von **Ref5** auf. Von den Benzyl- bzw. Phenethylmonomeren liegen **(13)** und **(17),** die eine tₘₐₓ von 15 s und eine t₉₅ zwischen 40 und 45 s aufweisen, im Bereich von **Ref4** und übertreffen **Ref2** und **Ref5** hinsichtlich der Photoreaktivität deutlich. Der Acrylatvertreter **(16)** weist in den Strukturen mit Benzyl-Seitengruppen die höchste Reaktivität auf. Und schließlich schneiden auch die Beispiele **(14)** und **(15)** hinsichtlich der Photoreaktivität gut ab.

Obwohl die Verbindungen **(2), (4), (5), (8)** und **(12)** in der Photo-DSC-Testreihe nicht getestet wurden, zeigten sie ausgezeichnete Photoreaktivität, als die Testproben für DMA-Messungen homopolymerisiert wurden. Daher kann für alle Beispiele der vorliegenden Offenbarung eine hohe Photoreaktivität festgestellt werden.

### Beispiel 27: Homopolymerisation und dynamisch-mechanische Analyse (DMA).

Es wurden DMA-Messungen von Homopolymerisaten der reinen Monomere durchgeführt, um ihre Formbeständigkeits- und Glasübergangstemperatur (T_{g}) zu untersuchen. Zu diesem Zweck wurde jedes Monomer mit 1 Gew.-% Photoinitiator (TPO-L) vermischt, auf zumindest 70 °C bzw. 10 °C über der Schmelztemperatur erhitzt und in einem "Uvitron International INTELLI-RAY 600 UV"-Ofen unter Verwendung einer Hg-Breitband-UV-Lampe mit 320-500 nm (600 W; UV-A: 125 mW/cm²; Vis: 125 mW/cm²) gehärtet. DMA-Messungen der so erhaltenen Photopolymere wurden unter Verwendung eines TA Instrument 2980 im 3-Punkt-Biegemodus, eingestellt auf eine Amplitude von 10 µm, einer statischen Kraft von 0,050 N und einer Autospannung von 125 % durchgeführt. Das Temperaturprogramm für jede Messung war auf -50 °C bis 200 °C und eine Heizrate von 3 K/min eingestellt. Tabelle 9 gibt die resultierenden Werte für die Speichermoduln bei 20 °C, G'_{(20°)}, die Glasübergangstemperaturen, die beim Maximum des tan-δ-Diagramms gemessen wurden, T_{g} und die Temperaturen an, bei denen G' 1000 MPa erreichte, T₁₀₀₀, an.

**Tabelle 9: Dynamisch-mechanische Analyse**

| **Photopolymer von Beispiel** | **G'₍₂₀₎ [MPa]** | **T_{g} [°C]** | **T₁₀₀₀ [°C]** |
|---|---|---|---|
| **2** | 2197 | 118 | 85 |
| **3** | 2779 | 112 | 85 |
| **4** | 2726 | 113 | 85 |
| **5** | 2746 | 114 | 68 |
| **6** | 2477 | 129 | 100 |
| **7** | 2027 | 111 | 72 |
| **8** | 2130 | 138 | 100 |
| **9** | 2267 | 154 | 105 |
| **10** | 2576 | 148 | 116 |
| **11** | 2654 | 123 | 97 |
| **12** | 1850 | 77 | 47 |
| **13** | 3101 | 90 | 71 |
| **14** | 3257 | 108 | 79 |
| **15** | 2353 | 69 | 36 |
| **16** | 2355 | 67 | 51 |
| **17** | 2958 | 81 | 63 |
| **Ref2** | 2437 | 87 | 57 |
| **Ref3** | 1080 | 59 | 22 |
| **Ref4** | 1540 | 149 | 75 |
| **Ref5** | 2198 | 78 | 59 |

Wie aus der industriellen Praxis bekannt, ergibt das flüchtige **Ref4** sehr gute thermomechanische Eigenschaften, im Speziellen hohe Glasübergangstemperaturen. Nichtsdestotrotz schnitten die Homopolymere der cycloaliphatischen Beispiele **(2)** bis **(11)** in dieser Hinsicht sehr gut ab, wobei alle eine T_{g} von 110 °C überschritten. Insbesondere **(9)** und **(10),** die eine T_{g} ∼150 °C aufweisen, können klar mit **Ref4** konkurrieren. **(12)** ergibt bereits Photopolymere mit niedrigerer T_{g}, da der längere C2-Alkyl-Spacer zwischen dem Ester und der cycloaliphatischen Gruppierung wahrscheinlich zu einem weicheren Material führt.

Im Gegensatz dazu ergab das relativ flüchtige **Ref2,** Methylsalicylatmethacrylat, das einen sehr kurzen Methylesterrest umfasst, ein Polymer mit einer T_{g} von nur 87 °C, was jedoch ein annehmbarer Wert ist. Weiters können längere aliphatische Ketten zwar in Bezug auf eine Verringerung der Flüchtigkeit vorteilhaft sein, senken aber gleichzeitig die Glasübergangs- und die Wärmeformbeständigkeitstemperatur. Dies wurde durch die Ergebnisse von **Ref3,** 2-Ethylhexylsalicylatmethacrylat, bestätigt, das eine T_{g} von 59 °C und den bei Weitem niedrigsten T₁₀₀₀-Wert von 22 °C ergab, was für eine Verwendung solcher Polymere als Komponenten von beispielsweise kieferorthopädischen Vorrichtungen deutlich zu niedrig ist. Es wurde davon ausgegangen, dass **Ref1** eine hohe T_{g} und gute thermomechanische Eigenschaften zeigt. Wiederholte Versuche, Proben zu formen, waren jedoch nicht erfolgreich. Grund dafür war die hohe Sprödigkeit der unter Verwendung von **Ref1** erhaltenen Photopolymere, die durchwegs zu gebrochenen Proben führte. Das flüchtigste Monomerbeispiel, **Ref5,** ergab ein Photopolymer mit einer ausreichenden T_{g} von 78 °C.

**Aus** einer Analyse der Benzyl- bzw. Phenethyl(meth)acryloyloxybenzoate **(13)** bis **(17)** ist ersichtlich, dass die T_{g} niedriger als jene des cycloaliphatischen (Meth)-acryloyloxybenzoats liegt und üblicherweise 100 °C nicht überschreitet. Trotzdem zeigen **(13), (14)** und **(17)** eine T_{g} über 80 °C, was immer noch höher als z. B. **Ref3** und **Ref5** ist. Beispiel **(15)** mit der Methacrylatgruppe in meta-Position zeigt eine relativ niedrige T_{g} von 69 °C, die wahrscheinlich auf die Isopropylgruppierung am aromatischen Ring zurückzuführen ist. Das Polymer von **(16)** führt zu dem Material mit der niedrigsten T_{g} (67 °C), was insofern nicht überrascht, als Polyacrylate typischerweise Materialien mit schlechteren thermomechanischer Leistung als Polymethacrylate ergeben. Die Beispiele **(15)** und **(16)** übertreffen das nichtzyklische, verzweigt-aliphatische Methacryloyloxybenzoat **(Ref3)** in Bezug auf die T_{g} und insbesondere den T₁₀₀₀-Wert deutlich.

Zusammenfassend kann festgestellt werden, dass alle getesteten homopolymerisierten Beispiele der vorliegenden Offenbarung gute thermomechanische Eigenschaften, insbesondere relativ hohe Glasübergangstemperaturen und T₁₀₀₀-Werte, zeigten.

### Beispiel 28: Verwendung als Reaktivverdünner.

Um die Zweckmäßigkeit der polymerisierbaren Monomere der vorliegenden Offenbarung als Reaktivverdünner zu zeigen, wurden **(3)** und **(13)** als repräsentative Beispiele herangezogen und als Reaktivverdünner mit hochviskosen, im Handel erhältlichen Harzen vermischt. Als beispielhafte Harze wurden BOMAR XR-741MS, ein niedermolekulares, difunktionelles aliphatisches Polyesterurethanmethacrylat, erhältlich von Dymax ("Bomar"), und Miramer UA5216, ein aliphatisches difunktionelles Acrylat mit einem zahlenmittleren Molekulargewicht von 30.000 Da, erhältlich von Miwon (ohne Isobornylacrylat) ("Miwon"), eingesetzt. Diese Harze wurden als solche (Vergleichsbeispiele 1 und 2) und im Gemisch (bei 90 °C) mit 30 Gew.-% **(3)** oder **(13)** jeweils als Reaktivverdünner (Beispiele **(29)** bis **(32))** getestet und polymerisiert, um vernetzte Polymere in Abwesenheit (Vergleichsbeispiele 3 und 4) oder Gegenwart (Beispiele **(33)** bis **(36)**) eines polymerisierbaren Monomers **(3)** oder **(13)** als Reaktivverdünner zu erhalten.

### Beispiel 37: Viskositäten von Photoharzen.

Um die Verdünnungswirkung des polymerisierbaren Monomers, das als Reaktivverdünner in einem viskosen Harz eingesetzt wurde, zu untersuchen, wurden Rheologiemessungen der obigen Harze durchgeführt, die einen Temperaturbereich von 25 °C bis 100 °C abdeckten. Die Messungen wurden unter Verwendung eines Anton Paar MCR 301 mit einem CTD-450-Ofen und einem CP-25-1-Messsystem erneut durchgeführt. Die Messungen wurden unter Verwendung eines Spaltabstands zwischen Stempel (Kegel) und Bodenplatte von 48 µm und einer konstanten Scherrate von 50 s⁻¹ durchgeführt.

**Tabelle 10: Viskositäten bei variierenden Temperaturen**

| **Beispiel** | **Harz** | **η bei 25 °C [Pa·s]** | **η bei 50 °C [Pa·s]** | **η bei 70 °C [Pa·s]** | **η bei 90 °C [Pa·s]** |
|---|---|---|---|---|---|
| **Vergl.-Bsp. 1** | Bomar | 29.070 | 167,7 | 12,2 | 1,8 |
| **29** | Bomar + **(3)** | 1.250 | 16,2 | 1,6 | 0,3 |
| **30** | Bomar + **(13)** | 124,6 | 3,9 | 0,6 | 0,2 |
| **Vergl.-Bsp. 2** | Miwon | 5.300 | 99,6 | 28,0 | 12,0 |
| **31** | Miwon + **(3)** | 734 | 78,7 | 20,0 | 6,7 |
| **32** | Miwon + **(13)** | 343,3 | 58,5 | 15,8 | 5,5 |

Wie in Tabelle 10 gezeigt, beginnt das reine Bomar-Harz aus Vergleichsbeispiel 1 bei einer sehr hohen Viskosität von mehr als 29.000 Pa·s, wobei es sich bei Raumtemperatur (25 °C) praktisch wie ein Feststoff verhält. Bei 50 °C wurde eine Viskosität von etwa 170 Pa·s gemessen, während die Viskosität bei 70 °C einen wünschenswerten Wert von etwa 10 Pa·s erreichte. Bei 90 °C betrug die Viskosität des reinen Bomar 1,8 Pa·s. Durch Zusatz von 30 Gew.-% von **(3)** zu dem Bomar-Harz in Beispiel **(29)** fiel die ursprüngliche Viskosität bei Raumtemperatur drastisch auf 1.250 Pa·s. Bei 50 °C führte die Verdünnungswirkung bereits zu einer Viskosität von etwa 16 Pa·s und sank weiter auf ausgezeichnete Werte von 1,6 und 0,3 Pa·s bei 70 °C bzw. 90 °C. Der Zusatz von 30 Gew.-% **(13)** zu Bomar in Beispiel **(30)** senkte die Viskosität bei 25 °C auf 124,6 Pa·s und bei 50 °C sank sie weiter auf 3,9 Pa·s und fiel bei einer Temperatur über 70 °C sogar unter 1 Pa·s.

Ähnliche Wirkungen konnten für das modifizierte Miwon-Harz (ohne Isobornylacrylat) in Vergleichsbeispiel 2 beobachtet werden. Beginnend bei einer Viskosität von 5.300 Pa·s bei 25 °C, verringerte eine Temperaturerhöhung auf 50 °C die Viskosität bereits auf etwa 100 Pa·s. Eine weitere Erhöhung auf 70 °C bzw. 90 °C führte zu Viskositäten von 28 Pa·s und 12 Pa·s. Vermischen des modifizierten Miwon-Harzes mit 30 Gew.-% **(3)** in Beispiel **(31)** führte erneut zu einer signifikanten Abnahme der Viskosität bei Raumtemperatur. Als die Temperatur auf 50 °C, 70 °C bzw. 90 °C erhöht wurde, waren die Viskositäten des Gemischs auch deutlich niedriger als jene des reinen Harzes. Dasselbe gilt für die Zugabe von 30 Gew.-% **(13)** in Beispiel **(32),** die sogar zu niedrigeren Viskositäten als bei Beispiel **(31)** führte, wobei der gesamte Temperaturbereich abgedeckt wird.

Folglich stellen die Beispiele **(3)** und **(13)** der Offenbarung ein leistungsstarkes Werkzeug zur Verdünnung hochviskoser kommerzieller Harze dar, um ihre Verarbeitbarkeit zu verbessern.

### Beispiel 38: DMA von Photopolymeren.

Um die thermomechanischen Eigenschaften der vernetzten Photopolymere, die aus den gehärteten Harzen resultieren, zu bestimmen, wurden die vier Gemische der Beispiele **(33)** bis **(36)** und der Vergleichsbeispiele 1 und 2 mit 1 Gew.-% Photoinitiator (TPO-L) vermischt. Die Proben wurden wie in Beispiel **27** beschrieben polymerisiert. Aufgrund der niedrigen Glasübergangstemperaturen, die aus dem Härten des Miwon-Harzes resultieren, wurde das Temperaturprogramm der Messungen auf -70 °C bis 100 °C und eine Heizrate von 3 K/min eingestellt. Tabelle 11 gibt wieder die erhaltenen Werte für die Speichermoduln bei 20 °C, G'_{(20°)}, die Glasübergangstemperaturen, die beim Maximum des tan-δ-Diagramms gemessen wurden, T_{g} sowie die Temperaturen, bei denen G' 1000 MPa erreichte, T₁₀₀₀, an.

**Tabelle 11: Dynamisch-mechanische Analyse der vernetzten Photopolymere**

| **Beispiel** | **Polymer** | **G'₍₂₀₎ [MPa]** | **T_{g} [°C]** | **T₁₀₀₀ [°C]** |
|---|---|---|---|---|
| **Vergl.-Bsp. 3** | Bomar | 2750 | 128 | 79 |
| **33** | Bomar + **(3)** | 2340 | 119 | 68 |
| **34** | Bomar + **(13)** | 3424 | 110 | 81 |
| **Vergl.-Bsp. 4** | Miwon | 5 | -18 | -42 |
| **35** | Miwon + **(3)** | 63 | -16/52 | -22 |
| **36** | Miwon + **(13)** | 22 | 21 | -22 |

Wie in Tabelle 11 gezeigt, ergab das reine Bomar-Harz aus Vergleichsbeispiel 3 eine hohe T_{g} (128 °C) sowie hohe Werte für G'₍₂₀₎ (2750 MPa) und T₁₀₀₀ (79 °C). Der Zusatz von 30 Gew.-% **(3)** als Reaktivverdünner in Beispiel **(33)** verringerte die T_{g} des resultierenden Photopolymers nur geringfügig auf 119 °C. Eine ähnlich geringfügige Abnahme von etwa 10 % wurde für den T₁₀₀₀-Wert beobachtet. Wie erwartet führte die Zugabe von **(3)** allerdings auch zu einem niedrigeren Wert für G'₍₂₀₎. Beimischen von 30 Gew.-% **(13)** zu Bomar in Beispiel **(34)** verringerte die T_{g} auf 110 °C, was 18 °C unter dem reinen Bomar-Photopolymer liegt und damit immer noch ein annehmbarer Wert war. Zusammengefasst hatte die Zugabe von **(3)** und **(13)** als beispielhafte Reaktivverdünner zu einem Harz, das hohe Formbeständigkeitstemperaturen ergab, nur geringe Auswirkungen auf die thermomechanischen Eigenschaften des Materials, verbesserte seine Verarbeitbarkeit aber beträchtlich.

Andererseits zeigte das Photopolymer, das aus dem reinen modifizierten Miwon-Harz in Vergleichsbeispiel 4 resultierte, bereits bei Raumtemperatur relativ schlechte thermomechanische Eigenschaften, d. h. eine sehr weiche Beschaffenheit, als die Probe mit den Fingern gebogen wurde. Diese Beobachtung wurde durch die mittels DMA bestimmten Werte bestätigt, wie in Tabelle 11 oben angegeben. In diesem Fall führte die Zugabe der polymerisierbaren Monomere **(3)** und **(13)** als Reaktivverdünner zu einer signifikanten Zunahme aller untersuchten Eigenschaften. In Bezug auf die T_{g} von Beispiel **(35)** ist anzumerken, dass zwei tan-δ-Maxima detektiert wurden: eines bei -16 °C, das aus den flexiblen Ketten von Miwon resultierte, und ein weiteres bei 52 °C. Beispiel **(36)** zeigt wiederum einen signifikanten Anstieg der T_{g} (21 °C) und der anderen thermomechanischen Eigenschaften. Allerdings war das Photopolymer, das von den Reaktivverdünnern **(3)** bzw. **(13)** stammenden Grundeinheiten enthält, bei Raumtemperatur viel steifer als das aus reinem Miwon resultierende Polymer.

Aus den Eigenschaften der neuen polymerisierbaren Monomere **(1)** bis **(21)** gemäß der vorliegenden Erfindung, wie in den obigen Tabellen 2 bis 11 angeführt, und den daraus ableitbaren Tendenzen kann eindeutig gefolgert werden, dass alle diese Monomere als Reaktivverdünner für eine große Bandbreite an hochviskosen Harzen geeignet sind und zu vorteilhaften thermomechanischen Eigenschaften der durch (Photo-)Polymerisation derselben erhaltenen Polymere führen.

Zusammengenommen dokumentieren die oben diskutierten Beispiele, Referenz- und Vergleichsbeispiele klar die Eignung von Cycloalkyl- sowie Benzyl- und Phenethyl(meth)acryloyloxybenzoaten gemäß der vorliegenden Offenbarung, dargestellt durch die Formeln (I), (II) und (III), als polymerisierbare Monomere und Reaktivverdünner für hochviskose Harze, die vorzugsweise unter Verwendung eines Lithographie-basierten Hochtemperatur-Photopolymerisationsprozesses polymerisiert werden sollen, um gegebenenfalls vernetzte (Photo-)Polymere mit vorteilhaften thermomechanischen Eigenschaften für eine mögliche Verwendung als kieferorthopädische Vorrichtungen zu erhalten.

Die hierin verwendeten Begriffe und Ausdrücke werden als beschreibende und nicht einschränkende Begriffe verwendet, und bei der Verwendung solcher Begriffe und Ausdrücke sollen keinerlei Äquivalente der gezeigten und beschriebenen Merkmale oder Teile davon ausgeschlossen sein, wobei es sich jedoch versteht, dass innerhalb des beanspruchten Schutzumfangs der Erfindung verschiedene Modifikationen möglich sind. Daher versteht es sich, dass, obwohl die vorliegende Erfindung anhand bestimmter Ausführungsformen, beispielhafter Ausführungsformen und optionaler Merkmale spezifisch offenbart wurde, Fachleute auf dem Gebiet der Erfindung auf Modifikationen und Variationen der hierin offenbarten Konzepte zurückgreifen können und dass solche Modifikationen und Variationen als im Schutzumfang der Erfindung, wie durch die beigefügten Ansprüche definiert, umfasst erachtet werden. Die hierin bereitgestellten spezifischen Ausführungsformen sind Beispiele für zweckmäßige Ausführungsformen der vorliegenden Erfindung, und Fachleuten auf dem Gebiet der Erfindung ist klar, dass die vorliegende Erfindung unter Verwendung zahlreicher Variationen der Vorrichtungen, Vorrichtungskomponenten und Verfahrensstufen, die in der vorliegenden Beschreibung angeführt sind, durchgeführt werden kann.

Wenn hierin eine Gruppe von Substituenten offenbart wird, versteht es sich, dass alle einzelnen Mitglieder dieser Gruppe und alle Untergruppen, einschließlich jeglicher Isomere, Enantiomere und Diastereomere der Gruppenmitglieder, separat offenbart werden. Wenn hierin eine Markush-Gruppe oder eine andere Gruppierung verwendet wird, ist beabsichtigt, dass alle einzelnen Mitglieder der Gruppe und alle für die Gruppe möglichen Kombinationen und Unterkombinationen einzeln in der Offenbarung umfasst sind. Wenn eine Verbindung so hierin beschrieben wird, dass ein bestimmtes Isomer, Enantiomer oder Diastereomer der Verbindung nicht spezifiziert ist, beispielsweise in einer Formel oder in einer chemischen Bezeichnung, soll diese Beschreibung alle Isomere und Enantiomere der beschriebenen Verbindung einzeln oder in einer beliebigen Kombination umfassen. Wenn nicht anders angegeben, sollen zusätzlich dazu alle Isotopenvarianten von hierin offenbarten Verbindungen von der Offenbarung umfasst sein. Spezifische Bezeichnungen für Verbindungen sind beispielhaft gemeint, zumal bekannt ist, dass Fachleute auf dem Gebiet der Erfindung dieselben Verbindungen unterschiedlich bezeichnen können.

Es ist anzumerken, dass die Singularformen "ein(e)" und "der/die/das", wie hierin und in den beigefügten Ansprüchen verwendet, auch Pluralbezüge umfassen, wenn der Kontext nicht klar etwas anderes vorgibt. Somit umfasst beispielsweise die Bezugnahme auf "eine Zelle" eine Vielzahl solcher Zellen und Äquivalente davon, die Fachleuten auf dem Gebiet der Erfindung bekannt sind, usw. Ebenso können die Begriffe "ein(e)", "ein(e) oder mehrere" und "zumindest ein(e)" hierin austauschbar verwendet werden. Es ist auch anzumerken, dass die Begriffe "umfassend", "einschließlich" und "aufweisend" austauschbar verwendet werden können.

Wenn nicht anders definiert, haben alle hierin verwendeten technischen und wissenschaftlichen Begriffe jene Bedeutungen, wie sie für gewöhnlich von Fachleuten auf dem Gebiet der Erfindung verstanden werden. Obwohl in der praktischen Durchführung oder Testung der vorliegenden Erfindung beliebige Verfahren und Materialien eingesetzt werden können, die zu den hierin beschriebenen ähnlich oder äquivalent sind, werden nun manche Verfahren und Materialien beschrieben. Nichts hierin soll als Zugeständnis ausgelegt werden, dass die Erfindung nicht als frühere Erfindung eine solche Offenbarung vorwegnehmen kann.

Jede hierin beschriebene oder exemplifizierte Formulierung oder Kombination von Komponenten kann eingesetzt werden, um die Erfindung praktisch durchzuführen, wenn nicht anders angegeben.

Sooft in dieser Patentschrift ein Bereich, beispielsweise ein Temperaturbereich, ein zeitlicher Bereich oder ein Zusammensetzungs- oder Konzentrationsbereich, angegeben ist, sollen alle Zwischen- und Unterbereiche sowie alle Einzelwerte, die in den gegebenen Bereichen umfasst sind, von der Offenbarung umfasst sein. Wie hierin verwendet, umfassen Bereiche spezifisch die Werte, die als Endpunktwerte des Bereichs bereitgestellt sind. Beispielsweise umfasst ein Bereich von 1 bis 100 spezifisch die Endpunktwerte 1 und 100. Es versteht sich, dass jegliche Unterbereiche oder Einzelwerte in einem Bereich oder Unterbereich, die in der Beschreibung hierin umfasst sind, von den Ansprüchen hierin ausgeschlossen werden können.

Wie hierin verwendet, ist "umfassend" synonym zu "einschließlich", "enthaltend" oder "gekennzeichnet durch" und ist einschließend oder offen und schließt zusätzliche, nicht genannte Elemente oder Verfahrensschritte nicht aus. Wie hierin verwendet, schließt "bestehend aus" jegliche(s)/jeglichen Element, Stufe oder Bestandteil, das/ die/der im Anspruchselement nicht spezifiziert ist, aus. Wie hierin verwendet, schließt "im Wesentlichen bestehend aus" Materialien oder Schritte, die die grundlegenden und neuen Merkmale des Anspruchs nicht erheblich beeinflussen, nicht aus. In jedem hierin umfassten Fall können beliebige der Begriffe "umfassend", "bestehend im Wesentlichen aus" und "bestehend aus" durch jeden der anderen beiden Begriffe ersetzt werden. Die hierin veranschaulichend beschriebene Erfindung kann entsprechend in Abwesenheit eines/r oder mehrerer beliebigen/r Elements oder Elemente, Einschränkung oder Einschränkungen, das/die hierin nicht spezifisch offenbart ist/sind, praktisch durchgeführt werden.

Dem Durchschnittsfachmann auf dem Gebiet der Erfindung ist klar, dass Ausgangsmaterialien, biologische Materialien, Reagenzien, Syntheseverfahren, Reinigungsverfahren, Analyseverfahren, Testverfahren und biologische Verfahren, die nicht die spezifisch exemplifizierten sind, bei der praktischen Durchführung der Erfindung ohne übermäßiges Experimentieren eingesetzt werden können. Alle fachbekannten funktionellen Äquivalente beliebiger solcher Materialien und Verfahren sollen in der vorliegenden Erfindung umfasst sein. Die eingesetzten Begriffe und Ausdrücke werden als beschreibende und nicht einschränkende Begriffe verwendet, und bei der Verwendung solcher Begriffe und Ausdrücke sollen keinerlei Äquivalente der gezeigten und beschriebenen Merkmale oder Teile davon ausgeschlossen sein, wobei jedoch anerkannt ist, dass innerhalb des beanspruchten Schutzumfangs der Erfindung verschiedene Modifikationen möglich sind. Daher versteht es sich, dass, obwohl die vorliegende Erfindung spezifisch durch bestimmte Ausführungsformen und optionale Merkmale offenbart wurde, Fachleute auf dem Gebiet der Erfindung auf Modifikationen und Variationen der hierin offenbarten Konzepte zurückgreifen können und dass solche Modifikationen und Variationen als vom durch die beiliegenden Ansprüche definierten Schutzumfang der Erfindung umfasst erachtet werden.

### AUSFÜHRUNGEN IN BEZUG AUF CHEMISCHE VERBINDUNGEN UND NOMENKLATUR

Wie hierin verwendet, kann sich der Begriff "Gruppe" auf eine funktionelle Gruppe einer chemischen Verbindung beziehen. Gruppen der vorliegenden Verbindungen beziehen sich auf ein Atom oder ein Kollektiv von Atomen, die Teil der Verbindung sind. Gruppen der vorliegenden Erfindung können über eine oder mehrere kovalente Bindungen an andere Atome der Verbindung gebunden sein. Gruppen können auch in Bezug auf ihren Valenzzustand charakterisiert werden. Die vorliegende Erfindung umfasst Gruppen, die durch einen einwertigen, zweiwertigen, dreiwertigen etc. Valenzzustand charakterisiert sind.

Wie hierin verwendet, bezieht sich der Begriff "substituiert" auf eine Verbindung, in der ein Wasserstoff durch eine andere funktionelle Gruppe ersetzt ist.

Alkylgruppen umfassen unverzweigte, verzweigte und zyklische Alkylgruppen. Alkylgruppen umfassen jene, die 1 bis 30 Kohlenstoffatome aufweisen. Alkylgruppen umfassen kleine Alkylgruppen mit 1 bis 3 Kohlenstoffatomen. Alkylgruppen umfassen Alkylgruppen mittlerer Länge mit 4 bis 10 Kohlenstoffatomen. Alkylgruppen umfassen lange Alkylgruppen mit mehr als 10 Kohlenstoffatomen, insbesondere jene mit 10 bis 30 Kohlenstoffatomen. Der Begriff "Cycloalkyl" bezieht sich spezifisch auf eine Alkylgruppe mit einer Ringstruktur, wie z. B. einer Ringstruktur, die 3 bis 30 Kohlenstoffatome, gegebenenfalls 3 bis 20 Kohlenstoffatome und gegebenenfalls 3 bis 10 Kohlenstoffatome, umfasst, einschließlich Alkylgruppen mit einem oder mehreren Ringen. Cycloalkylgruppen umfassen jene mit einem oder mehreren 3-, 4-, 5-, 6-, 7-, 8-, 9- oder 10-gliedrigen Kohlenstoffringen und insbesondere jene mit einem oder mehreren 3-, 4-, 5-, 6-, 7- oder 8-gliedrigen Ringen. Die Kohlenstoffringe in Cycloalkylgruppen können auch Alkylgruppen tragen. Cycloalkylgruppen können bizyklische und trizyklische Alkylgruppen umfassen. Alkylgruppen sind gegebenenfalls substituiert. Substituierte Alkylgruppen umfassen unter anderem jene, die mit Arylgruppen substituiert sind, die wiederum gegebenenfalls substituiert sind. Spezifische Alkylgruppen umfassen Methyl-, Ethyl-, n-Propyl-, Isopropyl-, Cyclopropyl-, n-Butyl-, sec-Butyl-, tert-Butyl-, Cyclobutyl-, n-Pentyl-, verzweigte Pentyl-, Cyclopentyl-, n-Hexyl-, verzweigte Hexyl- und Cyclohexylgruppen, die gegebenenfalls substituiert sind. Substituierte Alkylgruppen umfassen vollständig halogenierte oder semihalogenierte Alkylgruppen, wie z. B. Alkylgruppen, in denen ein oder mehrere Wasserstoffe durch ein oder mehrere Fluoratome, Chloratome, Bromatome und/oder lodatome ersetzt sind. Substituierte Alkylgruppen umfassen vollständig fluorierte oder semifluorierte Alkylgruppen, wie z. B. Alkylgruppen, in denen ein oder mehrere Wasserstoffe durch ein oder mehrere Fluoratome ersetzt sind. Eine Alkoxygruppe ist eine durch Bindung an Sauerstoff modifizierte Alkylgruppe, die durch die Formel R-O dargestellt werden kann und auch als Alkylethergruppe bezeichnet werden kann. Beispiele für Alkoxygruppen umfassen, ohne darauf eingeschränkt zu sein, Methoxy, Ethoxy, Propoxy, Butoxy und Heptoxy. Alkoxygruppen umfassen substituierte Alkoxygruppen, worin der Alkylteil der Gruppen substituiert ist, wie hierin in Verbindung mit der Beschreibung von Alkylgruppen erwähnt. Wie hierin verwendet, bezieht sich MeO- auf CH₃O-.

Alkenylgruppen umfassen unverzweigte, verzweigte und zyklische Alkenylgruppen. Alkenylgruppen umfassen jene mit 1, 2 oder mehr Doppelbindungen und jene, in denen zwei oder mehr der Doppelbindungen konjugierte Doppelbindungen sind. Alkenylgruppen umfassen jene mit 2 bis 20 Kohlenstoffatomen. Alkenylgruppen umfassen kleine Alkenylgruppen mit 2 bis 3 Kohlenstoffatomen. Alkenylgruppen umfassen Alkenylgruppen mittlerer Länge mit 4 bis 10 Kohlenstoffatomen. Alkenylgruppen umfassen lange Alkenylgruppen mit mehr als 10 Kohlenstoffatomen, insbesondere jene mit 10 bis 20 Kohlenstoffatomen. Cycloalkenylgruppen umfassen jene, in denen eine Doppelbindung im Ring oder in einer an einen Ring gebundenen Alkenylgruppe vorliegt. Der Begriff "Cycloalkenyl" bezieht sich spezifisch auf eine Alkenylgruppe mit einer Ringstruktur, einschließlich einer Alkenylgruppe mit einem oder mehreren 3-, 4-, 5-, 6-, 7-, 8-, 9- oder 10-gliedrigen Kohlenstoffringen, und insbesondere jene mit einem oder mehreren 3-, 4-, 5-, 6-, 7- oder 8- gliedrigen Ringen. Die Kohlenstoffringe in Cycloalkenylgruppen können auch Alkylgruppen tragen. Cycloalkenylgruppen können bizyklische und trizyklische Alkenylgruppen umfassen. Alkenylgruppen sind gegebenenfalls substituiert. Substituierte Alkenylgruppen umfassen u. a. jene, die mit Alkyl- oder Arylgruppen substituiert sind, wobei diese Gruppen wiederum gegebenenfalls substituiert sind. Spezifische Alkenylgruppen umfassen Ethenyl, Prop-1-enyl, Prop-2-enyl, Cycloprop-1-enyl, But-1-enyl, But-2-enyl, Cyclobut-1-enyl, Cyclo-but-2-enyl, Pent-1-enyl, Pent-2-enyl, verzweigtes Pentenyl, Cyclopent-1-enyl, Hex-1-enyl, verzweigtes Hexenyl, Cyclohexenyl, die alle gegebenenfalls substituiert sind. Substituierte Alkenylgruppen umfassen vollständig halogenierte oder semihalogenierte Alkenylgruppen, wie z. B. Alkenylgruppen, in denen ein oder mehrere Wasserstoffe durch ein oder mehrere Fluoratome, Chloratome, Bromatome und/oder lodatome ersetzt sind. Substituierte Alkenylgruppen umfassen vollständig fluorierte oder semifluorierte Alkenylgruppen, wie z. B. Alkenylgruppen, in denen ein oder mehrere Wasserstoffatome durch ein oder mehrere Fluoratome ersetzt sind.

Arylgruppen umfassen Gruppen mit einem oder mehreren 5-, 6-, 7- oder 8-gliedrigen aromatischen Ringen, einschließlich heterozyklischer aromatischer Ringe. Der Begriff "Heteroaryl" bezieht sich spezifisch auf Arylgruppen mit zumindest einem 5-, 6-, 7- oder 8-gliedrigen heterozyklischen aromatischen Ring. Arylgruppen können einen oder mehrere kondensierte aromatische Ringe enthalten, einschließlich eines oder mehrerer kondensierter heteroaromatischer Ringe und/oder einer Kombination aus einem oder mehreren aromatischen Ringen und einem oder mehreren nichtaromatischen Ringen, die kondensiert oder über kovalente Bindungen verbunden sein können. Heterozyklische aromatische Ringe können ein oder mehrere N-, O- oder S-Atome im Ring umfassen. Heterozyklische aromatische Ringe können jene mit einem, zwei oder drei N-Atomen, jene mit einem oder zwei O-Atomen und jene mit einem oder zwei S-Atomen oder Kombinationen aus einem oder zwei oder drei N-, O- oder S-Atomen umfassen. Arylgruppen sind gegebenenfalls substituiert. Substituierte Arylgruppen umfassen u. a. jene, die mit Alkyl- oder Alkenylgruppen substituiert sind, wobei diese Gruppen wiederum gegebenenfalls substituiert sind. Spezifische Arylgruppen umfassen Phenyl, Biphenylgruppen, Pyrrolidinyl, Imidazolidinyl, Tetrahydrofuryl, Tetrahydrothienyl, Furyl, Thienyl, Pyridyl, Chinolyl, Isochinolyl, Pyridazinyl, Pyrazinyl, Indolyl, Imidazolyl, Oxazolyl, Thiazolyl, Pyrazolyl, Pyridinyl, Benzoxadiazolyl, Benzothiadiazolyl und Naphthylgruppen, die alle gegebenenfalls substituiert sind. Substituierte Arylgruppen umfassen vollständig halogenierte oder semihalogenierte Arylgruppen, wie z. B. Arylgruppen, in denen ein oder mehrere Wasserstoffe durch ein oder mehrere Fluoratome, Chloratome, Bromatome und/oder lodatome ersetzt sind. Substituierte Arylgruppen umfassen vollständig fluorierte oder semifluorierte Arylgruppen, wie z. B. Arylgruppen, in denen ein oder mehrere Wasserstoffe durch ein oder mehrere Fluoratome ersetzt sind. Arylgruppen umfassen, ohne darauf eingeschränkt zu sein, aromatische Gruppen enthaltende oder heterozyklische aromatische Gruppen enthaltende Gruppen, die beliebigen der folgenden entsprechen: Benzol, Naphthalin, Naphthochinon, Diphenylmethan, Fluoren, Anthracen, Anthrachinon, Phenanthren, Tetracen, Tetracendion, Pyridin, Chinolin, Isochinolin, Indole, Isoindol, Pyrrol, Imidazol, Oxazol, Thiazol, Pyrazol, Pyrazin, Pyrimidin, Purin, Benzimidazol, Furane, Benzofuran, Dibenzofuran, Carbazol, Acridin, Acridon, Phenanthridin, Thiophen, Benzothiophen, Dibenzothiophen, Xanthen, Xanthon, Flavon, Cumarin, Azulen oder Anthracyclin. Wie hierin verwendet, umfasst eine Gruppe, die den oben angeführten Gruppen entspricht, ausdrücklich eine aromatische oder heterozyklische aromatische Gruppe, einschließlich einwertiger, zweiwertiger und mehrwertiger Gruppen der hierin angeführten aromatischen und heterozyklischen aromatischen Gruppen, die in einer kovalent gebundenen Konfiguration in den Verbindungen der Erfindung an einer beliebigen geeigneten Bindungsstelle bereitgestellt sind. In Ausführungsformen enthalten Arylgruppen zwischen 5 und 30 Kohlenstoffatomen. In Ausführungsformen enthalten Arylgruppen einen aromatischen oder heteroaromatischen sechsgliedrigen Ring und einen oder mehrere zusätzliche fünf- oder sechsgliedrige aromatische oder heteroaromatische Ringe. In Ausführungsformen enthalten Arylgruppen zwischen fünf und achtzehn Kohlenstoffatome in den Ringen. Arylgruppen weisen gegebenenfalls einen oder mehrere aromatische Ringe oder heterozyklische aromatische Ringe auf, die eine oder mehrere Elektronen abgebende Gruppen, Elektronen anziehende Gruppen und/oder Targeting-Liganden, die als Substituenten bereitgestellt sind, aufweisen.

Arylalkylgruppen sind Alkylgruppen, die mit einer oder mehreren Arylgruppen substituiert sind, wobei die Alkylgruppen gegebenenfalls zusätzliche Substituenten tragen und die Arylgruppen gegebenenfalls substituiert sind. Spezifische Alkylarylgruppen sind Phenyl-substituierte Alkylgruppen, z. B. Phenylmethylgruppen. Alkylarylgruppen werden alternativ als Arylgruppen beschrieben, die mit einer oder mehreren Alkylgruppen substituiert sind, wobei die Alkylgruppen gegebenenfalls zusätzliche Substituenten tragen und die Arylgruppen gegebenenfalls substituiert sind. Spezifische Alkylarylgruppen sind Alkyl-substituierte Phenylgruppen wie z. B. Methylphenyl. Substituierte Arylalkylgruppen umfassen vollständig halogenierte oder semihalogenierte Arylalkylgruppen, wie z. B. Arylalkylgruppen mit einer oder mehreren Alkyl- und/oder Arylgruppen, in denen ein oder mehrere Wasserstoffe durch ein oder mehrere Fluoratome, Chloratome, Bromatome und/oder lodatome ersetzt sind.

Wie hierin verwendet, werden die Begriffe "Alkylen" und "Alkylengruppe" synonym verwendet und beziehen sich auf eine zweiwertige Gruppe, die von einer Alkylgruppe wie hierin definiert herrührt. Die Erfindung umfasst Verbindungen mit einer oder mehreren Alkylengruppen. Alkylengruppen fungieren in manchen Verbindungen als Bindungs- und/oder Spacergruppen. Verbindungen der Erfindung können substituierte und/oder unsubstituierte C₁-C₂₀-Alkylen-, C₁-C₁₀-Alkylen- und C₁-C₅-Alkylengruppen aufweisen.

Wie hierin verwendet, werden die Begriffe "Cycloalkylen" und "Cycloalkylengruppe" synonym verwendet und beziehen sich auf eine zweiwertige Gruppe, die von einer Cycloalkylgruppe wie hierin definiert herrührt. Die Erfindung umfasst Verbindungen mit einer oder mehreren Cycloalkylengruppen. Cycloalkylgruppen fungieren in manchen Verbindungen als Bindungs- und/oder Spacergruppen. Verbindungen der Erfindung können substituierte und/oder unsubstituierte C₃-C₂₀-Cycloalkylen-, C₃-C₁₀-Cycloalkylen- und C₃-C₅-Cycloalkylengruppen aufweisen.

Wie hierin verwendet, werden die Begriffe "Arylen" und "Arylengruppe" synonym verwendet und beziehen sich auf eine zweiwertige Gruppe, die von einer Arylgruppe wie hierin definiert herrührt. Die Offenbarung umfasst Verbindungen mit einer oder mehreren Arylengruppen. In manchen Ausführungsformen ist ein Arylen eine zweiwertige Gruppe, die durch Entfernen von Wasserstoffatomen von zwei im Ring befindlichen Kohlenstoffatomen eines aromatischen Rings der Arylgruppe von einer Arylgruppe abgeleitet ist. Arylengruppen fungieren in manchen Verbindungen als Bindungs- und/oder Spacergruppen. Arylengruppen fungieren in manchen Verbindungen als Chromophor-, Fluorophor-, aromatische Antennen-, Farbstoff- und/oder Bildgebungsgruppen. Verbindungen der Offenbarung umfassen substituierte und/oder unsubstituierte C₆-C₃₀-Arylen-, C₆-C₁₈-Arylen-, C₆-C₁₄-Arylen- und C₆-C₁₀-Arylengruppen.

Wie hierin verwendet, werden die Begriffe "Heteroarylen" und "Heteroarylengruppe" synonym verwendet und beziehen sich auf eine zweiwertige Gruppe, die von einer Heteroarylgruppe wie hierin definiert herrührt. Die Offenbarung umfasst Verbindungen mit einer oder mehreren Heteroarylengruppen. In manchen Ausführungsformen ist ein Heteroarylen eine zweiwertige Gruppe, die durch Entfernen von Wasserstoffatomen von zwei im Ring befindlichen Kohlenstoffatomen oder im Ring befindlichen Stickstoffatomen eines heteroaromatischen oder aromatischen Rings der Heteroarylgruppe abgeleitet ist. Heteroarylengruppen fungieren in manchen Verbindungen als Bindungs- und/oder Spacergruppen. Heteroarylengruppen fungieren in manchen Verbindungen als Chromophor-, Fluorophor-, aromatische Antennen-, Farbstoff- und/oder Bildgebungsgruppen. Verbindungen der Offenbarung umfassen beispielsweise substituierte und/oder unsubstituierte Heteroarylengruppen mit 5 bis 30 Ringatomen, 5 bis 20 Ringatomen, 5 bis 10 Ringatomen, 5 bis 8 Ringatomen oder 5 bis 6 Ringatomen.

Wie hierin verwendet, werden die Begriffe "Alkenylen" und "Alkenylengruppe" synonym verwendet und beziehen sich auf eine zweiwertige Gruppe, die von einer Alkenylgruppe wie hierin definiert herrührt. Die Offenbarung umfasst Verbindungen mit einer oder mehreren Alkenylengruppen. Alkenylengruppen fungieren in manchen Verbindungen als Bindungs- und/oder Spacergruppen. Verbindungen der Offenbarung umfassen substituierte und/oder unsubstituierte C₂-C₂₀-Alkenylen-, C₂-C₁₀-Alkenylen- und C₂-C₅-Alkenylengruppen.

Wie hierin verwendet, werden die Begriffe "Cycloalkenylen" und "Cycloalkenylengruppe" synonym verwendet und beziehen sich auf eine zweiwertige Gruppe, die von einer Cycloalkenylgruppe wie hierin definiert herrührt. Die Offenbarung umfasst Verbindungen mit einer oder mehreren Cycloalkenylengruppen. Cycloalkenylengruppen fungieren in manchen Verbindungen als Bindungs- und/oder Spacergruppen. Verbindungen der Offenbarung umfassen substituierte und/oder unsubstituierte C₃-C₂₀-Cycloalkenylen-, C₃-C₁₀-Cycloalkenylen- und C₃-C₅-Cycloalkenylengruppen.

Wie hierin verwendet, werden die Begriffe "Alkinylen" und "Alkinylengruppe" synonym verwendet und beziehen sich auf eine zweiwertige Gruppe, die von einer Alkinylgruppe wie hierin definiert stammt. Die Offenbarung umfasst Verbindungen mit einer oder mehreren Alkinylengruppen. Alkinylengruppen fungieren in manchen Verbindungen als Bindungs- und/oder Spacergruppen. Verbindungen der Offenbarung umfassen substituierte und/oder unsubstituierte C₂-C₂₀-Alkinylen-, C₂-C₁₀-Alkinylen- und C₂-C₅-Alkinylengruppen.

Wie hierin verwendet, bezieht sich der Begriff "Halogen" auf eine Halogengruppe wie z. B. Fluor (-F), Chlor (-Cl), Brom (-Br) oder Iod (-I).

Der Begriff "heterozyklisch" bezieht sich auf Ringstrukturen, die zusätzlich zu Kohlenstoff zumindest eine andere Art von Atom im Ring enthalten. Beispiele für solche Heteroatome umfassen Stickstoff, Sauerstoff und Schwefel. Heterozyklische Ringe umfassen heterozyklische alizyklische Ringe und heterozyklische aromatische Ringe. Beispiele für heterozyklische Ringe umfassen, ohne darauf eingeschränkt zu sein, Pyrrolidinyl-, Piperidyl-, Imidazolidinyl-, Tetrahydrofuryl-, Tetrahydrothienyl-, Furyl-, Thienyl-, Pyridyl-, Chinolyl-, Isochinolyl-, Pyridazinyl-, Pyrazinyl-, Indolyl-, Imidazolyl-, Oxazolyl-, Thiazolyl-, Pyrazolyl-, Pyridinyl-, Benzoxadiazolyl-, Benzothiadiazolyl-, Triazolyl- und Tetrazolylgruppen. Die Atome heterozyklischer Ringe können an eine breite Vielzahl anderer Atome und funktioneller Gruppen, die beispielsweise als Substituenten bereitgestellt sind, gebunden sein.

Der Begriff "carbozyklisch" bezieht sich auf Ringstrukturen, die nur Kohlenstoffatome im Ring enthalten. Die Kohlenstoffatome carbozyklischer Ringe können an eine breite Vielzahl anderer Atome und funktioneller Gruppen, die beispielsweise als Substituenten bereitgestellt sind, gebunden sein.

Der Begriff "alizyklischer Ring" bezieht sich auf einen Ring oder eine Vielzahl kondensierter Ringe, der/die kein aromatischer Ring ist. Alizyklische Ringe umfassen sowohl carbozyklische als auch heterozyklische Ringe.

Der Begriff "aromatischer Ring" bezieht sich auf einen Ring oder eine Vielzahl kondensierter Ringe, die zumindest eine aromatische Ringgruppe umfasst. Der Begriff "aromatischer Ring" umfasst aromatische Ringe, die Kohlenstoff, Wasserstoff und Heteroatome umfassen. Ein aromatischer Ring umfasst carbozyklische und heterozyklische aromatische Ringe. Aromatische Ringe sind Komponenten von Arylgruppen.

Der Begriff "kondensierter Ring" oder "kondensierte Ringstruktur" bezieht sich auf eine Vielzahl von alizyklischen und/oder aromatischen Ringen, die in einer kondensierten Ringkonfiguration bereitgestellt sind, wie z. B. kondensierte Ringe, die zumindest zwei gemeinsame, im Ring befindliche Kohlenstoffatome und/oder Heteroatome aufweisen.

Wie hierin verwendet, bezieht sich der Begriff "Alkoxyalkyl" auf einen Substituenten der Formel Alkyl-O-alkyl.

Wie hierin verwendet, bezieht sich der Begriff "Polyhydroxyalkyl" auf einen Substituenten mit 2 bis 12 Kohlenstoffatomen und 2 bis 5 Hydroxylgruppen, wie z. B. einen 2,3-Dihydroxypropyl-, 2,3,4-Trihydroxybutyl- oder 2,3,4,5-Tetrahydroxypentylrest.

Wie hierin verwendet bezieht sich der Begriff "Polyalkoxyalkyl" auf einen Substituenten der Formel Alkyl(alkoxy)n-alkoxy, worin n eine ganze Zahl von 1 bis 10, vorzugsweise 1 bis 4 und noch bevorzugter für manche Ausführungsformen 1 bis 3, ist.

In Bezug auf die hierin beschriebenen Gruppen, die einen oder mehrere Substituenten enthalten, versteht es sich, dass solche Gruppen keine Substitution oder Substitutionsmuster enthalten, die sterisch unmöglich und/oder synthetisch nicht herstellbar sind. Zusätzlich dazu umfassen die Verbindungen der vorliegenden Erfindung alle stereochemischen Isomere, die aus der Substitution dieser Verbindungen hervorgehen. Eine optionale Substitution von Alkylgruppen umfasst eine Substitution mit einer oder mehreren Alkenylgruppen, Arylgruppen oder beidem, wobei die Alkenylgruppen oder Arylgruppen gegebenenfalls substituiert sind. Eine optionale Substitution von Alkenylgruppen umfasst eine Substitution mit einer oder mehreren Alkylgruppen, Arylgruppen oder beidem, wobei die Alkylgruppen oder Arylgruppen gegebenenfalls substituiert sind. Eine optionale Substitution von Arylgruppen umfasst eine Substitution des Arylrings mit einer oder mehreren Alkylgruppen, Alkenylgruppen oder beidem, wobei die Alkylgruppen oder Alkenylgruppen gegebenenfalls substituiert sind.

Optionale Substituenten für eine beliebige Alkyl-, Alkenyl- und Arylgruppe umfassen eine Substitution mit einem oder mehreren der folgenden Substituenten:
Halogen, einschließlich Fluor, Chlor, Brom oder Iod;
Pseudohalogeniden, einschließlich -CN, -OCN (Cyanat), -NCO (Isocyanat), -SCN (Thiocyanat) und -NCS (Isothiocyanat);
-COOR, worin R Wasserstoff oder eine Alkylgruppe oder eine Arylgruppe ist und worin R im Speziellen eine Methyl-, Ethyl-, Propyl-, Butyl- oder Phenylgruppe ist, wobei alle diese Gruppen gegebenenfalls substituiert sind;
-COR, worin R Wasserstoff oder eine Alkylgruppe oder eine Arylgruppe ist und worin R im Speziellen eine Methyl-, Ethyl-, Propyl-, Butyl- oder Phenylgruppe ist, wobei alle diese Gruppen gegebenenfalls substituiert sind;
-CON(R)₂, worin die R jeweils unabhängig voneinander Wasserstoff oder eine Alkylgruppe oder eine Arylgruppe sind und wobei R im Speziellen eine Methyl-, Ethyl-, Propyl-, Butyl- oder Phenylgruppe ist, wobei alle diese Gruppen gegebenenfalls substituiert sind; und wobei R und R gegebenenfalls einen Ring bilden, der eine oder mehrere Doppelbindungen und ein oder mehrere zusätzliche Kohlenstoffatome enthalten kann;
-OCON(R)₂, worin die R jeweils unabhängig voneinander Wasserstoff oder eine Alkylgruppe oder eine Arylgruppe sind und worin R im Speziellen eine Methyl-, Ethyl-, Propyl-, Butyl- oder Phenylgruppe ist, wobei alle diese Gruppen gegebenenfalls substituiert sind; und wobei R und R gegebenenfalls einen Ring bilden, der eine oder mehrere Doppelbindungen und ein oder mehrere zusätzliche Kohlenstoffatome enthalten kann;
-N(R)₂, worin die R jeweils unabhängig voneinander Wasserstoff oder eine Alkylgruppe oder eine Acylgruppe oder eine Arylgruppe sind und worin R im Speziellen eine Methyl-, Ethyl-, Propyl-, Butyl-, Phenyl- oder Acetylgruppe ist, die alle gegebenenfalls substituiert sind; und wobei R und R gegebenenfalls einen Ring bilden, der eine oder mehrere Doppelbindungen und ein oder mehrere zusätzliche Kohlenstoffatome enthalten kann;
-SR, worin R Wasserstoff oder eine Alkylgruppe oder eine Arylgruppe ist und worin R im Speziellen Wasserstoff, Methyl, Ethyl, Propyl, Butyl oder eine Phenylgruppe ist, die gegebenenfalls substituiert sind;
-SO₂R, oder -SOR, worin R eine Alkylgruppe oder eine Arylgruppe ist und worin R im Speziellen eine Methyl-, Ethyl-, Propyl-, Butyl- oder Phenylgruppe ist, die alle gegebenenfalls substituiert sind;
-OCOOR, worin R eine Alkylgruppe oder eine Arylgruppe ist;
-SO₂N(R)₂, worin die R jeweils unabhängig voneinander Wasserstoff oder eine Alkylgruppe oder eine Arylgruppe sind, die alle gegebenenfalls substituiert sind, und wobei R und R gg einen Ring bilden, der eine oder mehrere Doppelbindungen und ein oder mehrere zusätzliche Kohlenstoffatome enthalten kann; und
-OR, worin R H, eine Alkylgruppe, eine Arylgruppe oder eine Acylgruppe ist, die alle gegebenenfalls substituiert sind. In einem bestimmten Beispiel kann R ein Acyl sein, das -OCOR" ergibt, worin R" Wasserstoff oder eine Alkylgruppe oder eine Arylgruppe ist und worin R" im Speziellen ein Methyl-, Ethyl-, Propyl-, Butyl- oder Phenylgruppe ist, wobei alle diese Gruppen gegebenenfalls substituiert sind.

Spezifische substituierte Alkylgruppen umfassen Halogenalkylgruppen, insbesondere Trihalogenmethylgruppen und im Speziellen Trifluormethylgruppen. Spezifische substituierte Arylgruppen umfassen Mono-, Di-, Tri, Tetra- und Pentahalogensubstituierte Phenylgruppen; Mono-, Di-, Tri-, Tetra-, Penta-, Hexa- und Heptahalogensubstituierte Naphthalingruppen; 3- oder 4-Halogen-substituierte Phenylgruppen, 3- oder 4-Alkyl-substituierte Phenylgruppen, 3- oder 4-Alkoxy-substituierte Phenylgruppen, 3- oder 4-RCO-substituiertes Phenyl, 5- oder 6-Halogen-substituierte Naphthalingruppen. Im Speziellen umfassen substituierte Arylgruppen Acetylphenylgruppen, insbesondere 4-Acetylphenylgruppen; Fluorphenylgruppen, insbesondere 3-Fluorphenyl- und 4-Fluorphenylgruppen; Chlorphenylgruppen, insbesondere 3-Chlorphenyl und 4-Chlorphenylgruppen; Methylphenylgruppen, insbesondere 4-Methylphenylgruppen; und Methoxyphenylgruppen, insbesondere 4-Methoxyphenylgruppen.

In Bezug auf beliebige der obigen Gruppen, die einen oder mehrere Substituenten enthalten, versteht es sich, dass solche Gruppen keine Substitution oder Substitutionsmuster enthalten, die sterisch unmöglich und/oder synthetisch nicht herstellbar sind. Zusätzlich dazu umfassen die Verbindungen der vorliegenden Erfindung alle stereochemischen Isomere, die aus der Substitution dieser Verbindungen hervorgehen.

## Patentansprüche

1. Verwendung eines (Meth)acryloyloxy-substituierten Benzoesäureesters der allgemeinen Formel (III): 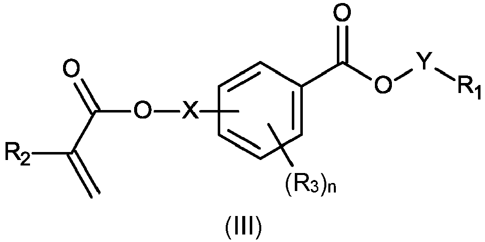 worin
R₁ für C₃-C₁₀-Cycloalkyl oder C₆-C₁₀-Aryl steht, wobei das C₃-C₁₀-Cycloalkyl und C₆-C₁₀-Aryl unsubstituiert oder mit einem oder mehreren Substituenten, ausgewählt aus C₁-C₆-Alkyl und C₁-C₆-Alkoxy substituiert sind;
R₂ für H oder CH₃ steht;
die R₃ jeweils unabhängig für C₁-C₆-Alkyl oder C₁-C₆-Alkoxy stehen;
n eine ganze Zahl von 0 bis 4 ist; und
X und Y jeweils unabhängig fehlen oder C₁-C₃-Alkylen sind, mit der Maßgabe, dass, wenn R₁ für C₆-C₁₀-Aryl steht, Y C₁-C₃-Alkylen ist;
als Reaktivverdünner in einem Verfahren zum Polymerisieren einer härtbaren Zusammensetzung, die zumindest eine Art von polymerisierbarer Spezies umfasst, wobei das Verfahren die Schritte des Bereitstellens der härtbaren Zusammensetzung, des Vermischens der härtbaren Zusammensetzung mit dem Reaktivverdünner und des Polymerisierens des Gemischs zu einem gegebenenfalls vernetzten Polymer umfasst, **dadurch gekennzeichnet, dass** die härtbare Zusammensetzung zumindest einen Photopolymerisationsinitiator umfasst und bestrahlt wird, um im Polymerisationsschritt Polymerisation zu initiieren,
in welchem Alkylgruppen unverzweigte, verzweigte und zyklische Alkylgruppen umfassen,
und Alkenylgruppen unverzweigte, verzweigte und zyklische Alkenylgruppen umfassen.

2. Verwendung nach Anspruch 1, **dadurch gekennzeichnet, dass** in Formel (III) R₁ für gegebenenfalls substituiertes C₅-C₁₀-Cycloalkyl oder gegebenenfalls substituiertes Phenyl steht.

3. Verwendung nach Anspruch 1 oder 2, **dadurch gekennzeichnet, dass** in Formel (III) X und Y jeweils unabhängig fehlen oder C₁-C₂-Alkylen sind.

4. Verwendung nach Anspruch 3, **dadurch gekennzeichnet, dass** in Formel (III) X fehlt.

5. Verwendung nach einem der Ansprüche 1 bis 4, **dadurch gekennzeichnet, dass** in Formel (III):
das C₃-C₁₀-Cycloalkyl und C₆-C₁₀-Aryl von R₁ jeweils unsubstituiert oder mit einem oder mehreren Substituenten, ausgewählt aus C₁-C₃-Alkyl und C₁-C₃-Alkoxy substituiert sind; und/oder
die R₃ jeweils unabhängig für C₁-C₃-Alkyl oder C₁-C₃-Alkoxy stehen; und/oder
n = 0 oder 1 ist.

6. Verwendung nach einem der Ansprüche 1 bis 5, **dadurch gekennzeichnet, dass** in Formel (III) R₁ für unsubstituiertes Phenyl steht.

7. Verwendung nach einem der Ansprüche 1 bis 5, **dadurch gekennzeichnet, dass** in Formel (III) R₁ aus der aus 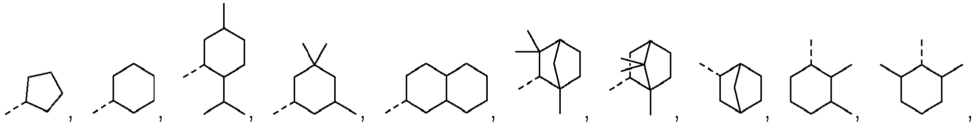 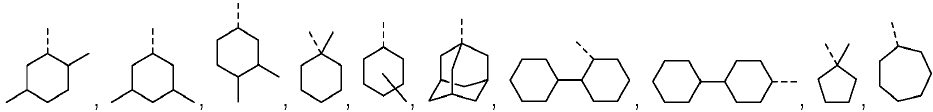 und 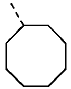 bestehenden Gruppe ausgewählt ist; wobei die gestrichelten Linien jeweils für die Stelle der Bindung an Y bzw. die Benzoat-Carboxylgruppe stehen.

8. Verwendung nach Anspruch 7, **dadurch gekennzeichnet, dass** in Formel (III) R₁ aus der aus 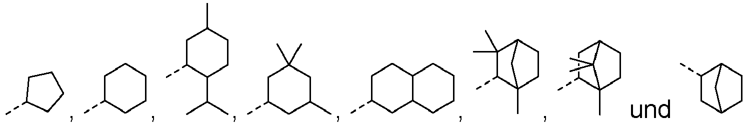 bestehenden Gruppe ausgewählt ist; wobei die gestrichelten Linien jeweils für die Stelle der Bindung an Y bzw. die Benzoat-Carboxylgruppe stehen.

9. Verwendung nach einem der Ansprüche 1 bis 6, **dadurch gekennzeichnet, dass** in Formel (III) -Y-R₁ aus den folgenden Strukturen ausgewählt ist: 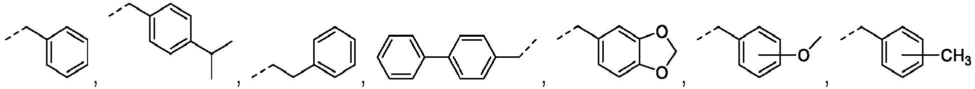 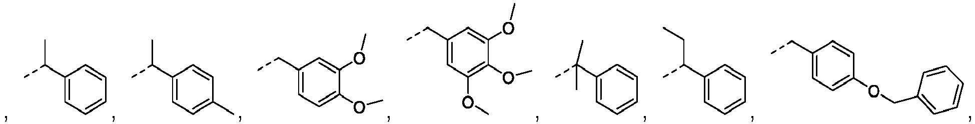 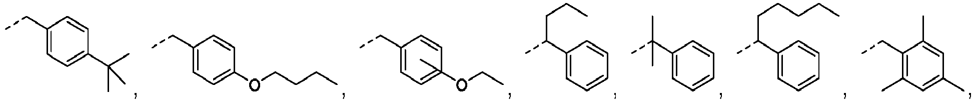 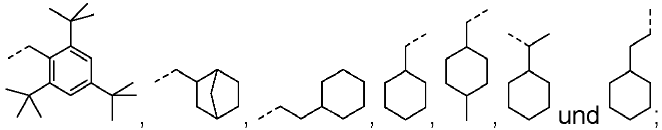 wobei die gestrichelten Linien jeweils für die Stelle der Bindung an die Benzoat-Carboxylgruppe stehen.

10. Verwendung nach einem der Ansprüche 1 bis 9, **dadurch gekennzeichnet, dass** der (Meth)acryloyloxy-substituierte Benzoesäureester der allgemeinen Formel (III) aus den folgenden chemischen Verbindungen ausgewählt ist:
2-(Methacryloyloxy)benzoesäurecyclopentylester (1);
2-(Methacryloyloxy)benzoesäurecyclohexylester (2);
2-(Methacryloyloxy)benzoesäure-2-isopropyl-5-methylcyclohexylester (3);
3-(Methacryloyloxy)benzoesäure-2-isopropyl-5-methylcyclohexylester (4);
4-(Methacryloyloxy)benzoesäure-2-isopropyl-5-methylcyclohexylester (5);
2-(Methacryloyloxy)benzoesäure-3,3,5-trimethylcyclohexylester (6);
2-(Acryloyloxy)benzoesäure-3,3,5-trimethylcyclohexylester (7);
2-(Methacryloyloxy)benzoesäuredecahydronaphthalin-2-ylester (8);
2-(Methacryloyloxy)benzoesäure-1,3,3-trimethyl-2-bicyclo[2.2.1]heptanylester (9);
2-(Methacryloyloxy)benzoesäure-1,7,7-trimethyl-2-bicyclo[2.2.1]heptanylester (10);
2-(Methacryloyloxy)benzoesäurebicyclo[2.2.1]heptan-2-ylmethylester (11);
2-(Methacryloyloxy)benzoesäure-2-cyclohexylethylester (12);
2-(Methacryloyloxy)benzoesäurebenzylester (13);
4-(Methacryloyloxy)benzoesäurebenzester (14);
3-(Methacryloyloxy)benzoesäure-4-isopropylbenzylester (15);
2-(Acryloyloxy)benzoesäurebenzylester (16);
2-(Methacryloyloxy)benzoesäurephenethylester (17);
4-(Methacryloyloxy)-3-methoxybenzoesäure-3-methoxybenzylester (18);
2-(Methacryloyloxy)benzoesäure-1-phenylethylester (19);
4-((Methacryloyloxy)methyl)benzoesäurecycloheptylester (20);
2-(Methacryloyloxy)benzoesäurecyclohexylmethylester (21).

11. Verwendung nach einem der Ansprüche 1 bis 10, **dadurch gekennzeichnet, dass** der Schritt des Vermischens der härtbaren Zusammensetzung mit dem Reaktivverdünner nach Erhitzen der härtbaren Zusammensetzung und/oder des Reaktivverdünners durchgeführt wird.

12. Verwendung nach einem der Ansprüche 1 bis 11, **dadurch gekennzeichnet, dass** die härtbare Zusammensetzung eine oder mehrere weitere Komponenten, ausgewählt aus der aus Polymerisationsinitiatoren, Polymerisationshemmern, Lösungsmitteln, Füllstoffen, Antioxidationsmitteln, Pigmenten, Farbstoffen, Oberflächenmodifikatoren und Gemischen davon bestehenden Gruppe umfasst.

13. Verwendung nach Anspruch 1 bis 12, **dadurch gekennzeichnet, dass** das Verfahren zum Polymerisieren der härtbaren Zusammensetzung Teil eines additiven Fertigungsverfahrens oder eines 3D-Druckverfahrens ist.

14. Verwendung nach Anspruch 1 bis 13, **dadurch gekennzeichnet, dass** die härtbare Zusammensetzung auf eine vorab festgelegte erhöhte Verfahrenstemperatur im Bereich von 90 °C bis 120 °C erhitzt wird, bevor sie mit Licht bestrahlt wird, das eine geeignete Wellenlänge aufweist, um von dem Photoinitiator absorbiert zu werden, wodurch eine Spaltung des Photoinitiators bewirkt wird, um die Polymerisation der härtbaren Zusammensetzung zu induzieren, um das gegebenenfalls vernetzte Polymer zu erhalten.

15. Verwendung nach einem der Ansprüche 1 bis 14, **dadurch gekennzeichnet, dass** die härtbare Zusammensetzung zumindest ein mehrwertiges Monomer als die zumindest eine Art von polymerisierbarer Spezies umfasst und zu einem vernetzten Polymer polymerisiert wird.

16. Kieferorthopädische Vorrichtung, umfassend ein vernetztes Polymer, das durch eine Verwendung nach einem der Ansprüche 1 bis 15 erhalten wird und Gruppierungen der nachstehenden Formel (IV) als von dem (Meth)acryloyloxy-substituierten Benzoesäureester der Formel (III) abgeleitete Grundeinheiten umfasst: 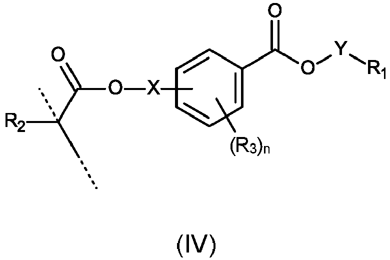 und die gestrichelten Linien jeweils für eine Bindung an ein weiteres Kohlenstoffatom in der Polymerkette stehen.

17. Kieferorthopädische Vorrichtung nach Anspruch 16, **dadurch gekennzeichnet, dass** sie ein Aligner, Expander oder Spacer ist.

18. (Meth)acryloyloxy-substituierter Benzoesäureester der allgemeinen Formel (III): 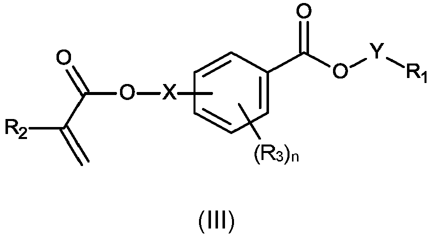 **dadurch gekennzeichnet, dass** R₁, R₂, R₃, X, Y und n jeweils so ausgewählt sind, dass sie:
2-(Methacryloyloxy)benzoesäurecyclopentylester (1) 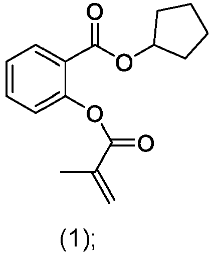 oder
2-(Methacryloyloxy)benzoesäurecyclohexylester (2) 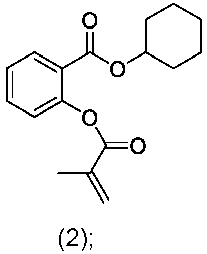 oder
2-(Methacryloyloxy)benzoesäure-2-isopropyl-5-methylcyclohexylester (3) 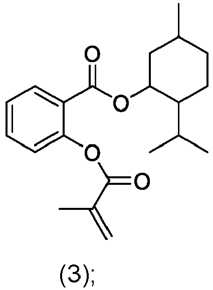 oder
3-(Methacryloyloxy)benzoesäure-2-isopropyl-5-methylcyclohexylester (4) 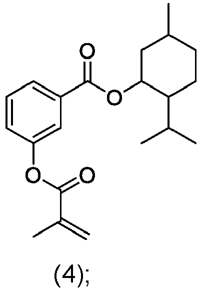 oder
4-(Methacryloyloxy)benzoesäure-2-isopropyl-5-methylcyclohexylester (5) 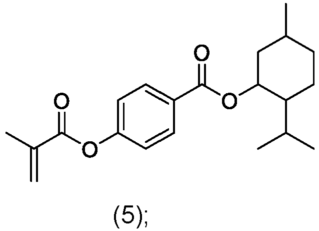 oder
2-(Methacryloyloxy)benzoesäure-3,3,5-trimethylcyclohexylester (6) 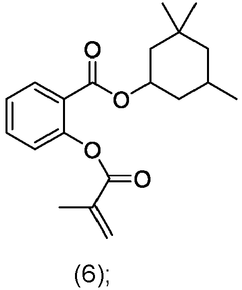 oder
2-(Acryloyloxy)benzoesäure-3,3,5-trimethylcyclohexylester (7) 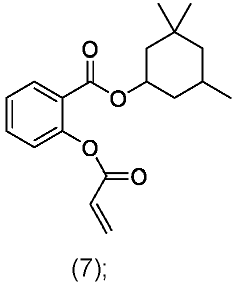 oder
2-(Methacryloyloxy)benzoesäuredecahydronaphthalin-2-ylester (8) 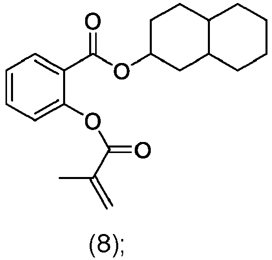 oder
2-(Methacryloyloxy)benzoesäure-1,3,3-trimethyl-2-bicyclo[2.2.1]heptanylester (9) 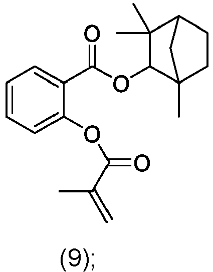 oder
2-(Methacryloyloxy)benzoesäure-1,7,7-trimethyl-2-bicyclo[2.2.1]heptanylester (10) 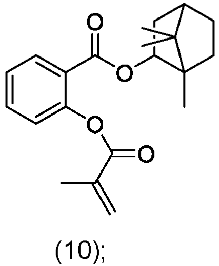 oder 2-(Methacryloyloxy)benzoesäurebicyclo[2.2.1]heptan-2-ylmethylester (11) 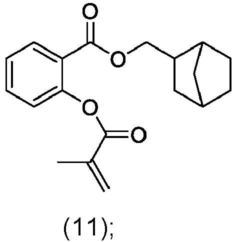 oder
2-(Methacryloyloxy)benzoesäure-2-cyclohexylethylester (12) 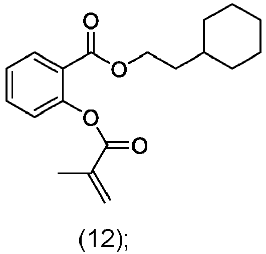 oder
2-(Methacryloyloxy)benzoesäurebenzylester (13) 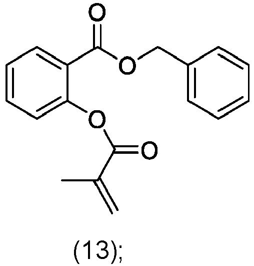 oder
3-(Methacryloyloxy)benzoesäure-4-isopropylbenzylester (15) 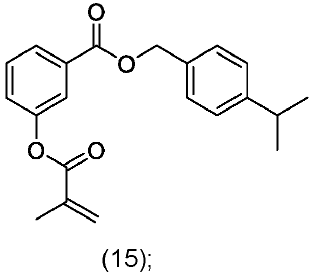 oder
2-(Acryloyloxy)benzoesäurebenzylester (16) 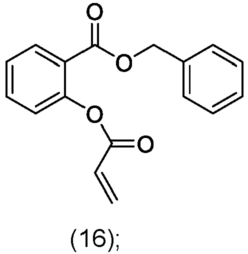 oder
2-(Methacryloyloxy)benzoesäurephenethylester (17) 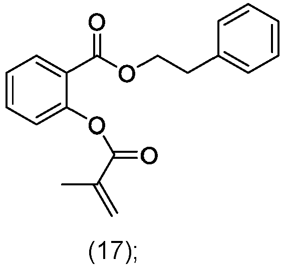 oder
4-(Methacryloyloxy)-3-methoxybenzoesäure-3-methoxybenzylester (18) 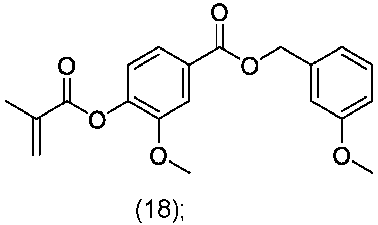 oder
2-(Methacryloyloxy)benzoesäure-1-phenylethylester (19) 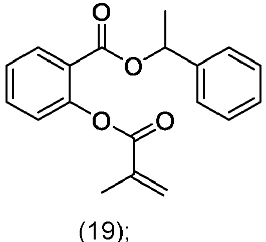 oder
4-((Methacryloyloxy)methyl)benzoesäurecycloheptylester (20) 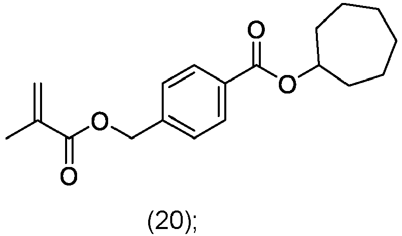 oder
2-(Methacryloyloxy)benzoesäurecyclohexylmethylester (21) 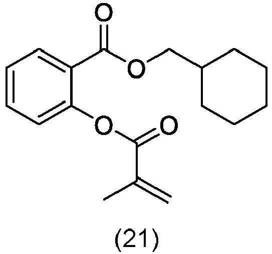 ergeben.

## Claims

1. Use of a (meth)acryloyloxy-substituted benzoic acid ester of general formula (III): 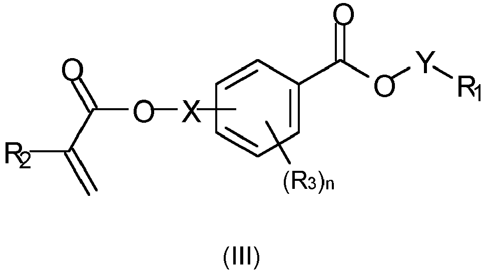 wherein
R₁ is C₃-C₁₀ cycloalkyl or C₆-C₁₀ aryl, wherein the C₃-C₁₀ cycloalkyl and C₆-C₁₀ aryl are unsubstituted or substituted with one or more substituents selected from C₁-C₆ alkyl and C₁-C₆ alkoxy;
R₂ is H or CH₃;
the R₃'s are each independently C₁-C₆ alkyl or C₁-C₆ alkoxy;
n is an integer from 0 to 4; and
X and Y are each independently absent or C₁-C₃ alkylene, with the proviso that, when R₁ is C₆-C₁₀ aryl, Y is C₁-C₃ alkylene;
as a reactive diluent in a process for polymerizing a curable composition comprising at least one type of polymerizable species, the process comprising the steps of providing the curable composition, mixing the curable composition with the reactive diluent, and polymerizing the mixture into an optionally crosslinked polymer,
**characterized in that** the curable composition comprises at least one photopolymerization initiator and is irradiated to initiate polymerization in the polymerization step;
**wherein the alkyl groups comprise linear, branched and cyclic alkyl groups; and**
**wherein the alkenyl groups comprise linear, branched and cyclic alkenyl groups.**

2. The use according to claim 1, **characterized in that** in formula (III) R₁ represents optionally substituted C₅-C₁₀ cycloalkyl or optionally substituted phenyl.

3. The use according to claim 1 or 2, **characterized in that** in formula (III) X and Y are each independently absent or C₁-C₂ alkylene.

4. The use according to claim 3, **characterized in that** X is absent in formula (III) .

5. The use according to any of claims 1 to 4, **characterized in that** in formula (III):
the C₃-C₁₀ cycloalkyl and C₆-C₁₀ aryl of R₁ are each unsubstituted or substituted by one or more substituents selected from C₁-C₃ alkyl and C₁-C₃ alkoxy; and/or
each R₃ is independently C₁-C₃ alkyl or C₁-C₃ alkoxy; and/or
n = 0 or 1.

6. The use according to any one of claims 1 to 5, **characterized in that** in formula (III) R₁ is unsubstituted phenyl.

7. The use according to any of claims 1 to 5, **characterized in that** in formula (III) R₁ is selected from the group consisting of 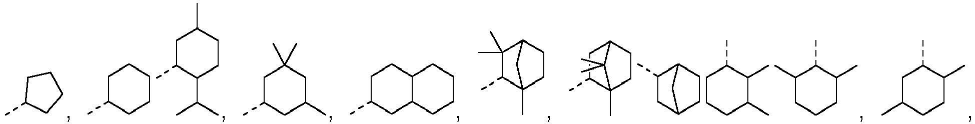 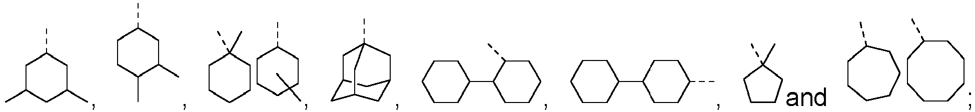 wherein the dotted lines represent the position of the bond to Y and the benzoate carboxyl group, respectively.

8. The use according to claim 7, **characterized in that** in formula (III) R₁ is selected from the group consisting of 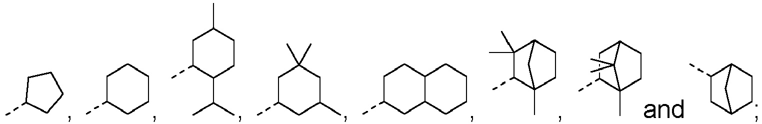 wherein the dotted lines represent the position of the bond to Y and the benzoate carboxyl group, respectively.

9. The use according to any one of claims 1 to 6, **characterized in that** in formula (III) -Y-R₁ is selected from the following structures: 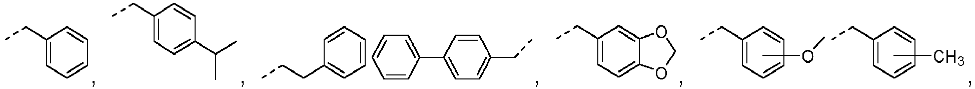 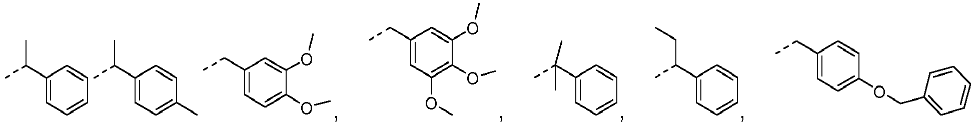 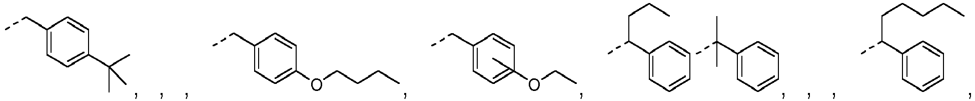 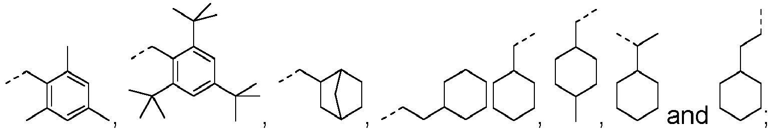 wherein the dotted lines each represent the position of the bond to the benzoate carboxyl group.

10. The use according to any one of claims 1 to 9, **characterized in that** the (meth)acryloyloxy-substituted benzoic acid ester of the general formula (III) is selected from the following chemical compounds:
2-(methacryloyloxy)benzoic acid cyclopentyl ester (1);
2-(methacryloyloxy)benzoic acid cyclohexyl ester (2);
2-(methacryloyloxy)benzoic acid-2-isopropyl-5-methylcyclohexyl ester (3);
3-(methacryloyloxy)benzoic acid-2-isopropyl-5-methylcyclohexyl ester (4);
4-(methacryloyloxy)benzoic acid-2-isopropyl-5-methylcyclohexyl ester (5);
2-(methacryloyloxy)benzoic acid-3,3,5-trimethylcyclohexyl ester (6);
2-(acryloyloxy)benzoic acid-3,3,5-trimethylcyclohexyl ester (7);
2-(methacryloyloxy)benzoic acid decahydronaphthalen-2-yl ester (8);
2-(methacryloyloxy)benzoic acid-1,3,3-trimethyl-2-bicyclo[2.2.1]heptanyl ester (9);
2-(methacryloyloxy)benzoic acid-1,7,7-trimethyl-2-bicyclo[2.2.1]heptanyl ester (10);
2-(methacryloyloxy)benzoic acid-bicyclo[2.2.1]heptan-2-yl methyl ester (11);
2-(methacryloyloxy)benzoic acid-2-cyclohexylethyl ester (12);
2-(methacryloyloxy)benzoic acid benzyl ester (13);
4-(methacryloyloxy)benzoic acid benzoate (14);
3-(methacryloyloxy)benzoic acid-4-isopropylbenzyl ester (15);
2-(acryloyloxy)benzoic acid benzyl ester (16);
2-(methacryloyloxy)benzoic acid phenethyl ester (17);
4-(methacryloyloxy)-3-methoxybenzoic acid-3-methoxybenzyl ester (18);
2-(methacryloyloxy)benzoic acid-1-phenylethyl ester (19);
4-((methacryloyloxy)methyl)benzoic acid cycloheptyl ester (20);
2-(methacryloyloxy)benzoic acid cyclohexyl methyl ester (21).

11. The use according to any one of claims 1 to 10, **characterized in that** the step of mixing the curable composition with the reactive diluent is performed after heating the curable composition and/or the reactive diluent.

12. The use according to any one of claims 1 to 11, **characterized in that** the curable composition comprises one or more further components selected from the group consisting of polymerization initiators, polymerization inhibitors, solvents, fillers, antioxidants, pigments, dyes, surface modifiers and mixtures thereof.

13. The use according to claims 1 to 12, **characterized in that** the process for polymerizing the curable composition is part of an additive manufacturing process or a 3D printing process.

14. The use according to claim 1 to 13, **characterized in that** the curable composition is heated to a predetermined elevated process temperature in the range of 90°C to 120°C before being irradiated with light having a suitable wavelength to be absorbed by the photoinitiator, thereby causing cleavage of the photoinitiator to induce polymerization of the curable composition to obtain the optionally crosslinked polymer.

15. The use according to any one of claims 1 to 14, **characterized in that** the curable composition comprises at least one polyvalent monomer as the at least one type of polymerizable species and is polymerized to form a crosslinked polymer.

16. Orthodontic device comprising a crosslinked polymer obtained by a use according to any one of claims 1 to 15 and comprising groupings of the below formula (IV) as repeating units derived from the (meth)acryloyloxy-substituted benzoic acid ester of formula (III): 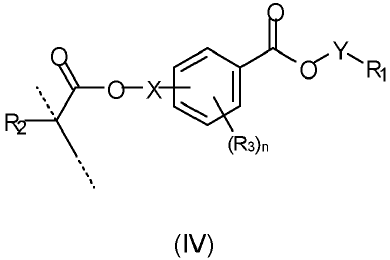 wherein R₁, R₂, R₃, X, Y and n are as defined in any one of claims 1 to 10 above; and the dotted lines each represent a bond to another carbon atom in the polymer chain.

17. The orthodontic device according to claim 16, **characterized in that** it is an aligner, expander or spacer.

18. (Meth)acryloyloxy-substituted benzoic acid esters of the general formula (III) : 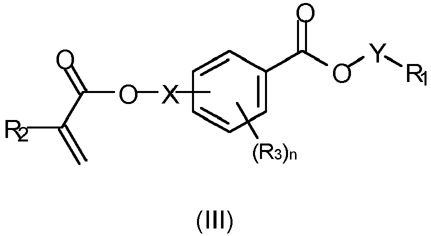 wherein R₁, R₂, R₃, X, Y and n are as defined in any one of claims 1 to 10 above, **characterized in that** R₁, R₂, R₃, X, Y and n are each selected such that they result in:
2-(methacryloyloxy)benzoic acid cyclopentyl ester (1) 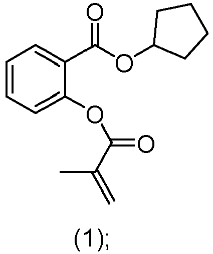 or
2-(methacryloyloxy)benzoic acid cyclohexyl ester (2) 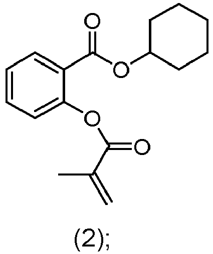 or
2-(methacryloyloxy)benzoic acid-2-isopropyl-5-methylcyclohexyl ester (3) 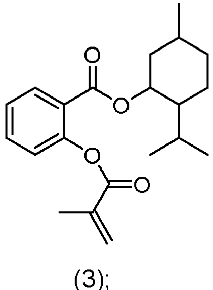 or
3-(methacryloyloxy)benzoic acid-2-isopropyl-5-methylcyclohexyl ester (4) 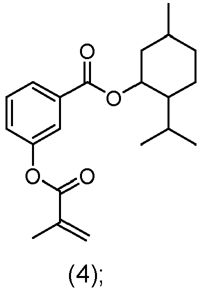 or
4-(methacryloyloxy)benzoic acid-2-isopropyl-5-methylcyclohexyl ester (5) 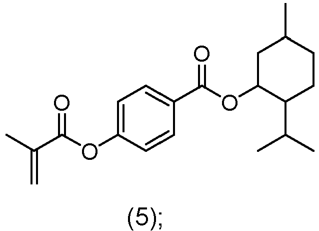 or
2-(methacryloyloxy)benzoic acid-3,3,5-trimethylcyclohexyl ester (6) 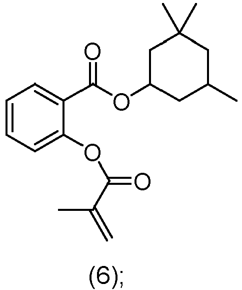 or
2-(Acryloyloxy)benzoic acid-3,3,5-trimethylcyclohexyl ester (7) 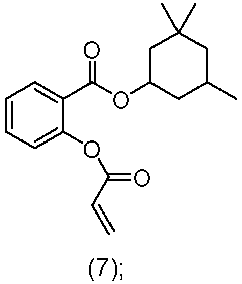 or
2-(methacryloyloxy)benzoic acid decahydronaphthalen-2-yl ester (8) 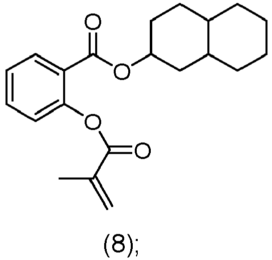 or
2-(methacryloyloxy)benzoic acid-1,3,3-trimethyl-2-bicyclo[2.2.1]heptanyl ester (9) 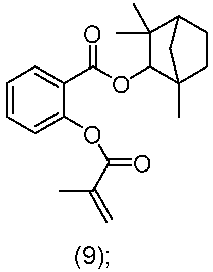 or
2-(methacryloyloxy)benzoic acid-1,7,7-trimethyl-2-bicyclo[2.2.1]heptanyl ester (10) 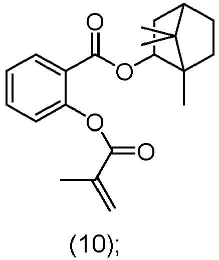 or
2-(methacryloyloxy)benzoic acid-bicyclo[2.2.1]heptan-2-ylmethyl ester (11) 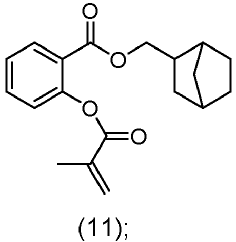 or
2-(methacryloyloxy)benzoic acid-2-cyclohexylethyl ester (12) 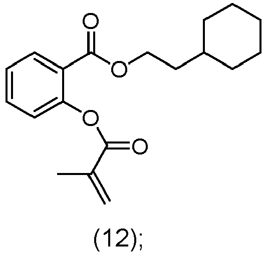 or
2-(methacryloyloxy)benzoic acid benzyl ester (13) 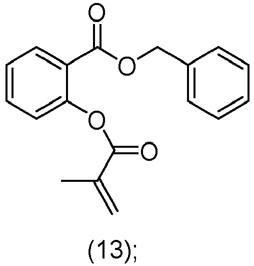 or
3-(methacryloyloxy)benzoic acid-4-isopropylbenzyl ester (15) 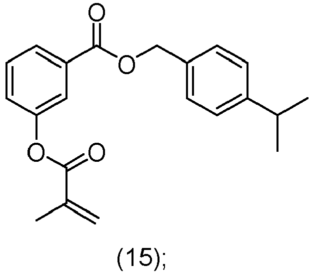 or
2-(acryloyloxy)benzoic acid benzyl ester (16) 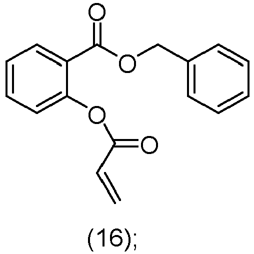 or
2-(methacryloyloxy)benzoic acid phenethyl ester (17) 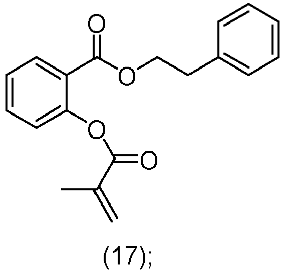 or
4-(methacryloyloxy)-3-methoxybenzoic acid-3-methoxybenzyl ester (18) 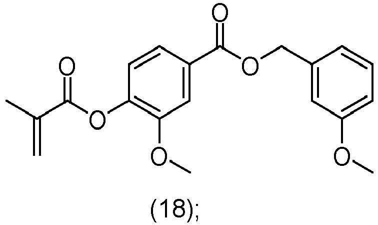 or
2-(methacryloyloxy)benzoic acid-1-phenylethyl ester (19) 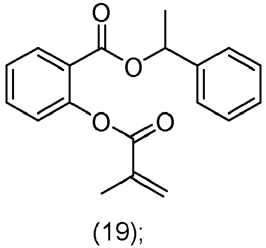 or
4-((methacryloyloxy)methyl)benzoic acid cycloheptyl ester (20) 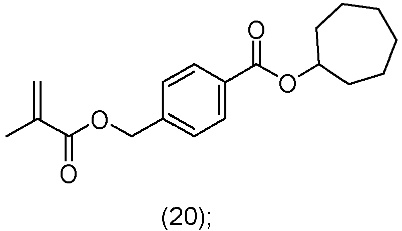 or
2-(methacryloyloxy)benzoic acid cyclohexyl methyl ester (21)

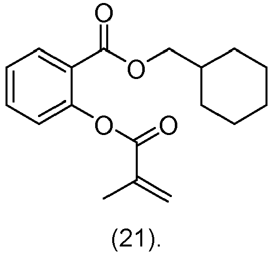

## Revendications

1. Utilisation d'un ester d'acide benzoïque à substitution (méth)acryloyloxy de formule générale (III) : 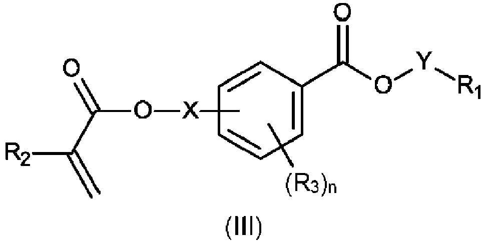 où
R₁ représente un cycloalkyle en C₃-C₁₀ ou un aryle en C₆-C₁₀, dans laquelle le cycloalkyle en C₃-C₁₀- et l'aryle en C₆-C₁₀ sont non substitués ou sont substitués par un ou plusieurs substituant(s) choisi(s) parmi alkyle en C₁-C₆ et alcoxy en C₁-C₆ ;
R₂ représente H ou CH₃ ;
les R₃ représentent respectivement et indépendamment les uns des autres un alkyle en C₁-C₆ ou un alcoxy en C₁-C₆ ;
n est un entier de 0 à 4 ; et
X et Y sont respectivement et indépendamment l'un de l'autre absents ou sont des alkylènes en C₁-C₃, à condition que Y soit un alkylène en C₁-C₃ lorsque R₁ représente un aryle en C₆-C₁₀,
en tant que diluant réactif dans un procédé de polymérisation d'une composition durcissable comprenant au moins un type d'espèce polymérisable, dans laquelle le procédé comprend les étapes consistant à fournir la composition durcissable, mélanger la composition durcissable avec le diluant réactif et polymériser le mélange pour former un polymère éventuellement réticulé,
**caractérisée en ce que** la composition durcissable comprend au moins un amorceur de photopolymérisation et est irradiée afin d'amorcer la polymérisation dans l'étape de polymérisation,
où les groupes alkyle comprennent des groupes alkyle linéaires, ramifiés et cycliques, et les groupes alcényle comprennent des groupes alcényle linéaires, ramifiés et cycliques.

2. Utilisation selon la revendication 1, **caractérisée en ce que**, dans la formule (III), R₁ représente un cycloalkyle en C₅-C₁₀ éventuellement substitué ou un phényle éventuellement substitué.

3. Utilisation selon la revendication 1 ou 2, **caractérisée en ce que**, dans la formule (III), X et Y sont respectivement et indépendamment l'un de l'autre absents ou sont un alkylène en C₁-C₂.

4. Utilisation selon la revendication 3, **caractérisée en ce que** X est absent dans la formule (III).

5. Utilisation selon l'une quelconque des revendications 1 à 4, **caractérisée en ce que**, dans la formule (III) :
le cycloalkyle en C₃-C₁₀ et l'aryle en C₆-C₁₀ de R₁ sont respectivement non substitués ou sont substitués par un ou plusieurs substituant(s) choisi(s) parmi alkyle en C₁-C₃ et alcoxy en C₁-C₃ ; et/ou
les R₃ représentent respectivement et indépendamment les uns des autres un alkyle en C₁-C₃ ou un alcoxy en C₁-C₃ ; et/ou
n est égal à 0 ou 1.

6. Utilisation selon l'une quelconque des revendications 1 à 5, **caractérisée en ce que**, dans la formule (III), R₁ représente un phényle non substitué.

7. Utilisation selon l'une quelconque des revendications 1 à 5, **caractérisée en ce que**, dans la formule (III), R₁ est choisi parmi le groupe constitué de 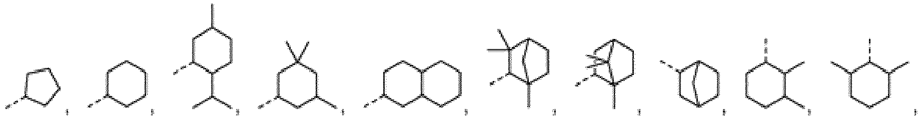 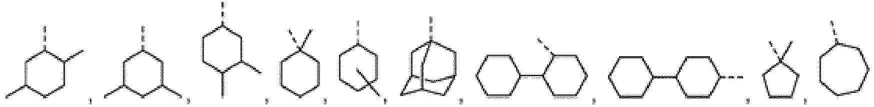 et 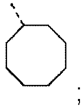 dans laquelle les lignes en pointillés représentent respectivement le point de liaison à Y ou au groupe benzoate-carboxyle.

8. Utilisation selon la revendication 7, **caractérisée en ce que**, dans la formule (III), R₁ est choisi parmi le groupe constitué de 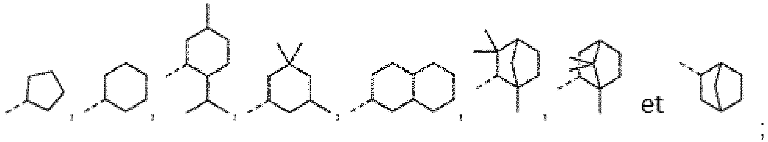 dans laquelle les lignes en pointillés représentent respectivement le point de liaison à Y ou au groupe benzoate-carboxyle.

9. Utilisation selon l'une quelconque des revendications 1 à 6, **caractérisée en ce que**, dans la formule (III), -Y-R₁ est choisi parmi les structures ci-dessous : 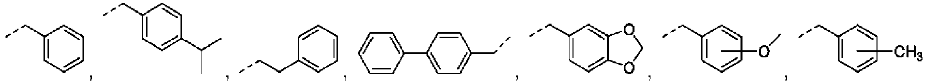 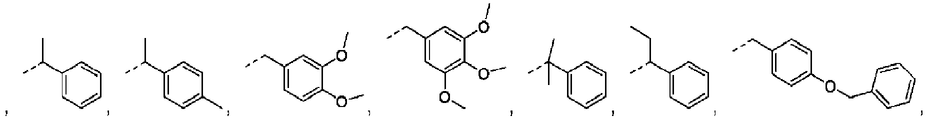 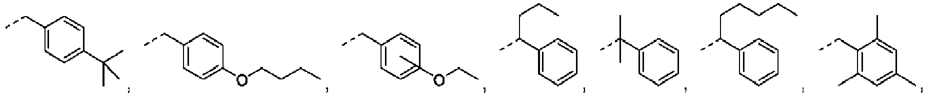 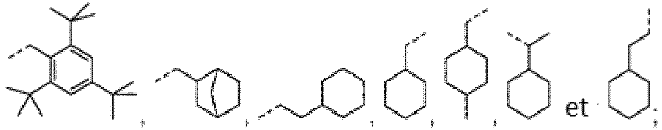 dans laquelle les lignes en pointillés représentent respectivement le point de liaison au groupe benzoate-carboxyle.

10. Utilisation selon l'une quelconque des revendications 1 à 9, **caractérisée en ce que** l'ester d'acide benzoïque à substitution (méth)acryloyloxy de formule générale (III) est choisi parmi les composés chimiques ci-dessous :
ester cyclopentylique d'acide 2-(méthacryloyloxy)benzoïque (1) ;
ester cyclohexylique d'acide 2-(méthacryloyloxy)benzoïque (2) ;
ester 2-isopropyl-5-méthylcyclohexylique d'acide 2-(méthacryloyloxy)benzoïque (3) ;
ester 2-isopropyl-5-méthylcyclohexylique d'acide 3-(méthacryloyloxy)benzoïque (4) ;
ester 2-isopropyl-5-méthylcyclohexylique d'acide 4-(méthacryloyloxy)benzoïque (5) ;
ester 3,3,5-triméthylcyclohexylique d'acide 2-(méthacryloyloxy)benzoïque (6) ;
ester 3,3,5-triméthylcyclohexylique d'acide 2-(acryloyloxy)benzoïque (7) ;
ester décahydronaphtalin-2-ylique d'acide 2-(méthacryloyloxy)benzoïque (8) ;
ester 1,3,3-triméthyl-2-bicyclo[2.2.1]heptanylique d'acide 2-(méthacryloyloxy)benzoïque (9) ;
ester 1,7,7-triméthyl-2-bicyclo[2.2.1]heptanylique d'acide 2-(méthacryloyloxy)benzoïque (10) ;
ester bicyclo[2.2.1]heptan-2-ylméthylique d'acide 2-(méthacryloyloxy)benzoïque (11) ;
ester 2-cyclohexyléthylique d'acide 2-(méthacryloyloxy)benzoïque (12) ;
ester benzylique d'acide 2-(méthacryloyloxy)benzoïque (13) ;
ester benzylique d'acide 4-(méthacryloyloxy)benzoïque (14) ;
ester 4-isopropylbenzylique d'acide 3-(méthacryloyloxy)benzoïque (15) ;
ester benzylique d'acide 2-(acryloyloxy)benzoïque (16) ;
ester phénéthylique d'acide 2-(méthacryloyloxy)benzoïque (17) ;
ester 3-méthoxybenzylique d'acide 4-(méthacryloyloxy)-3-méthoxybenzoïque (18) ;
ester 1-phényléthylique d'acide 2-(méthacryloyloxy)benzoïque (19) ;
ester cycloheptylique d'acide 4-((méthacryloyloxy)méthyl)benzoïque (20) ;
ester cyclohexylméthylique d'acide 2-(méthacryloyloxy)benzoïque (21).

11. Utilisation selon l'une quelconque des revendications 1 à 10, **caractérisée en ce que** l'étape de mélange de la composition durcissable avec le diluant réactif est mise en œuvre après chauffage de la composition durcissable et/ou du diluant réactif.

12. Utilisation selon l'une quelconque des revendications 1 à 11, **caractérisée en ce que** la composition durcissable comprend un ou plusieurs autre(s) composant(s) choisi(s) parmi le groupe constitué des amorceurs de polymérisation, inhibiteurs de polymérisation, solvants, charges, antioxydants, pigments, colorants, modificateurs de surface ainsi que des mélanges de-ceux-ci.

13. Utilisation selon les revendications 1 à 12, **caractérisée en ce que** le procédé de polymérisation de la composition durcissable fait partie d'un procédé de fabrication additive ou d'un procédé d'impression 3D.

14. Utilisation selon les revendications 1 à 13, **caractérisée en ce que** la composition durcissable est chauffée à une température de procédé élevée prédéterminée dans la plage comprise entre 90°C et 120°C avant d'être irradiée avec de la lumière présentant une longueur d'onde appropriée pour être absorbée par le photoamorceur, ce qui provoque un clivage du photoamorceur, afin d'induire la polymérisation de la composition durcissable, afin d'obtenir le polymère éventuellement réticulé.

15. Utilisation selon l'une quelconque des revendications 1 à 14, **caractérisée en ce que** la composition durcissable comprend au moins un monomère polyvalent tenant lieu du au moins un type d'espèce polymérisable et est polymérisée pour aboutir à un polymère réticulé.

16. Appareil orthodontique comprenant un polymère réticulé qui est obtenu grâce à une utilisation selon l'une quelconque des revendications 1 à 15 et comprend des groupements de formule (IV) ci-dessous tenant lieu d'unités de base dérivées de l'ester d'acide benzoïque à substitution (méth)acryloyloxy de formule (III) : 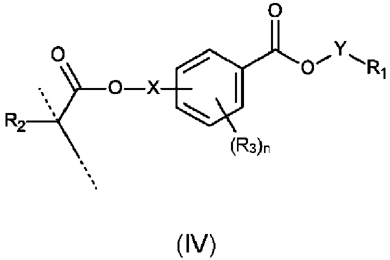 et les lignes en pointillés représentent respectivement une liaison à un autre atome de carbone au sein de la chaîne polymère.

17. Appareil orthodontique selon la revendication 16, **caractérisé en ce qu'**il s'agit d'un aligneur, d'un expanseur ou d'un espaceur.

18. Ester d'acide benzoïque à substitution (méth)acryloyloxy de formule générale (III) : 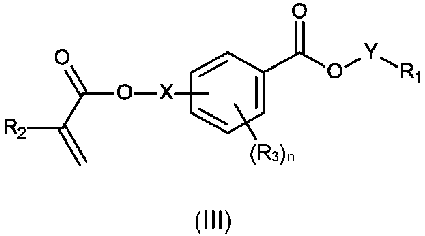 **caractérisé en ce que** R₁, R₂, R₃, X, Y et n sont respectivement choisis de sorte qu'il en résulte :
un ester cyclopentylique d'acide 2-(méthacryloyloxy)benzoïque (1) 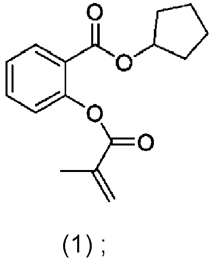 ou
un ester cyclohexylique d'acide 2-(méthacryloyloxy)benzoïque (2) 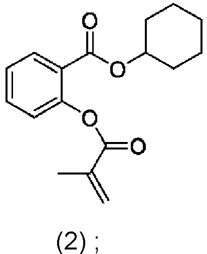 ou
un ester 2-isopropyl-5-méthylcyclohexylique d'acide 2-(méthacryloyloxy)benzoïque (3) 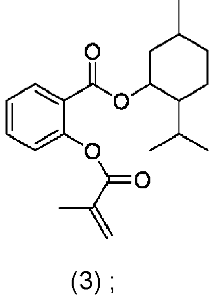 ou
un ester 2-isopropyl-5-méthylcyclohexylique d'acide 3-(méthacryloyloxy)benzoïque (4) 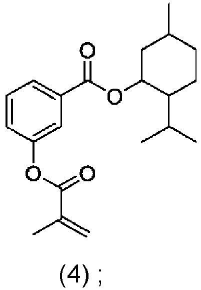 ou
un ester 2-isopropyl-5-méthylcyclohexylique d'acide 4-(méthacryloyloxy)benzoïque (5) 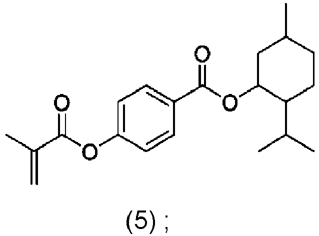 ou
un ester 3,3,5-triméthylcyclohexylique d'acide 2-(méthacryloyloxy)benzoïque (6) 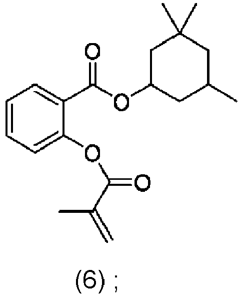 ou
un ester 3,3,5-triméthylcyclohexylique d'acide 2-(acryloyloxy)benzoïque (7) 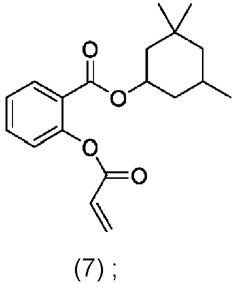 ou
un ester décahydronaphtalin-2-ylique d'acide 2-(méthacryloyloxy)benzoïque (8) 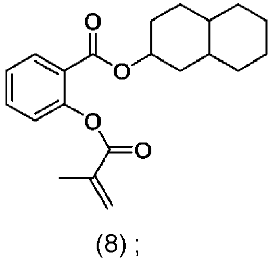 ou
un ester 1,3,3-triméthyl-2-bicyclo[2.2.1]heptanylique d'acide 2-(méthacryloyloxy)benzoïque (9) 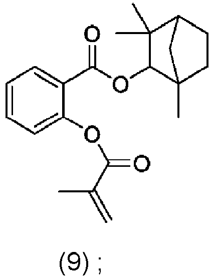 ou
un ester 1,7,7-triméthyl-2-bicyclo[2.2.1]heptanylique d'acide 2-(méthacryloyloxy)benzoïque (10) 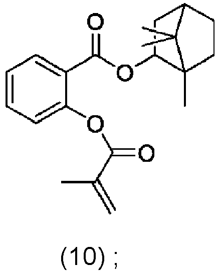 ou
un ester bicyclo[2.2.1]heptan-2-ylméthylique d'acide 2-(méthacryloyloxy)benzoïque (11) 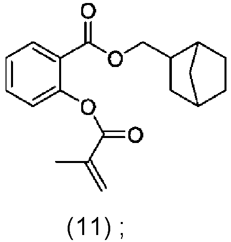 ou
un ester 2-cyclohexyléthylique d'acide 2-(méthacryloyloxy)benzoïque (12) 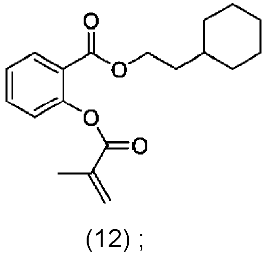 ou
un ester benzylique d'acide 2-(méthacryloyloxy)benzoïque (13) 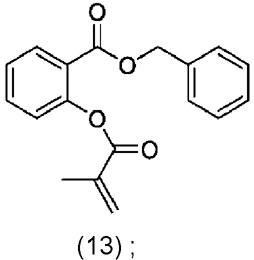 ou
un ester 4-isopropylbenzylique d'acide 3-(méthacryloyloxy)benzoïque (15) 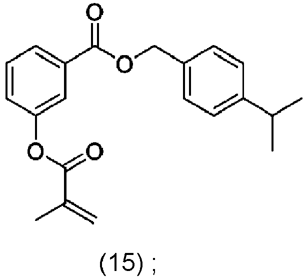 ou
un ester benzylique d'acide 2-(acryloyloxy)benzoïque (16) 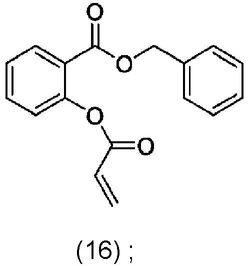 ou
un ester phénéthylique d'acide 2-(méthacryloyloxy)benzoïque (17) 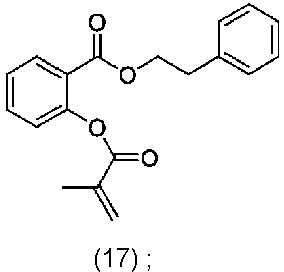 ou
un ester 3-méthoxybenzylique d'acide 4-(méthacryloyloxy)-3-méthoxybenzoïque (18) 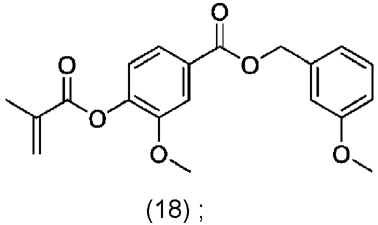 ou
un ester 1-phényléthylique d'acide 2-(méthacryloyloxy)benzoïque (19) 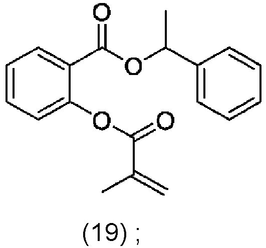 ou
un ester cycloheptylique d'acide 4-((méthacryloyloxy)méthyl)benzoïque (20) 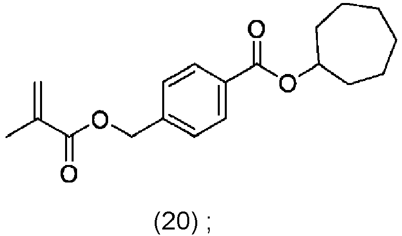 ou
un ester cyclohexylméthylique d'acide 2-(méthacryloyloxy)benzoïque (21)
